# FASCICULE DE BREVET EUROPEEN

(11) **EP 0 828 851 B1**
(45) Date de publication et mention de la délivrance du brevet: **20.07.2005**
(21) Numéro de dépôt: 96916181.9
(22) Date de dépôt: 09.05.1996
(51) Int. Cl.: C12Q 1/37, C07K 5/062, A61K 38/05

(54) **INHIBITEURS DE TRIPEPTIDYLPEPTIDASES**
TRIPEPTIDYLPEPTIDASE-INHIBITOREN
TRIPEPTIDYLPEPTIDASE INHIBITORS

(30) Priorité: 09.05.1995 FR 9505489
(43) Date de publication de la demande: 18.03.1998
(73) Titulaire: INSTITUT NATIONAL DE LA SANTE ET DE LA RECHERCHE MEDICALE (INSERM), 75654 Paris Cédex 13 (FR); BIOPROJET, 75003 Paris (FR)
(72) Inventeur: ROSE, Christiane, F-78320 Le Mesnil-Saint-Denis (FR); VARGAS, Froylan, F-75014 Paris (FR); BOURGEAT, Pierre, F-75013 Paris (FR); SCHWARTZ, Jean-Charles, F-75014 Paris (FR); BISHOP, Paul, Beaumont, London N19 3HZ (GB); BAMBAL, Ramesh, Babarao, Blue Bell, PA 19422 (US); GANELLIN, Charon, Robin, Welwyn, Herts AL6 0TD (GB); LEBLOND, Bertrand, F-76000 Rouen (FR); MOORE, Andrew, N., J., Ottawa, Ontario K1S 2A5 (CA); CHAN, Suzanne, Ken t DA6 7HG (GB); LIHUA, Zhao, London N4 2QT (GB)
(74) Mandataire: Bernasconi, Jean
(86) Numéro de dépôt international: PCT/FR1996/000700
(87) Numéro de publication internationale: WO 1996/035805

(56) Documents cités:
- WO-A-93/08259
- AT-A- 389 517
- DE-A- 2 727 670
- DE-A- 2 728 593
- FR-A- 2 460 292
- FR-A- 2 510 990
- US-A- 5 159 060
- NEUROCHEM. RES. (1993), 18(7), 743-9 CODEN: NEREDZ;ISSN: 0364-3190, XP000562808 WILSON, C. ET AL: "Purification and characterization of tripeptidylpeptidase-II from post-mortem human brain"
- PROCEEDINGS OF THE NATIONAL ACADEMY OF SCIENCES OF USA, vol. 85, WASHINGTON US, pages 8326-8330, XP002018477 C. ROSE ET AL: "A serine peptidase responsible for the inactivation of endogenous cholecystokinin in brain" cité dans la demande
- BIOCHEMICAL JOURNAL, vol. 304, pages 517-523, XP000562825 B. TOMKINSON: "Characterization of cDNA for murine tripeptidyl-peptidase II reveals alternative splicing"
- BIOCHEMISTRY, vol. 30, pages 168-174, XP000561825 B. TOMKINSON AND A-K JONSSON: "Characterization of cDNA for human tripeptidyl peptidase II"
- JOURNAL OF BIOLOGICAL CHEMISTRY, vol. 261, no. 5, MD US, pages 2409-2417, XP002018480 R-M BALÖW ET AL: "Purification, substrate specificity, and classification of tripeptidyl peptidase II" cité dans la demande
- ARCH. BIOCHEM. BIOPHYS. (1994), 314(2), 276-9 CODEN: ABBIA4;ISSN: 0003-9861, XP000562811 TOMKINSON, BIRGITTA ET AL: "Use of a dehydroalanine-containing peptide as an efficient inhibitor of tripeptidyl peptidase II"
- NATURE, vol. 380, 4 Avril 1996, LONDON GB, pages 403-409, XP002018482 C.ROSE E.A.: "Characterization and inhibition of a CCK-inactivating serine protease"
- LIFE SCIENCES vol. 34, 1984, pages 67 - 72
- J. CLIN. PSYCHIATRY vol. 52, no. 10, Octobre 1991, pages 21 - 28
- NEUROSCIENCE LETTERS vol. 131, 1991, pages 129 - 134
- BRAIN RESEARCH vol. 243, 1982, pages 176 - 179
- AM. JOURN. MEDICAL GENETICS vol. 114, 2002, pages 479 - 482
- BIOL. PSYCHIATRY vol. 34, 1993, pages 781 - 790
- PSYCHOLOGICAL MEDICINE vol. 22, 1992, pages 37 - 43

## Description

La présente invention se situe dans le domaine de la prévention de l'inactivation de neuropeptides endogènes tels que la cholécystokinine (CCK).

Aussi, l'invention concerne plus précisément un procédé de criblage de médicaments mettant en oeuvre l'enzyme intervenant dans ce type d'inactivation.

En outre, la présente invention a pour objet des composés chimiques de formule définie ainsi que leurs procédés de préparation et leur utilisation en tant qu'inhibiteurs des enzymes précitées, intervenant dans l'inactivation physiologique de neuropeptides endogènes c:omme en particulier la cholécystokinine (CCK).

L'invention a aussi pour objet des compositions physiologiquement acceptables comprenant lesdits composés.

Elle a également pour objet l'utilisation desdits composés dans la préparation de médicaments destinés à l'homme ou à l'animal.

On connaît le rôle physiologique important des neuropeptides endogènes et en particulier celui de la cholécystokinine (CCK). Cette dernière est à la fois une hormone digestive et un neurotransmetteur des systèmes nerveux central et périphérique. Dans ce dernier cas, elle se présente principalement sous forme d'un octapeptide sulfaté (CCK-8-S) répondant à la formule :

Asp-Tyr(SO₃H)-Met-Gly-Trp-Met-Asp-Phe-NH₂.

La CCK exerce des actions variées sur la motricité et les sécrétions digestives (contraction de la vésicule biliaire, inhibition de la sécrétion gastrique, etc.) ainsi que sur le système nerveux central (analgésie, action sur l'humeur et la cognition) et endocrinien (sécrétions hypophysaires). En outre, la CCK et certains dérivés possèdent une puissante activité anorexigène par facilitation de la satiété en stimulant des récepteurs périphériques et, éventuellement centraux (J.J. Vanderhaegen and J.M. Crawley, Ann. N.Y. Acad. Sci., 1985, **448** : 1-697).

Bien que l'on aurait pu attendre des effets intéressants de la stimulation pharmacologique des récepteurs CCK, aucun agent stimulant ces récepteurs n'est disponible actuellement en thérapeutique principalement pour des raisons de mauvaise biodisponibilité de la CCK et de ses dérivés. En outre, il a même été montré qu'une stimulation directe des récepteurs CCK était susceptible d'entraîner des effets anxiogènes indésirables.

D'autres études se sont orientées vers l'identification de l'enzyme ou des enzymes responsable(s) de l'inactivation physiologique de ces neuropeptides endogènes et en particulier de la CCK.

C'est ainsi qu'il a été suggéré l'implication de différentes enzymes supposées attaquer certaines liaisons peptidiques de la molécule CCK-8, comme notamment celle d'une aminopeptidase acide, distincte de l'aminopeptidase A (Deschodt-Lanckman et al., Peptides, 1983, 4 : 71), de l'enképhalinase et d'une aminopeptidase (Matsas et al., FEBS Lett., 1984, 175 : 124 ; Deschodt-Lanckman et al., Regul. Peptides, 1981, 2 : 15), celle de thiolpeptidases (Mc Dermott et al., Neurochem. Int., 1983, 5 : 641 ; Durieux et al., Neuropeptides, 1986, 7 :1) ainsi que celle d'une métalloendopeptidase (Steardo et al., J. Neurochem., 1985, 45 : 784).

Plus récemment, Rose et al. (Proc. Natl. Acad., Sci. USA, 1988, 85 :8326 ; Neurosci., 1989, 29 : 583) ont suggéré l'intervention d'une sérine-peptidase. Ils ont en outre constaté que des réactifs généraux des groupes sérine des protéines tels que le diisopropylfluorophosphorate (DFP) ou des chlorométhyl-cétones ou acides boroniques inhibiteurs d'élastases (une famille de sérine-peptidases) étaient capables d'empêcher la dégradation enzymatique de la CCK-8 endogène libérée par dépolarisation de coupes de cerveaux.

Toutefois, ces expériences n'ont pas permis d'identifier et de purifier l'enzyme responsable et les composés utilisés dans ces expériences in vitro ne sauraient être considérés comme des médicaments, en particulier, en raison de leur toxicité, leur absence de spécificité et/ou leur mauvaise biodisponibilité.

Plus récemment encore, il a été purifié et identifié une enzyme à partir d'extraits solubles de cerveau humain (Wilson C. et al., Neurochem. Res. 1993, 18(7), 743-749) présentant une forte similitude avec une protéase connue sous le nom de tripeptidylpeptidase TPP II précédemment isolée à partir de foie de rat ou d'erythrocytes humains (Balöw R.M. et al., J. Biol. Chem., 1986, 261:2409-2417 ; Tomkinson B. et al., Biochemistry 1991, vol. 30, 168-174). La séquence d'une murine TPP II de 4611 pb a également été décrite (Tomkinson B, Biochem. J., 1994, vol. 304, 517-523) dont la séquence codante présente une forte homologie avec la TPP II humaine précitée.

Cette enzyme purifiée a été démontrée capable d'hydrolyser plusieurs neuropeptides dont CCK-8 exogène mais ni son application dans l'inactivation de CCK-8 endogène ni la possibilité d'obtenir par son inhibition des réponses biologiques de type CCK n'avait été ni démontrée ni même suggérée.

Ainsi, on constate qu'il subsiste dans l'état de la technique le besoin de composés chimiques non toxiques, brodisponibles, spécifiques et pouvant être utilisés en tant que médicaments pour la prévention de l'inactivation de ces neuropeptides endogènes.

Par ailleurs, un inhibiteur de la TPPII de faible affinité a été décrit (Tomkinson et coll., Arch. Biochem. Biophys., 1994, 314: 276) mais celui-ci présente une structure oligopeptidergique laissant penser que sa biodisponibilité, notamment par voie orale est faible, qu'il ne peut donc constituer un médicament, utilisation qui n'est d'ailleurs pas envisagée par les auteurs.

L'invention a maintenant permis d'atteindre cet objectif en fournissant en particulier un procédé de criblage de médicaments mettant en oeuvre l'enzyme responsable isolée, ladite enzyme étant codée par la séquence nucléotidique donnée par les identifieurs SEQ ID NOS 1 et 2 permettant ainsi, selon que ladite enzyme est inhibée ou non, de distinguer des molécules susceptibles de constituer des médicaments efficaces dans le traitement des troubles ou affections où les neuropeptides endogènes, et en particulier la cholécystokinine, sont impliqués.

La présente invention fournit également des composés chimiques de formule définie ci-après, pouvant être utilisés dans la prévention de l'inactivation des neuropeptides endogènes et satisfait donc le besoin essentiel subsistant dans l'état de la technique.

Les inventeurs ont préparé une tripeptidylpeptidase membranaire à l'état pur, selon un procédé comprenant les étapes suivantes :
i) préparation de membranes de cerveau (cortex cérébral), par exemple de rat ;
ii) purification par chromatographie(s) liquide(s) haute performance (CLHP) ;
iii) vérification du produit obtenu, par réaction enzymatique utilisant un substrat CCK, par exemple les peptides CCK-8 (non sulfaté) de formule :

   Asp-Tyr-Met-Gly-Trp-Met-Asp-Phe-NH₂
ou CCK-5 de formule :

Gly-Trp-Met-Asp-Phe-NH₂

L'étude de la spécificité de cette enzyme purifiée sur une série de substrats-modèles a montré que celle-ci se comporte comme une aminotripeptidylpeptidase.

Les inventeurs ont effectué des travaux de séquençage. Un fragment de la protéine purifiée présente une forte similitude avec une protéase connue sous le nom de tripeptidylpeptidase II précédemment isolée à partir d'érythrocytes humains ou de foie de rat (Balöw R.M. et al., et Tomkinson et al.).

En utilisant des méthodes traditionnelles de clonage moléculaire, les inventeurs ont identifié deux clones distincts dans une librairie d'ADN complémentaire de cerveau de rat à l'aide de deux sondes A et B respectivement de 350 et 380 bases. Elles ont été obtenues par une réaction en chaîne de polymérase (PCR) pratiquée à l'aide des amorces suivantes :

SEQ ID N°3 (Sonde A, amorce sens) :
GACTGAGGAGCCCTTCCCTTTTCA

SEQ ID N°4 (Sonde A, amorce antisens) :
GCCTTAGGATAGAAGTCATAGCCA

SEQ ID N°5 (Sonde B, amorce sens) :
CCCTTTGTAGGAAAGGTTGTGCC

SEQ ID N°6 (Sonde B, amorce antisens) :
GAATACGCAATAATCGGGAGGATAC

Leur séquençage a indiqué que le premier clone est l'homologue rongeur de la tripeptidylpeptidase II (TPP II) humaine. Par contre, dans le second, la séquence diffère dans la partie 5' (à partir du nucléotide 293).

La séquence nucléotidique codant pour la protéine isolée est donnée par les identifieurs (SEQ ID N°1 et SEQ ID N°2) .

La séquence comporte un segment hydrophobe d'une vingtaine d'acides aminés indiquant l'existence d'un segment trans-membranaire. Ainsi, cette protéine, quoique dérivant vraisemblablement du même gène que la précédente par un processus d'épissage alternatif, se présente comme une ectopeptidase à sérine.

Aussi, la présente invention a pour objet un procédé de criblage de médicaments par mesure de l'activité de l'enzyme tripeptidylpeptidase II membranaire, par utilisation d'un substrat modèle de cette enzyme.

La tripeptidylpeptidase peut être préparée conformément au procédé précité.

Selon un autre mode de mise en oeuvre, des membranes de cerveau, préparées par simple centrifugation d'un homogenat, sont incubées en présence d'un substrat d'aminotripeptidylpeptidase (comme AAF-Amc) et d'inhibiteurs potentiels de l'activité enzymatique ainsi révélée.

Les inventeurs ont pu mettre au point des inhibiteurs cle la dégradation de neuropeptides endogènes, en particulier de CCK, en mesurant l'activité de ladite enzyme à l'état purifié mais aussi plus simplement à l'état natif sur des préparations de membranes à partir de tissus en utilisant un substrat modèle de la TPP II.

Aussi, la présente invention a encore pour objet des composés chimiques répondant à la formule générale (I) suivante : 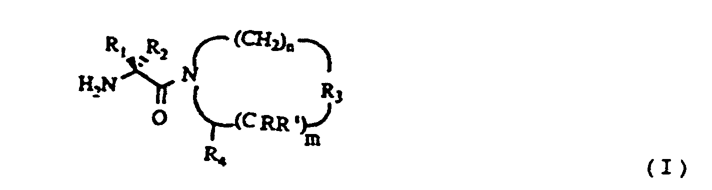 dans laquelle :
- R₁ représente un hydrogène ou un groupe alkyle en C₁-C₄ linéaire ou ramifié ;
- R₂ représente un hydrogène ou un groupe alkyle en C₁-C₂ ;
   l'un au moins de R₁ et R₂ représentant un hydrogène ;
- n = 0 ou 1 et m = 4 ou 1 avec n différent de m ;
- R et R' représentent chacun indépendamment un hydrogène ou un groupe alkyle en C₁-C₂ ;
- R₃ représente un radical bivalent constitué d'une chaîne alkyle -(CH₂)₂-; -CH₂-CH(cis.F)-, -CH₂-CH(CH₂Ph)-, d'un motif 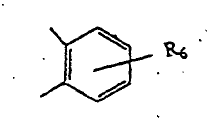
où R₆ représente H, F, OCH₃ ou OCH₂Ph, 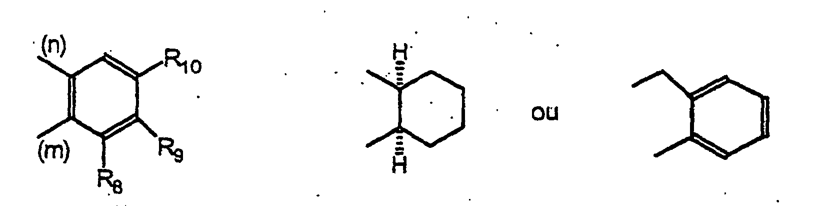 où R₈, R₉ et R₁₀ représentent chacun un atome d'hydrogène ou d'halogène, un groupe O (alkyle en C₁-C₄), OCH₂Ph, OH ou alkyle en C₁-C₄, y comprise R₆, (m) et (n) indiquant le sens de liaison respectivement au groupe (CH₂)ₙ (ou à N si n = 0) et au groupe (CRR')ₘ (ou à CHR₄ si m = 0).
- R₄ représente un groupe amide CO-NH-R₅ où R₅ représente un hydrogène ou un groupe alkyle en C₁-C₆ linéaire ou ramifié, -(CH₂)₃-SCH₃, -CH₂Ph, -CH₂C₈H₁₁, (CH₂)₃OH, 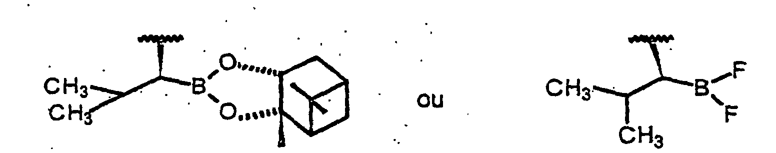 ainsi que leurs sels ou hydrates correspondants,
sous réserve que :
i) lorsque
   R₂ = R' = R = H ; R₃ = -CH₂-CH₂- ; n + m = 1 ; R₄ = -CO-NH-R₅ ; et R₅ = H
   alors
   R₁ est distinct de -CH₃; -CH₂-CH₃ ; -(CH₂)₂-CH₃; -(CH₂)₃-CH₃ ; -CH(CH₃)₂ ; -CH₂CH(CH₃)₂ ; et -CH(CH₃)CH₂-CH₃ ;
ii) lorsque
   R₂ = R' = R = H ; R₃ = -CH₂-CH₂- ; n + m = 1 ; R₄ = -CO-NH-R₅ ; et R₅ = -CH₂-CH₃;
   alors
   R₁ est distinct de -CH₃ ; et -CH₂CH(CH₃)₂ ;
iii) lorsque
   R₂ = R' = R = H ; R₃ = -CH₂-CH₂- ; n + m = 1 ; R₄ = -CO-NH-R₅; et R₅ = -(CH₂)₄-CH₃, -CH(CH₃)₂, CH₃ ou le motif : 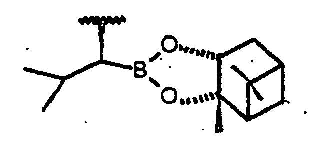 alors
   R₁ est distinct de -CH₃.

Dans toute la description, "Ph" désigne le radical phényle éventuellement substitué.

La présente invention a encore pour objet des composés chimiques répondant à la formule générale (I') suivante : 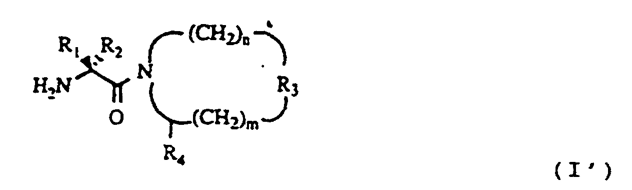 dans laquelle :
- R₁ représente un hydrogène ou un groupe alkyle en C₁-C₄ linéaire ou ramifié ;
- R₂ représente un hydrogène ou un groupe alkyle en C₁-C₂ ;
   l'un au moins de R₁ et R₂ représentant un hydrogène ;
- n = 0 ou 1 et m = 0 ou 1 avec n différent de m ;
- R₃ représente un radical bivalent constitué d'une chaîne alkyle -(CH₂)₂-, -CH₂-CH(cis.F)-, -CH₂-CH(CH₂Ph)-, d'un motif 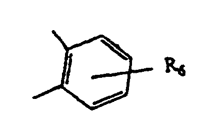
où R₆ représente H, F, OCH₃ ou OCH₂Ph, 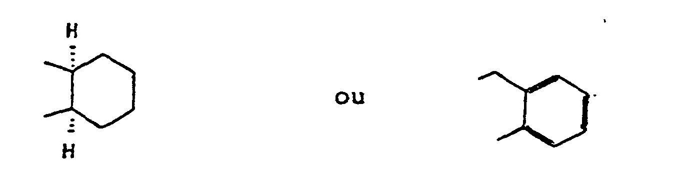
- R₄ représente un groupe amide CO-NH-R₅ où R₅ représente un hydrogène ou un groupe alkyle en C₁-C₆ linéaire ou ramifié, -(CH₂)₃-SCH₃, -CH₂Ph, -CH₂C₆H₁₁,

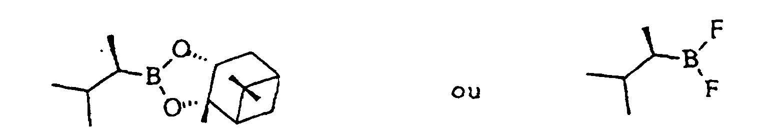

L'invention a pour objet des variantes de ces composées, qui sont définies aux revendications 8-23.

Selon un premier aspect, l'invention concerne plus particulièrement un premier groupe de ces composés dans lesquels R₃ représente le radical bivalent -(CH₂)₂-.

Des composés de ce type sont notamment les composés donnés aux exemples ci-après, n°1 à 7 (préparé selon la voie 1); n°8 (voie 2); n°9 (voie 5); n°10 (voie 6).

Parmi ce premier groupe de composés, ceux dans lesquels R₁ représente CH₂CH₃, (CH₂)₂CH₃, (CH₂)₃CH₃, CH(CH₂)₂, CH₂CH(CH₃)₂ et CH(CH₃)CH₂CH₃, R₂, R et R' représentent un hydrogène, R₄ représente le groupe amide CO-NH-R₅ et R₅ représente un hydrogène sont connus.

Le composé de formule (I) dans laquelle R₁ représente CH₂CH(CH₃)₂, R₂, R et R' représentent l'hydrogène, R₃ est le radical bivalent -(CH₂)₂- et R5 représente CH₂CH₃ est également connus.

Le composé de formule (I) dans laquelle R₁ représente CH₃, R₂, R et R' représentent l'hydrogène, R₃ est le radical -(CH₂)₂- et R₅ représente le motif 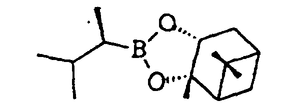 est également connu.

Selon un autre aspect, l'invention concerne plus particulièrement un second groupe de ces composés dans lesquels R₃ représente le motif cis.fluoroéthylène -CH₂-CH(cis.F)- (squelette proline substituée). Une sous-famille comprend les composés où R et R' sont chacun un hydrogène comme par exemple le composé de l'exemple 11 (voie 3) décrit ci-après.

Selon un autre aspect, l'invention concerne un troisième groupe de ces composés dans lesquels R₃ représente le motif benzyléthylène -CH₂-CH(CH₂Ph)-(squelette proline substituée). Une sous-famille comprend Les composés où R et R' sont chacun un hydrogène comme par exemple le composé de l'exemple 12 (voie 4) décrit ci-après.

Selon une seconde variante préférée, l'invention a pour objet des composés de formule (I) telle que définie ci-avant dans laquelle n = 0 et m = 1.

Selon un aspect de cette variante, l'invention concerne des composés dans lesquels R₃ représente le motif 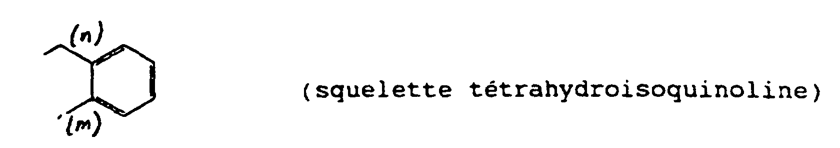 où (n) et (m) indiquent le sens de liaison comme précédemment.

Une sous-famille comprend les composés où R et R' sont chacun l'hydrogène dont notamment le composé de l'exemple 13 (voie 3) décrit ci-après.

Selon un deuxième aspect particulièrement préféré, l'invention a pour objet des composés de formule (I) où R et R' représentent l'hydrogène et où R₃ représente le motif 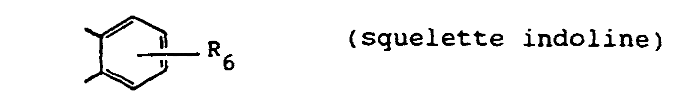 dont des exemples sont notamment les composés des exemples 14 à 17 (voie 3) ; des exemples 18 à 21 (voie 7) ; décrits ci-après.

Selon un autre aspect particulièrement préféré de cette seconde variante, l'invention concerne des composés de formule (I) dans laquelle R₃ représente le motif 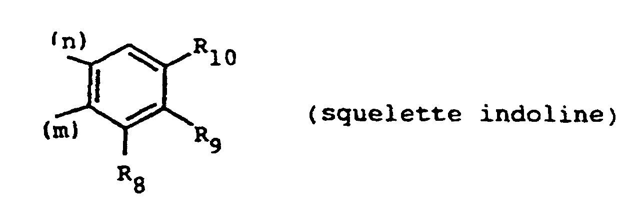 dont des exemples sont notamment les composés des exemples 24 à 26 (voie 8) et de l'exemple 27 (voie 9) ; 28 (voie 11) ; 29 et 30 (voie 10) ; 31 et 32 (voie 9 ; 33 et 34 (voie 12) ; 35 (voie 8) ; 38 (voie 13) et 37 (voie 14) décrits ci-après.

Cette classe de composés comprend les composés de formule (I) ou R₃ représente le motif 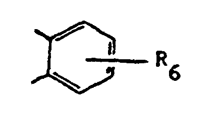 lorsque R et R' représentent chacun un hydrogène, deux des substituants R₈, R₉ et R₁₀ étant alors un hydrogène.

Selon un autre aspect de cette seconde variante, l'invention concerne des composés dans lesquels R₃ représente le motif

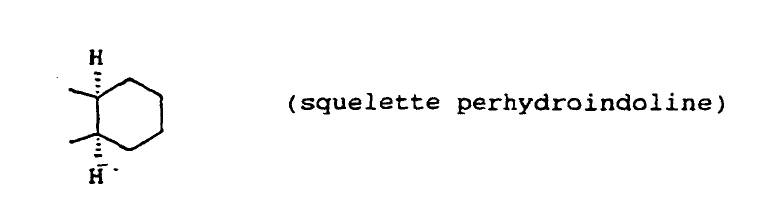

Une sous-famille comprend les composés où R et R' représentent chacun l'hydrogène comme notamment le composé de l'exemple 22 (voie 3) donné ci-après.

Enfin, selon une troisième variante, l'invention a pour objet les composés de formule (I) dans laquelle n = 1 et m = 0 et où R₃ représente le motif 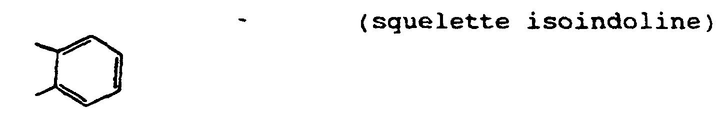 tels que le composé de l'exemple 23 (voie 4) décrit ci-après.

Les composés de formule (I) dans lesquels R₂ représente un hydrogène sont également particulièrement préférés.

L'effet inhibiteur de ces composés a été évalué par mesure de l'activité TPP II membranaire et exprimé par leur constante de dissociation apparente Ki comme décrit ci-après.

Les composés préférés selon l'invention sont les composés suivants :
- 2(S)-aminobutyryl-L-prolinamide ;
- L-valyl-L-proline n.hexylamide ;
- 1-(2(S)-aminobutyryl)-L-proline 3-(méthylthio) propylamide ;
- 1-(2(S)-aminobutyryl)-L-proline n.pentylamide ;
- 1-(2(S)-aminobutyryl)-L-proline n.butylamide ;
- 1-(2(S)-aminobutyryl)-L-proline [2(S)-méthyl] butylamide ;
- 1-(2(S)-aminobutyryl)-L-proline n.propylamide ;
- 1-(2(S)-aminobutyryl)-L-proline iso.butylamide ;
- L-valyl-L-proline n.butylamide ;
- L-alanyl-L-prolyl-L-borovaline pinanediol ester ;
- borohydrate de L-alanyl-L-prolyl-difluoro-L-borovaline ;
- 1-(2(S)-aminobutyryl)-(4(S)-fluoro)-L-proline n.butyl-amide ;
- 1-(2(S)-aminobutyryl)-(4(S)-benzyl)-L-proline n.butyl-amide ;
- acide 2-(2(S)-aminobutyryl)-1,2,3,4-tétrahydro-3(S)-iso-quinolinecarboxylique n.butylamide ;
- acide 1-(2(S)-aminobutyryl)-2(S)-indolinecarboxylique n.butylamide ;
- acide 1-(2(S)-aminobutyryl)-2(S)-indolinecarboxylique n.propylamide ;
- acide 1-(2(S)-aminobutyryl)-2(S)-indolinecarboxylique méthylamide ;
- acide 1-(2(S)-aminobutyryl)-2(S)-indolinecarboxylique éthylamide ;
- acide 1-(2(S)-aminobutyryl)-2(R/S)-(5-méthoxy)indoline carboxylique n.butylamide ;
- acide 1-(2(S)-aminobutyryl)-2(R/S)-(6-méthoxy)indoline carboxylique n.butylamide ;
- acide 1-(2(S)-aminobutyryl)-2(R/S)-(5-fluoro)indoline carboxylique n.butylamide ;
- acide 1-(2(S)-aminobutyryl)-2(R/S)-(5-benzyloxy)indoline carboxylique n.butylamide ;
- acide 1-(2(S)-aminobutyryl)-2(S)-[(3aS,7aS)-perhydro]-indolinecarboxylique n.butylamide ;
- acide 2-(2(S)-aminobutyryl)-1(R/S)-isoindoline carboxylique n.butylamide
ainsi que leurs sels ou hydrates correspondants.

Ces composés constituent des inhibiteurs préférés selon l'invention et présentent une constante Ki vis-à-vis de l'enzyme tripeptidylpeptidase selon l'invention, inférieure ou égale à 1 µM.

D'autres composés tout particulièrement préférés sont les composés de formule (I) présentant un squelette indoline à savoir les composés suivants:
- acide 1-(2(S)-aminobutyryl)-2(S)-indolinecarboxylique n.butylamide ;
- acide 1-(2(S)-aminobutyryl)-2(S)-indolinecarboxylique n.propylamide ;
- acide 1-(2(S)-aminobutyryl)-2(S)-indolinecarboxylique éthylamide ;
- acide 1-(2(S)-aminobutyryl)-2(R/S)-(5-méthoxy)indoline carboxylique n.butylamide ;
- acide 1-(2(S)-aminobutyryl)-2(R/S)-(5-fluoro)indoline carboxylique n.butylamide
ainsi que leurs sels ou hydrates correspondants.

Ces composés sont des inhibiteurs particulièrement préférés présentant une constante Ki n'excédant pas 0,02 µM.

D'autres composés tout particulièrement préférés sont encore les composés de formule (I) présentant un squelette indoline suivants :
- acide 1-(L-valyl)-5-méthoxyindoline-2(R/S)-carboxylique butylamide
- acide 1-(L-alanyl)-5-méthoxyindoline-2(R/S)-carboxylique butylamide
- acide 1-(L-alanyl)-5-méthoxyindoline-2(S)-carboxylique butylamide
- acide 1-(2(S)aminobutyryl)-4-méthoxy-indoline-2(R/S)-carboxylique butylamide
- acide 1-(2(S) aminobutyryl)-3,3-diméthylindoline-2(R/S) carboxylique butylamide
- acide 1-(2(S)-aminobutyryl)-3(R)-méthylindoline-2(R) carboxylique butylamide et acide 1-(2(S)-aminobutyryl)-2(S)-méthylindoline-2(S)-carboxylique butylamide
- acide 1-(2(S)-aminobutyryl)-3(R)-méthylindoline-2(S) carboxylique butylamide et acide 1-(2(S)-aminobutyryl)-3(S)-méthylindoline-2(R)-carboxylique butylamide
- acide 1-(2(S)-aminobutyryl)-4-éthoxyindoline-2(S)-carboxylique butylamide
- acide 1-(2(S)-aminobutyryl)-4,5-diméthoxyindoline-2(R/S)-carboxylique butylamide
- acide 1-2(S)-aminobutyryl-5-hydroxyindoline 2(S) carboxylique butylamide
- acide 1-2(S)-aminobutyryl-5-hydroxyindoline 2(R/S) carboxylique butylamide
- acide 1-2S-aminobutyryl-5-méthylindoline 2(R/S) carboxylique butylamide
- acide 1-(2(S)-aminobutyryl)-5-chloroindoline-2(S)-carboxylique butylamide
- acide 1-(2(S)-aminobutyryl) indoline-2(S)-carboxylique (3-hydroxy)propylamide
ainis que leurs sels ou hydrates correspondants.

La présente invention a également pour objet les procédés de préparation des composés de formule générale (I) décrits ci-avant.

Aussi, l'invention se rapporte plus particulièrement à un procédé (voie 1) pour la préparation d'un composé de formule générale (I) donnée ci-dessus dans laquelle :
- R₁ représente un groupe alkyle en C₁-C₄ linéaire ou ramifié ;
- R₂ représente un hydrogène ;
- R et R' représentent un hydrogène ;
- n = 0 ou 1 et m = 0 ou 1 avec n différent de m et R₃ représente -(CH₂)₂-, ; et
- R₄ représente CO-NH-R₅
- R₅ représente un hydrogène ou un groupe alkyle en C₁-C₆ linéaire ou ramifié, -(CH₂)₃-S-CH₃, -CH₂Ph ;
caractérisé en ce qu'il comprend :
i) la formation d'un composé de formule (III) 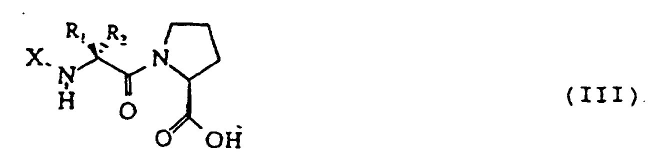 dans laquelle R₁ et R₂ ont les significations données ci-dessus et X représente un groupe protecteur, à partir d'un composé de formule (II) 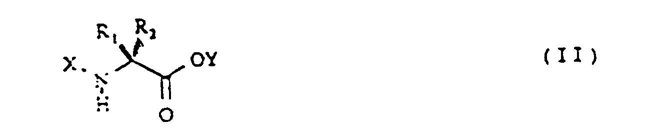 estérifié sur sa fonction acide par un groupement Y et dans laquelle X, R₁ et R₂ ont les significations données précédemment, par réaction avec la L-proline ;
ii) l'amidation de la fonction acide du composé (III) avec l'amine appropriée R₅NH₂
   où R₅ a la signification donnée ci-dessus, pour former le dérivé (IV) 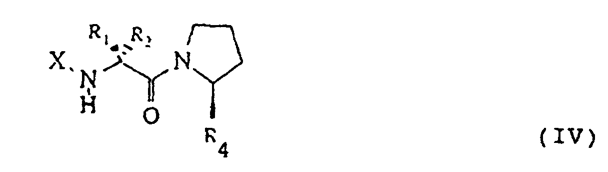 protégé sur sa fonction amine primaire par le groupe X ;
iii) l'élimination du groupe X du dérivé (IV), pour obtenir le composé (I) désiré.

Le groupe Y représente de préférence le motif

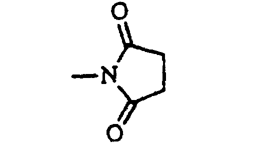

L'étape i) est effectuée en présence de triéthylamine et d'eau.

Par ailleurs, à l'étape ii), on forme l'anhydride mixte de l'acide (III) et du chloroformiate d'isobutyle, que l'on fait réagir ensuite in situ avec l'amine R₅NH₂.

Dans les composés préparés selon cette voie 1, R₁ représente de préférence un groupe ethyle ou isopropyle.

L'invention se rapporte également à un procédé (voie 2) pour la préparation d'un composé de formule générale (I) donnée ci-dessus dans laquelle :
- R₁ représente un groupe alkyle en C₁-C₄ linéaire ou ramifié ;
- R₂ représente un hydrogène ou un groupe méthyle ;
- R et R' représentent un hydrogène ;
- n = 0 ou 1 et m = 0 ou 1 avec n différent de m et R₃ représente un groupe -(CH₂)₂- ; et
- R₄ représente un groupe CO-NH-R₅ où R₅ représente un hydrogène ou un groupe alkyle en C₁-C₆ linéaire ou ramifié ;
   caractérisé en ce qu'il comprend :
   i) la formation d'un composé de formule (IV) 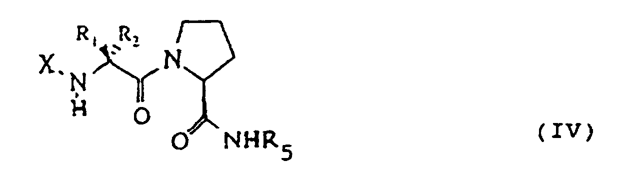 dans laquelle R₁, R₂ et R₅ ont les significations données ci-dessus et X représente un groupe protecteur, à partir d'un composé de formule (II) 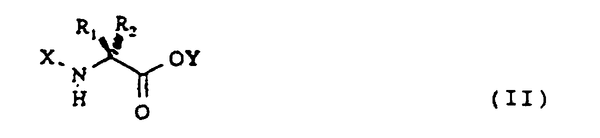 estérifié sur sa fonction acide à l'aide d'un groupe Y et dans laquelle R₁, R₂ et X ont les significations données précédemment, par réaction avec une prolineamide de formule (V) 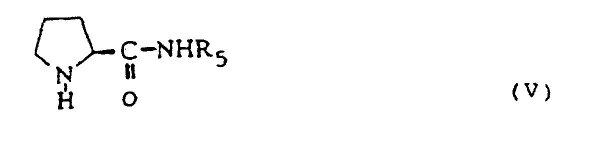 dans laquelle R₅ a la signification donnée ci-dessus ;
   ii) l'élimination du groupe protecteur X du composé (IV), pour obtenir le composé (I) désiré.

La prolineamide (V) peut être préparée par réaction de la L-proline 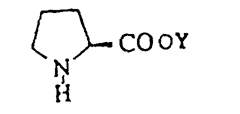 protégée sur sa fonction amine par un groupe protecteur X et estérifiée sur sa fonction acide à l'aide d'un groupe Y, avec l'amine R₅NH₂ appropriée.

Le groupe Y représente de préférence le motif

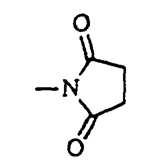

Pour former le composé (IV), on fait réagir la prolineamide ainsi obtenue avec le composé (II), en présence d'eau et de triéthylamine dans un solvant tel que le tétrahydrofuranne ou le dioxanne.

Dans les composés préparés selon cette voie 2, R₁ représente de préférence un groupe éthyle ou isopropyle et R₅ représente de préférence un hydrogène ou un groupe n.butyle.

L'invention se rapporte également à un procédé (voie 3) pour la préparation d'un composé de formule générale (I) donnée ci-dessus dans laquelle :
- R₁ représente un groupe alkyle en C₁-C₄ linéaire ou ramifié ;
- R₂ représente un hydrogène ;
- R et R' représentent un hydrogène ;
- n = 0 et m = 1 et R₃ représente un groupe 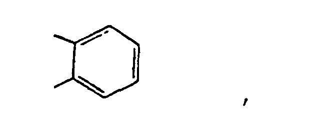 -CH₂-CH(cis.F)-, 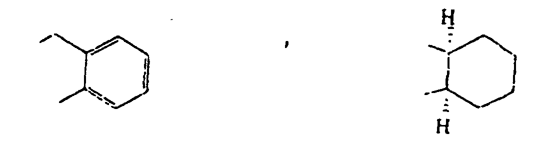
- R₄ représente un groupe CO-NH-R₅ où R₅ représente un hydrogène ou un groupe alkyle en C₁-C₆ linéaire ou ramifié ;
caractérisé en ce qu'il comprend :
i) la préparation d'un composé de formule (X) 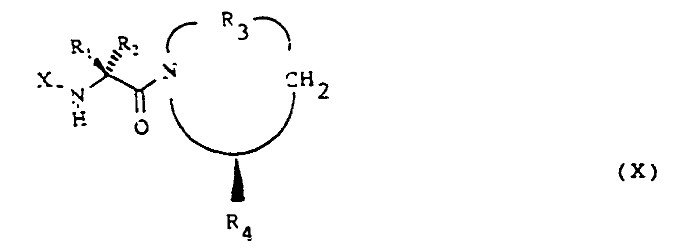 dans laquelle R₁, R₂, R₃ et R₄ ont les significations données ci-dessus et X représente un groupe protecteur, par réaction d'un composé de formule (VIII) 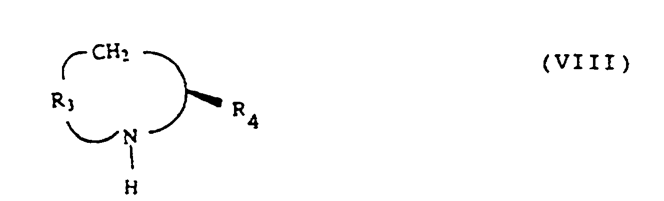 avec un composé de formule (IX) 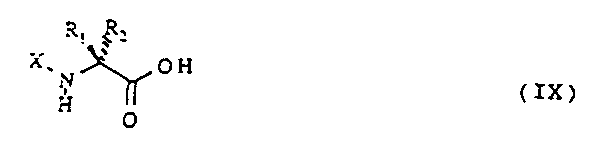 dans lesquelles R₁, R₂, R₄ et X ont les significations données précédemment ;
ii) l'élimination du groupement X du composé (X), pour former le composé (I) désiré.

Selon cette voie 3, on prépare le composé (VIII) par amidation de la fonction acide d'un composé (VI) 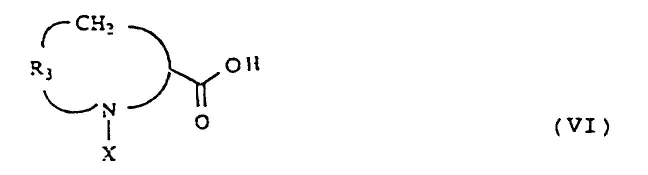 protégé sur sa fonction amine par un groupe protecteur X et dans laquelle R₃ a la signification donnée précédemment, avec l'amine R₅NH₂ appropriée, pour former le dérivé (VII) 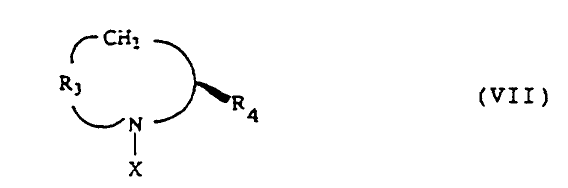 dans laquelle R₃, R₄ et X ont les significations données ci-dessus, puis par élimination du groupe protecteur X.

Pour cela, on forme avantageusement l'anhydride mixte de l'acide (VI) et de chloroformiate d'isobutyle en présence de N-éthylmorpholine dans du tétrahydrofuranne, que l'on fait ensuite réagir in situ avec l'amine R₅NH₂ appropriée. Ces conditions peuvent aussi constituer celles de l'étape i) sauf que l'on fait réagir le composé (VII) à la place de l'amine R₅NH₂.

Dans les composés préparés selon cette voie 3, R₁ représente de préférence un groupe éthyle et R₅ représente de préférence un hydrogène ou un groupe méthyle, éthyle, n.propyle ou n.butyle.

L'invention se rapporte aussi à un procédé (voie 4) pour la préparation d'un composé de formule générale (I) donnée ci-dessus dans laquelle :
- R₁ représente un groupe alkyle en C₁-C₄ linéaire ou ramifié ;
- R₂ représente un hydrogène ;
- n = 1 et m = 0 et R₃ représente un groupe -CH(CH₂Ph)-CH₂-, cu 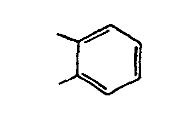
- R₄ représente un groupe amide CO-NH-R₅ où R₅ représente un hydrogène ou un groupe alkyle en C₁-C₆ linéaire ou ramifié ;
caractérisé en ce qu'il comprend :
i) l'obtention d'un composé (XII) 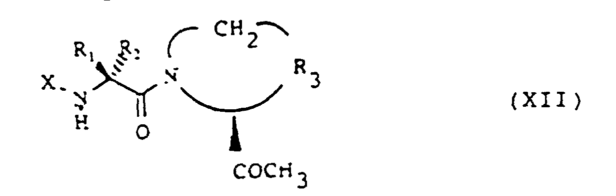 protégé sur sa fonction amine primaire par un groupe protecteur X et dans laquelle R₁, R₂ et R₃ ont les significations données ci-dessus, par réaction du composé de formule (IX) 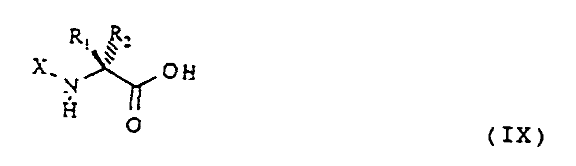 avec le composé de formule (XI) 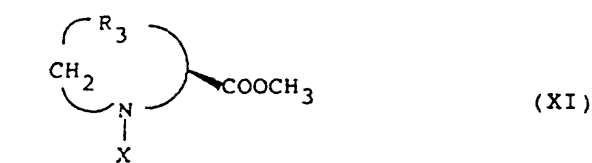 dans lesquelles R₁, R₂ et R₃ ont les significations données précédemment et X représente un groupe protecteur ;
ii) l'hydrolyse de la fonction ester du composé (XII) ainsi obtenu pour former le composé de formule (XIII) 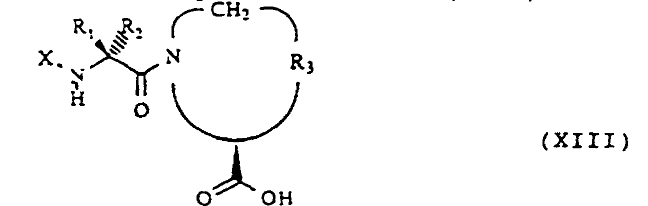 dans laquelle R₁, R₂, R₃ et x ont les significations données précédemment ;
iii) l'amidation de la fonction acide du composé (XIII) à l'aide de l'amine R₅NH₂ appropriée pour former le dérivé de formule (XIV) 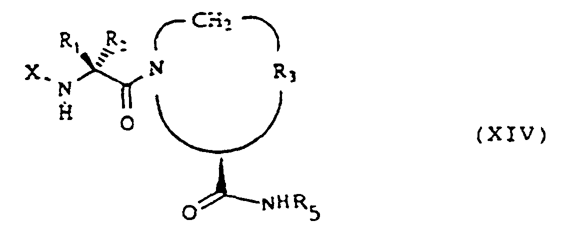 dans laquelle R₁, R₂, R₃, R₅ et X ont les significations données ci-dessus ;
iv) l'élimination du groupe protecteur X du composé (XIV) pour former le composé (I) désiré.

L'étape ii) d'hydrolyse est avantageusement effectuée en présence de soude dans du méthanol.

A l'étape iii), on forme l'anhydride mixte de l'acide (XIII) et du chloroformiate d'isobutyle, que l'on fait ensuite réagir in situ avec l'amine R₅NH₂ appropriée.

Dans les composés préparés selon cette voie 4, R₁ représente de préférence un groupe éthyle et R₅ représente de préférence un hydrogène ou un groupe n.butyle.

L'invention se rapporte encore à un procédé (voie 5) pour la préparation d'un composé de formule générale (I) donnée ci-dessus, dans laquelle :
- R₁ représente un groupe méthyle ;
- R₂ représente un hydrogène ;
- R et R' représentent un hydrogène ;
- n = 0 ou 1 et m = 0 ou 1 avec n différent de m et R₃ représente le groupe -(CH₂)₂- ; et
- R₄ représente un groupe amide CO-NH-R₅ où R₅ représente le motif 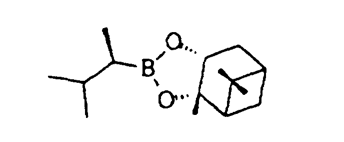
caractérisé en ce qu'il comprend :
i) l'amidation de la fonction acide du composé de formule (XIX) 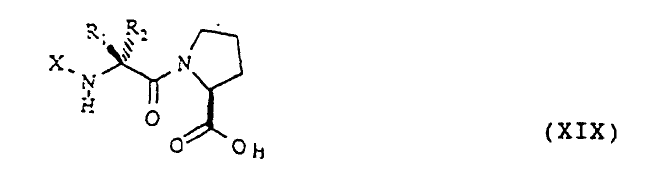 protégé sur sa fonction amine primaire par un groupe protecteur X et dans laquelle R₁ et R₂ ont les significations données ci-dessus, à l'aide de l'amine (XVIII) 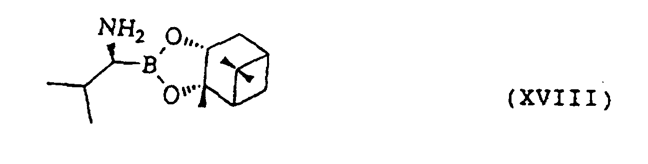 pour former le dérivé (XX) 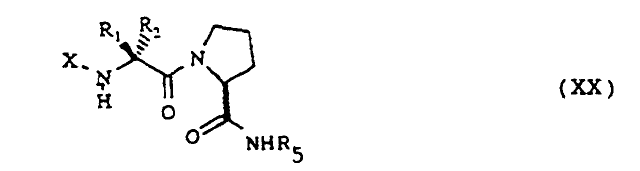 dans laquelle R₁, R₂, R₅ et X ont les significations données précédemment ;
ii) l'élimination du groupe protecteur X du composé (XX) pour obtenir le composé (I) désiré.

L'étape i) est avantageusement réalisée à l'aide de chloroformiate d'isobutyle pour former l'anhydride mixte de l'acide XIX, en présence de N-éthylmorpholine dans du tétrahydrofuranne.

L'étape ii) peut, quant à elle, être effectuée à l'aide d'acide trifluoroacétique dans du chlorure de méthylène.

L'amine (XVIII) est préparée
i) par réaction de l'acide isopropylboronique avec le (+)-pinanediol dans l'éther, pour obtenir le dérivé (XV) 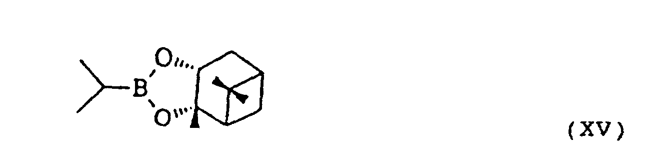
ii) par réaction du composé (XV) avec du butyllithium et. du chlorure de méthylène puis du chlorure de zinc ZnCl₂ dans du tétrahydrofuranne, à basse température, pour former le dérivé (XVI) 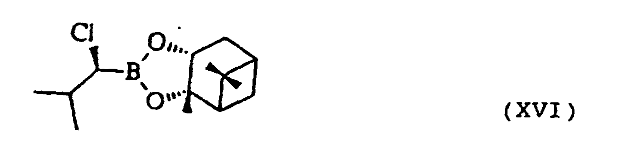
iii) substitution du chlore du composé (XVI) à l'aide de butyllithium et d'hexaméthyldisilazanne dans du tétrahydrofuranne à basse température, pour former le composé (XVII) 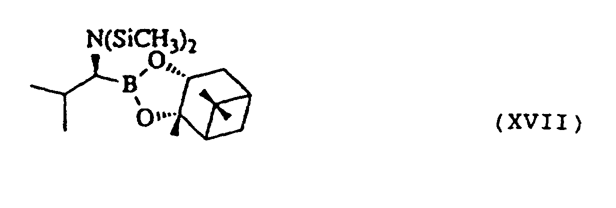
iv) libération de la fonction amine du composé (XVII) pour obtenir le composé (XVIII) désiré.

L'acide isopropylboronique est obtenu par addition de triéthylborate au chlorure d'isopropylmagnésium dans l'éther à basse température.

L'étape iv) précitée est avantageusement réalisée par l'acide trifluoroacétique dans du pentane.

L'invention se rapporte également à un procédé (voie 6) pour la préparation d'un composé de formule générale (I) donnée ci-dessus dans laquelle :
- R₁ représente un groupe méthyle ;
- R₂ représente un hydrogène ;
- R et R' représentent un hydrogène ;
- n = 0 ou 1 et m = 0 ou 1 avec n différent de m et R₃ représente un groupe -(CH₂)₂- ; et
- R₄ représente un groupe amide CO-NH-R₅ où R₅ représente (CH₃)₂-CH-BF₂ ;
caractérisé en ce qu'il comprend :
i) l'élimination du motif pinane du composé de formule (I) dans laquelle R₁, R₂, n, m R₃ et R₄ ont les significations données ci-dessus et R₅ représente 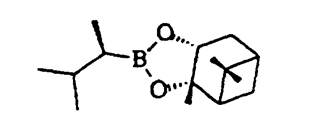 par action du trichlorure de bore dans du chlorure de méthylène suivie d'une hydrolyse, pour obtenir le dérivé (XXI) 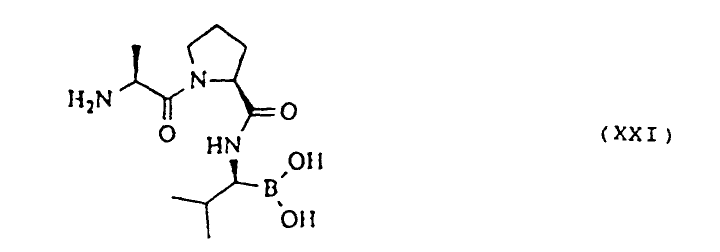
ii) le fait de faire réagir le composé (XXI) avec l'acide fluorhydrique pour former le composé (I) désiré.

L'invention se rapporte à un procédé (voie 7) pour la préparation d'un composé de formule générale (I) donnée ci-dessus, dans laquelle :
- R₁ représente un groupe alkyle en C₁-C₄ linéaire ou ramifié ;
- R₂ représente un hydrogène ;
- R et R' représentent un hydrogène ;
- n = 0 et m = 1 et R₃ représente le motif 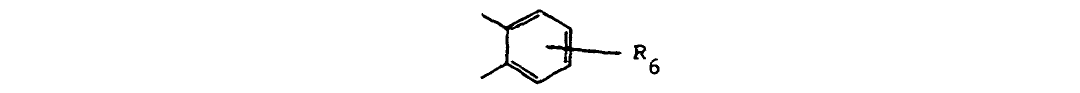
où R₆ représente un groupe OCH₃, OCH₂Ph, F ; et
- R₄ représente un groupe amide CO-NH-R₅ où R₅ représente un groupe alkyle en C₁-C₆ linéaire ou ramifié ;
caractérisé en ce qu'il comprend :
i) la formation de l'amide de formule (XXXXIV) 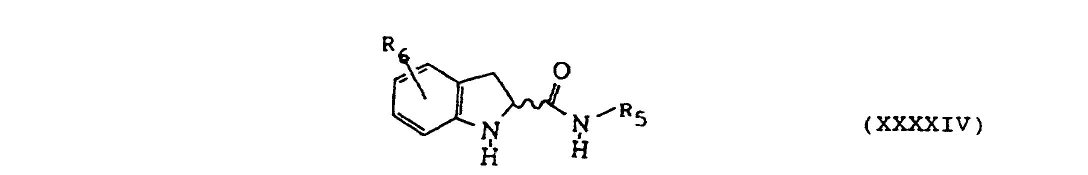 à partir de l'ester de formule (XXXXIII) 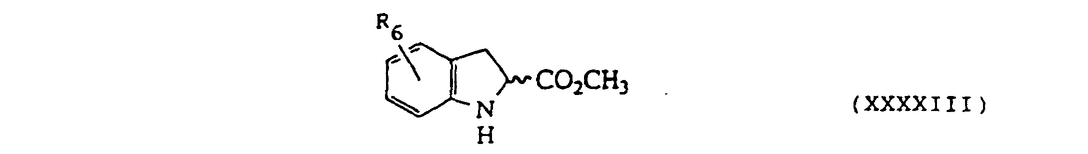 dans lesquelles R₆ a la signification donnée ci-dessus, par réaction avec l'amine R₅NH₂ appropriée ;
ii) la réaction du composé (XXXXIV) avec le composé de formule (IX) 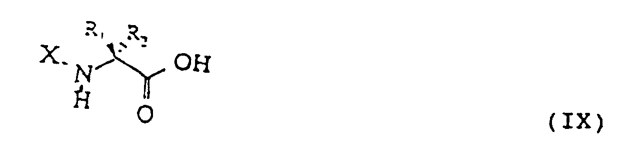 dans laquelle R₁ et R₂ ont les significations précitées et X représente un groupement protecteur, pour former le composé de formule (XXXXV) 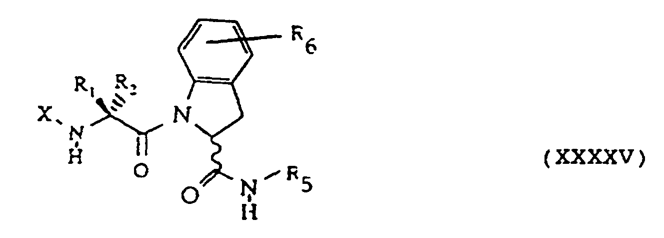 dans laquelle R₁, R₂, R₅, R₆ et X ont les significations données précédemment ;
iii) l'élimination du groupement X pour former le composé (I) désiré.

L'étape ii) s'effectue avantageusement en présence du chlorure de l'acide bis(2-oxo 3-oxazolodinyl)phosphinique et de triéthylamine dans du chlorure de méthylène.

Quant à l'ester méthylique (XXXXIII), il peut être obtenu
i) par action du nitrite de sodium en présence d'acide chlorhydrique, sur le composé de formule (XXXIX) 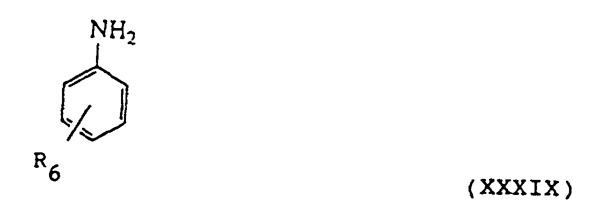 pour former le composé de formule (XXXX) 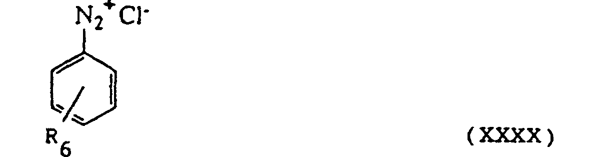 dans lesquelles R₆ a la signification indiquée précédemment
ii) par addition de 2-méthylacétoacétate d'éthyle au composé (XXXX) ainsi obtenu, en présence de nitrite de sodium dans l'éthanol, pour former le composé de formule (XXXXI) 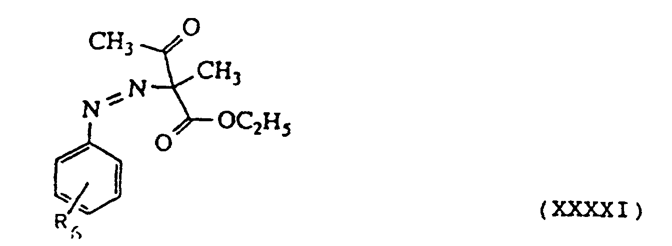 dans laquelle R₆ a la signification précitée ;
iii) par cyclisation en milieu acide, pour former l'ester éthylique (XXXXII) 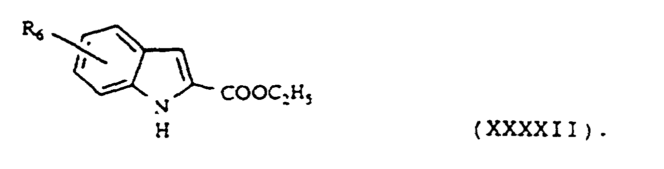
iv) par échange à partir de l'ester éthylique (XXXXII) ainsi obtenu, en présence de magnésium dans l'éthanol.

L'ester éthylique subit un échange d'ester et une réduction en présence de magnésium et de méthanol pour conduire à l'ester méthylique (XXXXIII).

Dans les composés préparés selon cette voie 7, R₁ représente de préférence un groupe éthyle et R₅ représente de préférence un groupe n.butyle.

L'invention se rapporte à un procédé (voie 8) pour la préparation d'un composé de formule générale (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 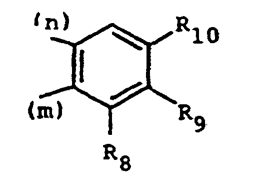
où R₈ et R₁₀ représentent un hydrogène et R₉ représente un groupe O (alkyle en C₁-C₄) ou alkyle en C₁-C₄
- R₄ représente un groupe amide CO-NH-R₅ où R₅ représente un groupe alkyle en C₁-C₆ linéaire ou ramifié ;
caractérisé en ce qu'il comprend :
i) la formation de l'amide de formule (49) 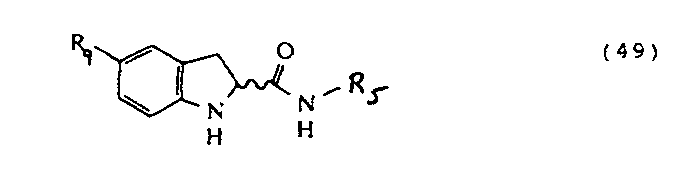 à partir de l'ester de formule (48) 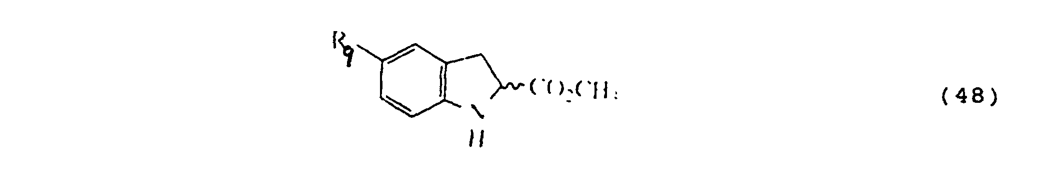 dans lesquelles R₉ et R₅ ont la signification donnée ci-dessus, par réaction avec l'amine R₅NH₂ appropriée ;
ii) la réaction du composé (49) avec le composé de formule (IX) 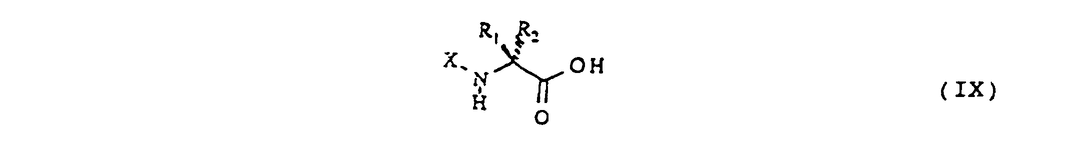 dans laquelle R₁ et R₂ ont les significations précitées et X représente un groupement protecteur, pour former le composé de formule (50) 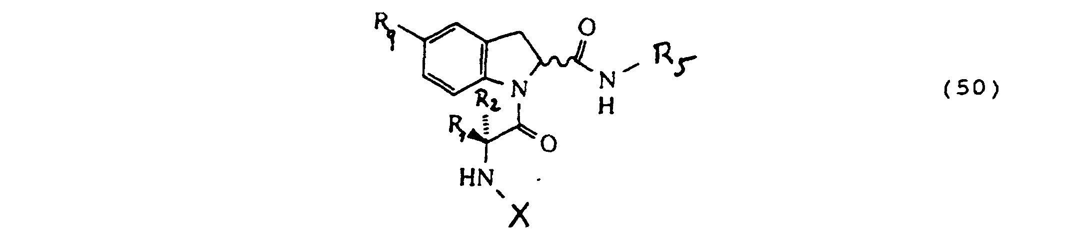 dans laquelle R₁, R₂, R₉ et X ont les significations données précédemment ;
iii) l'élimination du groupement X pour former le composé (I) désiré.

L'étape ii) s'effectue avantageusement en présence de chlorure bis(2-oxo 3-oxazolidinyl)phosphonique et de triéthylamine dans du chlorure de méthylène.

L'ester méthylique (48) peut être obtenu à partir de l'acide (46) correspondant 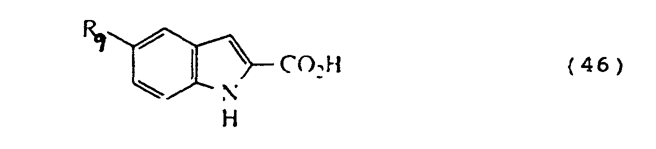 où R₉ a la signification donné précédemment
i) par traitement dans de l'éthanol ou du méthanol avec de l'acide sulfurique concentré, pour conduire à l'ester (47) correspondant 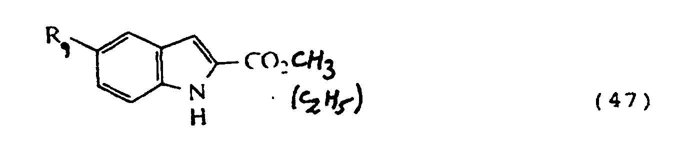 où R₉ est tel que défini ci-dessus
ii) puis le composé (47) est traité avec du magnésium dans le méthanol,

Dans les composés préparés selon cette voie 8, R₁ représente de préférence un groupe CH₃, C₂H₅ ou (CH₃)₂CH, R₉ représente de préférence un groupe OCH₃ ou CH₃ et R₅ représente de préférence un groupe n. butyle.

L'invention a encore pour objet un procédé (voie 9) pour la préparation d'un composé de formule générale (I) donnée ci-dessus, dans laquelle
- R₁ représente un groupe alkyle en C₁-C₄ linéaire ou ramifié ;
- R₂, R et R' représentent chacun un hydrogène ;
- n = 0 et m = 1 et R₃ représente le motif 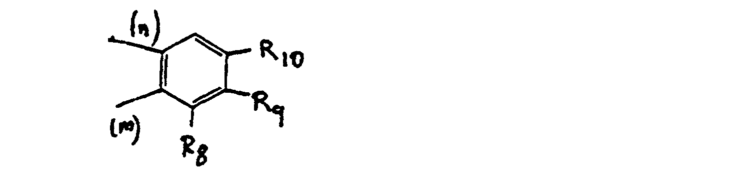
où R₈ et R₉ représentent un hydrogène ou un groupe O(alkyle en C₁-C₄), R₈ et R₉ ne pouvant représenter simultanément un hydrogène et R₁₀ représente un hydrogène
- R₄ représente un groupe amide CONHR₅ où R₅ est un groupe alkyle en C₁-C₆ linéaire ou ramifié,
caractérisé en ce qu'il comprend :
i) la formation du composé (54) 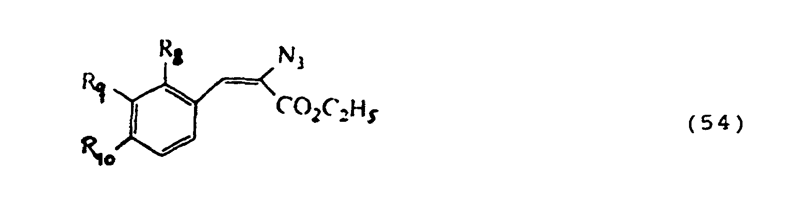 par réaction de l'aldéhyde (53) correspondant 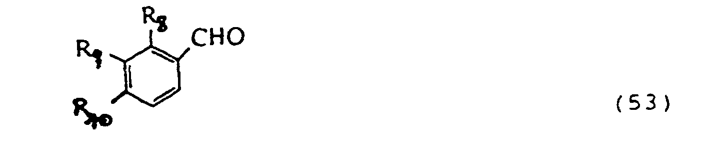 où R₈, R₉ et R₁₀ sont tels que définis ci-dessus avec de l'azidoacétate d'éthyle (52) 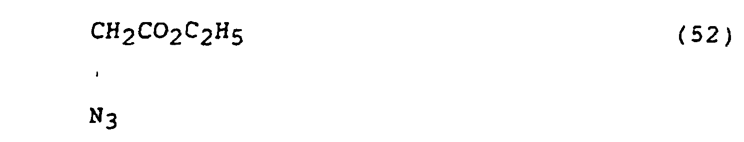
ii) la cyclisation du composé (54) pour conduire au composé (55) 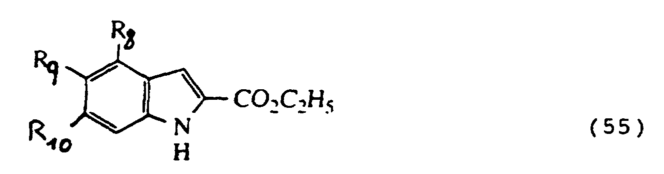 où R₈, R₉ et R₁₀ ont les significations données précédemment
iii) la formation de l'ester méthylique (56) 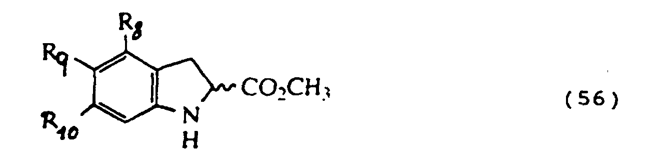 où R₈, R₉ et R₁₀ ont les significations données précédemment
   à partir du composé (55) en présence de magnésium dans du méthanol
iv) la réaction de l'ester (56) obtenu avec l'amine R₅NH₂ appropriée pour former l'amide (57) 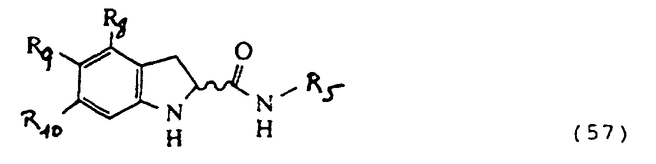 où R₈, R₉ et R₁₀ sont tels que définis ci-dessus
v) la réaction du composé (57) avec le composé de formule (IX) 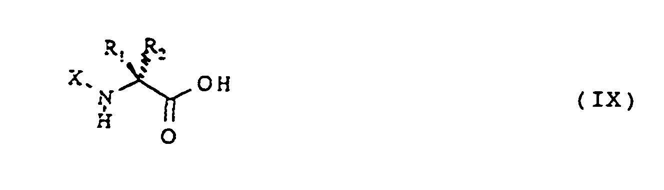 où R₁ et R₂ ont la signification précitée et X représente un groupement protecteur, pour former le composé (58) 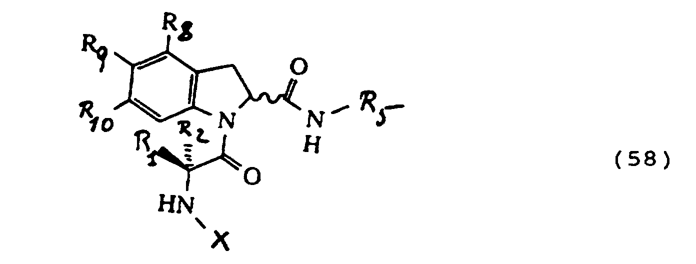 où R₁, R₂, R₅, R₈, R₉, R₁₀ et X ont la signification donnée précédemment,
vi) l'élimination du groupement X pour former le composé (I) désiré.

Le composé (52) peut être obtenu par action d'azoture de sodium sur le bromoacétate d'éthyle dans l'acétonitrile.

Selon l'étape i), on forme d'abord de l'éthylate de sodium puis on fait réagir les composés (52) et (53).

L'étape ii) de cyclisation est avantageusement réalisée par,reflux dans le toluène

L'étape v) s'effectue avantageusement en présence de chlorure bis (2-oxo 3-oxazolidinyl) phosphinique et de triethylamine dans le chlorure de méthylène.

Dans les composés préparés selon cette voie 9, R₁ représente de préférence le groupe CH₂CH₃, R₈ un groupe OCH₃ ou OC₂H₅ et R₉ un hydrogène, ou encore R₈ et R₉ représentent tous deux un groupe OCH₃, et R₅ représente de préférence un groupe n-butyle.

L'invention a encore pour objet un procédé (voie 10) pour la préparation d'un composé de formule (I) donnée ci-dessus, dans laquelle :
- R₁ représente un groupe alkyle en C₁-C₄ linéaire ou ramifié ;
- R₂ représente un hydrogène ;
- l'un des substituants R ou R' représente un hydrogène et l'autre un groupe alkyle en C₁-C₂.
- n = 0 et m = 1 et R₃ représente le motif 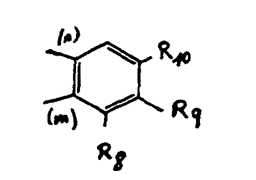 avec R₈, R₉ et R₁₀ représentant un hydrogène
- R₄ représente un groupe amide CONHR₅ où R₅ est un groupe alkyle en C₁-C₆ linéaire ou ramifié,
caractérisé en ce qu'il comprend :
i) la formation du composé (59) 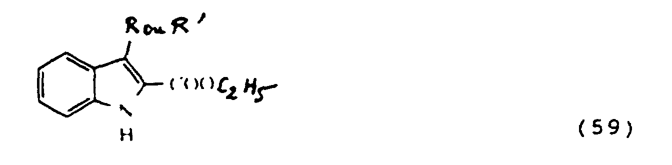 où R et R' sont tels que définis ci-dessus
   par réaction entre la phényl hydrazine et l'acide 2-céto-butyrique en milieu acide
ii) la formation du composé (60) à partir du composé (59) obtenu 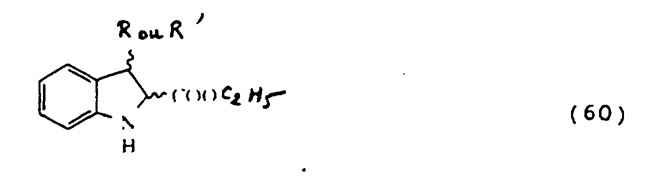 où R et R' sont tels que définis ci-dessus
   en présence de magnésium dans le méthanol,
iii) la formation de l'amide (61) correspondant au composé (60) par réaction avec l'amine appropriée R₅NH₂ 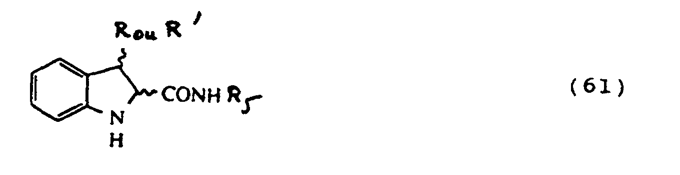 où R et R' sont tels que définis ci-dessus
iv) la séparation des isomères cis (61a) d'une part et des isomères trans (61b) d'autre part du composé (61) 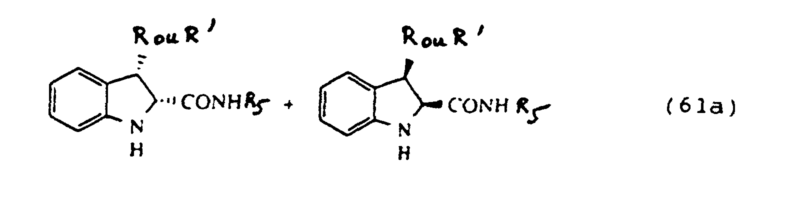 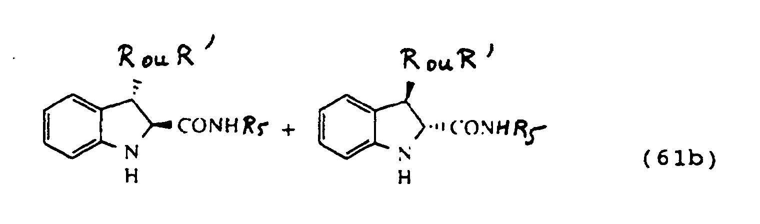
v) la réaction respectivement des composés (61a) et (61b) avec le composé (IX) 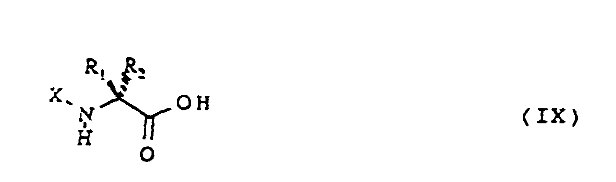 où R₁ et R₂ ont la signification donnée précédemment et X représente un groupe protecteur, pour former respectivement les mélanges (62a) et (62b) 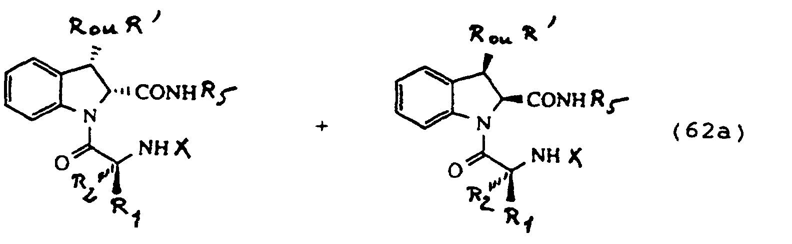 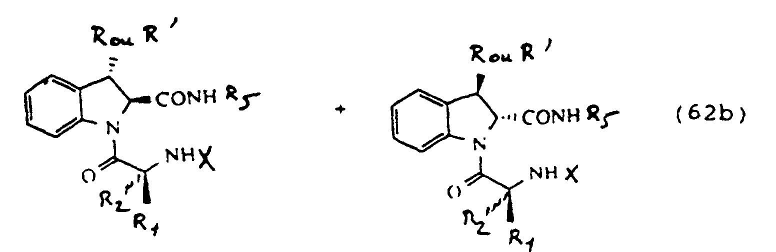 où R₁, R₂, R, R' et R₅ ont la signification donnée précédemment
vi) l'élimination du groupement protecteur x conduisant au composé (I) désiré sous forme respectivement des paires cis (63a) et trans (63b).

L'étape v) est effectuée avantageusement en présence de chlorure bis (2-oxo 3-oxazolidinyl) phosphinique et de tr-iethylamine dans le chlorure de méthylène.

Dans les composés préparés selon cette voie 10, R₁ représente de préférence le groupe CH₂CH₃, R ou R' représente le groupe CH₃ (l'autre substituant étant un hydrogène) et R₅ représente de préférence le groupe n. butyle.

L'invention a aussi pour objet un procédé (voie 11) pour la préparation d'un composé de formule (I) donnée ci-dessus, dans laquelle :
- R₁ représente un groupe alkyle en C₁-C₄ linéaire ou ramilfié ;
- R₂ représente un hydrogène ;
- R et R' représentent chacun un groupe alkyle en C₁-C₂, identiques ou différents ;
- n = 0 et m = 1 et R₃ représente le motif 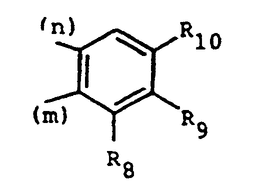
où R₈, R₉ et R₁₀ représentent chacun un hydrogène ;
- R₄ représente un groupe amide CONHR₅ où R₅ est un groupe alkyle en C₁-C₆ linéaire ou ramifié ;
caractérisé en ce qu'il comprend :
i) la formation du composé (65) de formule suivante : 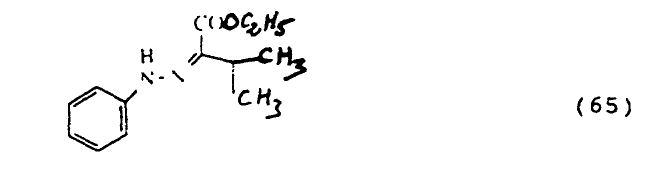 par réaction de la phénylhydrazine avec le 2-oxo 3-méthyl butanoate d'éthyle (64)
ii) la cyclisation du composé (65) en milieu acide, pour former le composé (66) suivant : 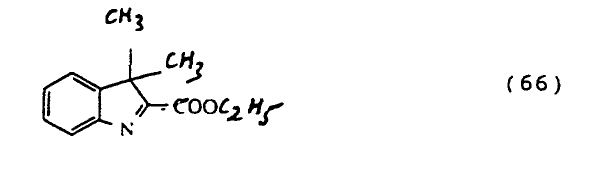
iii) l'hydrogenation du composé (66) conduisant au composé (67) suivant 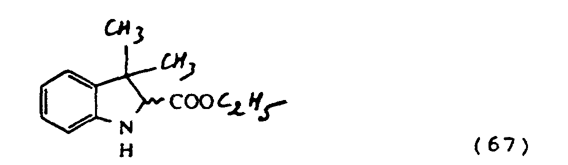
iv) la formation de l'amide correspondant (68) par action de LiNHR₅ où R₅ a la signification précitée 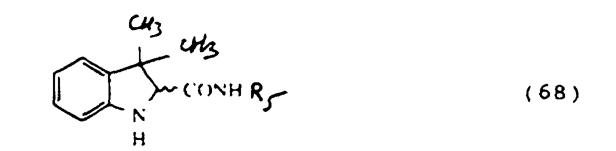
v) la réaction du composé (68) avec le composé de formule (IX) 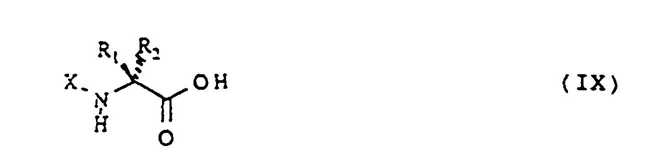 où R₁ et R₂ ont la signification donnée ci-dessus et X est un groupement protecteur, pour former le composé (69) 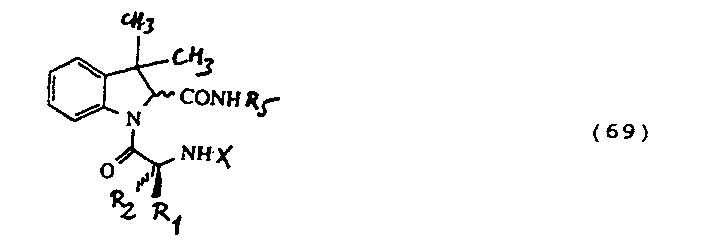
vi) l'élimination du groupement X pour former le composé (I) désiré.

La voie 11 est illustrée ci-dessus dans le cas où R et R' représentent un groupe méthyle mais R et R' peuvent représenter chacun un groupe alkyle, identiques ou différents. L'étape i) est alors réalisée avec le composé approprié, notamment un composé (64)

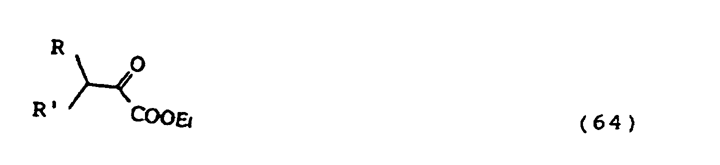

L'étape i) s'effectue avantageusement à une température de l'ordre de 60°C dans le toluène.

L'étape v) s'effectue avantageusement en présence de chlorure bis(2-oxo 3-oxazolidinyl)phosphinique et de triéthylamine dans le chlorure de méthylène.

Le 2-oxo 3-méthylbutanoate d'éthyle (64) peut être obtenu à partir de diéthyloxalate avec du chlorure d'isopropylmagnésium, dans l'éther à basse température.

Dans les composés préparés selon cette voie 11, R₁ représente de préférence un groupe éthyle et R₅ représente de préférence un groupe n.butyle.

L'invention concerne également un procédé (voie 12) pour la préparation d'un composé de formule (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 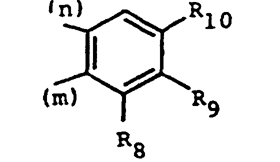
où R₉ représente un groupe OH et R₈ et R₁₀ représentent tous deux un hydrogène ;
- R₄ représente un groupe amide CONHR₅ où R₅ est un groupe alkyle en C₁-C₆ linéaire ou ramifié ;
caractérisé en ce qu'il comprend :
i) la formation du composé (71) 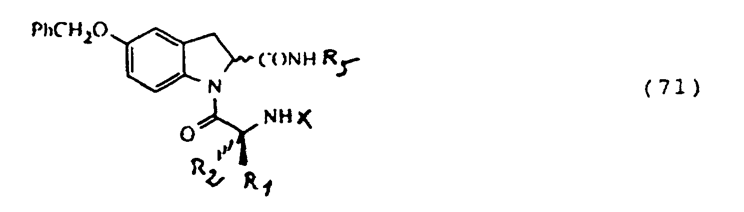 où R₉ représente un groupe OCH₂Ph, R₅ a la signification donnée précédemment et X représente un groupe protecteur, par réaction du composé (70) 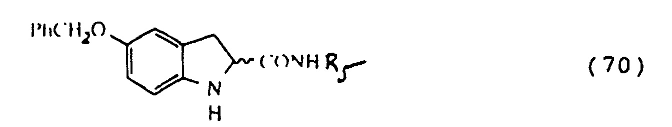 où R₅ est tel que défini ci-dessus, avec un composé (IX) 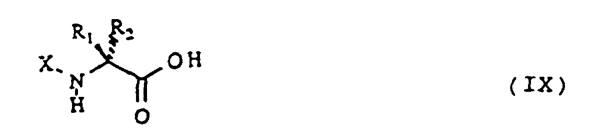 dans lequel R₁ et R₂ ont la signification donnée ci-dessus et X représente un groupement protecteur
ii) l'élimination des groupements CH₂Ph et X du composé (71) pour former le composé (I) désiré.

L'étape i) est avantageusement effectuée en présence de dicyclohexylcarbodiimide dans le chlorure de méthylène.

Le composé (70) peut être obtenu par le procédé décrit ci-avant (voie 7).

Dans les composés préparés selon cette voie 12, R₁ représente de préférence un groupe éthyle et R₅ un groupe n.butyle.

L'invention concerne encore un procédé (voie 13) pour la préparation d'un composé de formule (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 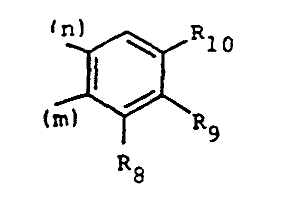
où R₉ représente un atome d'halogène et R₈ et R₁₀ représentent chacun un hydrogène
- R₄ représente un groupe amide CONHR₅ où R₅ est un groupe alkyle en C₁-C₆ linéaire ou ramifié ;
caractérisé en ce qu'il comprend :
i) la formation de l'ester méthylique (73) 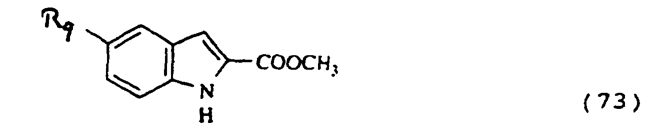 à partir de l'acide (72) correspondant 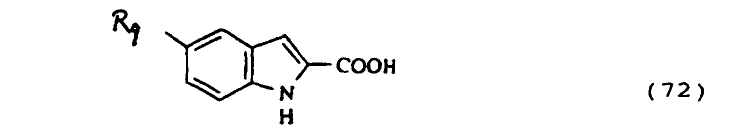 où R₉ est tel que défini ci-dessus,
   avec de l'acide sulfurique concentré dans du méthanol,
ii) la formation du composé (74) 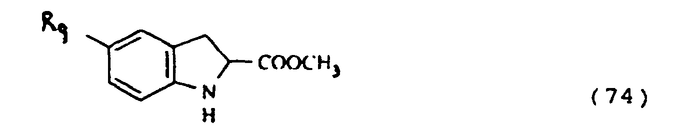 où R₉ est tel que défini ci-dessus,
   à partir du composé (73), avec du magnésium dans du méthanol,
iii) la formation de l'amide (75) correspondant par réaction avec l'amine appropriée R₅NH₂ 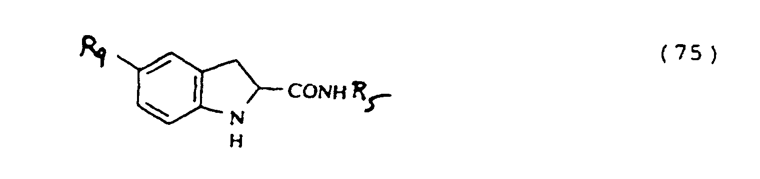 où R₅ et R₉ sont tels que définis ci-dessus
iv) la réaction du composé (75) avec un composé (IX) 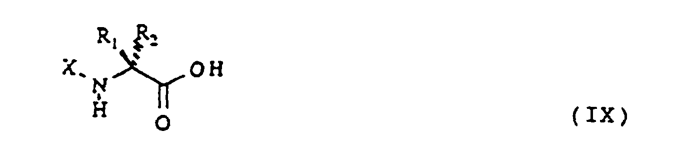 dans lequel R₁ et R₂ ont les significations données précédemment et X représente un groupe protecteur, pour former le composé (76) 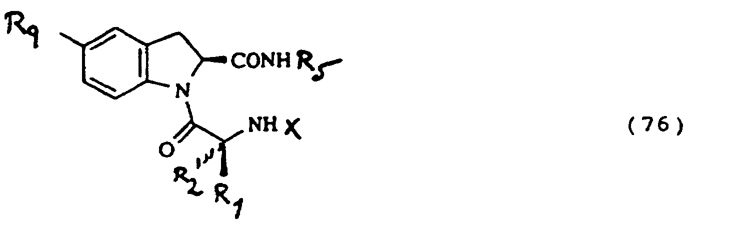 où R₁, R₂, R₅, R₉ et X ont les significations données précédemment
v) l'élimination du groupement X pour former le composé (I) désiré.

L'étape iv) est avantageusement réalisée en présence de chlorure bis(2-oxo-3-oxazolidinyl)phosphonique et d'éthylamine dans le chlorure de méthylène.

Dans les composés préparés selon cette voie 13, R₁ représente de préférence un groupe éthyle et R₅ représente de préférence un groupe n.butyle.

Enfin, l'invention a pour objet un procédé (voie 14) de préparation d'un composé de formule (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 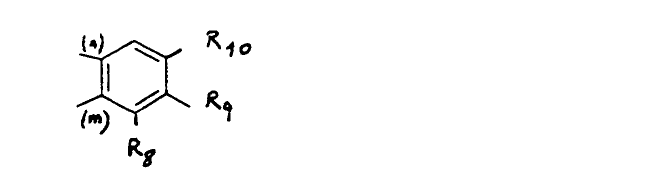
où R₈, R₉ et R₁₀ représentent chacun un hydrogène;
- R₄ représente un groupe amide CONHR₅ où R₅ est un groupe (CH₂)₃OH
caractérisé en ce qu'il comprend :
i) la réaction de l'ester méthylique de l'acide indoline 2S-carboxylique avec un composé de formule (IX) 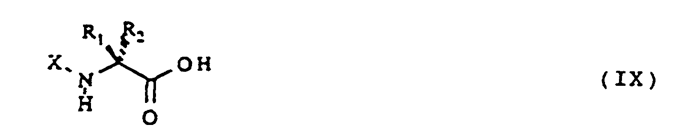 où R₁ et R₂ ont la signification donnée ci-dessus et X représente un groupe protecteur, pour former le composé (77) suivant 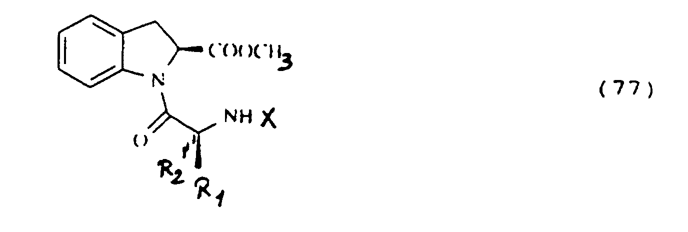 où R₁ et R₂ et X sont tels que définis ci-dessus
ii) la formation de l'amide (78) 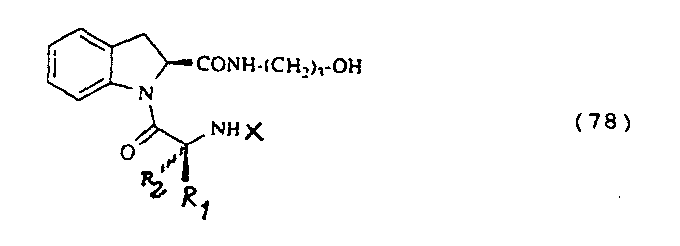 où R₁ et R₂ ont la signification précédente
   à partir du composé (77), par action de la 3-hydroxypropylamine dans le méthanol
iii) l'élimination du groupement X pour former le composé (I) désiré.

L'ester méthylique de l'acide indoline 2S-carboxylique peut être préparé à partir de l'acide indoline 2S-carboxylique avec du méthanol et de l'acide sulfurique concentré.

L'étape ii) est avantageusement réalisée en présence de chlorure bis(2-oxo-3-oxazolidinyl)phosphinique et de triéthylamine dans le chlorure de méthylène.

Dans les composés préparés selon cette voie 14, R₁ représente de préférence un groupe éthyle.

Dans tous les procédés décrits ci-desssus, la formation des anhydrides d'acides peut être effectuée par les méthodes classiques, avantageusement à l'aide de chloroformiate d'isobutyle en présence de N-éthylmorpholine dans du tétrahydrofuranne.

Par ailleurs, selon l'invention, X ou X' représente un groupe protecteur classique capable de protéger la fonction désirée dans une molécule donnée sans altérer les autres fonctions de celle-ci, tel que notamment les groupes benzyle, benzyloxycarbonyle ou tert-butoxycarbonyle. Ces groupes sont introduits par les méthodes classiques bien connues de l'homme du métier.

Il en est de même pour les étapes de déprotection qui sont également réalisées par des méthodes connues en soi comme en particulier l'hydrolyse acide ou encore l'hydrogénation catalytique notamment en présence de Pd/C.

Quant au groupement Y, il s'agit d'un radical capable d'estérifier une fonction acide sans modifier les autres fonctions de la molécule, et constituant un bon groupe partant afin de pouvoir être facilement éliminé lors d'une étape subséquente. Il s'agit de préférence de l'ester succinimidique.

Les travaux de la demanderesse ont montré que les composés de formule (I) présentaient de nombreuses propriétés thérapeutiques dans le domaine du traitement de troubles ou affections dans lesquels l'inactivation ou dégradation exagérée (ou pouvant être traités en retardant la dégradation physiologique) des neuropeptides endogènes desactivés par la tripeptidylpeptidase membranaire définie à la revendication 1 est impliquée comme en particulier les troubles ou affections liés à l'inactivation de la CCK. Donc, la présente invention a également pour objet l'utilisation d'un composé de formule (I) pour la préparation d'un médicament pour traiter, chez l'homme ou chez l'animal, en particulier les troubles de la prise alimentaire, de l'humeur, cognitifs ou moteurs, notamment l'anoréxie, la schizophrénie, la maladie de Parkinson et la dépression ainsi que les troubles du transit gastro-intestinal tel que le syndrome du côlon irritable, la boulimie ou les états d'obésité pathologique.

La présente invention a également pour objet l'utilisation d'un composé de formule (I) dans la préparation d'un médicament destiné au traitement, chez l'homme ou l'animal, des affections ou troubles provoqués par la dégradation physiologique de neuropeptides endogènes desactivés par la TPP membranaire définie à la revendication 1, en particulier celle de la CCK.

La présente invention a en particulier pour objet l'utilisation de composés de formule (I) dans la préparation d'un médicament destiné au traitement des troubles ou affections tels que précités.

Des composés de formule (I) utiles en tant que médicaments peuvent être administrés dans un support physiologiquement acceptable.

Aussi la présente invention a également pour objet des compositions pharmaceutiques comprenant une quantité thérapeutiquement efficace d'un composé de formule (I) en ccmbinaison avec un support physiologiquement acceptable.

Les inventeurs ont constaté que, d'une manière générale, parmi les composés de fomrules (I), ceux présentant une chiralité L,L (c'est-à-dire S,S selon la nomenclature Ingold-Kahn-Prelog) sont les plus actifs. Dans les exemples donnés ci-après, la configuration des autres carbones optiquement actifs est également indiquée pour les composés de formules (I) présentant plus de deux centres d'asymétrie.

Dans toutes les utilisations précitées, on préfère choisir les composés selon l'invention présentant une constante Ki inférieure ou égale à 1 µM tels que ceux exemplifiés et d'une manière tout-à-fait préférentielle, les composés de formule (I) présentant un squelette indoline.

Selon l'invention, toutes les utilisations précitées comprennent également celle des composés de formules (I) déjà connus, comme indiqués précédemment, c'est-à-dire les composés de formule (I) dans laquelle R₂, R et R' représentant un hydrogène, n = 0 et m = 1 ou n = 1 et m = 0, R₃ le radical bivalent -(CH₂)₂-, R₄ un groupe amide CONHR₅, R₁ représente un groupe CH₂CH₃, (CH₂)₂CH₃, (CH₂)₃CH₃, CH(CH₃)₂, CH₂CH(CH₃)₂ ou CH(CH₃)CH₂CH₃ avec R₅ représentant un hydrogène, R₁ représente CH₂CH(CH₃)₂ avec R₅ représentant un groupe CH₂CH₃ et R₁ représente CH₃ avec R₅ représentant le motif

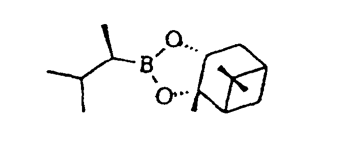

D'autres avantages et caractéristiques de la présente invention apparaîtront à la lecture des résultats expérimentaux qui suivent, en particulier d'une méthode d'isolement et de caractérisation de la tripeptidylpeptidase selon l'invention ainsi que des exemples de préparation donnés à titre illustratif et non limitatif.

### ISOLEMENT ET CARACTERISATION DE L'ENZYME INACTIVANT LA CCK

Les inventeurs ont utilisé des membranes de cerveau (cortex cérébral) de rat comme matériel d'origine et des peptides CCK-8 non sulfaté (Asp-Tyr-Met-Gly-Trp-Met-Asp-Phe-NH₂) et CCK-5 (Gly-Trp-Met-Asp-Phe-NH₂) comme substrats, les produits caractéristiques de la réaction (CCK-5 et Gly-Trp-Met) étant mesurés fluorimétriquement après isolement par chromatographie liquide haute performance (CLHP) selon Camus et al., (Neurosci., 1989, 29 : 595).

Les étapes successives de purification CLHP mises en jeu sont décrites dans le tableau I suivant qui indique aussi les facteurs de purification obtenus en utilisant CCK-8 comme substrat.

### TABLEAU I

**TABLEAU I**

| Etapes [éluant] | Temps de rétention ou molarité de l'éluant au pic d'activité | Facteur de purification |
|---|---|---|
| Membranes | | 1 |
| | | |
| Solubilisation de membranes (détergent : 1%) | | 0.4 |
| | | |
| Echange d'ions (DEAE 5PW) [NaCl 0-300 mM] | 300 mM | 15 |
| | | |
| Hydroxyapatite (Bio-Gel HPHT) [phosphate 0-300 mM] | 220 mM | 45 |
| | | |
| Filtration sur gel (Protein Pak 300SW) [phosphate 50 mM] | 45 min | 3.500 |
| | | |
| Colonne hydrophobe (Phenyl-5 PW) [(NH₄)₂ SO₄ 1M-0] | 0 | 8.571 |

Dès le deuxième stade de purification, le profil de chromatographie ne comporte qu'un seul pic d'activité enzymatique auquel est superposé le pic obtenu en mesurant l'hydrolyse de CCK-5, indiquant par là qu'une enzyme unique est responsable des deux coupures de la molécule CCK-8.

Après la dernière étape de purification, une électrophorèse sur gel de sodium dodécylsulfate (SDS) indique une bande unique marquée irréversiblement par DFP-³H à une masse apparente de 135 kDa.

L'étude de la spécificité de l'enzyme purifiée sur une série de substrats-modèles a montré que celle-ci se comporte comme une aminotripeptidylpeptidase capable, notamment, d'hydrolyser les fragments Ala-Ala-Phe-p.nitroanilide ou Ala-Ala-Phe-amidométhylcoumarine (A-AP-Amc) en libérant nitroaniline ou aminométhylcoumarine.

### EXEMPLES DE PREPARATION DE COMPOSES DE FORMULE (I)

Pour les données spectrales indiquées dans tous les exemples ci-dessous, les abréviations suivantes ont pour signification :
s = singulet, m = multiplet, d = doublet, dd = doublet de doublets, t = triplet, dt = doublet de triplets, q = quadruplet, tq = triplet de quadruplets et tt = triplet de triplets.

### Exemple 1 : Préparation de la L-valyl-L-proline n.hexylamide ; voie 1

R₁ = CH(CH₃)₂ ; R₂ = H ; n = 0 ou 1 et m = 0 ou 1 avec n différent de m ; R = R' = H ; R₃ = -(CH₂)₂- ; R₄ = CO-NH-R₅ ; R₅ = (CH₂)₅CH₃

### a/ Préparation de la N-benzyloxycarbonyl-L-valine

On dissout 10 g de L-valine (85 mmoles) dans KOH 4 M (40 ml) et on refroidit le mélange dans la glace. On y ajoute 21,9 g de chlorure de benzyloxycarbonyle (128 mmoles) en 5 minutes et il se forme un solide blanc. On agite le mélange à 0°C pendant une heure puis on laisse la température remonter et on agite encore une heure à température ambiante. On dilue alors le mélange réactionnel avec KOH 4 M (200 ml) et on l'extrait à l'éther (2 x 100 ml). On porte alors la phase aqueuse à pH acide < 1 avec de l'acide chlorhydrique concentré puis on l'extrait à l'acétate d'éthyle (3 x 200 ml). Les extraits sont combinés et sèchés (Na₂SO₄) et on évapore le solvant . On obtient une huile claire.
RMN-¹H (200 MHz, CDCl₃, ppm) : 7,42-7,52 (1H, d, NH) ; 7,24-7,36 (5H, m, Ph) ; 5,00 (2H, s, Ph-CH₂) ; 3,78-3,88 (1H, m, α-CH acide) ; 1,98-2,08 (1H, m, β-CH acide) ; 0,80-0,88 (6H, m, 2 x CH₃).

### b/ Préparation de l'ester succinimidique de la N-benzyloxycarbonyl-L-valine

On dissout dans du diméthoxyéthane (100 ml) 15,63 g (62 mmoles) du produit obtenu au a/ et 7,16 g de N-hydroxy-succinimide (62 mmoles) et on refroidit le mélange dans de la glace. On ajoute 12,8 g de dicyclohexylcarbodiimide (62 mmoles) en agitant et le mélange est réfrigéré pendant une nuit. On élimine par filtration le solide blanc qui se forme et on concentre le filtrat pour obtenir un solide blanc par cristallisation dans l'isopropanol. On obtient le produit désiré : PF = 105-6°C (litterature : 116-7°C)
RMN-¹H : 7,35 (5H, s, ArCH) ; 5,27-5,41 (1H, d, NH) ; 5,11 (2H, s, PhCH₂) ; 4,58-4,71 (1H, m, α-CH valine) ; 2,80 (4H, s, 2 x CH₂) ; 2,19-2,41 (1H, m, β-CH valine) ; 0,99-1,37 (6H, m, 2 x CH₃).

### c/ Préparation de la N-benzyloxycarbonyl-L-valyl-L-proline

On dissout dans l'eau (100 ml) 3,4 g de L-proline (29,9 mmoles) et on ajoute 4,5 g de triéthylamine (44,8 mmoles). On dissout dans du tétrahydrofuranne (100 ml) 10 g (29,9 mmoles) du produit obtenu au b/ que l'on refroidit avec de la glace. On ajoute la solution de proline en 40 minutes et on agite le mélange pendant une heure à 0°C puis à température ambiante pendant une nuit. On réduit environ de moitié le volume de solvant, on acidifie (pH < 1) avec HCl concentré et on extrait à l'acétate d'éthyle (3 x 200 ml). On réunit les phases organiques que l'on sèche (Na₂SO₄) et on évapore le solvant. On obtient une huile.

Une analyse CCM (acétate d'éthyle/acide acétique 1%) a montré la présence de 4 composants.

On redissout le produit obtenu dans l'acétate d'éthyle et on extrait avec KOH 4M (3 x 200 ml). On acidifie les phases aqueuses (pH < 1) avec HCl concentré et on extrait à l'acétate d'éthyle (3 x 200 ml). On sèche les phases organiques combinées (Na₂SO₄) et on évapore le solvant. On obtient une huile.
RMN-¹H (200 MHz, CDCl₃, ppm) : 7,25-7,30 (5H, m, Ph) ; 5.90-5,95 (1H, d, NH) ; 5,04-5,10 (2H, m, PhCH₂) ; 4,50-4,57 (1H, m, α-CH valine) ; 4,26-4,34 (1H, m, α-CH proline) ; 3,63-3,79 (2H, m, CH₂ proline) ; 1,95-2,14 (4H, m, 2 x CH₂proline) ; 0,87-1,00 (6H, m, 2 x CH₃).
Spectre de masse (EI) : 349 (M⁺ + 1).

### d/ Préparation de la N-benzyloxycarbonyl-L-valyl-L-proline hexylamide

On dissout dans du tétrahydrofuranne (50 ml, sec, distillé) 2 g (5,7 mmoles) du produit obtenu au c/ et on refroidit dans un bain glace/sel. On ajoute 660 mg de N-éthylmorpholine (5,7 mmoles) et 780 mg de chloroformiate d'isobutyle (5,7 mmoles) et on maintient le mélange refroidi pendant 30 minutes. On ajoute 580 mg de n.hexylamine (5,7 mmoles), on laisse la température du mélange remonter à la température ambiante et on laisse le mélange ainsi pendant une nuit. On dissout le mélange dans l'eau (50 ml) et on l'extrait à l'acétate d'éthyle (2 x 50 ml). On combine et on sèche (Na₂SO₄) les phases organiques et on évapore le solvant. On obtient 2,95 g d'une huile brune. Par cristallisation dans un mélange acétate d'éthyle/éther de pétrole, on obtient 370 mg d'une poudre blanche. On concentre les filtrats. Par chromatographie en utilisant de l'acétate d'éthyle comme éluant, on obtient le produit désiré.
RMN-¹H (200 MHz, CDCl₃, ppm) : 7,33 (5H, s, Ph) ; 5,45-5,50 (1H, d, NH) ; 5,07-5,08 (2H, d, PhCH₂) ; 4,52-4,57 (1H, m, α-CH valine) ; 4,28-4,36 (1H, m, α-CH proline) ; 3,56-3,72 (2H, m, CH₂ proline) ; 3,11-3,21(2H, m, α-CH₂ amide) ; 1, 69-2, 39 (5H, m, β-CH valine, 2 x CH₂ proline) ; 1,15-1,45 (8H, m, 4 x CH₂) ; 0,81-0,98 (9H, m, 3 x CH₃).

### e/ Préparation du composé en titre sous forme d'oxalate

On dissout dans du méthanol 100 ml) 900 mg (208 mmoles) du produit obtenu au d/ et on ajoute 300 mg de palladium sur charbon. On hydrogène le mélange pendant 3 heures puis on élimine par filtration le catalyseur. Après élimination du solvant, on obtient 602 mg (94,5 %) d'une huile pâle.

Une analyse par CCM a montré qu'il ne restait pas de produit de départ.

On ajoute 182 mg d'acide oxalique (202 mmoles) dans 5 ml d'éthanol et on dissout l'huile. Après addition d'éther (50 ml), on obtient une solution trouble qui est réfrigérée. On obtient le produit désiré sous forme de sel d'oxalate.
P.F. = 146-7°C
Analyse élémentaire : C₁₆H₃₁N₃O₂.C₂H₂O₄
Trouvé : C = 55,99 % ; H = 8,75 % ; N = 10,63 % ;
Théorique : C = 55,80% ; H = 8,58 % ; N = 10,84 % ;
RMN-¹H (200 MHz, DMSO, ppm) : 7,85-7,91 (1H, t, NH) ; 4,25-4,32 (1H, q, α-H proline) ; 3,93-3,96 (1H, d, αH valine) ; 3,43-3,72 (2H, m, CH₂ proline) ; 2,95-3,07 (2H, m, αCH₂amide) ; 1,66-2,15 (5H, m, β-CH valine, 2 x CH₂ proline) ; 1,25-1,39 (8H, m, 4 x CH₂) ; 0,8-1,2 (6H, m, 3 x CH₃).
IR (cm⁻¹) : 3400, 3333 (N-H), 2700-3200 (OH), 1683, 1639 (C=O), 1195 (CONH).
Spectre de masse (FAB) : 298 (M⁺ + 1).

### Exemple 2 : Préparation de la 1-(2(S)-aminobutyryl)-L-proline 3-(méthylthio)propylamide ; voie 1

R₁ = CH₂CH₃ ; R₂ = H ; n = 0 ou 1 et m = 0 ou 1 avec n différent de m ; R = R' = H ; R₃ = -(CH₂)₂- ; R₄ = CO-NH-R₅ ; R₅ = (CH₂)₃SCH₃ ;

### a/ Préparation de l'acide N-(tert.butoxycarbonyl)-2(S)-aminobutyrique

On dissout dans un mélange de dioxanne (155 ml), d'eau (78 ml) et de NaOH 1N (78 ml), 8 g d'acide 2(S)-aminobutyrique (77,6 mmoles). On refroidit la solution dans un bain glace/sel et on ajoute 18,62 g de dicarbonate de di-tert.butyle (85,4 mmoles). On laisse la solution se réchauffer jusqu'à la température ambiante et on agite pendant une heure. On concentre alors le mélange sous vide jusqu'à un volume d'environ 90 ml puis on l'acidifie avec une solution de KHSO₄. On extrait la solution résultante au chlorure de méthylène (2 x 200 ml), on sèche les extraits réunis et on les fait évaporer, ce qui conduit à une huile. Par sèchage prolongé sous vide, on obtient un solide cireux blanc. P.F. = 65-66°C

### b/ Préparation de l'ester succinimidique de l'acide N-(tert.butoxycarbonyl)-2(S)-aminobutyrique

On dissout dans du tétrahydrofuranne sec, sous azote à 0°C, 14,2 g d'acide (70 mmoles) obtenu au a/. On ajoute 14,4 g de dicyclohexylcarbodiimide (70 mmoles) et 8,1 g de N-hydroxy-succinimide (70 mmoles), et on agite le mélange pendant une nuit. On élimine par filtration le précipité formé, on le rince une fois avec de l'acétate d'éthyle et on élimine le solvant sous vide, ce qui conduit à une huile. Le produit est utilisé dans les étapes subséquentes sans purification supplémentaire.
RMN-¹H (CDCl₃, ppm) : 4,6 (1H, m, αH) ; 2,6 (4H, s, CH₂CH₂succinimide) ; 1,5-1,7 (2H, m, CH₂aminobutyryle) ; 1,2 (9H, s, (CH₃)₃) ; 0,9 (3H, t, CH₃aminobutyryle).

### c/ Préparation de la 1-(N-(tert.butoxycarbonyl)-2(S)-aminobutyryl)-L-proline

On dissout dans du tétrahydrofuranne (150 ml), 6,933 g d'ester (23,1 mmoles) obtenu au b/ et on refroidit le mélange à 0°C. On ajoute une solution de 3,99 g de L-proline (34,7 mmoles) et 3,51 g de triéthylamine (34,7 mmoles) dans l'eau (50 ml). On maintient l'agitation pendant 48 heures à température ambiante. On élimine sous vide le tétrahydrofuranne et on le remplace par de l'eau puis on porte le pH à 2 environ avec du KHSO₄ dilué. On extrait la solution acide au chlorure de méthylène, on sèche les extraits et on les fait évaporer, ce qui conduit à une huile.
RMN-¹H (CDCl₃, ppm) : 4,3 (1H, m, αH) ; 4,2 (1H, m, αH)⁻ ; 3,6-3,8 (2H, m, CH₂Nproline) ; 1,9-2,1 (4H, m, CH₂CH₂CH₂Nproline) ; 1,6-1,8 (2H, m, CH₂aminobutyryle) ; 1,5 (9H, s, (CH₃)₃) ; 0,9 (3H, t, CH₃aminobutyryle).

### d/ Préparation de la 1-(N-(tert.butoxycarbonyl)-2(S)-aminobutyryl)-L-proline 3-(méthylthio)propylamide

On dissout dans du tétrahydrofuranne (100 ml), sous azote, 2,47 g (8,23 mmoles) du produit obtenu au c/, en refroidissant dans un bain glace/sel. On ajoute 0,948 g de N-éthylmorpholine (8,23 mmoles) puis 1,125 g de chloroformiate d'isobutyle (8,23 mmoles). Après 30 minutes, on ajoute 0,864 g de 3-(méthyl-thio)propylamine (8,23 mmoles). On maintient l'agitation pendant une nuit. On verse la suspension dans du chlorure de méthylène (250 ml), on lave avec de l'acide citrique 10 % (3 x 100 ml) puis avec NaHCO₃ 5 % (3 x 100 ml), on sèche et on fait évaporer, ce qui conduit à une huile.
RMN-¹H (CDCl₃, ppm) : 4,4 (1H, m, αH) ; 4,3 (1H, m, αH) ; 3,5-3,8 (2H, m, CH₂Nproline) ; 3,2 (2H, m, CH₂N) ; 2,2 (2H, m, CH₂CH₂Nproline) ; 2,0 (3H, s, CH₃S) ; 1,6-1,8 (8H, m, CH₂CH₂CH₂Nproline, CH₂CH₂SCH₃, CH₂aminobutyryle) ; 1,5 (9H, s, (CH₃)₃) ; 0,9 (3H, t, CH₃aminobutyryle).

### e/ Préparation du composé en titre sous forme de sel d'oxalate

On dissout dans de l'acide trifluoroacétique refroidi (100 ml), 5 g (12,9 mmoles) du produit obtenu au d/, en refroidissant dans un bain glace/sel et on agite le mélange pendant 30 minutes. On élimine sous vide l'acide, ce qui conduit à une huile claire. On traite cette huile par NaHCO₃ 5 % et on l'extrait au chlorure de méthylène. On sèche la phase organique et on la fait évaporer, ce qui conduit à la base libre (huile claire). On dissout l'huile obtenue dans une petite quantité d'éthanol et on ajoute de l'acide oxalique (1,1 équivalent). Après addition d'éther, on obtient des cristaux blancs du produit désiré sous forme de sel d'oxalate. Ce produit est suffisamment pur sans qu'une recristallisation soit nécessaire.
P.F. = 120-121°C
RMN-¹H (CD₃OD, ppm) : 4,5 (1H, m, αH) ; 4,3 (1H, m, αH) ; 3,6 (2H, m, CH₂Nproline) ; 2,5 (2H, m, CH₂Namide) ; 2,1 (3H, s, SCH₃) ; 1,75-2,0 (10H, m, SCH₂CH₂CH₂CH₂CH₂Nproline, CH₂ aminobutyryle) ; 1,0 (3H, t, CH₃aminobutyryle).
Analyse élémentaire : C₁₃H₂₅N₃O₂S.C₂H₂O₄.0,5H₂O
Trouvé : C = 47,17 % ; H = 7,05 % ; N = 10,67 % ;
Calculé : C = 46,62 % ; H = 7,30 % ; N = 10,87 % ;

### Exemple 3 : Préparation de la 1-(2(S)-aminobutyryl)-L-proline n.pentylamide ; voie 1

R₁ = CH₂CH₃ ; R₂ = H ; n = 0 ou 1 et m = 0 ou 1 avec n différent de m ; R = R' = H ; R₃ = -(CH₂)₂- ; R₄ = CO-NH-R₅ ; R₅ = (CH₂)₄CH₃

### a/ Préparation de l'acide N-benzyloxycarbonyl-2(S)-amino-butyrique

On dissout dans une solution aqueuse de KOH 4M (100 ml), 10 g d'acide 2(S)-aminobutyrique (97 mmoles) et on refroidit à 0°C. On ajoute 24,7 g de chloroformiate de benzyle (145 mmoles) et on agite le mélange en laissant la température du mélange remonter jusqu'à la température ambiante pendant 2 heures 30 minutes. On dilue alors le mélange avec de l'eau (100 ml) et on l'extrait à l'ether (2 x 200 ml). On acidifie la phase aqueuse jusqu'à un pH < 1 avec HCl concentré et on extrait à l'acétate d'éthyle (3 x 200 ml). On combine les fractions d'acétate d'éthyle, on les sèche (Na₂SO₄) et on élimine le solvant. On obtient un solide blanc.
RMN-¹H (ppm) : 7,32 (5H, s, ArCH) ; 5,22-5,28 (1H, d, NH) ; 5,10 (2H, s, Ph-CH₂) ; 4,02-4,44 (1H, m, α-CH) ; 1,58-2,02 (2H, m, CH₂) ; 0,9-1,2 (3H, t, CH₃).

### b/ Préparation de l'ester succinimidique de l'acide N-benzyloxycarbonyl-2(S)aminobutyrique

On dissout dans du diméthoxyéthane (160 ml), 20 g d'acide (84 mmoles) obtenu au a/ et 9,7 g de N-hydroxysuccinimide (20 mmoles) et on refroidit la solution dans de la glace. On ajoute par portions 17,3 g de dicyclohexyl-carbodiimide (84 mmoles) en refroidissant et il se forme rapidemment un précipité blanc. On refrigère le mélange pendant une nuit, on le filtre et on élimine le solvant. On obtient une huile visqueuse. Par cristallisation dans de l'isopropanol, on obtient un solide blanc.
RMN-¹H (ppm) : 7,31 (5H, s, ArCH) ; 5,3-5,6 (1H, d, NH) ; 5,1 (2H, s, Ph-CH₂) ; 4,8-4,9 (1H, m, α-CH) ; 2,9 (4H, s, 2 x CH₂ester) ; 1,80-2,05 (2H, m, CH₂aminobutyryle) ; 0,95-1,25 (3H, m, CH₃aminobutyryle).

### c/ Préparation de la 1-(N-benzyloxycarbonyl-2(S)-amino-butyryl)-L-proline

On dissout dans du tétrahydrofuranne (50 ml), 5 g d'ester (14,95 mmoles) obtenu au b/ et on refroidit dans de la glace. On y ajoute alors 1,75 g de L-proline (14,95 mmoles), 2,25 g de triéthylamine (22,4 mmoles) et de l'eau (50 ml). On agite le mélange et on le refroidit pendant un heure puis on le porte à la température ambiante pendant une nuit. On reduit la teneur en solvant à environ la moitié de son volume, on acidifie et on extrait à l'acétate d'éthyle (3 x 100 ml). On sèche les fractions d'acétate d'éthyle combinées (Na₂SO₄) et on élimine le solvant. On obtient une huile épaisse.
RMN-¹H (ppm) : 7,38 (5H, s, ArCH) ; 5,9-6,0 (1H, d, NH) ; 5,08 (2H, s, Ph-CH₂) ; 4,42-4,54 (2H, m, 2 x α-CH) ; 3,52-3,85 (2H, m, CH₂proline) ; 1,52-2,30 (6H, m, 2 x CH₂proline, CH₂aminobutyryle) ; 0,85-1,0 (3H, m, CH₃).

### d/ Préparation de la 1-(N-benzyloxycarbonyl-2(S)-amino-butyryl)-L-proline n.pentylamide

On dissout dans du tétrahydrofuranne, sec, distillé (50 ml), 2 g (5,98 mmoles) du produit obtenu au c/, et on refroidit dans un bain glace/sel. On y ajoute 0,813 g de chloroformiate d'iso.butyle (5,98 mmoles) et 0,687 g de N-éthylmorpholine (5,98 mmoles) en agitant et la solution devient trouble. On poursuit l'agitation et le refroidissement pendant 30 minutes puis on ajoute 0,52 g de n.pentylamine (5,98 mmoles). On agite le mélange à la température ambiante pendant une nuit puis on le dilue avec de l'eau (50 ml) et on l'extrait à l'acétate d'éthyle (2 x 50 ml). On sèche les extraits organiques réunis (Na₂SO₄) et on élimine le solvant. On obtient 2,54 g d'une huile. Des analyses CLHP et CCM ont montré qu'il restait de l'acide de départ. On redissout donc l'huile obtenue dans de l'acétate d'éthyle (100 ml), on la lave avec KOH 4M (50 ml) et de l'eau puis on la sèche (Na₂SO₄) et on élimine le solvant. Or obtient le produit désiré.
RMN-¹H (ppm) : 7,33 (5H, s, ArCH) ; 5,07-5,08 (2H, m, CH₂Ph) ; 3,96-4,07 (2H, m, α-CH) ; 3,48-3,9 (2H, m, CH₂ proline) ; 1,1-2,5 (12H, m, 6 x CH₂) ; 0,75-1,0 (6H, m, 2 x CH₃).

### e/ Préparation du composé en titre sous forme d'oxalate

On dissout dans du méthanol (100 ml), 1 g (2,48 mmoles) du produit obtenu au d/ et on ajoute 200 mg de Pd/C (10 %). On hydrogène ce mélange sous pression pendant 3 heures dans un appareil de Parr. On élimine le catalyseur par filtration puis on élimine le solvant et on ajoute 223 mq d'acide oxalique (2,48 mmoles). Par cristallisation dans un mélange éthanol/éther, on obtient le produit désiré.
P.F. = 140-141°C
Analyse élémentaire :
C₁₄H₂₇N₃O₂.0,9C₂H₂O₄.0,2C₂H₅OH.0,5H₂O
Trouvé : C = 52,63 % ; H = 8,44 % ; N = 11,24 % ;
Calculé : C = 52,78 % ; H = 8,48 % N = 11,40 % ;
Spectre de masse (FAB) : 270 (M⁺ + 1)
RMN-¹H (DMSO-d₆, ppm) : 7,64-7,69 (1H, m, NH) ; 4,24-4,30 (1H, m, α-CH) ; 4,03-4,09 (1H, m, α-CH) ; 3,30-3,74 (2H, m, CH₂proline) ; 2,90-3,10 (-NH-CH₂-) ; 1,52-2,10 (6H, m, 2 x CH₂proline, CH₂ chaîne butyle) ; 0,72-1,44 (12H, m, 2 x CH₃, 3 x CH₂ chaîne pentyle).

### Exemple 4 : Préparation de la 1-(2(S)-aminobutyryl)-L-proline n.butylamide ; voie 1

R₁ = CH₂CH₃ ; R₂ = H ; n = 0 ou 1 et m = 0 ou 1 avec n différent de m ; R = R' = H ; R₃ = -(CH₂)₂- ; R₄ = CO-NH-R₅ ; R₅ = (CH₂)₃CH₃

### a/ Préparation de la 1-(N-(tert.butoxycarbonyl)-2(S)-aminobutyryl)-L-proline n.butylamide

On dissout dans du tétrahydrofuranne sec (15 ml) 2,42 g de 1-(N-(tert.butoxycarbonyl)-2(S)-aminobutyryl)-L-proline (8,05 mmoles) préparé comme à l'exemple 2 c/. On refroidit la solution dans un bain glace/sel puis on ajoute 0,927 g de N-éthylmorpholine (8,05 mmoles) et 1,10 g de chloroformiate d'isobutyle (8,05 mmoles). La solution est agitée pendant 45 minutes et on ajoute 0,59 g (8,05 mmoles) de n.butylamine. Après avoir agité pendant une nuit, on ajoute du chlorure de méthylène (200 ml) et on lave le mélange avec de l'acide citrique 10 % (2 x 100 ml) puis avec NaHCO₃ 5 % (2 x 100 ml). On sèche la phase organique (Na₂SO₄) et on évapore sous vide. On obtient un sirop. On réalise la déprotection sans caractérisation.

### b/ Préparation du composé en titre sous forme d'oxalate

On traite par de l'acide trifluoroacétique (TFA) froid (28 ml) le produit obtenu au a/ en agitant dans un bain glace/sel pendant 30 minutes. On élimine le TFA sous vide. On traite l'huile résultante par NaHCO₃ 5 % froid jusqu'à ce que le pH soit d'environ 10 puis on extrait au chlorure de méthylène. On sèche la phase organique (Na₂SO₄) et on évapore. On reprend l'huile dans un faible volume d'éthanol et on traite par l'acide oxalique. Après addition d'ether, on obtient des cristaux blancs du produit désiré sous forme de sel d'oxalate. Ce composé est suffisammment pur sans recristallisation.
P.F. = 165-167°C
RMN-¹H (CD₃OD, ppm) : 4,4 (1H, m, α-H) ; 4,2 (1H, m, αH) ; 3,4-3,7 (2H, m, CH₂N proline) ; 3,0-3,2 (2H, m, CH₂NC₄H₉) ; 1,6-2,2 (4H, m, NCH₂CH₂CH₂ proline) ; 1,2-1,5 (4H, m, CH₂ aminobutyryle, NCH₂CH₂C₄H₉) ; 1,1 (3H, t, CH₃ aminobutyryle) ; 0,9 (3H, t, CH₃ de n.butyle).
Analyse élémentaire : C₁₃H₂₅N₃O₂.C₂H₂O₄.1,3H₂O
Trouvé : C = 49,15 % ; H = 7,62 % ; N = 11,06 % ;
Théorique : C = 48,85 % ; H = 8,09 % ; N = 11,39 % ;

### Exemple 5 : Préparation de la 1-(2(S)-aminobutyryl)-L-proline [2(S)-méthyl]butylamide ; voie 1

R₁ = CH₂CH₃ ; R₂ = H ; n = 0 ou 1 et m = 0 ou 1 avec n différent de m ; R = R' = H ; R₃ = -(CH₂)₂- ; R₄ = CO-NH-R₅ ; R₅ = (S)CH₂CH(CH₃)CH₂CH₃ ;

### a/ Préparation de la 1-(N-benzyloxycarbonyl-2(S)-amino-butyryl)-L-proline

On traite par une solution de 4,138 g d'ester succinimidique de l'acide N-benzyloxycarbonyl-2(S)-aminobutyrique (12,4 mmoles) préparé comme à l'exemple 3 b/, dans du diméthoxyéthane (25 ml), une solution de 2,1395 g de L-proline (18,6 mmoles) et de 1,56 g de NaHCO₃ (18,6 mmoles) dans l'eau (20 ml). Après 5 heures, on ajoute 12,4 ml d'eau, on acidifie le mélange résultant jusqu'à pH = 2 avec HCl concentré et on l'extrait à l'acétate d'éthyle. On sèche les extraits et on les fait évaporer ce qui conduit à un sirop.
RMN-¹H (CDCl₃, ppm) : 7,2 (5H, m, H-Aryle) ; 5,2 (1H, m, αH) ; 5,0 (3H, m, CH₂benzyloxycarbonyle, αH) ; 4,0-4,1 (2H, m, CH₂N) ; 1,8-2,0 (4H, m, CH₂CH₂CH₂N) ; 1,1-1,2 (2H, m, CH₂aminobutyryle) ; 0,9 (3H, t) ; 3,8 (2H, m, CH₂Aryle) ; 3,5 (2H, m, CH₂cycle) ; 3,2 (2H, m) ; 2,0 (2H, m) ; 0,9 (3H, t, CH₃aminobutyryle).

### b/ Préparation de la 1-(N-benzyloxycarbonyl-2(S)-amino-butyryl)-L-proline [2(S)-méthyl]butylamide

On dissout dans du tétrahydrofuranne (100 ml), 3,8 g (11,4 mmoles) du produit obtenu au a/, et on refroidit le mélange sous azote dans un bain glace/sel. On ajoute 1,31 g de N-éthyl-morpholine (11,4 mmoles) puis 1,487 g de chloroformiate d'isobutyle (11,4 mmoles). Après 30 minutes, on ajoute 0,99 g de 2(S)-méthylbutylamide (11,4 mmoles) et on poursuit l'agitation pendant une nuit. On verse la suspension dans du chlorure de méthylène (250 ml), on lave avec de l'acide citrique 10 % (3 x 100 ml) puis avec NaHCO₃ 5 % (3 x 100 ml), on sèche et on fait évaporer. On obtient une huile.
RMN-¹H (CDCl₃, ppm) : 7,2 (5H, m, H-Aryle) ; 5,0 (2H, d, CH₂benzyloxycarbonyle) ; 4,3-4,5 (2H, m, 2αH) ; 3,2-3,6 (2H, m, CH₂N) ; 2,9-3,1 (2H, m, CH₂Nproline) ; 1,0-2,1 (18H, m, CH₂CH₂CH₂N, CH₃CH₂aminobutyryle, NCH₂CH(CH₃)CH₂CH₃).

### c/ Préparation du composé en titre sous forme d'oxalate

On dissout dans du méthanol 1,96 g du produit obtenu au b/ et on ajoute 0,24 g de palladium sur charbon (10 %, humide). On hydrogène cette suspension dans un appareil de Parr sous 40 Psi pendant 4 heures. On élmimine le catalyseur par filtration et on élimine le solvant sous vide. On dissout l'huile résultante dans un peu d'éthanol et on ajoute de l'acide oxalique (1,1 équivalent). Après addition d'éther, on obtient le produit désiré sous forme de sel d'oxalate.
P.F. = 135-136°C
RMN-¹H (CD₃OD, ppm) : 4,4 (1H, m, αH) ; 4,25 (1H, m, αH) ; 3,65 (2H, m, CH₂Nproline) ; 3,0 (2H, m, CH₂Namide) ; 2,0 (5H, m, CH₂CH₂Nproline, CH₃CH₂CH(CH₃)CH₂N) ; 1,5 (2H, m, CH₂aminobutyryle) ; 1,1 (3H, t, CH₃aminobutyryle) ; 0,9 (6H, m, CH₃CH₂CH(CH₃)CH₂N).
Analyse élémentaire : C₁₄H₂₇N₃O₂.C₂H₂O₄.5H₂O
Trouvé : C = 52,04 % ; H = 7,82 % ; N = 11,27 %
Calculé : C = 52,16 % ; H = 8,21 % ; N = 11,41 %

### Exemple 6 : Préparation de la 1-(2(S)-aminobutyryl)-L-proline n.propylamide ; voie 1

R₁ = CH₂CH₃ ; R₂ = H ; n = 0 ou 1 et m = 0 ou 1 avec n différent de m ; R = R' = H ; R₃ = -(CH₂)₂- ; R₄ = CO-NH-R₅ ; R₅ = (CH₂)₂CH₃

### a/ Préparation de la 1-(N-(tert.butoxycarbonyl)-2(S)-aminobutyryl)-L-proline n.propylamide

On dissout dans du tétrahydrofuranne (40 ml), 2 g de 1-(N-(tert.butoxycarbonyl)-2(S)aminobutyryl)-L-proline (6,6 mmoles) préparée comme à l'exemple 2 c/. On ajoute alors à cette solution, à -10°C et sous azote, 0,86 ml de chloroformiate d'iso.butyle (6,6 mmoles) et 0,85 ml de N-éthylmorpholine (6,6 mmoles). Après agitation à -10°C pendant 20 minutes, on ajoute 2,75 ml de n.propylamine (33,0 mmoles). On laisse agiter le mélange à 0°C pendant une heure puis à température ambiante pendant une nuit. On fait évaporer le solvant sous vide et on obtient une matière semi-solide blanche. On la dissout dans de l'acétate d'éthyle (150 ml), on la lave avec de l'acide citrique 5 % (30 ml), une solution saturée de bicarbonate de sodium (30 ml) et de la brine (20 ml). On filtre après sèchage (Na₂SO₄) et on fait évaporer le solvant. On obtient une huile que l'on purifie par chromatographie liquide sur colonne (éluant : acétate d'éthyle) et on obtient un solide blanc.
RMN-¹H (CDCl₃, ppm) : 6,86 (1H, s large, NH) ; 5,22 (1H, d, NH) ; 4,56 (1H, dd, α-CHaminobutyryle) ; 4,37 (1H, m, α-CH proline) ; 3,54 (2H, m, N-CH₂proline) ; 3,13 (2H, m, N-CH₂ propyl) ; 2,39-1,31 (8H, m, 4 x CH₂) ; 1,24 (9H, s, (CH₃)₃-) ; 0,91 (3H, t, CH₃propyle) ; 0,86 (3H, t, CH₃ aminobutyryle).
Spectre de masse (FAB) : 342 (MH⁺)

### b/ Préparation du composé en titre sous forme de trifluoroacétate

On dissout dans du dichlorométhane (5 ml), 2,0 g (5,9 mmoles) du produit obtenu au a/ et on ajoute de l'acide trifluoroacétique (5 ml) à cette solution refroidie. On agite le mélange réactionnel pendant 2 heures dans un bain de glace. On fait évaporer l'acide et on obtient par précipitation avec de l'éther éthylique et séchage pendant une nuit à 30°C sous 0,1 mmHg, le produit désiré.
P.F. = 46-51°C (capillaire ouvert)
RMN-¹H (D₂O/TSP, ppm) : 4,41 (1H, t, α-CHaminobutyryle) ; 4,33 (1H, t, α-CHproline) ; 3,71 (2H, m, N-CH₂proline) ; 3,17 (2H, dt, N-CH₂amide) ; 2,21 (2H,, dt, CH₂proline) ; 1,86-2,03 (4H, m, 2 x CH₂) ; 1,54 (2H, tq, CH₂amide) ; 1,04 (3H, t, CH₃ aminobutyryle) ; 0,89 (3H, t, CH₃ amide).
Spectre de masse (FAB) : 242 (MH⁺)
Analyse élémentaire : C₁₂H₂₃N₃O₂.1,3CF₃COOH
Trouvé : C = 44,75 % ; H = 6,13 % ; N = 10,59 % ;
Calculé : C = 45,01 % ; H = 6,29 % ; N = 10,79 % ;

### Exemple 7 : Préparation de la 1-(2(S)-aminobutyryl)-L-proline iso.butylamide ; voie 1

R₁ = CH₂CH₃ ; R₂ = H ; n = 0 ou 1 et m = 0 ou 1 avec n différent de m ; R₃ = -(CH₂)₂- ; R₄ = CO-NH-R₅ ; R₅ = CH₂CH(CH₃)₂

### a/ Préparation de la 1-(N-(tert.butoxycarbonyl)-2(S)-aminobutyryl)-L-proline iso.butylamide

On procède comme à l'exemple 6 a/ en utilisant de l'iso.butylamine au lieu de la n.propylamine. On obtient une huile.
RMN-¹H (CDCl₃, ppm) : 6,93 (1H, s large, NH) ; 5,24 (1H, s large, NH) ; 4,56 (1H, dd, α-CHaminobutyryle) ; 4,36 (1H, tt, α-CHproline) ; 3,53 (2H, m, N-CH₂proline) ; 3,00 (2H, m, NCH₂); 2,39-1,58 (7H, m, CH et CH₂) ; 1,37 (9H, s, (CH₃)₃-) ; 0,94 (3H, t, CH₃) ; 0,84 (6H, d, 2 x CH₃).
Spectre de masse (FAB) : 356 (MH⁺)

### b/ Préparation du composé en titre sous forme de trifluoroacétate

On procède comme à l'exemple 6 b/ à partir du produit préparé au a/ et on obtient le produit désiré par précipitation avec de l'éther éthylique et sèchage à température ambiante sous vide (0,1 mmHg) pendant une nuit.
P.F. = 58-61°C (capillaire ouvert)
RMN-¹H (D₂O/TSP, ppm) : 4,43 (1H, t, α-CHaminobutyryle) ; 4,33 (1H, t, α-CHproline) ; 3,70 (2H, m, N-CH₂proline) ; 3,04 (2H, dd, N-CH₂amide) ; 2,23 (2H, m, CH₂proline) ; 1,96(4H, m, CH₂) ; 1,78 (1H, m, CH) ; 1,04 (3H, t, CH₃aminobutyryle) ; 0,89 (6H, d, 2 x CH₃).
Spectre de masse (FAB) : 256 (MH⁺)
IR (pastille KBr, cm⁻¹) : 3298 et 3089 (N-H), 2962 (C-H), 1662 (C=O).
Analyse élémentaire : C₁₃H₂₅N₃O₂.1,2CF₃COOH
Trouvé : C = 47,15 % ; H = 6,75 % ; N = 10,72 % ;
Calculé : C = 47,16 % ; H = 6,73 % ; N = 10,71 ;

### Exemple 8 : Préparation de la L-valyl-L-proline n.butylamide ; voie 2

R₁ = CH(CH₃)₂ ; R₂ = H ; n = 0 ou 1 et m = 0 ou 1 avec n différent de m ; R = R'= H ; R₃ = -(CH₂)₂- ; R₄ = CO-NH-R₅ ; R₅ = (CH₂)₃CH₃

### a/ Préparation de la N-(tert.butoxycarbonyl)-L-proline n.butylamide

On dissout dans du tétrahydrofuranne (50 ml), 5,0 g d'ester succinimidique de la N-(tert.butorycarbonyl)-L-proline (0,016 mmole) commercialisé par NOVABIOCHEM. on y ajoute alors à 0°C, en 10 minutes, 1,533 ml de n.butylamine (0,015 mmole) et on agite le mélange pendant une heure. On élimine le solvant et on purifie la matière brute résultante par chromatographie sur silice en utilisant comme éluant de l'acétate d'éthyle. On obtient une huile.
RMN-¹H (200MHz, CDCl₃, ppm) : 6,3 (1H, s large, NH) ; 4,25 (1H, m, méthine) ; 3,6-3,1 (4H, m, méthylène) ; 2,9 (2H, m, méthylène ; 1,95 (9H, s, méthyle) ; 1,6-1,2 (6H, m, méthylène).

### b/ Préparation de la L-proline n.butylamide, trifluoroacétate

On agite à température ambiante pendant une heure, 4,32 g du produit obtenu au a/ (0,014 mmole) et 15 ml d'acide trifluoroacétique. On fait évaporer sous vide l'acide en excès et on fait sécher sous vide pendant 3 heures. On obtient le produit désiré.
RMN-¹H (400 MHz, CDCl₃, ppm) : 9,3 (1H, s large, NH) ; 7,65 (1H, s large, NH) ; 4,7 (1H, m, méthine) ; 3,5 (2H, m, méthylène) ; 3,25 (2H, m, méthylène) ; 2,1 (4H, m, méthylène) ; 1,5 (2H, m, méthylène) ; 1,3 (2H, m, méthylène) ; 0,9 (3H, t, méthyle).

### c/ Préparation de la N-(tert.butoxycarbonyl)-L-valyl-L-proline n.butylamide

On dissout dans du tétrahydrofuranne (30ml), 3,707 g d'ester succinimidique de la N-(tert.butoxycarbonyl)-L-valine (0,0117 mole) commercialisé par NOVABIOCHEM. On y ajoute une solution de 3,35 g du produit obtenu au b/ (0,0117 mole) dans 20 ml de tétrahydrofuranne en agitant à 25°C. On ajoute ensuite 3,0 ml de triéthylamine pour neutraliser le sel de trifluoroacétate et on agite le mélange pendant 20 heures. On fait évaporer le solvant et on purifie la matière brute résultante par chromatographie sur silice en utilisant comme éluant un mélange d'éther de pétrole et d'acétate d'éthyle 1/1. On obtient le produit désiré sous forme d'une huile incolore.
RMN-¹H (200 MHz, CDCl₃, ppm) : 6,9 (1H, s large, NH) ; 5,2 (1H, d, NH) ; 4,5 (1H, dd, méthine) ; 4,25 (1H, dd, méthine) ; 3,6 (2H, m, méthylène) ; 3,2 (2H, q, méthylène) ; 1,1 (5H, m, méthine et méthylène) ; 1,4 (9H, s, CH₃tert.butyle) ; 1,3 (4H, m, méthylène) ; 0,9 (6H, dd, méthyle) ; 0,8 (3H, t, méthyle).

### d/ Préparation de la L-valyl-L-proline n.butylamide, trifluoroacétate

On agite à 25°C pendant 45 minutes, 1,5 g du produit obtenu au c/ (4,8 mmoles) et 6 ml d'acide trifluoroacétique. On fait évaporer l'excès d'acide et on purifie la matière brute résultante par chromatographie sur silice en utilisant comme éluant un mélange éther/acétate d'éthyle/acide acétique (1/1/0,1) puis acétate d'éthyle/méthanol/acide acétique (1/1/0,1). On réalise une purification supplémentaire par CLHP préparative dans les conditions suivantes : colonne Lichrosorb r.p. select B ; éluant : eau/méthanol 60/40 + acide trifluoroacétique 0,1 %. On obtient le produit sous la forme d'une huile.

### e/ Préparation du composé en titre sous forme d'oxalate

On dissout dans Na₂CO₃ en solution aqueuse à 20 % (5 ml), 1,7 g du produit obtenu au d/ (4,4 mmoles), on agite pendant 10 minutes et on extrait à l'acétate d'éthyle (3 x 15 ml). On sèche les extraits organiques (Na₂SO₄) et on concentre de façon à obtenir une huile (0,734 g). On dissout 1 g d'acide oxalique dans 3 ml d'éthanol et on ajoute cette solution à l'huile précédemment obtenue, on chauffe dans un bain d'eau pendant 10 minutes puis on refroidit. Par addition d'éther éthylique, on obtient un précipité blanc que l'on filtre et qu'on lave avec de l'éther éthylique. On le recristallise dans un mélange méthanol/acétate d'éthyle/éther (1/1/3), ce qui conduit à des cristaux blancs.
P.F. = 165-166°C
CCM (acétate d'éthyle/méthanol/acide acétique 6/2/2) : R_{f} = 0,25
IR (KBr, cm⁻¹) : 3432 (NH), 3072 (⁺NH₃), 2958 (C-H), 1718 (C=O), 1641 (C=O), 1554 (N-H).
RMN-¹H (200 MHz, DMSO-d₆, ppm) : 7,9 (1H, t, NH) ; 7,3 (2H, s large, NH₂) ; 4,25 (1H, t, méthine) ; 3,95 (1H, d, méthine) ; 3,65 (1H, m, CH₂ proline) ; 3,45 (1H, m, CH₂ proline) ; 3,0 (2H, m, méthylène) ; 2,2-1,5 (5H, m, méthine et méthylène) ; 1,3 (4H, m, méthylène) ; 0,9 (6H, dd, méthyle) ; 0,8 (3H, t, méthyle).
Spectre de masse : 269 (M⁺) (2 %).
Analyse élémentaire : C₁₆H₂₉N₃O₆.0,25(COOH)₂
Trouvé : C = 52,12 % ; H = 8,01 % ; N = 11,07 % ;
Calculé : C = 52,19 % ; H = 7,85 % ; N = 11,13 % ;

### Exemple 9 : Préparation de la L-alanyl-L-prolyl-L-borovaline pinanediol ester ; voie 5

R₁ = CH₃ ; R₂ = H ; n = 0 ou 1 et m = 0 ou 1 avec n différent de m ; R = R' = H ; R₃ = -(CH₂)₂- ; R₄ = CO-NH-R₅ ;
R₅ =

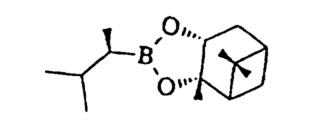

### a/ Préparation de l'acide isopropylboronique

On ajoute simultanément, sous atmosphère d'azote et agitation vigoureusement, une solution de 149 g de triéthylborate (1 mole) dans 300 cm³ d'éther et une solution 2M de 103 g de chlorure d'isopropylmagnésium (1 mole) dans 500 cm³ d'éther, dans de l'éther éthylique (500 cm³) agitée sous azote à -78°C, en maintenant la température en dessous de -70°C pendant 2 heures. On laisse la température du mélange réactionnel remonter à la température ambiante et on poursuit l'agitation encore pendant 24 heures. On acidifie alors le mélange avec de l'acide sulfurique dilué à 40 % froid (250 cm³) et on agite en maintenant la température en dessous de 15°C. On agite pendant une période supplémentaire de 16 heures et on dilue encore avec de l'eau (250 cm³) pour éliminer l'émulsion. On sépare la phase éthérée et on extrait la phase aqueuse à l'éther (3 x 200 cm³). On réunit les phases éthérées et on les sèche (sulfate de magnésium) puis on les concentre. On cristallise le solide résultant dans l'eau. On obtient le produit désiré (humide) qu'il est nécessaire de conserver humide ou dans l'éther afin d'éviter une autooxydation.
RMN-¹H (DMSO-d₆), ppm) : 3,4 (2H, s large, OH) ; 0,8 (7H, s, méthine et méthyle).

[L'acide isopropylboronique a été décrit par R.M. Washburn et al., Org. Syn., 1963, Coll. Vol. IV : 68-72].

### b/ Préparation de l'hexahydro-2(1-méthyléthyl)-3a,5,5-triméthyl-[3aS-(3aα,4α,6α,7aα)]-4,6-méthano-1,3,2-benzodiox aborole (ou acide isopropylboronique pinanediol ester) : composé XV.

On agite pendant 48 heures 30 g d'acide (0,34 mole) préparé au a/ et 40 g de (+)-pinanediol (0,23 mole) dans 150 cm³ d'éther. On dilue le mélange réactionnel avec 150 cm³ d'éther et 100 cm³ d'eau puis on sépare la phase éthérée. On extrait la phase aqueuse à l'éther (3 x 50 cm³), on combine les phases organiques, on les sèche (sulfate de magnésium) et on les concentre. On purifie l'huile résultante par chromatographie sur silice en éluant avec un mélange éther de pétrole/acétate d'éthyle (9/1). On purifie encore l'huile obtenue par distillation sous vide et on recueille le produit désiré avec un point d'ébullition de 64-68°C sous 0,3 Torr.
RMN-¹H (CDCl₃, ppm) : 4,25 (1H, dd, méthine) ; 2,4-1,7 (6H, m, méthylène et méthine) ; 1,35 (3H, s, méthyle) ; 1,25 (3H, s, méthyle) ; 1,0 (7H, s, méthyle et méthine) ; 0,8 (3H, s, méthyle).

[Ce composé a été décrit par C.A. Kettner et al., J. Biol. Chem., 1984, **259**, 15106].

### c/ Préparation du 2-(1-chloro-2-méthylpropyl)-hexahydro-3a,5,5-triméthyl-[3aS-[2(1S),3aα,4α,6α,7aα]-4,6-m éthano-1,3,2-benzodioxaborole : composé XVI.

On agite à -105°C sous atmosphère d'azote, 200 cm³ de tétrahydrofuranne et 10,6 g de dichlorométhane (0,124 mole). On introduit à l'aide d'une seringue, à -105°C en 40 minutes, 46 cm³ de butyllithium 1,6M dans l'hexane (0,084 mole). On agite ce mélange pendant 15 minutes puis on introduit lentement, à l'aide d'une seringue, un mélange de 15 g de composé XV (0,0675 mole) obtenu au b/ dans 60 cm³ de tétrahydrofuranne, en maintenant la température à -105°C. On agite le mélange réactionnel pendant 30 minutes puis on introduit à -105°C en 20 minute, à l'aide d'une seringue, 40,5 cm³ de chlorure de zinc 1M dans l'éther éthylique (0,0405 mole). On laisse la température remonter à la température ambiante et on agite encore pendant 16 heures. On élimine le solvant sous pression réduite et on reprend l'huile résultante dans 500 cm³ d'éther. On lave l'extrait éthéré avec une solution aqueuse saturée de chlorure d'ammonium (100 cm³) et on sépare la phase aqueuse. On l'extrait à l'éther (2 x 100 cm³), on sèche (sulfate de magnésium) les solutions éthérées réunies et on les concentre. On distille sous vide l'huile résultante et on obtient le composé XVI désiré dans la fraction principale avec un point d'ébullition de 74-108°C sous 0,2 Torr.
RMN-¹H (CDCl₃, ppm) : 4,35 (1H, d, O-CH-ester pinanediol) ; 3,32 (1H, d, CHCl) ; 2,45-1,9 (7H, m, méthylène-méthine ester de pinanediol) ; 1,35 (3H, s, méthyle) ; 1,25 (3H, s, méthyle) ; 1,0 (6H, dd, CH₃isopropyle) ; 0,8 (3H, s, méthyle).

### d/ Préparation de l'hexabydro-3a,5,5-triméthyl-α-(1-méthyléthyl)-N,N-bis(triméthylsilyl)-[3aS-[2(1R),3aα,4α,6α, 7aα]-4,6-méthano-1,3,2-benzodioxaborole-2-méthaneamine : composé XVII

On agite à -72°c sous atmosphère d'azote, 24,3 cm³ d'hexaméthyldisilazanne (0,115 mole) et 120 cm³ de tétrahydrofuranne. On y ajoute progressivement 61 cm³ de n.butyllithium 1,6M dans l'hexane (0,0974 mole) entre -72°C et -70°C en 40 minutes. On laisse la température remonter à 0°C pendant 3 heures et on refroidit à nouveau le mélange réactionnel à -72°C. On y introduit lentement en 30 minutes à -72°C, un mélange de 24 g du composé XVI (0,0886 mole) obtenu au c/, dans 100 cm³ de tétrahydrofuranne. On porte alors la température à 20°C et on agite pendant 20 heures puis on concentre. On traite l'huile résultante à l'éther (250 cm³) et à l'eau (100 cm³). On sépare la phase organique et on extrait la phase aqueuse à l'éther éthylique (2 x 100 cm³) puis on réunit les solutions éthérées, on les sèche (sulfate de sodium) et on les concentre. On distille sous vide l'huile résultante (en éliminant la tête) et on obtient le composé XVII avec un point d'ébullition de 120-155°C sous 0,2 Torr.
RMN-¹H (CDCl₃, ppm) : 4,25 (1H, d, O-CH ester de pinanediol) ; 2,4-1,7 (7H, m, méthylène et méthine) ; 1,35 (3H, s, méthyle) ; 1,25 (3H, s, méthyle) ; 1,15 (1H, d, méthine) ; 0,9 (6H, dd, CH₃isopropyle) ; 0,8 (3H, s, méthyle).

### e/ Préparation de l'hexahydro-3a,5,5-triméthyl-α-(1-méthyléthyl)-[3aS-[2(1R),3aα,4α,6α,7aα]-4,6-méthano-1,3,2-b enzodioxaborole-2-méthaneamine, trifluoroacétate (ou trifluoroacétate de borovaline pinanediol ester) : composé XVIII

On agite à 0°C sous atmosphère d'azote, 1 g (0,0258 mole) du produit obtenu au d/ dans 10 cm³ de pentane. On y introduit lentement 0,584 cm³ d'acide trifluoroacétique (0,0758 mole) et on agite ce mélange pendant une heure à 0°C. Après concentration sous pression réduite puis aspiration sous un vide poussé pendant 48 heures, on obtient le composé XVIII sous la forme d'un solide blanc.
RMN-¹H (DMSO-d₆, ppm) : 7,7 (2H, s large, NH₂) ; 4,3 (1H, d, O-CH) ; 2,8 (1H, m, α-CH) ; 2,4-1,8 (7H, m, méthine et méthyle) ; 1,35 (3H, s, méthyle) ; 1,25 (3H, s, méthyle) ; 1,0 (6H, dd, méthyle) ; 0,8 (3H, s, méthyle).
[α]^{D}₂₃ + 25° (c 1,0, CHCl₃)

### f/ Préparation de la N-(tert.butoxycarbonyl)-L-alanyl-L-proline : composé XIX

On dissout dans 30 cm³ de NaOH aqueux 0,5M et 30 cm³ de dioxanne, 3 g de L-alanyl-L-proline sous forme d'hydrate (0,0147 mole) et on refroidit le mélange à 0°C sous agitation. On y introduit à 0°C à l'aide d'une seringue, 3,53 g de dicarbonate de di-tert.butyle (0,0162 mole). On agite le mélange réactionnel à 0°C pendant 30 minutes puis à 20°C pendant une heure. On ajoute 30 cm³ d'acétate d'éthyle et on refroidit le ménage à 0°C. On le traite alors avec KHSO₄ aqueux dilué jusqu'à atteindre pH = 2 et il se forme un précipité. On le filtre, on le lave à l'éther et on le sèche sous vide. On obtient le produit désiré.

RMN (DMSO-d₆, ppm) : 6,9 (1H, d, NH) ; 4,2 (2H, m, α-CH alanine et proline) ; 3,5 (2H, m, méthylène) ; 2,1 (1H, m, CH₂proline) ; 1,9 (3H, m, CH₂proline) ; 1,3 (9H, s, méthyle) ; 1,1 (3H, d, méthyle).

[Ce composé a été décrit notamment par E. Wuensch et al., Int. J. Pept. Protein Res., 1988, 32, 368].

### g/ Préparation de la N-(tert.butoxycarbonyl)-L-alanyl-L-prolyl-L-borovaline pinanediol ester : composé XX

On dissout dans 30 cm³ de tétrahydrofuranne agité sous azote, 3,17 g du composé obtenu au f/ et on refroidit à -20°C. On y introduit à -20°C, 2,27 g de N-éthylmorpholine (0,0190 mole) puis 1,59 g de chloroformiate d'isobutyle (0,0116 mole) et il se forme un précipité. On agite le mélange réactionnel à -20°C pendant 30 minutes puis on introduit un mélange de 4,05 g de composé XVIII sous forme de trifluoroacétate (0,0110 mole) tel qu'obtenu au e/ dans 20 cm³ de tétrahydrofuranne. On agite le mélange à -20°C pendant 30 minutes puis on le laisse réchauffer à 20°C et on l'y maintient pendant 30 minutes. On ajoute alors 30 cm³ d'eau et on extrait à l'acétate d'éthyle (3 x 50 cm³). On sèche les phases organiques réunies et on les concentre. On purifie l'huile résultante par chromatographie sur silice en éluant avec un mélange chloroforme/méthanol (9/1). Par séchage sous vide de l'huile résultante, on obtient le composé désiré sous forme d'un solide blanc.
RMN-¹H (CDCl₃, ppm) : 7,0 (1H, d, NH) ; 5,4 (1H, d, NH) ; 4,65 (1H, d, α-CH) ; 4,45 (1H, t, α-CH) ; 4,30 (1H, d, O-CH) ; 3,5 (2H, m, méthine) ; 3,1 (1H, t, α-CHborovaline) ; 2,5-1,5 (11H, m, méthine et méthylène proline et pinane) ; 1,4 (9H, s, méthyle et tert.butyle) ; 1,35 (3H, s, méthyle) ; 1,3 (3H, dd, méthyle alanine) ; 0,9 (6H, dd, méthyle) ; 0,75 (3H, s, méthyle).

### h/ Préparation du composé en titre sous forme de trifluoroacétate

On dissout dans 2 cm³ de dichlorométhane, 0,5 g du composé obtenu au g/ (0,000962 mole) et on refroidit le mélange à 0°C sous azote. On y introduit, à 0°C sous azote, 3,7 g d'acide trifluoroacétique (0,0324 mole) et on agite le mélange pendant 30 minutes. On élimine l'acide en excès sous pression réduite puis sous vide. On purifie l'huile résultante par CLHP préparative sur une colonne Kromasil équipée d'un détecteur à 215 nm en éluant avec une mélange CF₃COOH aqueux 0,1 %/CF₃COOH méthanolique 0,1 % 3/7 et on obtient un solide incolor qui présente un temps de rétention de 9,7 minutes.
P.F. = 124-125°C
IR (KBr, cm⁻¹) : 3475 (NH₂), 1671 (C=O amide), 1614 (C=O amide).
RMN-¹H (CDCl₃, ppm) : 7,9 (1H, s, NH) ; 4,5 (s large, NH₂ et α-CH alanine) ; 4,2 (1H, d, B-O-H) ; 3,95 (1H, m, α-CH proline) ; 3,45 (2H, m, CH₂ proline) ; 2,4-1,6 (11H, m, méthylène et méthine) ; 1,4 (3H, d, CH₃ alanine) ; 1,3 (3H, s, méthyle) ; 1,2 (4H, s, méthyle et méthine) ; 0,8 (6H, dd, méthyle) ; 0,7 (3H, s, méthyle).
Analyse élémentaire : C₂₂H₃₈BN₃O₄.2CF₃COOH
Trouvé : C = 47,8 % ; H = 6,50 % ; N = 6,31 % ;
Calculé : C = 48,2 % ; H = 6,23 % ; N = 6,49 % ;

**Exemple 10 : Préparation du borohydrate de L-alanyl-L-prolyl-difluoro-L-borovaline ; voie 6** R₁ = CH₃ ; R₂ = H ; n = 0 ou 1 et m = 0 ou 1 avec n différent de m ; R = R' = H ; R₃ = -(CH₂)₂- ; R₄ = CO-NH- R₅ ; R₅ =

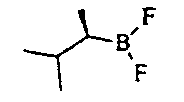

On refroidit à -50°c, une solution de 0,20 g de BCl₃ 1M dans CH₂Cl₂ (0,000384 mole). On y ajoute à l'aide d'une seringue sous atmosphère d'azote et agitation, une solution de 0,20 g du composé obtenu à l'exemple 9 (0,000384 mole) dans 1 cm³ de CH₂Cl₂. On agite ainsi ce mélange réactionnel pendant 15 minutes puis on poursuit l'agitation à 0°C pendant une heure. On y ajoute 1 cm³ d'eau puis 5 cm³ d'acétate d'éthyle. On sépare la phase aqueuse et on évapore à sec à 20°C. On dissout le solide blanc résultant dans 2 cm³ d'eau et 1 cm³ d'acétone et on traite par de l'acide fluorhydrique aqueux 0,5M (5 cm³) pendant 15 minutes. On élimine le solvant à 20°C sous vide poussé. On agite le solide résiduel dans du méthanol et on élimine par filtration le résidu insoluble puis on fait évaporer le filtrat. On obtient un solide blanc que l'on cristallise dans un mélange acétone/eau/éther. On obtient le composé désiré sous forme de borohydrate.
P.F. = 207-208°C
IR (KBr, cm⁻¹) : 3439 (NH), 3190 (NH₃), 1652 (C=O amide), 1629 (C=O amide), 727 (B-F).
RMN-¹H (D₂O, ppm) : 4,8 (1H, m, α-CH proline) ; 4,5 (1H, m, α-CH alanine) ; 3,8 (2H, m, CH₂ proline) ; 2,31 (1H, m, αCH valine) ; 2,0 (4H, m, CH₂ proline) ; 1,7 (1H, m, CH valine) ; 1,55 (3H, d, CH₃ alanine) ; 0,88 (6H, dd, méthyle).
Analyse élémentaire : C₁₂H₂₂BF₂N₃O₂.0,95 H₃BO₃
Trouvé : C = 41,51 % ; H = 6,86 ; N = 11,72 % ;
Calculé : C = 41,43 % ; H = 7,20 % ; N = 12,08 % ;

### Exemple 11 : Préparation de la 1-(2(S)-aminobutyryl)-(4(S)-fluoro)-L-proline n.butylamide ; voie 3

R₁ = CH₂CH₃ ; R₂ = H ; n = 0 ou 1 et m = 0 ou 1 avec n différent de m ; R = R' = H ; R₃ = -CH₂-CH(cis.F)- ; R₄ = CO-NH-R₅ ; R₅ = n.C₄H₉ ;

### a/ Préparation de l'ester méthylique de la N-benzyloxycarbonyl-4(S)-fluoro-L-proline

On dissoüt, sous azote, dans du dichlorométhane (90 ml), 4,83 g (17,29 mmoles) d'ester méthylique de la N-benzyloxy-carbonyl-4(R)-hydroxy-L-proline (commercialisé par ALDRICH, SIGMA) et on refroidit à -70°C puis on traite ce mélange goutte à goutte avec 3 ml (22,71 mmoles) de trifluorure de diéthylaminosulfure (DAST). On réchauffe en une heure le mélange résultant et on l'agite pendant 18 heures. On verse alors le mélange réactionnel dans un mélange eau-glace contenant du NaHCO₃, on l'extrait au dichlorométhane, on sèche (MgSO₄) l'extrait et on le concentre sous pression réduite. On purifie l'huile jaune obtenue par chromatographie sur silice en éluant avec un mélange éther de pétrole/acétate d'éthyle 40/60 puis 50/50. Après évaporation sous vide, on obtient une huile ; R_{f} = 0,5 (éther de pétrole/acétate d'éthyle 50/50)
RMN-¹H (CDCl₃, ppm) : 7,40-7,27 (5H, m, ArH) ; 5,22 (1H, dt, -CHF-, J_{HF} = 52,4 Hz, J = 3,7 Hz) ; 5,14-5,06 (2H, m, CH₂benzyle) ; 4,58 (dd, αH, J = 30,1 Hz, J = 9,5 Hz); 3,96-3,62 (2H, m, -CH₂N-) ; 3,64, 3,75 (3H, OCH₃) ; 2,59-2,47 (1H, m, -CH₂) ; 2,47-2,24 (1H, m, -CH₂-).
Spectre de masse (EI) : m/e (% intensité) = 281 (M⁺, 1,36) ; 261 (M⁺-HF, 0 01) ; 91 (Ph-CH₂⁺, 100).

### b/ Préparation de la N-benzyloxycarbonyl-4(S)-fluoro-L-proline

On dissout dans du méthanol (30 ml), 3,29 g (11,70 mmoles) du composé préparé au a/ et on ajoute goutte à goutte, à -5°C, 7 ml (14 mmoles) de soude 2M. On laisse alors le mélange à la température ambiante pendant 17 heures. Après addition d'eau (100 ml), on élimine sous vide le méthanol et on lave la solution aqueuse 2 fois avec du dichlorométhane. On acidifie alors cette solution à pH = 3 avec de l'acide citrique (20 %) puis on extrait avec du dichlorométhane (3 x 50 cm³). On sèche (MgSO₄) les extraits, on filtre et on élimine le solvant sous vide (en ajoutant de l'éther à la fin). On obtient ainsi un solide blanc.
P.F. = 123-124°C
RMN-¹H (400 MHz, CDCl₃, ppm) : 7,38-7,26 (5H, m, ArH) ; 5,32-5,15 (3H, m, -CHF- J_{HF} = 52,16 Hz, CH₂ benzyle) ; 4,61 (1H, dd, αH J = 27,6 Hz, J = 9,6 Hz) ; 3,98-3,60 (2H, m, CH₂N); 2,78-2,54 (1H, m, -CH₂-) ; 2,52-2,24 (1H, m, -CH₂-).
CCM (acétate d'éthyle) : R_{f} = 0,04
Spectre de masse (EI) : m/e (% intensité) = 267 (M⁺, 1,82) ; 247 (M⁺-HF, 0,02) ; 91 (PhCH₂⁺, 100).

### c/ Préparation de la N-benzyloxycarbonyl-4(S)-fluoro-L-proline n.butylamide

On dissout, sous argon, dans du tétrahydrofuranne sec (40 ml), 1,40 g (5,24 mmoles) du composé obtenu au b/ et on ajoute 0,66 ml (5,24 mmoles) de 4-éthylmorpholine à 0°C puis 0,685 ml (5,24 mmoles) de chloroformiate d'isobutyle à -5°C. Un précipité blanc de chlorhydrate de 4-éthylmorpholine se forme. Après agitation pendant 20 minutes à -5°C, on ajoute 0,54 ml (5,46 mmoles) de n.butylamine et on agite le mélange réactionnel 40 minutes à 0°C puis une heure à la température ambiante. On élimine par filtration le solide, on lave avec du tétrahydrofuranne sec puis on fait évaporer sous vide la solution résultante. On traite alors le résidu incolor par 50 ml d'une solution d'acide citrique à 5 % et 120 ml d'acétate d'éthyle. Après avoir secouer rigoureusement, on sépare les phases, on lave la phase organique avec 50 ml de KHCO₃ 10 % puis avec 50 ml de brine. Après séchage (MgSO₄) et élimination sous vide du solvant, on obtient un solide blanc qu'on lave à l'éther de pétrole et qu'on sèche sous vide sur P₂O₅. On obtient le produit désiré sous la forme d'un solide blanc.
P.F. = 79-80°C
CCM (acétate d'éthyle/éther de pétrole 50/50) : 0,5
RMN-¹H (400 MHz, CDCl₃, ppm) : 7,34 (5H, s large, ArH) ; 6,50-6,00 (1H, d large, -NH-) ; 5,21 (1H, dt, -CHF-J_{HF} = 51,9 Hz, J = 3,4 Hz) ; 5,20-5,00 (2H, m, CH₂benzyle) ; 4,48 (1H, d, αH J = 1,4 Hz) ; 4,02-3,38 (2H, m, -CH₂N proline) ; 3,32-3,02 (2H, m, -CH₂-NH- NHbutyle) ; 2,90-2,52 (1H, m, -CH₂- proline) ; 2,48-2,08 (1H, m, -CH₂-proline) ; 1,54-1,16 (4H, m, -CH₂- NHbutyle) ; 0,87 (3H, s large, CH₃ NH butyle).

### d/ Préparation de la 4(S)-fluoro-L-proline n.butylamide

On dissout dans 40 ml de méthanol, 1,48 g (4,59 mmoles) du composé obtenu au c/ et on ajoute 0,56 g de Palladium sur charbon activé humide (10 %). On hydrogène le mélange réactionnel sous 60 psi pendant 3 heures puis on filtre la solution et on la concentre sous vide. On obtient une huile qui cristallise après sèchage sous vide sur P₂O₅ pendant 18 heures, ce qui conduit au produit désiré.
P.F. = 30°C
RMN-¹H (400 MHz, CDCl₃, ppm) : 7,53 (s large, 1H, -NH-) ; 5,16 (1H, dt, -CHF- J₁ = 53,1 Hz, J₂ = 3,7 HZ) ; 3,82 (1H, dd, αH, J₁ = 10,3 Hz, J₂ = 3,3 Hz) ; 3,40-3,10 (4H, m, -CH₂-N- et -CO-N-CH₂-) ; 2,48-2,20 (2H, m, -CH₂-proline) ; 2,12 (1H, s large, -NH-) ; 1,42-1,52 (2H, m, -CH₂- NHbutyle) ; 1,37-1,28 (2H, m, -CH₂- NHbutyle) ; 0,91 (3H, t, J = 7,34 Hz, -CH₃).

### e/ Préparation de la 1-(N-(tert.butoxycarbonyl)-2(S)-aminobutyryl)-(4(S)-fluoro)-L-proline n.butylamide

On procède comme à l'étape c/ à partir de 0,81 g (3,98 mmoles) d'acide N-(tert.butoxycarbonyl)-2(S)-aminobutyrique préparé comme à l'exemple 2 a/ et en utilisant le composé obtenu au d/ au lieu de la n.butylamine. Le produit brut obtenu présente deux tâches en CCM avec des R_{f} similaires. On sépare ces deux produits par chromatographie sur gel de silice en éluant avec un gradient du mélange acétate d'éthyle/éther de pétrole de 20/80 à 60/40. On obtient l'amide pur (second produit) qui cristallise après séchage sur P₂O₅ sous vide pendant 5 jours.
P.F. = 67-68°C
(isomère trans : T et isomère cis : C)
RMN-¹H (400 MHz, CDCl₃, ppm) : 7,52 (0,37 H, s large,-NH- C) ; 6,55 (0,63H, s large, -NH- T) ; 5,31 (0,37H, dt, -CHF- T J₁ = 52,3 Hz, J₂ = 4,0 Hz) ; 5,23 (0,63H, dt, -CHF-C J₁ = 50,1 Hz, J₂ = 3,2 Hz) ; 5,19 (0,63H, d large, -NH- T J = 8,3 Hz) ; 5,19 (0,37H, d large, -NH- C J = 6,7 Hz) ; 4,76 (0,63H, d, αH T J = 9,5 nz) ; 4,43 (0,37H, d, αH C, J = 9,2 Hz) ; 4,33 (0,63H, q, αH T J₁ = 8,5 Hz, J₂ = 6,7 Hz) ; 4,09-3,76 (2,37H, m, -CH₂-N-proline et αH C) ; 3,46-2,85 (3H, m, -CO-N-CH₂ et -CH₂aminobutyryle) ; 2,34-2,07 (1H, m, -CH₂ aminobutyryle) ; 1,89-1,20 (15H, m, tert.C₄H₉ et -CH₂- proline et NHbutyle) ; 1,00 (1,89H, t, CH₃aminobutyryle T J = 7,3 Hz) ; 1,00 (1,11H, t, CH₃aminobutyryle C J = 7,5 Hz) ; 0,89 (3H, t, CH₃ NHbutyle, J = 7,3 Hz).
Spectre de masse (FAB) : trouvé : MH⁺ = 374 (11,7) ; 58 (100,0) ; calculé : M = 373

### f/ Préparation du composé en titre sous forme de trifluoroacétate

On refroidit à 0°C une solution de CF₃COOH/CH₂Cl₂ 50/50 et on y ajoute 0,20 g (0,547 mmole) du composé préparé au e/. On agite à la température ambiante pendant une heure et 15 minutes puis on élimine l'acide et le solvant sous vide, ce qui conduit à une huile jaune. On ajoute ensuite 2 x 6 ml de CH₂Cl₂ et on fait évaporer sous vide pour éliminer l'acide en excès. On lave le produit brut résultant avec 3 x 20 ml de diéthyléther anhydre et on obtient un solide blanc qur l'on sèche pendant 48 heures à la température ambiante sous 0,25 mmHg. On obtient le produit désiré.
P.F. = 60-63°C
(T : isomère trans et C : isomère cis)
RMN-¹H (400 MHz, CD₃OD, ppm) : 5,34 (0,73H, dm, -CHF-T) ; 5,27 (0,27H, dm, -CHF- C) ; 4,65 (1H, dd, α-Haminobutyryle) ; 4,13 (0,73H, dd, α-Hproline T) ; 4,05-3,63 (2H, m, CH₂-Nproline et 0,27H, α-Hproline C) ; 3,28-3,08 (2H, m, -N-CH₂- -NHbutyle); 2,68-2,25 (2H, m, -CH₂aminobutyryle) ; 2,10-1,75 (2H, m, -CH₂proline) ; 1,54-1,42 (2H, m, -CH₂-CH₂-CH₃) ; 1,40-1,29 (2H, m, -CH₂-CH₂-CH₃) ; 1,11 (2,19H, t, CH₃aminobutyryle T) ; 1,01 (0,81H, t, CH₃aminobutyryle C) ; 0,94 (0,81H, t, CH₃ NHbutyle C) ; 0,92 (2,19H, t, CH₃ NHbutyle T).
Spectre de masse (FAB) : MH⁺ trouvé = 274 (100 %) ;
calculé = 273
IR (pastille de KBr, cm⁻¹) : 3438 (m, amide) ; 3073 (m, NH₃⁺) ; 1668 (s, C=O).
Microanalyse : C₁₃H₂₄O₂N₃F.1,1CF₃COOH
Trouvé : C = 45,90 % ; H = 6,30 % ; N = 10,48 %
Calculé : C = 45,78 % ; H = 6,34 % ; N = 10,54 % ;

### Exemple 12 : Préparation de la 1-(2(S)-aminobutyryl)-(4(S)-benzyl)-L-proline n.butylamide ; voie 4

R₁ = CH₂CH₃ ; R₂ = H ; n = 0 ou 1 et m = 0 ou 1 avec n différent de m ; R = R' = H ; R₃ = -CH₂-CH(CH₂Ph)- ; R₄ = CO-NH-R₅ ; R₅ = n.C₄H₉ ;

### a/ Préparation de l'ester méthylique de la N-benzyloxy-carbonyl-4-céto-L-proline

On dissout dans 300 ml d'acétone (qualité réactif), 5,0 g (17,90 mmoles) d'ester méthylique de la N-benzyloxycarbonyl-4-hydroxy-L-proline (commercialisée par ALDRICH, SIGMA). On agite cette solution magnétiquement et on ajoute 15 ml d'acide chromique dans l'acide sulfurique approximativement 8N sur une période d'environ 3 minutes. On continue l'agitation pendant une heure puis on détruit l'excès d'oxydant par addition de 5 ml d'isopropanol en 20 minutes. On élimine le solvant puis on dissout le résidu dans de l'éther et on filtre sur fluorisil afin d'éliminer les sels de chrome. On purifie le produit brut obtenu après évaporation par chromatographie éclaire sur silice en éluant avec un gradient de mélange acétate d'éthyle/éther de pétrole (P.Eb. < 40°C) 15/85 à 40/60. On obtient une huile incolore après sèchage sous pression réduite pendant une nuit.
R_{f} = 0,85 (acétate d'éthyle)
RMN-¹H (200 MHz, CDCl₃, ppm) : 7,30 (5H, s large, ArH) ; 5,20-5,00 (2H, m, CH₂benzyle) ; 4,85 (1H, t, α-H) ; 3,95 (2H, s large, -CH₂-Nproline) ; 3,70-3,60 (3H, d, -OCH₃) ; 3,05-2,80 (1H, m, -CH₂proline) ; 3,65-3,50 (1H, m, CH₂proline).
Spectre de masse (FAB) : m/e (% intensité) = 300 ((M + Na)⁺, 100,0) ; 278 (MH⁺, 13,0) ; 154 (43,5) ; 136 (12) ; 107 (12,5) ; 91 (100).

### b/ Préparation de l'ester méthylique de la N-benzyloxy-carbonyl-4-benzylidène-L-proline

On met en suspension dans 30 ml de tétrahydrofuranne sec, 3,68 g (9,46 mmoles) de chlorure de benzyltriphénylphosphonium et on ajoute ce mélange à une solution de 0,22 g (9,65 mmoles) d'hyrure de sodium (95 %) dans 20 ml de tétrahydrofuranne sec, sous azote. On ajoute ensuite 25 ml de DMSO sec et on chauffe le mélange à 70°C jusqu'à ce qu'il devienne homogène (4 heures). Après refroidissement de la solution à 50°C, on ajoute en 5 minutes, un mélange de 2,00 g (7,90 mmoles) de l'ester obtenu au a/ dans 10 ml de tétrahydrofuranne sec. On chauffe alors le mélange réactionnel à 70°C pendant 16 heures puis on le verse dans 200 ml d'eau/glace contenant 1,4 g de KHCO₃. On ajoute ensuite 150 ml de dichlorométhane puis on sépare la phase organique et on extrait la phase aqueuse une fois avec 100 ml de dichlorométhane. Après séchage (MgSO₄) des phases organiques et évaporation sous vide, on purifie le produit brut obtenu par chromatographie éclaire sur silice en éluant avec un mélange acétate d'éthyle/éther de pétrole 15/85 puis 35/65. Après sèchage sous vide sur P₂O₅ pendant une nuit, on obtient une huile incolore.
R_{f} = 0,90 (acétate d'éthyle)
RMN-¹H (400 MHz, CDCl₃, ppm) : 7,50-7,05 (10H, m, ArP) ; 6,53-6,13 (1H, m, H vinylique) ; 5,30-5,00 (2H, m, H benzyle) ; 4,73-4,30 (3H, m, α-H et N-CH₂proline) ; 3,80-3,50 (3H, dd, OCH₃) ; 3,33-3,10 (1H, m, -CH₂proline) ; 3,05-2,67 (1H, m, -CH₂proline) ;
Spectre de masse (FAB, (matrice MNOBA + NaI) : m/e (% intensité) = 300 (MH⁺ + Na, 97) ; 278 (MH⁺, 13) ; 154 (44) ; 91 (C₇H₇⁺, 100).

### c/ Préparation de l'ester méthylique de la 4(S)-benzyl-L-proline

On ajoute 0,95 g de palladium sur charbon activé (humide à 10 %) à une solution de 2,10 g (5,97 mmoles) de l'ester obtenu au b/ dans 250 ml de méthanol et on hydrogène ce mélange réactionnel pendant 2 heures et 30 minutes sous 60 psi. Après filtration de la solution, concentration sous vide et sèchage sur P₂O₅ pendant une nuit, on obtient le produit désiré.

Le spectre RMN montre la présence de deux diastéréoisomères dans un rapport ci/trans 90/10.
(T = isomère trans et C = isomère cis)
RMN-¹H (400 MHz, ppm) : 7,32-7,12 (5H, m, ArH) ; 3,82 (1H, t, α-H, J = 8,0 HZ) ; 3,74 (3H, s, OCH₃) ; 3,08-3,02 (1H, m, -CH₂-Nproline) ; 2,80-2,73 (1H, m, -CH₂N proline) ; 2,72-2,58 (2H, m, Hbenzyle) ; 1,60-1,70 (0,1H, m, PhCH₂CH T) ; 1,50-1,59 (0,9H, m, PhCH₂CH C).

### d/ Préparation de l'ester méthylique de la 1-(N-benzyloxy-carbonyl-2(S)-aminobutyryl)-(4(S)-benzyl)-L-proline

On ajoute à 0°C 0,66 g (4,01 mmoles) de 3-hydroxy-1,2,3-benzotriazine-4(3H)-one et 0,83 g (4,01 mmoles) de dicylohexylcarbodiimide (DCC) à une solution de 0,815 g (3,43 mmoles) d'acide N-benzyloxycarbonyl-2(S)-aminobutyrique préparé comme à l'exemple 3 a/ dans un mélange de 5 ml de diméthylformamide et 5 ml de chlorure de méthylène. Après une heure et 15 minutes, on ajoute un mélange de 0,88 g (4,01 mmoles) de l'ester obtenu au c/ dans 5 ml de CH₂Cl₂. On agite à 0°C pendant 15 minutes puis deux jours à la température ambiante. Après élimination sous vide du solvant, on reprend le résidu dans de l'acétate d'éthyle. On élimine par filtration la dicyclohexylurée, on lave le filtrat successivement avec NaHCO₃ 4 %, de l'acide citrique 10 % puis on fait évaporer sous vide. On purifie le produit brut ainsi obtenu par chromatographie éclaire sur silice en éluant avec un gradient du mélange acétate d'éthyle/ éther de pétrole 15/85 à 35/65. Après évaporation, on obtient une pâte jaune.
RMN-¹H (200 MHz, CDCl₃, ppm) : 7,50-6,95 (10H, m, ArH) ; 5,60 (1H, d, -NH-) ; 5,05 (2H, m, PhCH₂O) ; 4,50-4,35 (2H, m, α-H) ; 3,95-3,60 (4H, m, OCH₃ et -CH₂-Nproline) ; 3,35-3,20 (1H, m, -CH₂-Nproline) ; 2,80-2,10 (4H, m, PhCH₂C et -CH₂proline) ; 1,95-1,50 (3H, m, -CH₂aminobutyryle et H proline) ; 0,95 (3H, t, CH₃aminobutyryle).
R_{f} = 0,61 (acétate d'éthyle)

### e/ Préparation de la 1-(N-benzyloxycarbonyl-2(S)-amino-butyryl)-(4(S)-benzyl)-L-proline

On saponifie 1,30 g (2,96 mmoles) de l'ester obtenu au d/ avec 1,75 ml (3,50 mmoles) de NaOH 2M dans 30 ml de méthanol pendant 20 heures à la température ambiante. Après évaporation sous vide, reprise dans 100 ml d'eau distillée, lavage deux fois à l'éther puis acidification à pH = 1 avec HCl aqueux 5N, on extrait trois fois avec 150 ml d'éther éttiylique, on sèche (MgSO₄) les phases organiques, on filtre et on fait évaporer sous vide. On obtient le produit désiré sous la forme d'un solide blanc.
P.F. = 56-57°C
RMN-¹H (200 MHz, CDCl₃, ppm) : 7,5-7,0 (10H, m, ArH) ; 5,7 (1H, d, -NH-) ; 5,05 (2H, s large, PhCH₂O) ; 4,50-4,25 (2H, m, α-H) ; 4,00-3,80 (1H, m, -N-CH₂proline) ; 3,30-3,10 (1H, m, -N-CH₂proline) ; 2,80-2,10 (4H, m, PhCH₂C et -CH₂proline) ; 1,90-1,40 (3H, m, -CH₂aminobutyryle et Hproline) ; 0,90 (3H, t, CH₃aminobutyryle).

### f/ Préparation de la 1-(N-benzyloxycarbonyl-2(S)-amino-butyryl)-(4(S)-benzyl)-L-proline n.butylamide

On procède de la manière indiquée à l'exemple 11 c/ à partir du composé obtenu précédemment au c/. On purifie le produit brut résultant par chromatographie éclaire sur silice en éluant avec un gradient de mélange acétate d'éthyle/éther de pétrole 15/85 à 50/50. On obtient deux échantillons du produit désiré.

### g/ Préparation du composé en titre sous forme d'hydrate d'oxalate

On hydrogène sous 60 psi pendant 2 heures et 30 minutes une solution de 0,75 g du composé obtenu précédemment au f/ dans du méthanol, à l'aide de 0,30 g de palladium 10 % sur charbon (humide). Après élimination du catalyseur par filtration et du solvant sous vide, on purifie le produit brut obtenu par chromatographie éclaire en utilisant comme premier éluant un mélange acétate d'éthyle/éther de pétrole 50/50 puis un mélange acétate d'éthyle/méthanol 50/50. Après évaporation du solvant, on reprend l'huile résultante dans de l'isopropanol et on filtre pour éliminer la silice. On obtient 350 mg (1,01 mmole) de base libre. On y ajoute 0,12 g (1,32 mmole) d'acide oxalique dans de l'éthanol et on ajoute de l'éther. Après cristallisation, on obtient le produit désiré.
P.F. = 89-93°C (capillaire ouvert)
RMN-¹H (400 MHz, CD₃OD, ppm) : = 7,32-7,15 (5H, m, ArH) ; 4,48-4,28 (1H, m, α-H) ; 4,22-4,10 (1H, m, α-H) ; 3,87-3,68 (1H, m, -CH₂-Nproline) ; 3,42-3,06 (3H, m, -CH₂Nproline et -CH₂-N NH₂butyle) ; 2,84-2,44 (3H, m, -CH₂Ph et CH₂proline) ; 2,36-2,10 (1H, m, -CH₂proline) ; 2,02-1,76 (2H, m, -CH₂aminobutyryle) ; 1,67-1,26 (5H, m, -CH₂CH₂ NH₂butyle et Hproline) ; 1,12-0,86 (6H, m, -CH₃aminobutyryle et -CH₃ NH₂butyle).
Microanalyse : C₂₀H₃₁N₃O₂.1,05(COOH)₂.0,9H₂O
Trouvé . C = 58,19 % ; H = 7,24 % ; N = 8,95 %
Calculé : C = 58,18 % ; H = 7,71 % ; N = 9,21 % ;
Spectre de masse (FAB) : m/e (% intensité) = 368 (M⁺ + Na, 14) ; 346 (M⁺, 100) ; 261 (68) ; 160 "(47) ; 91 (C₇H₇⁺, 7).

### Exemple 13 : Préparation de l'acide 2-(2(S)-aminobutyryl)-1,2,3,4-tétrahydro-3(S)-isoquinolinecarboxyli que: n. butylamide ; voie 3

R₁ = CH₂CH₃ ; R₂ = H ; n = 0 et m = 1 ; R = R' = H ; R₃ = 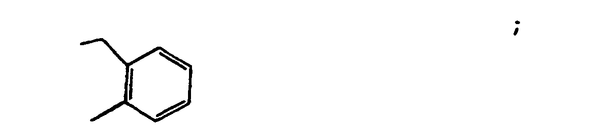 R₄ = CO-NH-R₅ ; R₅ = n.C₄H₉

### a/ Préparation de l'acide 1,2,3,4-tétrahydro-3(S)-iso-quinolinecarboxylique

On dissout dans 50 ml d'acide chlorhydrique concentré, 5,0 g (30,27 mmoles) de L-phénylalanine et on ajoute 4,0 g de paraformaldéhyde et 1 ml d'acide sulfurique concentré. On porte au reflux cette solution pendant deux jours. Après refroidissement et filtration, on dissout le solide résultant dans un mélange chaud eau/éthanol : 1/1 (v/v) et on ajoute NH₄OH aqueux (30 %) jusqu'à ce qu'un pH 7,0 soit atteint. On récupère par filtration les cristaux qui ont précipité après refroidissement de la solution, on les lave à l'éthanol et on les sèche. On obtient ainsi le produit désiré.
P.F. = 315°C (litt.)23
Spectre de masse (FAB) : m/e (% intensité) = 178 (MH⁺, 23) ; 154 (100).

### b/ Préparation de l'acide N-benzyloxycarbonyl-1,2,3,4-tétrahydro-3(S)-isoquinolinecarboxylique

On refroidit dans un bain glace-eau, une solution de 2,50 g (14,11 mmoles) de l'acide préparé au a/ dans NaOH 2N (8 ml) et on agite vigoureusement avec un agitateur magnétique. On ajoute alternativement, en 10 portions environ, 2,21 ml (15,52 mmoles) de chloroformiate de benzyle et 8 ml de NaOH 2N. On maintient la température du mélange réactionnel entre 0 et 5°C en controllant la vitesse d'addition des réactifs. On ajoute 9 ml de NaOH 2N et on maintient le mélange à la température ambiante pendant 30 minutes. On lave la solution alcaline à l'éther (4 x 50 ml), puis on acidifie par addition de HCl 5N (pH < 2). On extrait ensuite au chlorure de méthylène, on sèche sur MgSO₄, on filtre et on fait évaporer sous vide. Après séchage du solide résultant sur P₂O₅, on obtient le produit désiré.
P.F. = 52-55°C
RMN-¹H (200 MHz, CDCl₃, ppm) : 7,55-7,00 (9H, m, Ar-H) ; 5,10 (2H, d, H benylique) ; 5,00-4,50 (3H, m, α-H et -CH₂-Ntétra-hydroisoquinoline) ; 3,20-3,00 (2H, m, -CH₂tétrahydro-isoquinoline).
Spectre dee masse (FAB) : m/e (% intensité) = 334 ((M + Na)⁺, 0,1) ; 312 (MH⁺, 3,5) ; 91 (C₇H₇⁺, 100).

### c/ Préparation de l'acide N-benzyloxycarbonyl-1,2,3,4-tétrahydro-3(S)-isoquinolinecarboxylique n.butylamide

On procède comme à l'exemple 11 c/ à partir de l'acide obtenu au b/. On agite le mélange réactionnel à 0°C pendant 40 minutes puis à la température ambiante pendant une nuit. On ajoute ensuite 100 ml de chlorure de méthylène et on lave la solution avec 3 x 50 ml d'acide citrique 10 %. On sépare les phases après avoir secouer énergiquement puis on lave la phase organique avec 2 x 50 ml de NaHCO₃ 5 %. Après séchage sur MgSO₄, on purifie le produit brut obtenu par chromatographie éclaire en éluant avec un mélange acétate d'éthyle/éther de pétrole 70/30 (v/v). Après évaporoation sous vide, on fait sécher sur P₂O₅ pendant une nuit le produit résultant. On obtient ainsi l'amide pur sous la forme d'une huile.
RMN-¹H (200 MHz, CDCl₃, ppm) : 7,45-7,05 (9H, m, Ar-H) ; 5,80 (1H, d, -NH-) ; 5,25-5,06 (2H, m, -CH₂-Ph) ; 4,90-4,40 (3H, m, α-H et -NH-CH₂tétrahydroisoquinoline) ; 3,42-2,78 (4H, m, -NH-CH₂ NH₂butyle et -CH₂tétrahydroisoquinoline) ; 3,60-3,40 (2H, m, -CH₂tétrahydroisoquinoline) ; 1,18-0,66 (7H, m, -CH₂CH₂CH₃ NHbutyle).
R_{f} (acétate d'éthyle) = 0,60
Spectre de masse (FAB) : m/e (% intensité) = 367 (MH⁺, 13,9) ; 91 (C₆H₅⁺, 100).

### d/ Préparation de l'acide 1,2,3,4-tétrahydro-3(S)-isoquinolinecarboxylique n.butylamide

On procède à une hydrogénation du composé obtenu au c/, dans les conditions de l'exemple 11 d/. On obtient le produit désiré.
P.F. = 58-60°C
RMN-¹H (400 MHz, CDCl₃, ppm) : 7,26-7,02 (5H, m, Ar-H et -NHamide) ; 4,08-3,98 (2H, m, α-H et -CH₂Ntétrahydroisoquinoline) ; 3,51-3,58 (1H, m, -CH₂-NHtétrahydroisoquinoline) ; 3,32-3,20 (3H, m, -CH₂amide et 1H -CH₂tétrahydroisoquinoline) ; 2,76-2,88 (1H, m, -CH₂tétrahydroisoquinoline) ; 1,81 (1H, s large, -NHtétrahydroisoquinoline) ; 1,55-1,46 (2H, m, -NH-CH₂-CH₂-CH₂-CH₃) ; 1,40-1,29 (2H, m, -NH-CH₂-CH₂-CH₂-CH₃) ; 0,93 (3H, t, CH₃amide J = 7,3 Hz).

### e/ Préparation de l'acide 2-(N-benzyloxycarbonyl-2(S)-aminobutyryl)-1,2,3,4-tétrahydro-3(S)-isoquinoline carboxylique n.butylamide

On procède comme à l'étape c/, à partir d'acide N-benzyl-oxycarbonyl-2(S)-aminobutyrique préparé comme à l'exemple 3 a/ et à partir du composé obtenu au d/. On laisse reposer le mélange réactionnel pendant un nuit à la température ambiante, puis on ajoute 40 ml de chlorure de méthylène et on lave cette solution avec 3 x 50 ml d'acide citrique 10 %. Après avoir secouer énergiquement, on sépare les phases et on lave la phase organique avec 2 x 50 ml de NaHCO₃ 5 %. Après sèchage sur MgSO₄ et évaporation sous vide, on purifie le produit brut résultant par chromatographie éclaire sur silice en éluant avec un mélange acétate d'éthyle/éther 15/85 puis 35/65. On obtient le produit désiré sous la forme d'une huile que l'on fait sécher sous vide sur P₂O₅ pendant une nuit.
Spectre de masse (FAB) : m/e (% intensité) = 474 ((M + Na)⁺, 0,1) ; 452 (M⁺, 2,5) ; 91 (C₇H₇⁺, 100).
RMN-¹H (400 MHz, CDCl₃, ppm) : 7,42-7,08 (9H, m, ArH) ; 6,03 (1H, s large, -NHamide) ; 5,68 et 5,39 (1H, d, -NHamide) ; 5,20-4,88 (3H, m, Hbenzyle et α-H) ; 4,86-4,38 (3H, m, -CH₂Ntétrahydroisoquinoline et α-H) ; 3,68-2,90 (4H, m, -CH₂N NH₂butyle et -CH₂tétrahydroisoquinoline) ; 2,00-1,60 (2H, m, -CH₂aminobutyryle) ; 1,44-1,16 (2H, m, -CH₂-CH₂-CH₂-CH₃) ; 1,16-0,96 (2H, m, -CH₂-CH₂-CH₂-CH₃) ; 0,36 et 0,78 (3H, t, CH₃aminobutyryle).

### f/ Préparation du composé en titre sous la forme d'oxalate

On procède à une hydrogénation du composé obtenu au e/, dans les conditions de l'étape d/. On ajoute ensuite à la solution filtrée, 0,16 g (1,78 mmole) d'acide oxalique et on concentre cette solution sous vide. On ajoute alors de l'éthanol puis de l'éther (rapport 1/50), ce qui conduit à un produit cristallisé. Après filtration et lavage au diéthyléther, on fait sècher le solide sur P₂O₅ sous vide à 30°C pendant 24 heures et on obtient le composé désiré.
P.F. = 105-110°C (capillaire ouvert)
RMN-¹H (400 MHz, CD₃OD, ppm) : 7,34-7,15 (4H, m, Ar-H) ; 4,86-4,54 (4H, m, 2 α-H et -CH₂Ntétrahydroisoquinoline) ; 3,43-2,88 (4H, m, -CH₂tétrahydroisoquinoline et -NH-CH₂ NHbutyle) ; 2,14-1,80 (2H, m, -CH₂aminobutyryle) ; 1,48-1,18 (4H, m, -CH₂-CH₂ -NHbutyle) ; 1,0-1,2 (3H, t, CH₃aminobutyryle) ; 0,7-0,95 (3H, t, CH₃ -NHbutyle).
Spectre de masse (FAB) : m/e (% intensité) = 318 (MH⁺, 96,1) ; 245 (30,9) ; 233 (40,3) ; 149 (100,0) ; 132 (65,4) ; 71 (38,8) ; 58 (53,7) ; 57 (73,0).
IR (pastille KBr, cm⁻¹) : 3072 (m, NH) ; 2956 (s, CH) ; 1652 (s, C=O).
Microanalyse : C₁₈H₂₇N₃O₂.1,05(COOH)₂.0,5H₂O
Trouvé : C = 57,63 % ; H = 7,08 % ; N = 9,53 % ;
Calculé : C = 57,35 % ; H = 7,21 % ; N = 9,98 % ;

### Exemple 14 : Préparation de l'acide 1-(2(S)-aminobutyryl)- 2(S)-indolinecarboxylique n.butylamide ; voie 3

R₁ = CH₂CH₃ ; R₂ = H ; n = 0 et m = 1 ; R₃ = 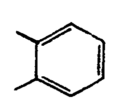 (R₆ = H), R = R' = H ;
R₄ = CO-NH-R₅ ; R₅ = n.C₄H₉

### a/ Préparation de l'acide N-benzyloxycarbonyl-2(S)-indolinecarboxylique n.butylamide

On dissout dans du tétrahydrofuranne (150 ml), sous azote, 7,699 g (25,9 mmoles) d'acide N-benzyloxycarbonyl-2(S)-indolinecarboxylique préparé comme à l'exemple 16 a/. On ajoute à 0°C 2,986 g (25,9 mmoles) de N-éthylmorpholine puis 3,54 g (25,9 mmoles) de chloroformiate d'isobutyle. Après 45 minutes, on ajoute 1,903 g (25,9 mmoles) de n.butylamine et on agite la suspension pendant une nuit. On ajoute du chlorure de méthylène (300 ml) et on lave la phase organique avec de l'acide citrique 10 % (3 x 100 ml) puis NaHCO₃ (3 x 100 ml). Après sèchage et évaporation, on obtient un solide blanc que l'on recristallise dans du méthanol.
P.F.164-165°C
RMN-¹H (CDCl₃, ppm) : 6,9-7,8 (9H, m, Ar-H) ; 5,2 (2H, m, CH₂benzyloxycarbonyle) ; 4,9 (1H, m, αH) ; 2,9-3,8 (4H, m, CH₂cycle CH₂N) ; 1,0-1,5 (4H, m, CH₂CH₂CH₂N) ; 0,8-1,0 (3H, m, CH₃).

### b/ Préparation de l'acide 2(S)-indolinecarboxylique n.butylamide

On dissout dans du méthanol le composé préparé au a/ et on ajoute du palladium sur charbon (10 %, humide, 10 % en poids). On hydrogène le mélange pendant 4 heures sous 40 psi dans un appareil de Parr. Après élimination du catalyseur et évaporation du méthanol, on obtient des cristaux suffisamment purs sans recristallisation.
P.F. = 110-111°C
RMN-¹H (CDCl₃, ppm) : 6,8-7,1 (4H, m, Ar-H) ; 4,3 (1H, m, αH) ; 2,9-3,5 (4H, m, CH₂cycle CH₂N) ; 1,0-1,9 (4H, m, CH₂CH₂CH₂N) ; 0,9 (3H, t, CH₃).

### c/ Préparation de l'acide 1-(N-benzyloxycarbonyl-2(S)-aminobutyryl)-2(S)-indolinecarboxylique n.butylamide

On procède comme à l'exemple 15 c/ en utilisant 0,646 g (2,73 mmoles) d'acide N-benzyloxycarbonyl-2(S)-aminobutyrique préparé comme à l'exemple 3 a/ et 0,595 g (2,73 mmoles) de l'amide préparé à l'étape b/. On obtient une huile que l'on recristallise dans CCl₄.
P.F. = 178-179°C
RMN-¹H (CDCl₃, ppm) : 7,0-7,2 (9H, m, Ar-H) ; 4,9-5,3 (4H, m, 2 x αH CH₂benzyloxycarbonyle) ; 3,5 (2H, m, CH₂cycle) ; 3,1 (2H, m, CH₂N) ; 1,0-2,0 (9H, m, CH₂CH₂CH₂N CH₂aminobutyryle CH₃ aminobutyryle) ; 0,9 (3H, m, CH₃ butyle).

### d/ Préparation du composé en titre sous la forme d'hydrate d'oxalate

On hydrogène le composé obtenu au c/ comme à l'exemple 5 c/ pour la N-benzyloxycarbonyl-2(S)-aminobutyryl-L-proline [2(S)-méthyl] n.butylamide et on isole le composé en titre sous la forme de sel d'oxalate. Il est suffisamment pur sans recristallisation.
P.F. = 142°C
RMN-¹H (CD₃OD, ppm) : 8,25 (1H, m, NH) ; 7,25 (4H, m, Ar-H) ; 5,1 (2H, m, 2 x αH (caché par le colvant)) ; 3,8 (2H, m, CH₂Ar) ; 3,5 (2H, m, CH₂cycle) ; 3,2 (2H, m, CH₂N) ; 2,0 (2H, m, CH₂aminobutyryle) ; 0,9-1,8 (10H, m, CH₃aminobutyryle CH₃CH₂CH₂CH₂N).
Analyse : C₁₇H₂₅N₃O₂.C₂H₂O₄.0,75 H₂O
Trouvé : C = 56,25 % ; H = 6,99 % ; N = 9,84 % ;
Calculé : C = 56,08 % H = 7,06 % N = 10,33 % ;

### Exemple 15 : Préparation de l'acide 1-(2(S)-aminobutyryl)-2(S)-indolinecarboxylique n.propylamide ; voie 3

R₁ = CH₂CH₃ ; R₂ = H ; n = 0 et m = 1 ; R₃ = 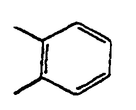 (R₆ = H) ; R = R' = H ; R₄ = CO-NH-R₅ ; R₅ = n.C₃H₇ ;

### a/ Préparation de l'acide N-benzyloxycarbonyl-2(S)-indolinecarboxylique n.propylamide

On procède comme à l'exemple 14 a/ à partir de l'acide N-benzyloxycarbonyl-2(S)-indolinecarboxylique préparé comme à l'exemple 16 a/ et de n.propylamine. On obtient un solide blanc que l'on recristallise dans du CCl₄.
P.F. = 185-186°C
RMN-¹H (CDCl₃, ppm) : 7,0-7,25 (9H, m, Ar-CH) ; 5,20 (2H, s, Ph-CH₂) ; 4,90 (1H, m, NCHCO) ; 3,90 (2H, m, CH₂indoline) ; 3,0-3,5 (NCH₂propyle) ; 0,9-1,8 (5H, m, CH₃CH₂).

### b/ Préparation de l'acide 2(S)-indolinecarboxylique n.propylamide

On effectue une hydrogénation du composé obtenu à l'étape a/ dans les conditions de son analogue n.butylamide de l'exemple 14 b/. Après évaporation du méthanol, on obtient un solide blanc qui est suffisamment pur sans recristallisation.
RMN-¹H (CDCl₃, ppm) : 6,85-7,2 (4H, ArCH) ; 4,3 (1H, m, NCHCO) ; 2,9-3,8 (4H, m, NCH₂, CH₂indoline) ; 1,5 (2H, m, NCH₂CH₂) ; 0,9 (3H, t, CH₃).

### c/ Préparation de l'acide 1-(N-benzyloxycarbonyl-2(S)-aminobutyryl)-2(S)-indolinecarboxylique n.propylamide

On dissout, sous azote, dans du tétrahydrofuranne (50 ml) 0,439 g (1,85 mmole) d'acide N-benzyloxycarbonyl-2(S)-amino-butyrique préparé comme à l'exemple 3 a/ et on refroidit dans un bain glace/eau. On ajoute 0,213 g (1,85 mmole) de N-éthyl-morpholine puis 0,253 g (1,85 mmole) de chloroformiate d'isobutyle. Après 45 minutes, on ajoute 0,378 g (1,85 mmole) du composé obtenu à l'étape b/ et on maintient l'agitation pendant 12 heures. On verse la suspension dans du CH₂Cl₂ (100 ml), on lave avec de l'acide citrique 10 % (3 x 100 ml) puis avec NaHCO₃ 5 % (3 x 100 ml). Après sèchage et évaporation, on obtient un solide blanc qui cristallise dans CCl₄.
P.F. = 176-177°C
RMN-¹H (CDCl₃, ppm) : 7,0-7,25 (9H, m, H-Ar) ; 5,0 (2H, s, CH₂benzyloxycarbonyle) ; 4,8 (1H, m, αH) ; 4,1 (1H, αH) ; 3,3 (2H, m, CH₂cycle) ; 3,0 (2H, m, CH₂N) ; 1,0-2,0 (4H, m, CH₃CH₂CH₂N CH₂aminobutyryle) ; 0,70-1,00 (CH₃aminobutyryle CH₃propyle).

### d/ Préparation du composé en titre sous forme d'hydrate d'oxalate

On procède à une hydrogénation du composé issu de l'étape c/ dans les conditions de l'exemple 5 c/ pour la N-benzyloxy-carbonyl-2(S)-aminobutyryl-L-proline [2(S)-méthyl)] n.butyl-amide. On obtient le produit désiré sous la forme de son sel d'oxalate qui est suffisamment pur sans recristallisation.
P.F. = 130-131°C
RMN-¹H (CD₃OD, ppm) : 7,70 (1H, m, NH) ; 6,55-6,90 (4H, m, H-Ar) ; 4,20-4,60 (2H, m, 2 x αH) ; 2,90-3,40 (2H, m, CH₂ cycle) ; 2,50-2,80 (CH₂N) ; 1,40-1,80 (2H, m, CH₂CH₃propyle) ; 1,00 (2H, m, CH₂aminobutyryle) ; 0,40-0,70 (6H, m, CH₃aminobutyryle CH₃propyle).
Microanalyse : C₁₆H₂₃N₃O₂.(COOH)₂.1,25H₂O
Trouvé : C = 53,84 % ; H = 6,68 % ; N = 10,62 % ;
Calculé : C = 53,79 % ; H = 6,90 % ; N = 10,45 % ;

### Exemple 16 : Préparation de l'acide 1-(2(S)-aminobutyryl)-2(S)-indolinecarboxylique méthylamide ; voie 3

R₁ = CH₂CH₃ ; R₂ = H ; n = 0 et m = 1 ; R = R' = H ; R₃ = 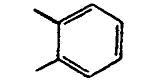 (R₆ = H) ; R₄ = CO-NH-R₅ ; R₅ = CH₃ ;

### a/ Préparation de l'acide N-benzyloxycarbonyl-2(S)-indolinecarboxylique

On refroidit dans un bain glace-eau une solution de 20 g (122,56 mmoles) d'acide 2(S)-indoline carboxylique dans NaOH 2N (62 ml) et on agite avec un agitateur magnétique puissant. On y ajoute alternativement 20,0 ml (140,09 mmoles) de chloroformaite de benzyle et 68 ml de NaOH 2N en environ 10 portions en 60 minutes. On maintient la température du mélange réactionnel entre 0° et 5°C en controllant la vitesse d'addition des réactifs. On ajoute ensuite 50 ml de NaOH et on maintient le mélange à la température ambiante pendant 30 minutes. On lave la solution alcaline à l'éther (4 x 150 ml) puis on l'acidifie par addition de HCl 5N (80 ml, pH < 2). Une masse cristalline précipite après une nuit à la température ambiante. On la filtre et on la lave à l'eau distillée (500 ml). Après sèchage sur P₂O₅, on obtient le produit désiré.
P.F. = 117-118°C
RMN-¹H (400 MHz, CDCl₃, ppm) : 8,30-6,90 (10H, m, ArH et COOH) ; 5,45-5,14 (2H, m, CH₂-Ph) ; 5,08-4,92 (1H, m, α-H) ; 3,65-3,46 (1H, m, CH₂indoline) ; 3,30-3,15 (1H, m, CH₂indoline).
Spectre de masse (FAB) : m/e (% intensité) = 342 ((M-H + 2Na)⁺, 0,2) ; 320 ((M + Na)⁺, 4,3) ; 298 (MH⁺, 5,7) ; 297 (M⁺ ; 9,3) ; 91 (C₇H₇⁺, 100).

### b/ Préparation de l'acide N-benzyloxycarbonyl-2(S)-indolinecarboxylique méthylamide

On dissout dans 90 ml de tétrahydrofuranne sec, 4,00 g (13,45 mmoles) d'acide obtenu au a/ et on ajoute 1,71 ml (13,45 mmoles) de 4-éthylmorpholine à 0°C. On refroidit cette solution à -5°C et on ajoute, goutte à goutte, 0,62 ml (4,78 mmoles) de chloroformiate d'isobutyle. Après 30 minutes à -5°C, on sature la solution avec de la méthylamine (gaz). On laisse le mélange une nuit à la température ambiante puis on ajoute 150 ml de dichlorométhane et on lave cette solution avec 3 x 150 ml d'acide citrique 10 %. Après avoir secouer énergiquement, on sépare les phases, on lave la phase organique avec 2 x 150 ml de NaHCO₃ 5 %. Après séchage sur MgSO₄, on purifie le produit brut par lavage avec CCl₄ chaud. On obtient un solide blanc correspondant à l'amide pur.
P.F. = 210-211°C
RMN-¹H (400 MHz, CDCl₃, ppm) : 7,75 (1H, s large, COOH) ; 7,45-6,95 (9H, m, Ar-H) ; 5,90 (1H, s large, NH) ; 5,37-5,10 (2H, m, CH₂-Ph) ; 4,93 (1H, dd, α-H) ; 3,62-3,16 (2H, m, CH₂indoline) ; 2,71 (3H, s, CH₃).
Spectre de masse (FAB) : m/e (% intensité) : 311 (MH⁺, 11,3) ; 310 (M⁺, 11,4) ; 91 (C₇H₇⁺, 100).

### c/ Préparation de l'acide N-benzyloxycarbonyl-2(S)-indolinecarboxylique méthylamide

On hydrogène sous 60 psi pendant 3 heures, une solution de 2,91 g (9,38 mmoles) du composé obtenu à l'étape b/ dans 120 ml de méthanol en présence 1,20 g de palladium 10 % sur charbon activé (humide). Après filtration et concentration sous vide, l'huile résultante crrstallise après sèchage une nuit sur P₂O₅.
P.F. = 125-130°C
RMN-¹H (200 MHz, CDCl₃, ppm) : 7,22-7,03 (3H, m, NHamide et 2 ArH) ; 6,82 (1H, t, J = 7,4 Hz, ArH) ; 6,73 (1H, d, J = 7,8 Hz, ArH) ; 4,42 (1H, t, α-H J = 9,4 Hz) ; 4,14 (1H, s large, NH) ; 3,65 -3,55 (1H, m, CH₂) ; 3,14-3,02 (1H, m, CH₂) ; 2,84 (3H, d, J = 15,0 Hz, CH₃).
Spectre de masse (FAB) : m/e = 177 (MH⁺, 42,4) ; 176 (M⁺, 33,2) ; 118 (100,0).

### d/ Préparation de l'acide 1-(N-benzyloxycarbonyl-2(S)-aminobutyryl)-2(S)-indolinecarboxylique méthylamide

On dissout, sous argon, dans 40 ml de tétrahydrofuranne sec, 1,73 g (8,51 mmoles) d'acide N-(tert.butoxycarbonyl)-2(S)-amino-butyrique préparé selon l'exemple 2 a/ et on ajoute 1,07 ml (8,51 mmoles) de 4-éthylmorpholine à 0°C puis 1,11 ml (8,51 mmoles) de chloroformiate d'isobutyle à -5°C. Un précipité blanc se forme. Après 30 minutes d'agitation à -5°C, on ajoute 1,50 g (8,51 mmoles) du composé obtenu à l'étape c/. On agite alors le mélange à 0°C pendant 40 minutes puis une nuit à la température ambiante. On ajoute ensuite à cette solution, 100 ml de dichlorométhane puis on la lave avec 3 x 50 ml d'acide citrique 10 %. Après avoir secouer énergiquement, on sépare les phases, on lave la phase organique avec 2 x 50 ml de NaHCO₃ 5 %. Après sèchage sur MgSO₄, on élimine le solvant sous vide et on purifie le produit brut obtenu par recristallisation dans CCl₄.
P.F. = 196-197°C
RMN-¹H (400 MHz, CDCl₃, ppm) : 8,12 (1H, d, NH) ; 7,45-6,95 (9H, m, ArH) ; 5,30 (1H, d, NH) ; 5,25-5,0 (2H, m, CH₂Ph) ; 4,82 (1H, t, α-H) ; 4,14 (1H, m, α-H) ; 3,60-3,45 (2H, m, CH₂indoline) ; 2,80 (3H, d, CH₃méthylamide) ; 2,0-1,55 (2H, m, CH₂aminobutyryle) ; 1,03 (1H, t, CH₃aminobutyryle).
Spectre de masse (FAB) : m/e (% intensité) = 396 (MH⁺, 9,9) ; 91 (C₆H₅⁺, 100).

### e/ Préparation du composé en titre sous forme d'hydrate d'oxalate

On dissout 2,15 g (5,44 mmoles) du composé obtenu à l'étape d/ dans 200 ml de méthanol et on ajoute 0,85 g de palladium 10 % sur charbon activé (humide). On hydrogène le mélange réactionnel sous 60 psi pendant 3 heures. On filtre la solution et on ajoute 1,3 équivalent (0,64 g) d'acide oxalique (7,07 mmoles) puis on concentre la solution sous vide. On ajoute de l'éthanol puis de l'éther (rapport 1:50) et on obtient un produit cristallisé. Après filtration et levage au diéthyléther, on sèche le solide sous vide pendant 24 heures sur P₂O₅ à 35°C. On obtient le produit désiré.
P.F. = 138-139°C
RMN-¹H (400 MHz, CD₃OD, ppm) : 8,20 et 8,02 (1H, d, ArH) ; 7,36-7,05 (3H, m, ArH) ; 5,18-5,02 et 4,70-4,62 (1H, m, αHindoline) ; 5,01-4,94 et 4,28-4,22 (1H, m, αHaminobutyryle) ; 3,92-3,00 (2H, m, CH₂indoline) ; 2,78 et 2,73 et 2,33 (3H, s, CH₃amide) ; 2,26-2,12 et 2,12-2,86 (2H, m, CH₂aminobutyryle) ; 1,16 et 1,07 et 1,04 (3H, t, CH₃aminobutyryle).
Spectre de masse (FAB) : m/e (% intensité) : 262 (MH⁺, 43,0) ; 154 (100) ; 137 (65,1) ; 136 (81,6).
Spectre IR (disque KBr, cm⁻¹) : 3440 (vague, NH) ; 3289 (m, NH) ; 2969 (m, CH) ; 2935 (m, CH) ; 1668 (s, C=O).
Microanalyse : C₁₄H₁₉N₃O₂.(COOH)₂. 1,25 H₂O
Trouvé : C = 51,55 % ; H = 6,05 % ; N = 11,28 % ;
Calculé : C = 51,40 % ; H = 6,34 % ; N = 11,24 % ;

### Exemple 17 : Préparation de l'acide 1-(2(S)-aminobutyryl)-2(S)-indolinecarboxylique éthylamide ; voie 3

R₁ = CH₂CH₃ ; R₂ = H ; n = 0 et m = 1 ; R₃ = 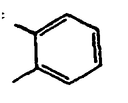 (R₆ = H) ; R = R' = H ; R₄ = CO-NH-R₅ ; R₅ = CH₂CH₃ ;

### a/ Préparation de l'acide N-benzyloxycarbonyl-2(S)-indolinecarboxylique éthylamide

On procède comme à l'exemple 16 b/ en utilisant de l'éthylamine (gas). On purifie le produit brut obtenu par recristallisation dans CCl₄. Par filtration, on obtient l'amide pur sous la forme d'un solide blanc.
P.F. = 201,0-201,5°C
R_{f} = 0,58 (acétate d'éthyle)
RMN-¹H (400 MHz, CDCl₃, ppm) : 7,70 (1H, s large,COOH) ; 7,45-6,98 (9H, m, ArH) ; 5,90 (1H, s large, NH) ; 5,35-5,18 (2H, m, CH₂benzyle); 4,90 (1H, dd, α-H) ; 3,64-3,04 (4H, m, CH₂indolinyle et NH-CH₂) ; 0,99 (3H, s large,CH₃).

### b/ Préparation de l'acide 2(S)-indolinecarboxylique éthylamide

On procède comme à l'exemple 16 c/ à partir du composé obtenu à l'étape a/. L'huile résultante cristallise après sèchage sur P₂O₅ pendant une nuit. On obtient le produit désiré.
P.F. = 92-95°C
R_{f} = 0,28 (acétate d'éthyle)
RMN-¹H (400 MHz, ppm) : 7,22-7,00 (3H, m, NHamide et ArH) ; 6,82 (1H, t, ArH) ; 6,73 (1H, d, ArH) ; 4,42 (1H, t, α-H) ; 4,14 (1H, s large, NH) ; 3,65-3,50 (1H, m, CH₂indoline) ; 3,45-3,20 (2H, m, CH₂CH₃) ; 3,14-3,02 (1H, m, CH₂indoline) ; 1,15 (3H, t, CH₃).

### c/ Préparation de l'acide 1-(N-benzyloxycarbonyl-2(S)-aminobutyryl)-2(S)-indolinecarboxylique éthylamide

On procède comme à l'exemple 16 d/ en utilisant de l'acide N-benzyloxycarbonyl-2(S)-aminobutyrique préparé comme à l'exemple 3 a/ et le composé issu de l'étape b/. On obtient l'amide pur sous la forme d'un solide blanc.
P.F. = 184,5-185°C
RMN-¹H (400 MHz, CDCl₃, ppm) : 8,15 (1H, d, Haromatique J = 7,9 Hz) ; 7,44-7,08 (8H, m, ArH) ; 7,04 (s large, NHamide) ; 5,37 (1H, d, NHamide) ; 5,0-5,2 (2H, m, PhCH₂) ; 4,84 (1H, m, α-H) ; 4,18 (1H, m, α-H) ; 3,62-3,48 (2H, m, CH₂indoline) ; 3,34-3,14 (2H, m, CH₂éthylamide) ; 2,10-1,60 (2H, m, CH₂aminobutyryle) ; 1,20-0,98 (3H, m, CH₃).

### d/ Préparation du composé en titre

On procède comme à l'exemple 16 c/ pour le composé issu de l'étape c/. Après sèchage du solide sur P₂O₅ pendant 24 heures à 35°C, on obtient le produit désiré.
P.F. = 115-120°C
RMN-¹H (400 MHz, D₂O, ppm) : 7,06-8,08 (4H, m, ArCH) ; 5,12 (1H, m, αH) ; 3,91 (1H, m, αH) ; 3,78-3,50 (1H, m, CH₂Nindoline) ; 3,25-2,96 (3H, m, CH₂Nindoline et NH-CH₂) ; 2,25-1,90 (2H, m, CH₂aminobutyryle) ; 1,28-0,98 (m, CH₃aminobutyryle et CH₃éthylamide).
Spectre de masse (FAB) : m/e (% intensité) : 276 (MH⁺, 49,9) ; 191 (80,8) ; 118 (100) ; 89 (16,0) ; 77 (37,2) ; 58 (74,1) ; 51 (21,3) ; 50 (22,7) ; 46 (19,9) ; 39 (28,2) ; 31 (29,5).
Spectre IR (Disque KBr, cm⁻¹) : 3395 (m, amide) ; 3057 (m, NH₃⁺) ; 1718 et 1669 (s, C=O).
Microanalyse : C₁₅H₂₁N₃O₂.(COOH)₂.H₂O
Trouvé : C = 53,09 % ; H = 6,54 % ; N = 10,84 % ;
Calculé : C = 53,26 % ; H = 6,57 % ; N = 10,96 % ;

### Exemple 18 : Préparation de l'acide 1-(2(S)-aminobutyryl)-2(R/S)-(5-méthoxy)indolinecarboxylique n-butylamide ; voie 7

R₁ = CH₂CH₃ ; R₂ = H ; n = 0 et m = 1 ; R₃ = 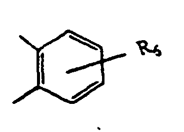 R₆ = OCH₃ ; R = R' = H ; R₄ = CO-NH-R₅ ; R₅ = n. C₄ H₉

### a/ Préparation de l'ester éthylique de l'acide 5-méthoxy-2-indolecarboxylique : composé XXXXII

On traite de la p-Anisidine (61,5 g, 499 mmoles) dans 500 ml d'HCl 15 % contenant 500 g de glace par adjonction goutte à goutte pendant 5 minutes d'une solution de nitrite de sodium (37,5 g, 543 mmoles) dans l'eau (100 ml). On ajoute 3 g de charbon activé et on agite le mélange 10 minutes. Après filtration, le filtrat est rapidement ajouté à du 2-méthylacétoacétate d'éthyle (80 g, 554 mmoles) et de l'acétate de sodium anhydre (410 g, 4,998 moles), dans de l'éthanol (500 ml) contenant 500 g de glace. Le mélange est agité 2 heures puis extrait au toluène (3 x 500 ml). Les extraits sont séchés (Na₂SO₄) et le solvant éliminé pour obtenir du 1-(p-méthoxyphénylazo)-1-méthyl acétoacétate d'éthyle brut sous forme d'une huile. Cette huile est ajoutée à une solution froide de HCl dans l'éthanol (38-40 % en poids). Le mélange est porté et maintenu au reflux pendant 40 minutes. Le mélange est agité 2 heures à température ambiante, additionné d'eau (150 ml) puis réfrigéré. Le produit se sépare sous forme de cristaux orange qui sont reccueillis et lavés à l'éthanol froid puis à l'eau.
RMN-¹H (CDCl₃, ppm) : 9,03 (1H, s large, NH) ; 7,31 (1H, dd, ArCH) ; 7,14 (1H, t, ArCH) ; 7,07 (1H, d, ArCH) ; 6,99 (1H, dd, ArCH) ; 4,40 (2H, q, CH₂) ; 3,84 (3H, s, CH₃O) ; 1,43 (3H, t, CH₃CH₂).

### b/ Préparation de l'acide 5-méthoxy-2(R/S)-indoline carboxylique n.butylamide : composé XXXXIV.

Le produit de l'étape a/ (10 g, 53 mmoles) est dissout dans du méthanol (100 ml) et traité par du magnésium (3,6 g, 150 mmoles). La température de réaction est maintenue à 15-20°C par refroidissement externe et le mélange agité pendant la nuit. Puis il est dilué par 300 ml d'eau et extrait par du chlorure de méthylène (4 x 100 ml). Les extraits sont séchés (Na₂SO₄) et le solvant éliminé pour donner l'ester méthylique de l'acide 5-méthoxy-2(R/S)-indolinecarboxylique (composé XXXXIII). Celui-ci est dissout dans la butylamine (40 ml) et amené au reflux sous azote pendant 3 heures. L'amine en excès est éliminée et le résidu cristallisé à partir d'éther pour donner le composé XXXXIV.
P.F. 119-121°C.
RMN-¹H (CDCl₃, ppm) : 7,2 (1H, s, NHCO) ; 6,6-6,7 (3H, m, ArCH) ; 4,37 (1H, t, NHCHCO) ; 3,90 (1H, s, NH indoline) ; 3,73 (3H, s, OCH₃) ; 3,54 (1H, dd, ArC(H)H) ; 3,27 (2H, m, NHCH₂) ; 3,05 (1H, dd, Ar-C(H)H) ; 1,2-1,5 (4H, m, CH₃(CH₂)₂), 0.89 (3H, t, CH₃(CH₂)₂).

### c/ Préparation de l'acide 1-(N-benzyloxycarbonyl)-2(S)-aminobutyryl)-2(R/S)-(5-méthoxy)indolinecarboxylique n.butylamide : composé XXXXV

2,37 g (10 mmoles) d'acide N-benzyloxycarbonyl-2(S)-amino-butyrique, obtenu selon l'exemple 3a/, et le composé obtenu par l'étape b/ (2,48 g, 10 mmoles) sont dissous dans du chlorure de méthylène anhydre et refroidit à 0°C. Le mélange est traité par de la triéthylamine (2,03 g, 20 mmoles) puis par le chlorure bis(2-oxo-3-oxazolidinyl)phosphinique (2,8 g, 10 mmoles). Le mélange réactionnel est agité pendant la nuit à température ambiante. Il est lavé par une solution d'acide citrique à 10 % (3 x 100 ml) puis une solution de bicarbonate de sodium à 5 % (3 x 100 ml), sèché (Na₂SO₄) et le solvant est éliminé. Le produit est purifié par chromatographie sur gel de silice, éluant acétate d'éthyle/pétrole 1:2.
RMN-¹H (CDCl₃, ppm) : 8,0 (0,5H, m, NH) ; 7,2-7,3 (5H, m, Ph) ; 6,6-7,1 (3,5H, m, NH et ArCH indoline) ; 4,7-5,7 (4H, m, PhCH₂, NH, NCHCO de l'acide butyrique) ; 4,0-4,3 (1H, m, NCHCO indoline) ; 3,78 (3H, s, OCH₃) ; 3,45 (1H, m, ArCH(H) indoline) ; 3,0-3,2 (3H, m, NHCH₂, ArCH(H) indoline) ; 0,7-2,0 (12H, 3 x CH₂, 2 x CH₃).
Spectre de masse (FAB) : m/e = 468 (M⁺ + 1).

### d/ Préparation du composé en titre sous la forme de sel d'oxalate

Le compose de l'étape c/ (2,8 g, 6 mmoles) est dissous dans 100 ml de méthanol contenant du palladium sur charbon (10 %, 0,8 g, 50 % d'humidité) et le mélange est hydrogéné à 40 psi pendant 2,5 heures. Le catalyseur est éliminé par filtration et le solvant éliminé. Le résidu est traité par l'acide oxalique (0,85 g, 9,4 mmoles) dans de l'éthanol (10 ml) puis de l'éther (500 ml) le produit précipite.
Microanalyse : C₁₈H₂₇N₃O₃.(COOH)₂.0,25H₂O :
Trouvé : C = 56,14 % ; H = 6,98 % ; N = 9,57 % ;
Calculé : C = 56,13 % ; H = 6,95 % ; N = 9,82 % ;
Spectre de masse (FAB) : m/e = 333 (M⁺)
RMN-¹H (DMSO-d₆, ppm) : 8,3-9,1 (ArCH masqué) ; 7,97
(1H, dd, CONH) ; 6,7-7,0 (2H, m, ArCH) ; 4,9-5,3 (1H, m, NCHCO indoline) ; 3,2-3,9 (5H, m, CH₃O, NCHCO butyle, ArCH(H)) ; 2,7-3,2 (3H, m, CONHCH₂, ArCH(H)) ; 1,0-2,0 (6H, m, CH₃CH₂, CH₃(CH₂)₂) ; 0,7-1,0 (2 x CH₃CH₂).

### e/ Séparation optique de l'acide 1-(2(S)-aminobutyryl)-2(R/S)-(5-méthoxy)indolinecarboxylique n-butylamide

L'acide 1-(N-benzyloxycarbonyl-2(S)-aminobutyryl)-2(R/S)-(5-méthoxy)indolinecarboxylique n.butylamide (composé XXXXV) est séparé en ses diastéréoisomères par chromatographie sur colonne sur gel de silice en utilisant comme éluant un mélange chlorure de méthylène/diéthyléther (10:1).

L'isomère (S,S) est élue en premier. Il est recueilli et recristallisé dans du méthanol puis le groupe protecteur benzyloxycarbonyle est éliminé par hydrogénation en présence de palladium sur charbon dans le méthanol pendant 2 heures et 30 minutes sous une pression d'hydrogène de 60 psi, comme décrit ci-dessus. Le catalyseur est éliminé par filtration et le résidu est traité avec de l'acide oxalique dans le méthanol puis l'éther pour conduire au sel d'oxalate.
P.F. = 185-190°C
Microanalyse : C₁₈H₂₇N₃O₃.1,4C₂H₂O₄.0,5H₂O :
Trouvé : C = 53,3 % ; H = 6,81 % ; N = 9,05 % ;
Calculé : C = 53,3 % ; H = 6,63 % ; N = 8,97 % ;
L'isomère (S,R) est élué en second. Il est traité d'une manière similaire à l'isomère (S,S) pour obtenir le sel d'oxalate.
P.F. = 95-105°C
Microanalyse : C₁₈H₂₇N₃O₃.1,35C₂H₂O₄ :
Trouvé : C = 54,4 % ; H = 6,55 % ; N = 9,45 % ;
Calculé : C = 54,1 % ; H = 6,58 % ; N = 9,24 % ;

### Exemple 19 : Préparation de l'acide 1-(2(S)-aminobutyryl)-2(R/S)-(6-méthoxy)indolinecarboxylique n-butylamide ; voie 7

R₁ = CH₂CH₃ ; R₂ = H ; n = 0 et m = 1 ; R₃ = 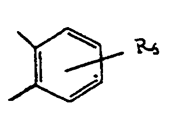 R₆ = OCH₃ ; R = R' = H ; R₄ = CO-NH-R₅ ; R₅ = n.C₄H₉

On procède comme à l'exemple 18. On obtient le composé en titre sous forme de sel d'oxalate.
Microanalyse : C₁₈H₂₇N₃O₃.(COOH)₂.H₂O :
Trouvé : C = 54,54 % ; H = 7,08 % ; N = 9,52 % ;
Calculé : C = 54,41 % ; H = 7,08 % ; N = 9,52 % ;
RMN-¹H (DMSO-d₆, ppm) : 8,5 (1H, b, NH) ; 7,3-8,2 (6H, m, NH₃⁺,ArCH, H₂O) ; 7,0-7,2 (1H, m, ArCH) ; 6,5-6,7 (1H, m, ArCH) ; 5,05 (1H, dd, NCHCOindoline) ; 3,2-4,1 (5H, m, CH₃O, ArCH(H), NCHCO butyryle) ; 2,7-3,2 (3H, m, NCH₂, ArCH(H)) ; 1,5-2,0 (2H, m, CH₂) ; 1,0-1,5 (4H, m, 2 x CH₂) ; 0,7-1,0 (6H, m, 2 x CH₃).

### Exemple 20 : Préparation de l'acide 1-(2(S)-aminobutyryl)-2(R/S)-(5-fluoro)indolinecarboxylique n-butylamide ; voie 7

R₁ = CH₂CH₃ ; R₂ = H ; n = 0 et m = 1 ; R₃ = 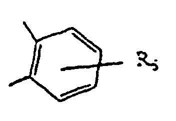 R₆ = F ; R = R' = H ; R₄ = CO-NH-R₅ ; R₅ = n.C₄H₉

### a/ Préparation de l'ester éthylique de l'acide (5-fluoro)-2-indole carboxylique : composé XXXXII

On procède comme à l'exemple 18a/ pour préparer le 1-(p-fluorophénylazo)-1-méthyl acétoacétate d'éthyle à partir de p-fluoroaniline et de 2-méthylacétoacétate d'éthyle. On le dissout dans 270 ml d'acide acétique contenant 15 ml d'acide sulfurique, puis le mélange est porté au reflux pendant 15 minutes. Après refroissement à la température ambiante, on ajoute 500 ml d'eau et on extrait le mélange à l'éther (500 ml) et au chlorure de méthylène (500 ml). Les extraits mélangés sont lavés à l'eau (1000 ml) et avec une solution de carbonate de potassium à 10% (300 ml), séchés et le solvant éliminé. Le produit est alors obtenu par cristallisation dans l'éthanol.
RMN-¹H (CDCl₃, ppm) : 7,0-7,4 (4H, m, ArCH) ; 4,41 (2H, q, CH₂) ; 1,40 (3H, t, CH₃).

### b/ Préparation de l'acide 5-fluoro-2(R/S)-indoline carboxylique n.butylamide : composé XXXXIV.

On procède comme à l'exemple 18 b/.
P. F. : 93-5°C.
RMN-¹H (CDCl₃, ppm) : 7,12 (1H, b, NH) ; 6,6-6,9 (3H, m, ArCH) ; 4,42 (1H, t, NHCHCO) ; 4,03 (1H, b, NH) ; 3,56 (1H, dd, ArCH(H)) ; 3,27 (2H, m, CH₂NH) ; 3,05 (1H, m, ArCH(H)) ; 1,2-1,7 (CH₃(CH₂)₂) ; 0,90 (3H, t, CH₃).
Spectre de masse (FAB) : m/e = 237 (M⁺ + 1).

### c/ Préparation de l'acide 1-(N-(benzyloxycarbonyl)-2(S)-aminobutyryl)-2(R/S)-(5-fluoro)indolinecarboxylique n.butylamide : composé XXXXV

On procède comme à l'exemple 18c/, puis on purifie le produit par une chromatographie sur gel de silice (éluant : acétate d'éthyle/pétrole 1:1).
P.F. 192-3°C.
RMN-¹H (CDCl₃, ppm) : 8,05 (1H, b, NH) ; 7,2-7,4 (5H, m, Ph) ; 6,8-7,1 (4H, m, NH, ArCH indoline) ; 4,7-5,4 (4H, m, PhCH₂, 2 x NCHCO) ; 3,5 (2H, m, ArCH₂ indoline) ; 3,2 (2H, m, CH₂NHCO) ; 0,7-2,0 (12H, m, CH₃CH₂ et CH₃(CH₂)₂).

### d/ Préparation du composé en titre sous forme de sel d'oxalate

On procède comme à l'exemple 18 d/.
Microanalyse : C₁₇H₂₄FN₃O₂.(COOH)₂.0,75H₂O :
Trouvé : C = 53,58 % ; H = 6,60 % ; N = 10,25 % ;
Calculé : C = 53,70 % ; H = 6,52 % ; N = 9,89 % ;
RMN-¹H (DMSO-d₆, ppm) : 8,60 (1H, b, NH) ; 7,8-8,1 (1H, m, ArCH) ; 7,2 -7,8 (4H, b, NH₃⁺ et H₂O) ; 6,8-7,2 (2H, m, ArCH) ; 5,0-5,2 (1H, m, NHCHCO indoline) ; 2,6-3,8 (5H, m, NCHCO butyryle, ArCH₂, CH₂NHCO) ; 1,5-2,0 (2H, m, CH₂) ; 1,0-1,5 (4H, m, 2 x CH₂) ; 0,6-1,0 (6H, m, 2 x CH₃).
Spectre de masse (FAB) : m/e = 322 (M⁺+1).

### e/ Séparation optique de l'acide 1-(2(S)-aminobutyryl)-2(R/S)-(5-fluoro)indolinecarboxylique n-butylamide

On sépare l'acide 1-(N-(benzyloxycarbonyl)-2(S)-aminobutyryl)-2(R/S)-(5-fluoro)indolinecarboxylique en ses diastéréoisomères par chromatographie sur colonne sur gel de silice en utilisant comme éluant un mélange éther de pétrole (P.E. = 60-80°C)/éthanoate d'éthyle (2:1).

L'isomère (S,R) est élue en premier. Il est recristallisé dans un mélange pétrole/diéthyléther puis déprotégé par hydrogénation comme indiqué à l'exemple 18 e/. On obtient l'isomère (S,R) sous forme de sel d'oxalate.
P.F. = 173-177°C
Analyse : C₁₇H₂₄FN₃O₂.C₂H₂O₄.H₂O :
Trouvé : C = 52,9 % ; H = 6,37 % ; N = 9,52 % ;
Calculé : C = 53,14 % ; H = 6,57 % ; N = 9,78 % ;
L'isomère (S,S) est élué en second et traité d'une manière analogue au premier isomère pour sa purification et sa déprotection. On obtient l'isomère (S,S) également sous forme de sel d'oxalate.
P.F. = 105-110°C
Microanalyse : C₁₇H₂₄FN₃O₂.C₂H₂O₄.H₂O :
Trouvé : C = 52,9 % ; H = 6,37 % ; N = 9,70 % ;
Calculé : C = 53,14 % ; H = 6,57 % ; N = 9,78 % ;

### Exemple 21 : Préparation de l'acide 1-(2(S)-aminobutyryl)-2(R/S)-(5-benzyloxy)indolinecarboxylique n-butylamide ; voie 7

R₁ = CH₂CH₃ ; R₂ = H ; n = 0 et m = 1 ; R₃ =

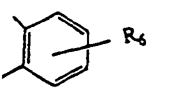

R₆ = OCH₂Ph ; R = R' = H ; R₄ = CO-NH-R₅ ; R₅ = n.C₄Hg

On procède comme à l'exemple 18.

L'acide 1-(N-(tert-butoxycarbonyl)-2(S)-aminobutyryl)-2(R/S)-(5-benzyloxy)indolinecarboxylique n-butylamide (3,56 g, 7 mmoles) est dissout dans l'acétate d'éthyle (20 ml) puis traité par HCl dans de l'acétate d'éthyle (3 M, 25 ml). Le mélange est agité à température ambiante pendant 1h et le solide formé est recueilli, lavé et séché. Le composé en titre est isolé sous forme de chlorhydrate.
Microanalyse : C₂₄H₃₁N₃O₃.HCl.H₂O
Trouvé . C = 63,28 % ; H = 6,92 % ; N = 9,20 % ;
Calculé : C = 63,36 % ; H = 7,31.% ; N = 9,24 % ;
RMN-¹H (DMSO-d₆, ppm) : 8,7 (0,9H, s large, NH d'un diastéréoisomère) ; 8,5 (s large, NH₃⁺ et H₂O) ; 8,07 (0,1H, NH du second diastéréoisomère) ; 7,93 (1H, d, ArCH) ; 7,2-7,5 (5H, m, ArCH) ; 6,93 (1H, s, ArCH) ; 6,84 (1H, dd, ArCH) ; 5,35 (0,9H, dd, NCHCO indoline d'un diastéréoisomère) ; 4,9-5,2 (2,1H, PhCH₂, NCHCO indoline du second diastéréoisomère) ; 4,7 (0,1H, m, NCHCO butyryle d'un diastéréoisomère) ; 3,92 (0,9H, m, NCHCO butyryle du second diastéréoisomère) ; 3,4-3,7 (1H, m, ArCH(H) indoline) ; 2,8-3,1 (3H, m, ArCH(H) indoline, NCH₂) ; 1,5-2,0 (2H, m, CH₂) ; 1,05-1,5 (4H, m, 2 x CH₂) ; 0,7-1,0 (6H, m, 2 x CH₃).
Spectre de masse (FAB) : m/e = 410 (M⁺+1).

### Exemple 22 : Préparation de l'acide 1-(2(S)-aminobutyryl)-2(S)-[(3aS,7aS)-perhydro]indolinecarboxylique n-butylamide ; voie 3

R₁ = CH₂CH₃ ; R₂ = H ; n = 0 et m = 1 ; R₃ = 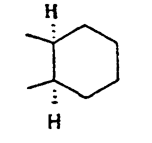 R = R' = H ; R₄ = CO-NH-R₅ ; R₅ = n.C₄H₉

### a/ Préparation de l'acide 2(S)-[(3aS,7aS)perhydro]indoline carboxylique sous forme de sel de N-acétate.

Une solution d'acide 2(S)-indolinecarboxylique (2,5 g, 15,32 mmoles) dans l'acide acétique contenant PtO₂ (1g) est hydrogénée à 55°C pendant 2 heures à 60 psi. Le catalyseur est éliminé par filtration et le solvant éliminé sous vide pour donner un résidu brut qui est cristallisé à partir de diéthyl éther pour donner, après filtration et lavage à l'éther, un solide blanc.
P.F. 247-248°C.
RMN-¹H (200 MHz, CD₃OD, ppm) : 4,05-3,90 (1H, m, H junction) ; 3,70-3,55 (1H, m, αH) ; 2,45-2,20 (2H, m, CH₂-COOH) ; 2,15-1,25 (12H, m, CH₂cyclohexyle, H junction et CH₃ AcOH).

### b/ Préparation de l'acide N-(benzyloxycarbonyl)-2(S)-[(3aS,7aS)perhydro]indolinecarboxylique.

Une solution du composé obtenu par l'étape a/ (1,28 g, 5,58 mmoles) dans NaOH 2N (30 ml) est refroidie dans un bain eau-glace et agitée vigoureusement. Du chloroformiate de benzyle (0,95 ml, 6,65 mmoles) et de la soude 2N (30 ml) sont ajoutés (en environ 10 parties). La température du mélange réactionnel est maintenue entre 5 et 10°C en jouant sur la vitesse d'addition des réactifs. 40 ml de soude 2N sont ajoutés et le mélange est maintenu à température ambiante pendant 30 minutes. La solution alcaline est lavée avec de l'éther (4 x 50 ml) puis acidifiée à pH < 2 par addition de HCl 5N. La solution est extraite par CH₂Cl₂ et les extraits sont séchés sur MgSO₄, filtrés et évaporés sous vide. L'huile obtenue est séchée sous vide sur P₂O₅ pour obtenir le produit désiré.
RMN-¹H (200 MHz, CDCl₃, ppm) : 7,14 (5H, s large, ArCH) ; 5,05 (2H, s large, H benzyle) ; 4,20-4,10 (1H, m, H junction) ; 4,00-3,75 (1H, m, αH) ; 2,20-1,00 (11H, m, CH₂ cyclohexyle, H junction, CH₂COOH).

### c/ Préparation de l'acide N-benzyloxycarbonyl-2(S)-[(3aS,7aS)perhydro]indolinecarboxylique n.butylamide

On procède comme à l'exemple 11 étape c/ en utilisant le produit obtenu à l'exemple 22 étape b/.

On dissout 1,40 g du produit issu de l'étape précédente (4,61 mmoles) dans 50 ml de tétrahydrofuranne anhydre et 0,58 ml de 4-éthylmorpholine (4,61 mmoles) sont ajoutés à une température de 0°C. Cette solution est refroidie à -5°C et 0,60 ml d'isobutylchloroformiate (4,61 mmoles) sont ajoutés goutte à goutte. Après 30 minutes à -5°C, 0,46 ml de butylamine (4,61 mmoles) sont ajoutés à la solution puis le mélange est laissé durant une nuit à température ambiante. On ajoute alors 70 ml de dichlororométhane et cette solution est lavée avec 3x50 ml d'acide citrique 10%. Après agitation vigoureuse, les phases sont séparées. La phase organique est lavée avec 2x50 ml de NaHCO₃ 5%. Après séchage sur MgSO₄, la solution est évaporée sous vide et on obtient l'amide brut qui est utilisé sans autre purification.
RMN-¹H (200 MHz, CDCl₃, ppm) : 7,30 (5H, s large, ArCH) ; 5,05 (2H, s large, H benzyle) ; 4,20 (1H, m, αH) ; 4,00-3,80 (1H, m, H junction) ; 3,80-3,60 (1H, m, NH) ; 3,20-3,00 (2H, m, CH₂-NH butylamide) ; 2,30-1,00 (15H, m, CH₂ cyclohexyle, CH₂-CH₂-BuNH, H junction, CH₂-COOH) ; 1,00-0,80 (6H, m, CH₃ butylamide).

### d/ Préparation de l'acide 2-[(2S,3aS,7aS)perhydro]indoline carboxylique n. butylamide

Le produit de l'étape c/ (1,70 g, 4,54 mmoles) en solution dans 200 ml de méthanol est hydrogéné à température ambiante pendant 2 heures 30 minutes à 60 psi en utilisant comme catalyseur 0,6 g de Pd/C (10%, humide). L'amide est isolé sous forme d'huile.
RMN-¹H (200 MHz, CDCl₃, ppm) : 7,80 (1H, s large, NH) ; 3,90-3,80 (1H, m, αH) ; 3,50-3,00 (3H, m, -CH₂-NH-BuNH-, H junction) ; 3,00-1,05 (15H, m, -CH₂- cyclohexyle, H junction, -CH₂- butylamide) ; 0,95 (3H, t, CH₃ butylamide).

### e/ Préparation de l'acide 1-(N-benzyloxycarbonyl)-2(S)-aminobutyryl)-2(S)-[(3aS,7aS) perhydro]indolinecarboxylique n.butylamide.

On dissout de l'acide N-benzyloxycarbonyl-2(S)-aminobutyrique obtenu comme à l'exemple 3 a/ (0,95 g, 4 mmoles) dans 45 ml de tétrahydrofuranne anhydre et on rajoute 0,51 ml de 4-éthylmorpholine (4,01 mmoles) à 0°C. Cette solution est refroidie à -5°C et traitée goutte à goutte par de l'isobutylchloroformiate (0,52 ml, 4,01 mmoles). Après 30 minutes à -5°C, on ajoute au mélange le composé de l'étape d/ (0,90 g, 4,01 mmoles) en solution dans 5 ml de tétrahydrofuranne anhydre et le mélange ainsi obtenu est laissé durant une nuit à température ambiante. On ajoute ensuite 50 ml de dichlorométhane, puis le mélange est lavé avec 3 x 50 ml d'une solution d'acide citrique 10 %. La phase organique est lavée avec 2 x 50 ml de NaHCO₃ 5%. Après séchage sur MgSO₄, la solution est évaporée sous-vide et on obtient un produit brut que l'on purifie par chromatographie éclair sur silice en éluant avec un mélange acétate d'éthyle/éther (15:85 puis 35:65). Ceci permet d'obtenir le produit désiré sous forme d'huile. On la sèche durant une nuit à pression réduite sur P₂O₅.
RMN-¹H (200 MHz, CDCl₃, ppm) : 7,20 (5H, s large, ArCH) ; 6,90 (1H, s large, -NH-) ; 5,45 (1H, d, -NH-) ; 5,10-4,95 (2H, m, H benzyle) ; 4,50-4,30 (2H, m, H junction et αH) ; 4,10-4,00 (1H, m, αH) ; 3,20-3,00 (2H, m, -CH₂-butylamide) ; 2,65-1,00 (17H, m, -CH₂- cyclohexyle, H junction, -CH₂- aminobutyryle, -CH₂- butylamide) ; 1,00-0,90 (6H, m, CH₃ aminobutyryle et CH₃ butylamide).
Spectre de masse (FAB) : m/e (% intensité) : 444 (MH⁺, 13,5) ; 225 (56) ; 124 (100) ; 91 (91).

### f/ Préparation du composé en titre

Une solution du composé obtenu à l'étape e/ (0,66 g, 1,49 mmole) dans 100 ml de méthanol est hydrogénée pendant 3 heures en utilisant un catalyseur Pd/C (0,25 g, 10%, humide) à 60 Psi. Le catalyseur est éliminé par filtration et le solvant évaporé sous vide. On ajoute 0,18 g d'acide oxalique (1,98 mmole) à l'huile brute ainsi obtenue, dans de l'éthanol, puis on y ajoute de l'éther. Après cristallisation, filtration et séchage sous vide, on obtient le produit désiré.
P. F. : 168-9 °C (capillaire ouvert)
RMN-¹H (400 MHz, CD₃OD, ppm) : 4,30-4,25 (1H, m, αH) ; 4,05-3,85 et 3,40-3,35 (2H, m, αH et 7aH) ; 3,15-2,98 (2H, m, -CH₂-NH- BuNH₂) ; 2,33-2,15 (1H, m, 3aH) ; 2,05-1,50 (9H, m, -CH₂- aminobutyryle, -CH₂- perhydroindoline) ; 1,45-1,05 (7H, m, -CH₂-CH₂- BuNH₂ et H proline) ; 0,95-0,75 (6H, m, -CH₃ aminobutyryle et -CH₃ BuNH₂).
Spectre de masse (FAB) : m/e (% intensité) = 332 (MH + Na)⁺, 1) ; 310 (MH⁺, 100) ; 225 (75) ; 124 (66) ; 59 (C₇H₇⁺, 43).
IR (disque KBr, cm⁻¹) : 3342 (NH₃⁺, vague) ; 3049 (NH, vague) ; 2937 (CH, s) ; 2857 (CH, s) ; 1745 (C=O, s) ; 1682 (s) ; 1652 (s).
Microanalyse : C₁₇H₃₁N₂O₂.1,0C₂H₂O₄.0,3H₂O :
Trouvé : C = 56,34 % ; H = 8,37 % ; N = 10,33 % ;
Calculé : C = 56,36 % ; H = 8,36 % ; N = 10,38 % ;

### Exemple 23 : Préparation de l'acide 2-(2(S)-aminobutyryl)-1-(R/S)-isoindolinecarboxylique n-butylamide ; voie 4

R₁ = CH₂CH₃ ; R₂ = H ; n = 1 et m = 0 ; R₃ =

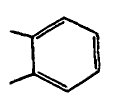

R = R' = H ; R₄ = CO-NH-R₅ ; R₅ = n.C₄H₉

### a; Préparation de l'acide o.(bromométhyl)phénylacétique.

On dissout de l'acide o-tolylacétique (40 g, 266 mmdles), du N-bromosuccinimide (52,147 g, 293 mmoles) et du peroxyde de benzoyle (0,174 g, catalytique) dans 300 ml de CCl₄. La suspension est portée au reflux et y est maintenue pendant 12 heures. Après refroidissement, les particules solides sont séparées par filtration et le CCl₄ est évaporé sous vide, laissant apparaître un solide blanc. Celui-ci est suffisamment pur sans recristallisation.
RMN-¹H (CDCl₃, ppm) : 7,25 (4H, m, ArH) ; 4,3 (2H, s, CH₂COOH) ; 3,7 (2H, s, CH₂Br).

### b/ Préparation de l'ester méthylique de l'acide o.(bromométhyl)phénylbromoacétique.

L'acide issu de l'étape a/ est dissout dans 44 ml de SOCl₂ et chauffé pendant 3 heures à 55°C. Le SOCL₂ est éliminé sous vide et, l'huile brune qui en résulte est traitée par 26 ml de Br₂ en irradiant par une lampe UV de 450 w pendant 2 heures. Après addition, le brome est évacué par distillation sous vide et le résidu noir est versé dans 305 ml de méthanol. Le méthanol est ensuite éliminé et il reste une huile noire qui est distillée sous une pression de 0,1 mm Hg. Les fractions de distallation qui sortent entre 120 et 130°C sont récupérées.
RMN-¹H (CDCl₃, ppm) : 7,0-7,50 (4H, m, ArH) ; 5,7 (1H, s, CH) ; 4,3 (2H, d, CH₂Br) ; 3,7 (3H, s, CH₃).

### c/ Préparation de l'ester méthylique de l'acide N-benzyl-1(R/S)-isoindoline carboxylique sous forme de chlorhydrate.

L'ester méthylique (46,4 g, 144 mmoles) issu de l'étape b/ est dissout dans du toluène anhydre (288 ml) sous azote. On ajoute 46,35 g de benzylamine (864 ml) en utilisant un mélange glace/sel. Le mélange est agité pendant 72 heures. Ensuite, l'hydrobromure d'amine est éliminé par filtration et le toluène évacué sous vide. On dissout dans l'éther l'huile jaune obtenue et on y fait buller du HCl pour obtenir le chlorhydrate.
P.F. : 160-161°C
RMN-¹H (D₂O, ppm) : 7,00 (9H, m, ArH) ; 5,20 (1H, s, αH) ; 4,10 (2H, d, CH₂ cycle) ; 4,00 (2H, d, CH₂Bn) ; 3,10 (3H, s, CH₃).

### d/ Préparation de l'ester méthylique de l'acide 1(R/S)-isoindoline carboxylique sous forme de chlorhydrate

Le produit de l'étape c/ (5 g, 16mmoles) est dissout dans 100 ml d'éthanol et on ajoute du Pd/C (10%, humide). La suspension est agitée sous 40 psi d'hydrogène à 50°C et pendant 8 heures. Le catalyseur est extrait par filtration et l'éthanol concentré à un petit volume. L'addition d'éther permet de faire apparaître des critaux blancs. Le degré de pureté du produit est suffisant et ne nécessite pas une recristallisation.
P. F.: 159-161°C
RMN-¹H (CD₃OD, ppm) : 7,25-7,30 (4H, m, ArH) ; 5,50 (1H, s, αH) ; 4,50 (2H, d, CH₂ cycle) ; 3,8 (3H, s, CH₃).

### e/ Préparation de l'ester méthylique de l'acide 2-(N-(benzyloxycarbonyl)-2(S)-aminobutyryl)-1(R/S)-isoindoline carboxylique.

L'acide N-benzyloxycarbonyl-2(S)-aminobutyrique (composé de l'exemple 3 étape a/) (2,62 g, 11,04 mmoles) est dissout dans 100 ml de tétrahydrofuranne sous azote et refroidi sur un mélange glace/sel. On ajoute de la N-étitylmorpholine (1,27 g, 11,04 mmoles) puis du chloroformiate d'isobutyle (1,51 g, 11,04 mmoles). Après 30 minutes, on ajoute le composé issu de l'étape d/ (2,347 g, 11,04 mmoles). L'agitation est maintenue durant toute une nuit. Le mélange est alors versé dans 200 ml de CH₂Cl_{2'} lavé avec 3x100 ml d'acide citrique (10%) puis avec 3x100 ml de NaHCO₃ (5%), séché et évaporé jusqu'à obtenir une huile claire.
RMN-¹H (CDCl₃, ppm) : 7,25 (9H, m, ArH) ; 5,75 (1H, m, αH) ; 5,10 (2H, s, CH₂ benzyloxycarbonyle) ; 4,60-5,00 (3H, m, αH, CH₂ cycle) ; 3,8 (3H, s, OCH₃) ; 1,60-2,00 (2H, m, CH₂ aminobutyryle) ; 1,0 (3H, m, CH₃ aminobutyryle).

### f/ Préparation de l'acide 2-(N-benzyloxycarbonyl-2(S)-aminobutyryl)-1(R/S)-isoindolinecarboxylique.

Le composé de l'étape e/ (3,422 g, 8,6 mmoles) est dissout dans 69 ml de méthanol. On ajoute 8,6 ml de NaOH 2N et la solution est mise sous agitation à température ambiante pendant 24 heures. On ajoute ensuite 172 ml d'eau et on évacue le méthanol sous vide. La solution est acidifiée avec du HCl concentré jusqu'à pH 2 et la suspension qui en résulte est extraite par CH₂Cl₂. Après séchage, le CH₂Cl₂ est évacué. Il reste une huile.
RMN-¹H (CDCl₃, ppm) : 7,00-7,30 (9H, m, ArH) ; 5,75 (2H, m, CH₂ cycle) ; 4,8-5,2 (3H, m, αH, CH₂ benzyloxycarbonyle) ; 4,60 (1H, m, αH) ; 1,90 (2H, m, CH₂ aminobutyryle) ; 1,0 (3H, m, CH₃ aminobutyuryle).

### g/ Préparation de l'acide 2-(N-benzyloxycarbonyl-2(S)-aminobutyryl)-1(R/S)-isoindolinecarboxylique n-butylamide.

Le composé de l'étape f/ (2,70g, 7,07mmoles) est dissout dans 100 ml de tétrahydrofuranne sous azote et refroidi sur un mélange glace/sel. On ajoute ensuite de la N-éthylmorpholine (0,814 g, 7,07 mmoles), puis de l'isobutylchloroformiate (0,966 g, 7,07 mmoles). Après 30 minutes, on ajoute de la n-butylamine (0,517 g, 7,07 mmoles) et on maintient l'agitation durant toute une nuit. Le mélange est versé dans 200 ml de CH₂Cl₂, lavé avec 3x100 ml d'acide citrique (10%) puis avec 3x100 ml de NaHCO₃ (5%), séché et évaporé jusqu'à obtention d'une huile claire.
RMN-¹H (CDCl₃, ppm) : 7,25 (9H, m, ArH) ; 5,20-5,30 (2H, m, CH₂ cycle) ; 4,80-5,10 (3H, m, αH, CH₂ benzyloxycarbonyle) ; 4,00-4,25 (1H, m, αH) ; 3,0 (2H, m, CH₂N) ; 1,00-1,90 (6H, m, CH₂ aminobutyrle, CH₃CH₂CH₂) ; 0,8-1,5 (6H, m, CH₃ aminobutyryle, CH₃ butyle).

### h/ Préparation du composé en titre sous la forme de sel d'oxalate

Le composé de l'étape g/ est hydrogéné de la même manière qu'à l'exemple 5 étape c/, et le produit est isolé en tant que sel d'oxalate.
P. F. : 90-92°C.
RMN-¹H (CD₃OD, ppm) : 7,00-7,25 (4H, m, ArH) ; 5,30 (1H, m, NCHCO) ; 4,8-5,0 (2H, m, CH₂ isoindoline) ; 3,25 (1H, m, NCHCO) ; 2,90-3,00 (2H, m, CH₂N) ; 1,50-2,00 (2H, m, CH₂ aminobutyryle) ; 0,8-1,2 (10H, m, CH₃CH₂CH₂ CH₃ aminobutyryle).
Microanalyse : C₁₇H₂₅N₃O₂.C₂H₂O₄.1,25H₂O :
Trouvé : C = 54,98 % ; H = 7,30 % ; N = 10,66 % ;
Calculé : C = 54,86 % ; H = 7,15 % ; N = 10,10 % ;

Les exemples 24 à 36 correspondent à des composés de formule (I) dans laquelle :
R₂ = H, n = 0 et m = 1, R₃ représente le motif 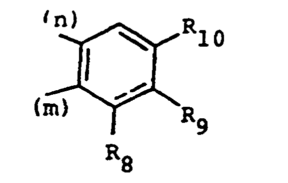 R₄ = CONHR₅ et R₅ = n. butyle,
R₁, R, R', R₈, R₉ et R₁₀ étant tels que définis à chaque exemple.

### EXEMPLE 24 : voie 8

### Préparation du butylamide de l'acide 1-(N-benzyloxycarbonyl-L-valyl)-5-méthoxyindoline-2(R/S)-carboxylique :

A une solution refroidie (0°C) de butylamide de l'acide 5-méthoxyindoline-2(R/S)-carboxylique (1,000 g, 4,03 mmol) et de N-benzyloxycarbonyl-L-valine (1,135 g, 4,50 mmol) dissous dans du dichlorométhane (50 ml), on a ajouté de la triéthylamine (2,25 ml, 16 mmol) et du chlorure bis(2-oxo-3-oxazolidinyl)phosphinique (1,539 g, 6,054 mmol) sous azote. Le mélange a été agité à température ambiante pendant 22 heures, puis lavé avec de l'acide citrique à 10% (3 x 50 ml), du NaHCO₃ à 5% (3 x 50 ml) et de l'eau (50 ml). La phase organique a été séchée et concentrée: le résidu a été purifié par chromatographie sur une colonne de gel de silice, en utilisant un mélange essence de pétrole - acétate d'éthyle 3:2 comme éluant. On a obtenu un solide blanc.
Point de Fusion: 124 - 126°C.
RMN ¹H (CDCl₃), δ (ppm) 8,03 (1H, m, CONH), 7,24 - 7,47 (5H, m, ArH), 6,71 - 6,89 (3H, m, ArH), 4,86 - 5,65 (4H, m, COCHNH de la valine, PhCH₂), 3,75 - 4,15 (4H, m, COCHN de l'indoline, OCH₃), 3,46 - 3,51 (1H, m, CH(H) de l'indoline), 3,09 - 3,21 (3H, m, CH(H) de l'indoline, CONCH₂), 1,85 - 2,12 (1H, m, CHMé₂), 0,78 - 1,42 (13H, m, CH₂CH₂CH₃ du butyle, 2 x CH₃ de la valine).

### Préparation de l'oxalate du butylamide de l'acide 1-(L-valyl)-5-méthoxyindoline-2(R/S)-carboxylique : R₁ = CH(CH₃)₂, R = R' R₈ = R₁₀ = H, R₉ = OCH₃ ;

A une solution de butylamide de l'acide 1-(*N*-benzyloxycarbonyl-L-valyl)-5-méthoxyindoline-2(R/S)-carboxylique (0,502 g, 1,04 mmol) dans le méthanol (20 ml), on a ajouté du palladium sur charbon activé (0,2 g, à 10% d'humidité). Le mélange a été hydrogéné pendant deux heures et demie à température ambiante. Après avoir retiré le catalyseur par filtration, de l'acide oxalique (0,130 g, 1,44 mmol) a été ajouté au filtrat; le solvant a été éliminé et le résidu, qui a cristallisé dans un mélange méthanol ether diéthylique, a donné un solide blanc.
Point de Fusion: 112 - 120°C.
C₁₉H₂₉N₃O₃ 1,25 (COOH)₂,
requis: C 56,14%, H 6,90%, N 9,13%,
trouvé: C 56,01%, H 6,93%, N 9,30%.
RMN ¹H (DMSO-d₆), δ (ppm) 8,46 - 8,53 (1H, m, CONH), 7,91 - 8,06 (1H, d, ArH), 6,77 - 6,94 (2H, m, ArH), 4,95 - 5,30 (1H, m, COCHN de l'indoline), 2,94 - 3,91 (8H, m, COCHN de la valine, CH₃₀, CH₂ de l'indoline, CONHCH₂), 2,05 - 2,24 (1H, m, CHMe₂), 0,81 - 1,46 (13H, m, CH₂CH₂CH₃, 2 x CH₃ de la valine).

### EXEMPLE 25 : voie 8

### Préparation du butylamide de l'acide 1-(N-benzyloxycarbonyl-L-alanyl)-L-méthoxyindoline-2(R/S)-carboxylique :

A une solution refroidie (0°C) de butylamide de l'acide 5-méthoxyindoline-2(R/S)-carboxylique (0,500 g, 2,01 mmol) et de N-benzyloxycarbonyl-L-alanine (0,4956 g, 2,22 mmol) dissous dans du dichlorométhane (25 ml), on a ajouté de la triéthylamine (1,12 ml, 8 mmol) et du chlorure bis(2-oxo-3-oxazolidinyl)phosphinique (0,815 g, 3,2 mmol), sous azote. Le mélange a été agité à température ambiante pendant 22 heures. Il a ensuite été lavé avec de l'acide citrique à 10% (3 x 30 ml), du NaHCO₃ à 5% (3 x 30 ml) et de l'eau (30 ml). La phase organique a été séchée et concentrée, et le résidu a été purifié par chromatographie sur une colonne de gel de silice, en utilisant un mélange essence de pétrole - acétate d'éthyle 1:1 comme éluant. On a obtenu un solide blanc.
Point de Fusion: 106 - 109°C.
RMN ¹H (CDCl₃), δ (ppm) 8,00 - 8,07 (1H, m, CONH), 7,30 - 7,36 (5N, m, ArH), 6,72 - 7,02 (3H, m, ArH), 5,35 - 6,0 (1H, m, NH de l'alanine), 4,95 - 5,20 (3H, m, PhCH₂. COCHN de l'alanine), 4,20 - 4,80 (1H, m, COCHN de l'indoline), 3,76 (3H, s, OCH₃), 3,40 - 3,60 (1H, m, CH(H) de l'indoline), 3,09 - 3,30 (3H, m, CH(H) de l'indoline, CONCH₂), 1,10 - 1,50 (7H, m, CH₂CH₂, CH₃ de l'alanine), 0,78 - 0,87 (3H, m, CH₃ du butyle).

### Préparation de l'oxalate du butylamide de l'acide 1-(L-alanyl)-5-méthoxyindoline-2(R/S)-carboxylique : R₁ = CH₃, R = R' = R₈ = R₁₀ = H, R₉ = OCH₃ ;

A une solution de butylamide de l'acide 1-(N-benzyloxycarbonyl-L-alanyl)-5-méthoxyindoline-2(R/S)-carboxylique (0,130 g, 0,28 mmol) dans du méthanol (6 ml), on a ajouté du palladium sur charbon activé (0,04 g, à 10% d'humidité). Le mélange a été hydrogéné pendant deux heures et demie, à température ambiante. Le catalyseur a été retiré par filtration, puis de l'acide oxalique (0,028 g, 0,31 mmol) a été ajouté au filtrat qui a été concentré. De l'éther diéthylique a été ajouté pour faire précipiter le produit qui a été obtenu sous la forme d'un solide blanc.
Point de Fusion: 112 - 118°C.
C₁₇H₂₅N₃O₃ 1,14 (COOH)₂ 0,9 H₂O,
requis: C 51,51%, H 6,46%, N 9,10%,
trouvé: C 51,37%, H 6,28%, N 9,40%.
RMN ¹H (DMSO-d₆), δ (ppm) 8,44 - 8,47 (1H, m, CONH), 7,97 - 8,04 (1H, m, ArH), 6,78 - 6,92 (2H, m, ArH), 4,75 - 5,25 (1H, m, COCHN de l'indoline), 3,35 - 4,10 (5H, m, COCHN de l'alanine, CH(H) de l'indoline, CH₃O), 2,99 - 3,13 (3H, m, CH(H) de l'indoline, CONCH₂), 1,23 - 1,50 (7H, m, CH₂CH₂, CH₃ de l'alanine), 0,87 (3H, t, CH₃ du butyle).

### EXEMPLE 26 : voie 8

### Préparation du butylamide de l'acide 1-(N-benzyloxycarbonyl-L-alanyl)-5-méthoxyindoline-2(S)-carboxylique :

Du butylamide de l'acide 1-(N-benzyloxycarbonyl-L-alanyl)-5-méthoxyindoline-2(R/S)-carboxylique a été préparé comme décrit plus haut. Une chromatographie prudente sur une colonne de gel de silice, en utilisant un mélange essence de pétrole - acétate d'éthyle 1:1 comme éluant, a donné deux diastéréoisomères. Le premier à éluer était l'isomère (R,S), puis l'isomère (S,S).
Données pour la forme (S,S):
RMN ¹H (CDCl₃), δ (ppm) 8,02 - 8,05 (1H, m, CONH), 7,30 - 7,38 (5H, m, ArH), 6,73 - 7,00 (3H, m, ArH), 4,77 - 5,63 (4H, m, NH de l'alanine, PHCH₂, COCHN de l'alanine), 4,24 - 4,28 (1H, m, COCHN de l'indoline), 3,78 (s, 3H, OCH₃), 3,49 - 3,54, 3,17 - 3,22 (m, 4H, CH₂ de l'indoline, CONCH₂), 1,16 - 1,55 (m, 7H, CH₂CH₂, CH₃ de l'alanine), 0,79 - 0,89 (m, 3H, CH₃ du butyle).

### Préparation de l'oxalate du butylamide de l'acide 1-(L-alanyl)-5-méthoxyindoline-2(S)-carboxylique : R₁ = CH₃, R = R' = R₈ = R₁₀ = H, R₉ = OCH₃ ;

A une solution de butylamide de l'acide 1-(N-benzyloxycarbonyl-L-alanyl)-5-méthoxyindoline-2(S)-carboxylique (0,110 g , 0,243 mmol) dans du méthanol (6 ml), on a ajouté du palladium sur charbon activé (0,04 g, à 10% d'humidité). Le mélange a été hydrogéné pendant deux heures et demie, à température ambiante. Le catalyseur a été retiré par filtration, puis de l'acide oxalique (0,0219 g, 0,25 mmol) a été ajouté au filtrat qui a été concentré. De l'éther diéthylique a été ajouté pour précipiter le produit qui a été obtenu sous la forme d'un solide blanc.
Point de Fusion: 141 - 143°C.
C₁₇H₂₅N₃O₃ 1,75(COOH)₂ 0,25(H₂O),
requis: C 51,14%, H 6,07%, N 8,73%,
trouvé: C 51,00%, H 5,98%, N 8,88%.
RMN ¹H (DMSO-d₆), δ (ppm) 8,48 - 8,51 (1H, m, CONH), 8,01 (1H, d, ArH), 6,89 (1H, s, ArH), 6,79 (1H, d, ArH), 5,00 - 5,03 (1H, m, COCHN de l'indoline), 3,61 - 3,73 (5H, m, CH₃O, COCHN de l'alanine, CH(H) de l'indoline), 3,04 - 3,10 (3H, m, CH(H) de l'indoline, CONCH₂), 1,25 - 1,51 (7H, m, CH₂CH₂ du butyle, CH₃ de l'alanine), 0,87 (3H, t, CH₃ du butyle).

### EXEMPLE 27 : voie 9

### Préparation de l'azidoacétate d'éthyle :

Une solution de bromoacétate d'éthyle (150 g, 0,901 mol) dans de l'acétonitrile sec (800 ml) a été traitée avec de l'azoture de sodium (58,57 g, 0,901 mol) sous une atmosphère d'azote, et le mélange a été chauffé pendant 20 heures à reflux. Après refroidissement, de l'eau (50 ml) a été ajoutée et le mélange a été agité pendant une demi - heure; la phase supérieure a été séparée et la phase inférieure a été traitée avec un sel et extraite avec de l'éther diéthylique. Les phases organiques ont été combinées et le solvant a été éliminé à une température n'excédant pas 50°C, pour donner une huile jaune.
RMN ¹H (CDCl₃), δ (ppm) 4,24 (2H, q, OCH₂), 3,84 (2H, s, N₃CH₂), 1,29 (3H, t, CH₃).

### Préparation du 2-azido-3-(2-méthoxyphényl) propénoate d'éthyle :

Des morceaux de sodium (3,678 g, 160 mmol) ont été ajoutés par portions à de l'éthanol (200 ml) sur une période de 30 minutes. La solution résultante a été refroidie à -18°C, et ensuite on a ajouté, sur une période d'une heure, un mélange de 2-méthoxybenzaldéhyde (4,832 g, 40 mmol) et d'azidoacétate d'éthyle (160 mmol) à une vitesse qui permette de maintenir la température au dessous de -15°C. Après 3 heures, la solution a été stockée à 5°C pendant deux jours pour donner un produit cristallin qui a été recueilli par filtration et lavé avec de l'hexane froid.
RMN ¹H (CDCl₃), δ (ppm) 8,19 - 8,20 (1H, m, ArCH-), 7,22 - 7,40 (2H, m, ArH), 6,84 - 7,04 (2H, m, ArH), 4,35 (2H, q, OCH₂), 3,84 (3H, s, OCH₃), 1,36 (3H, t, CH₃).

### Préparation du 4-méthoxyindole-2-carboxylate d'éthyle :

Du 2-azido-3-(2-méthoxyphényl)-propénoate d'éthyle (3,566 g, 144 mmol) a été mis en suspension dans du toluène (800 ml) et le mélange a été chauffé à reflux pendant trois heures, puis refroidi et agité à température ambiante pendant la nuit. Le 4-méthoxyindole-2-carboxylate d'éthyle s'est formé sous l'apparence d'un solide jaune et a été recueilli.
Point de Fusion: 168 - 170°C.
RMN ¹H (CDCl₃), 6 (ppm) 8,92 (1H, m, NH), 7,34 - 7,35 (1H, m, ArH), 7,23 - 7,27 (1H, m, ArH), 6,99 - 7,03 (1H, dd, ArH), 6,48 - 6,52 (1H, d, ArH), 4,39 (2H, q, OCH₂-), 3,95 (3H, s, CH₃O), 1,39 (3H, t, -CH₃).

### Préparation du 4-méthoxyindoline-2(R/S)-carboxylate de méthyle :

Du 4-méthoxyindole-2-carboxylate d'éthyle (0,95 g, 4,33 mmol) a été dissous dans du méthanol (10 ml), puis des copeaux de magnésium (0,471 g, 19,37 mmol) ont été ajoutés tout en agitant, à température ambiante, sous une atmosphère d'azote. Après le démarrage de la réaction, un bain d'eau a été utilisé pour maintenir la température de la réaction entre 15 - 20°C. Ce mélange a été agité jusqu'au lendemain. Après la fin de la réaction, du dichlorométhane (200 ml) puis une solution de chlorure d'ammonium (200 ml) ont été ajoutés au mélange. La phase organique a été séparée et la phase aqueuse a été extraite avec du dichlorométhane (3 x 50 ml). Les phases organiques combinées ont été séchées sur du sulfate de magnésium et le solvant a été éliminé pour donner une huile marron.
RMN ¹H (CDCl₃), δ (ppm) 6,99 - 7,06 (1H, m, ArH), 6,29 - 6,40 (2H, dd, ArH), 4,43 (2H, m, H-N, COCHN de l'indoline), 3,79 (3H, s, H₃COO), 3,77 (3H, s, H₃CO), 3,28 - 3,32 (2H, m, CH₂ de l'indoline).

### Préparation du butylamide de l'acide 4-méthoxyindoline-2(R/S)-carboxylique :

Du 4-méthoxyindoline-2(R/S)-carboxylate de méthyle (0,89 g, 4,3 mmol) a été dissous dans de la butylamine (40 ml) sous azote, et ensuite, la solution a été agitée à reflux pendant trois heures. Après que le mélange ait refroidi, la butylamine restante a été éliminée sous vide. Le produit brut a été recristallisé dans l'éther diéthylique pour donner un solide blanc - cassé.
RMN ¹H (CDCl₃), δ (ppm) 7,01 - 7,09 (2H, m, CONH, ArH), 6,35 - 6,36 (2H, dd, ArH), 4,35 - 4,45 (1H, m, COCHN de l'indoline), 4,13 - 4,16 (1H, m, NH de l'indoline), 3,79 (3H, s, OCH₃), 3,49 - 3,63 (1H, m, CH(H) de l'indoline), 3,21 - 3,31 (2H, m, CONCH₂), 2,89 - 3,01 (1H, m, CH(H) de l'indoline), 1,26 - 1,55 (4H, m, CH₂CH₂)_{'} 0,90 (3H, t, CH₃).

### Préparation du butylamide de l'acide 1-(benzyloxycarbonyl-2(S)-aminobutyryl)-4-méthoxyindoline-2(R/S)-carboxylique :

Du butylamide de l'acide 4-méthoxyindoline-2(R/S)-carboxylique (0,935 g, 3,77 mmol) et de l'acide N-benzyloxycarbonyl-2(S)-aminobutyrique (1,422 g, 6,00 mmol) ont été dissous dans du chlorure de méthylène séché (50 ml). La solution.a été refroidie à 0°C, puis de la triéthylamine (1,67 ml, 12 mmol) et du chlorure bis (2-oxo-3-oxazolidinyl)phosphinique (1,527 g, 6,00 mmol) ont été ajoutés. Le mélange a été agité à température ambiante pendant 22 heures, puis lavé avec de l'acide chlorhydrique 3N (3 x 50 ml), du bicarbonate de sodium à 5% (3 x 50 ml) et de l'eau (50 ml), et séché sur du sulfate de sodium. Après avoir retiré le solvant, le résidu a été purifié par chromatographie sur une colonne de gel de silice, en utilisant un mélange dichlorométhane : acétate d'éthyle 3:2 comme éluant. On a obtenu un solide blanc - cassé.
RMN ¹H (CDCl₃), δ (ppm) 7,73 - 7,75 (1H, m, CONH), 7,18 - 7,33 (6H, m, ArH), 6,62 - 6,73 (2H, m, ArH), 4,82 - 5,66, 4,18 - 4,19 (5H, m, PHCH₂, NH de l'Abu, 2 x COCHN), 3,81 (3H, s, CH₃O), 3,16 - 3,49 (4H, m, CONCH₂, CH₂ de l'indoline), 0,79 - 1,83 (12H, m, CH₂CH₂CH₃, CH₂CH₃).
(Abu = aminobutyryle).

### Préparation de l'oxalate du butylamide de l'acide 1-(2(S)-aminobutyryl)-4-méthoxy-indoline-2(R/S)-carboxylique : R₁ = CH₂CH₃, R = R' = R₉ = R₁₀ = H, R₈ = OCH₃ ;

Du butylamide de l'acide 1-(benzyloxycarbonyl-2(S)-aminobutyryl)-4-méthoxyindoline-2(R/S)-carboxylique (0,100 g, 0,21 mmol) a été dissous dans du méthanol sec (20 ml), puis du palladium sur charbon activé (0,04 g) a été ajouté. Le mélange a été hydrogéné pendant 2,5 heures a température ambiante. Le catalyseur a été retiré et de l'acide oxalique (0,020 g, 0,22 mmol) a été ajouté à la solution qui a été concentrée. De l'éther a été ajouté et un solide blanc - cassé a été obtenu.
Point de Fusion: 117 - 119°C.
C₁₈H₂₇N₃O₃ 1,0(COOH)₂ 1,0 (H₂O)
requis: C 54,41%, H 7,08%, N 9,52%,
trouvé: C 54,48%, H 6,87%, N 9,50%.
RMN ¹H (DMSO-d₆), δ (ppm) 6,5 - 10,0 (CONHBu, hydrogènes acides), 7,6 - 7,85, 7,15 - 7,35, 6,65 - 6,90 (m, ArH), 4,6 - 5,5 (1H, m, COCHN de l'indoline), 2,5 - 4,1 (8H, m, CH₃O, CH₂ de l'indoline, CONCH₂-, COCHN de l'Abu), 0,7 - 2,2 (12H, m, CH₁CH₂CH_{3'} CH₂CH₃ de l'Abu).

### EXEMPLE 28 : voie 11

### Préparation du 2-oxo-3-méthyle-butanoate d'éthyle :

A une solution de diéthyloxalate (29,23 g, 20,0 mmol) dans l'éther diéthylique séché (100 ml) à -70°C, on a ajouté du chlorure d'isopropylmagnésium (2,0 M dans l'éther, 200 mmol) sur une période d'une heure. Le mélange a été agité à -70°C pendant une demi - heure supplémentaire avant d'être immédiatement versé dans une suspension vigoureusement agitée de glace (80 g), d'éther diéthylique (100 ml) et d'HCl concentré (18 ml). La phase aqueuse a été séparée et la phase organique lavée avec de l'eau (100 ml), puis séchée (MgSO₄), et le solvant a été retiré pour laisser le produit désiré sous la forme d'une huile incolore. RMN ¹H (CDCl₃), δ (ppm) 4,28 (2H, q, OCH₂), 3,18 - 3,25 (1H, m, COCH), 1,31 (3H, t, CH₃ de l'éthoxy), 1,16 (6H, d, 2 x CH₃ du propyle).

### Préparation de la phénylhydrazone du 2-oxo-3-méthylbutanoate d'éthyle :

Un mélange de 2-oxo-3-méthyl - butanoate d'éthyle (23,76 g) et de phénylhydrazine (25 ml) a été chauffé à 60°C dans du toluene pendant environ une heure, durant laquelle un léger vide a été utilisé pour éliminer l'eau. Après avoir retiré le solvant, le résidu a été soumis à une chromatographie sur du gel de silice, en utilisant un mélange essence de pétrole : acétate d'éthyle 4:1 comme éluant, pour fournir le produit désiré sous forme d'une huile jaune.
RMN ¹H (CDCl₃), δ (ppm) 7,10 - 7,33 (4H, m, ArH), 6,86 - 6,98 (1H, m, ArH), 4,28 (2H, q, OCH₂), 2,88 - 3,06 (1H, m, N=CCH), 1,36 (3H, t, CH₃ de l'éthoxy), 1,06 (6H, d, 2 x CH₃ du propyle).

### Préparation du 3,3-diméthyl-3H-indole-2-carboxylate d'éthyle :

De l'éthanol saturé en acide chlorhydrique (320 ml, à environ 34%) a été préparé. Ensuite, de la phénylhydrazone 2-oxo-3-méthyl-butanoate d'éthyle (composé 65) (20 g, 85,4 mmol) a été ajouté à cette solution. Le mélange jaune résultant a été chauffé à reflux pendant dix minutes. Le mélange a été refroidi et l'éthanol a été retiré. Le résidu a été ajouté à de l'éther (100 ml) et traité avec du carbonate de sodium à 5% jusqu'à ce que l'effervescence cesse. La phase organique a été séparée et la phase aqueuse a été extraite avec de l'éther (3 x 50 ml). Les extraits organiques combinés ont été lavés avec de l'eau (3 x 100 ml) jusqu'à ce qu'ils soient neutres, et ils ont été séchés (MgSO₄). Le solvant a été éliminé pour laisser des cristaux dorés. Une recristallisation dans de l'essence de pétrole, à - 30°C, a donné des cristaux pâles, crème.
Point de Fusion: 76 - 77°C.
RMN ¹H (CDCl₃), δ (ppm) 7,70 - 7,80 (1H, m, ArH), 7,34 - 7,36 (3H, m, ArH), 4,43 (2H, q, OCH₂), 1,49 (6H, s, 2 x CH₃ de l'indole), 1,42 (3H, t, CH₃ de l'éthoxy).

### Préparation du 3,3-diméthylindoline-2(R/S)-carboxylate d'éthyle :

Du 3,3-diméthyl-3H-indole-2-carboxylate d'éthyle (4,00 g, 18,4 mmol) a été dissous dans de l'éthanol (20 ml), puis du palladium sur charbon activé (0,5 g) a été ajouté à cette solution. Ce mélange a été hydrogéné pendant cinq heures. Après filtration pour retirer le catalyseur, le filtrat a été séché (MgSO₄) et le solvant retiré pour donner une huile marron.
RMN ¹H (CDCl₃), δ (ppm) 6,94 - 7,08 (2H, m, ArH), 6,62 - 6,80 (2H, m, ArH), 4,06 - 4,34 (4H, m, NH de l'indoline, COCHN de l'indoline, CH₂ de l'éthoxy), 1,44 (3H, s, 1 x CH₃ de l'indoline), 1,24 (3H, t, CH₃ de l'éthoxy), 1,07 (3H, s, 1 x CH₃ de l'indoline).

### Préparation du butylamide de l'acide 3,3-diméthylindoline-2(R/S)-carboxylique:

De la butylamine (15 ml) dans du toluène (40 ml) a été refroidie jusqu'à -78°C et une solution de butyl-lithium 2,5M dans l'hexane (12,91 ml, 31,5 mmol) a été ajoutée goutte à goutte. Après une demi - heure, on a ajouté de l'ester éthylique de l'acide 3,3-diméthylindoline-2(R/S)-carboxylique (4,600 g, 21 mmol) à la solution, et ce mélange a été agité sous azote pendant trois heures. Ensuite, le mélange a été versé dans un mélange eau-glace (50 ml) et extrait avec de l'acétate d'éthyle; les extraits ont été séchés sur du sulfate de magnésium et le solvant a été retiré pour donner une huile marron. Celle-ci a été purifiée par chromatographie sur une colonne de gel de silice, en utilisant un mélange essence de pétrole : acétate d'éthyle 7:3 comme éluant pour donner un solide jaune.
RMN ¹H (CDCl₃), δ (ppm) 7,02 - 7,08 (3H, m, 2ArH, CONH), 6,84 (1H, t, ArH), 6,70 (1H, d, ArH), 3,96 - 4,68 (2H, m, NH de l'indoline, COCHN de l'indoline), 3,20 - 3,38 (2H, m, CONCH₂), 1,31 - 1,68 (7H, m, CH₂CH₂ du butyle, 1 x CH₃ de l'indoline), 1,08 (3H, s, 1 x CH₃ de l'indoline), 0,90 (3H, t, CH₃ du butyle).

### Préparation du butylamide de l'acide 1-(N-benzyloxycarbonyl-2(S)-aminobutyryl)-3,3-diméthylindoline-2(R/S)-carboxylique:

Du butylamide de l'acide 3,3-diméthylindoline-2(R/S)-carboxylique (1,000 g, 4,08 mmol) et de l'acide N-benzyloxycarbonyl-2(S)-aminobutyrique (1,451 g, 6,12 mmol) ont été dissous dans du tétrahydrofurane sec (25 ml). La solution a été refroidie à 0°C, puis de la triéthylamine (2,44 ml, 13,78 mmol) et du chlorure bis(2-oxo-3-oxazolidinyl)phosphinique (2,338 g, 9,183 mmol) ont été ajoutés. Le mélange a été agité à température ambiante pendant 22 heures, puis de l'acétate d'éthyle (40 ml) a été ajouté et le mélange a été lavé avec de l'acide chlorhydrique 3N (3 x 50 ml), du bicarbonate de sodium à 5% (3 x 50 ml), de l'eau (50 ml), et séché sur du sulfate de sodium. Après avoir éliminé le solvant, plusieurs purifications successives par chromatographie sur une colonne de gel de silice ont données le produit sous la forme d'un solide blanc - cassé. RMN ¹H (CDCl₃), δ (ppm) 8,08 - 8,2 (1H, m, CONH), 7,02 - 7,5 (9H, m, ArH), 4,04 - 5,82 (5H, m, CH₂ du benzyle, CH, NH de l'Abu, COCHN de l'indoline), 3,00 - 3,30 (2H, m, CONCH₂), 0,70 - 1,98 (18H, m, CH₂CH₃ de l'Abu, 2 x CH₃ de l'indoline, CH₂CH₂CH₃ du butyle).

### Préparation de l'oxalate du butylamide de l'acide 1-(2(S)-aminobutyryl)-3,3-diméthylindoline-2(R/S)-carboxylique : R₁ = CH₂CH₃, R = R' = CH₃, R₈ = R₉ = R₁₀ = H ;

Du butylamide de l'acide 1-(N-benzyloxycarbonyl-2(S)-aminobutyryl)-3,3-diméthyl-2(R/S)-carboxylique (0,0660 g, 0,142 mmol) a été dissous dans du méthanol sec (6 ml), puis du palladium sur charbon activé (0,022 g) a été ajouté. Ce mélange a été hydrogéné sous 60 psi d'hydrogène pendant 2,5 heures, à température ambiante. Après filtration pour retirer le catalyseur, de l'acide oxalique (0,013 g, 0,14 mmol) a été ajouté à la solution. La plus grande partie du méthanol a été éliminée par évaporation, puis de l'éther a été ajouté et un solide blanc - cassé a été précipité. Celui-ci a été recueilli et séché.
Point de Fusion: 127 - 133°C.
C₁₉H₂₉N₃O₂ 1,0(COOH)₂ 1,0 H₂O,
requis: C 57,39%, H 7,57%, N 9,56%,
trouvé: C 57,25%, H 7,52%, N 9,70%.
RMN ¹H (DMSO-d₄), δ (ppm) 8,45 - 8,75 (1H, m, CONH), 7,99 - 8,23 (1H, m, ArH), 7,07 - 7,29 (3H, m, ArH), 4,63 - 4,83, 3,61 - 4,00 (2H, m, CH de l'Abu, COCHN de l'indoline), 3,00 - 3,16 (2H, m, CONCH₂), 0,85 - 1,88 (18H, m, CH₂CH₃ de l'Abu, CH₂CH₂CH₃ du butyle, 2 x CH₃ de l'indoline).

### EXEMPLE 29 : voie 10

### Préparation de l'ester éthylique de l'acide 3-méthylindole-2-carboxylique:

De la phénylhydrazine (14,87 g, 0,137 mol) a été mélangée avec de l'acide 2-cétobutyrique (14 g, 0,137 mol) et après un court moment, une solution d'acide sulfurique (14 g, 0,142 mol) dans l'éthanol absolu, a été ajoutée. L'hydrazone brute s'est dissoute, et la solution marron a été portée à ébullition modérée pendant cinq heures. Le mélange a été maintenu pendant la nuit à 4°C. Un solide a précipité. Celui-ci a été recueilli et lavé avec de l'eau (30 ml) et de l'alcool (10 ml) pour donner un solide blanc- cassé.
RMN ¹H (CDCl₃), δ (ppm) 8,66 - 8,80 (1H, m, NH), 7,67 (1H, dd, ArH), 7,14 - 7,36 (3H, m, ArH), 4,42 (2H, q, OCH₂), 2,61 (3H, s, CH₃ de l'indole), 1,43 (3H, t, CH₃ de l'ester).

### Préparation du 3-(R/S)-méthylindoline-2(R/S) carboxylate de méthyle :

Du 3-méthylindole-2-carboxylate d'éthyle (4,06 g, 20 mmol) a été dissous dans du méthanol (50 ml), puis des copeaux de magnésium (2,431 g, 100 mmol) ont été ajoutés avec agitation, à température ambiante, sous une atmosphère d'azote. Après que la réaction ait démarrée, un bain d'eau a été utilisé pour maintenir la température de la réaction entre 15 - 20°C. Après la fin de la réaction, on a ajouté du dichlorométhane (200 ml), puis une solution de NH₄Cl (200 ml) au mélange. La phase organique a été séparée et la phase aqueuse a été extraite avec du dichlorométhane (3 x 50 ml). Les phases organiques combinées ont été séchées sur du sulfate de magnésium et le solvant a été éliminé pour donner une huile marron.
RMN ¹H (CDCl₃), 6 (ppm) 7,03 - 7,22 (2H, m, ArH), 6,73 - 6,78 (2H, m, ArH), 4,48 - 4,82 (1H, m, H-N), 4,00, 4,50 (1H, dd, COCHN de l'indoline), 3,78, 3,77 (3H, s, H₃COOC), 3,47 - 3,70 (1H, m, CH(CH₃) de l'indoline), 1,44, 1,22 (3H, dd, CH₃ de l'indoline).

### Préparation du butylamide de l'acide 3-(R/S)-méthyldindoline-2(R/S)-carboxylique :

Du 3-(R/S)-méthyl-indoline-2(R/S)-carboxylate de méthyle (3,60 g, 18,8 mmol) a été dissous dans de la butylamine (100 ml) sous azote, et ensuite, la solution a été agitée à reflux pendant 3 heures. Après que le mélange ait refroidi, la butylamine restante a été éliminée sous vide. Le produit brut a été purifié par chromatographie sur une colonne de gel de silice, en utilisant un mélange essence de pétrole : acétate d'éthyle 7:3, puis 5:5 comme éluant. Deux fractions principales ont été recueillies.
La première était un solide blanc qui est l'isomère trans.
RMN ¹H (CDCl₃), δ (ppm) 7,01 - 7,24 (3H, m, 2ArH, CONH), 6,83 (1H, t, ArH), 6,71 (1H, d, ArH), 4,05 - 4,07 (1H, m, NH de l'indoline), 3,89 - 3,92 (1H, m, COCHN de l'indoline), 3,23 - 3,27 (3H, m, CH(CH₃) de l'indoline, CONCH₂), 1,43 - 1,63 (5H, m, CH₃ de l'indoline, CH₂ du butyle), 1,29 - 1,37 (2H, m, CH₂ du butyle), 0,90 (3H, t, CH₃ du butyle).
Le second était un solide blanc - cassé qui est l'isomère cis. RMN ¹H (CDCl₃), δ (ppm) 7,04 - 7,09 (3H, m, 2ArH, CONH), 6,79 - 6,83 (1H, m, ArH), 6,69 (1H, d, ArH), 4,43 - 4,47 (1H, dd, COCHN de l'indoline), 4,02 - 4,03 (1H, m, NH de l'indoline), 3,67 - 3,71 (1H, m, CH(CH₃) de l'indoline), 3,18 - 3,39 (2H, m, CONCH₂), 1,43 - 1,63 (2H, m, CH₂ du butyle), 1,29 - 1,37 (2H, m, CH₂ du butyle), 1,12 (3H, d, CH₃ de l'indoline), 0,90 (3H, t, CH₃ du butyle).

### Préparation du butylamide de l'acide 1-(N-benzyloxycarbonyl-2(S)-aminobutyryl)-3(R)-méthylindoline-2(R)-carboxylique et du butylamide de l'acide 1-(N-benzyloxycarbonyl-2(S)-aminobutyryl)-3(S)-méthylindoline-2(S)-carboxylique :

Le butylamide de l'acide trans-3-méthylindoline-2-carboxylique (0,530 g, 2,284 mmol) et l'acide N-benzyloxycarbonyl-2(S)-aminobutyrique (0,65 g, 2,74 mmol) ont été dissous dans du dichlorométhane sec (5ml). A cette solution on a ajouté de la triéthylamine (1,15 ml, 8,224 mmol) et du chlorure bis(2-oxo-3-oxazolidinyl)phosphinique (1,047 g, 4,112 mmol). Le mélange a été agité à température ambiante pendant 22 heures, puis lavé avec de l'acide chlorhydrique 3N (3 x 50 ml), du bicarbonate de sodium à 5% (3 x 50 ml), de l'eau (50 ml), et séché sur du sulfate de sodium. Après élimination du solvant, une chromatographie sur colonne de gel de silice, utilisant un mélange essence de pétrole : acétate d'éthyle 3:2 a fourni le produit sous la forme d'un solide blanc - cassé.
RMN ¹H (CDCl₃), δ (ppm) 8,08 - 8,20 (1H, m, CONH), 6,60 - 7,55 (9H, m, ArH), 4,60 - 5,70 (4H, m, CH₂ du benzyle, COCHNH de l'Abu), 4,04 - 4,44 (1H, m, COCHN de l'indoline), 2,96 - 3,78 (3H, m, CH(CH₃) de l'indoline, CONCH₂), 0,70 - 1,84 (15H, m, CH₂CH₃ de l'Abu, CH₃ de l' indoline, CH₂CH₂CH₃ du butyle).

### Préparation de l'oxalate du butylamide de l'acide 1-(2(S)-aminobutyryl)-3(R)-méthylindoline -2(R)-carboxylique et de l'oxalate du butylamide de l'acide 1-(2(S)-aminobutyryl)-3(S)-méthylindoline-2(S)-carboxylique : R₁ = CH₂CH₃, R ou R' = CH₃ et R' ou R = H, R₈ = R₉ = R₁₀ = H ;

Du butylamide de l'acide 1-(N-benzyloxycarbonyl-2(S)-aminobutyryl)-3(R)-méthyl-indoline-2(R)-carboxylique et du butylamide de l'acide 1-(N-benzyloxycarbonyl-2(S)-aminobutyryl)-3(S)-méthyl-indoline-2(S)-carboxylique (140 mg, 0,310 mmol) ont été dissous dans du méthanol sec (6 ml), puis du palladium sur charbon activé (0,040 g) a été ajouté. Ce mélange a été hydrogéné pendant 2,5 heures à température ambiante. Après filtration, de l'acide oxalique (0,028 g, 0,320 mmol) a été ajouté à la solution. Après avoir éliminé la plus grande partie du méthanol par évaporation, de l'éther a été ajouté. On a obtenu un solide blanc.
Point de Fusion: 120,8 - 125,9°C.
C₁₈H₂₇N₃O₂ 0,75(COOH)₂ 1,1H₂O,
requis: C 57,86%, H 7,64%, N 10,36%,
trouvé: C 57,59%, H 7,40%, N 10,21%.
RMN ¹H (DMSO-d₆), δ (ppm) 8,53 - 8,57 (1H, m, CONH), 8,03 - 8,11 (1H, m, ArH), 7,00 - 7,40 (3H, m, ArH), 4,59 - 4,81, 3,59 - 3,88, 2,99 - 3,38 (5H, m, COCHN de l'Abu, 2 x CH de l'indoline, CONCH₂), 1,58 - 1,85, 1,21 - 1,45, 0,81 - 1,08 (15H, m, CH₂CH₃ de l'Abu, CH₂CH₂CH₃ du butyle, CH₃ de l'indoline).

### EXEMPLE 30 : voie 10

### Préparation du butylamide de l'acide 1-(N-benzyloxycarbonyl-2(S)-aminobutyryl)-3(R)-méthylindoline-2(S)-carboxylique et du butylamide de l'acide 1-(2(S)-N-benzyloxycarbonyl-2(S)-aminobutyryl)-3(S)-méthylindoline-2(R)-carboxylique :

Du butylamide de l'acide cis-3-méthyl-indoline-2-carboxylique (0,220 g, 0,948 mmol) et de l'acide N-benzyloxycabonyl-2(S)-aminobutyrique (0,355 g, 1,50 mmol) ont été dissous dans du dichlorométhane sec (5 ml). A cette solution on a ajouté de la triéthylamine (0,455 ml, 4,5 mmol) et du chlorure bis(2-oxo-3-oxazolidinyl)-phosphinique (0,573 g, 2,25 mmol). Le mélange a été agité à température ambiante pendant 22 heures, puis lavé avec de l'acide chlorhydrique 3N (3 x 50 ml), du bicarbonate de sodium à 5% (3 x 50 ml), de l'eau (50 ml), et séché sur du sulfate de sodium. Après avoir éliminé le solvant, le résidu a été purifié par chromatographie sur une colonne de gel de silice, en utilisant un mélange essence de pétrole : acétate d'éthyle 3:2 comme éluant. Les fractions principales ont été recristallisées dans l'éther diéthylique pour permettre de récolter un produit blanc - cassé.
RMN ¹H (CDCl₃), δ (ppm) 8,08 - 8,16 (H, m, CONH), 7,0 - 7,40 (9H, m, ArH), 4,76 - 5,98 (4H, m, CH₂ du benzyle, CH, NH de l'Abu), 4,18 - 4,44 (1H, m, COCHN de l'indoline), 2,96 - 3,98 (3H, m, CH(CH₃) de l'indoline, CONCH₂), 0,70 - 1,94 (15H, m, CH₂CH₃ de l'Abu, CH₃ de l'indoline, CH₂CH₂CH₃ du butyle).

### Préparation de l'oxalate du butylamide de l'acide 1-(2(S)-aminobutyryl)-3(R)-méthylindoline-2(S)-carboxylique et de l'oxalate du butylamide de l'acide 1-(2(S)-aminobutyryl)-3(S)-méthylindoline-2(R)-carboxylique : R₁ = CH₂CH₃, R ou R' = CH₃ et R' ou R = H, R₈ = R₉ = R₁₀ = H ;

Du butylamide de l'acide 1-(N-benzyloxycarbonyl-2(S)-aminobutyryl)-3(R)-méthylindoline-2(S)-carboxylique et du butylamide de l'acide 1-(N-benzyloxycarbonyl-2(S)-aminobutyryl)-3(S)-méthylindoline-2(R)-carboxylique (60 mg, 0,133 mmol) ont été dissous dans du méthanol sec (6 ml), puis du palladium sur charbon activé (0,020 g) a été ajouté. Ce mélange a été hydrogéné pendant 2,5 heures à température ambiante. Après filtration, de l'acide oxalique (0,013 g, 0,140 mmol) a été ajouté à la solution. Après avoir éliminé la plus grande partie du méthanol par évaporation, de l'éther a été ajouté et on a obtenu un solide blanc.
Point de Fusion: 158,7 - 162°C.
C₁₈H₂₇N₃O₂ 1,4 (COOH)₂ 0,7 H₂O,
requis: C 54,76%, H 6,90%, N 9,22%,
trouvé: C 54,45%, H 6,70%, N 9,54%.
RMN ¹H (DMSO-d₆), δ (ppm) 8,59 - 8,60 (1H, m, CONH), 8,06 - 8,08 (1H, m, ArH), 7,07 - 7,23 (3H, m, ArH), 5,00 (1H, d, COCHN de l'indoline), 3,87 - 3,91 (1H, m, COCHN de l'Abu), 3,60 - 3,58 (1H, m, CH(CH₃) de l'indoline), 3,04 - 3,16 (2H, m, CONCH₂), 1,79 - 1,95 (2H, m, CH₂ du butyle), 1,21 - 1,44 (m, 7H, CH₂ du butyle, Abu et CH₃ de l'indoline), 0,98 (3H, t, CH₃ de Abu), 0,870 (3H, t, CH₃ du butyle).

### EXEMPLE 31 : voie 9

### Préparation du 2-azido-3-(2-éthoxyphényl)-propénoate d'éthyle :

Des morceaux de sodium (3,678 g, 160 mmol) ont été ajoutés à de l'éthanol (200 ml), sur une période de 30 minutes. La solution résultante a été refroidie jusqu'à -18°C, et ensuite un mélange de 2-éthoxybenzaldéhyde (6,007 g, 40 mmol) et d'azidoacétate d'éthyle (160 mmol) a été ajouté, sur une heure, à une vitesse qui permettait de maintenir la température au dessous de -15°C. Après trois heures, la solution a été stockée à 5°C pendant deux jours pour donner un produit cristallin qui a été recueilli et lavé avec de l'hexane froid pour donner un solide blanc - cassé.
RMN ¹H (CDCl₃), δ (ppm) 8,20 - 8,16 (1H, dd, ArH), 7,44 (1H, s, CH), 7,24 - 7,33 (1H, m, ArH), 6,84 - 7,00 (2H, m, ArH), 4,36 (2H, q, OCH₂), 4,07 (2H, q, COOCH₂), 1,44 (3H, t, CH₃ de l'éther), 1,39 (3H, t, CH₃ de l'ester).
Celui-ci a été recristallisé dans le méthanol pour donner l'ester méthylique.

### Préparation du 4-éthoxyindole-2-carboxylate de méthyle :

Du 2-azido-3-(2-éthoxyphényl)-propénoate de méthyle (2,061 g, 10 mmol) a été mis en suspension dans le toluène (500 ml) et le mélange a été chauffé à reflux pendant trois heures et ensuite maintenu à température ambiante pendant la nuit. Les solides ont été recueillis par filtration pour donner le produit désiré sous la forme d'un solide jaune.
RMN ¹H (CDCl₃), δ (ppm) 8,92 (1H, m, NH), 7,37 - 7,38 (1H, m, ArH), 7,18 - 7,26 (1H, m, ArH), 6,94 (1H, d, ArH), 6,49 (1H, d, ArH), 4,18 (2H, q, OCH₂), 3,93 (3H, s, CH₃O), 1,49 (3H, t, CH₃ de l'éthoxy).

### Préparation du 4-éthoxyindoline-2(R/S)-carboxylate de méthyle :

Du 4-éthoxyindole-2-carboxylate de méthyle (1,02 g, 4,568 mmol) a été dissous dans du méthanol (20 ml), puis des copeaux de magnésium (0,55 g, 22,6 mmol) ont été ajoutés tout en agitant, à température ambiante, sous azote. Après que la réaction ait démarrée, on a utilisé un bain d'eau pour maintenir la température de la réaction entre 15-20°C. Après la fin de la réaction, on a ajouté du dichlorométhane (200 ml), puis une solution de NH₄Cl (200 ml) au mélange. La phase organique a été séparée et la phase aqueuse a été extraite avec du dichlorométhane (3 x 50 ml). Les phases organiques combinées ont été séchées sur sulfate de magnésium et le solvant a été éliminé pour donner une huile marron.
RMN ¹H (CDCl₃), δ (ppm) 6,96 - 7,03 (1H, m, ArH), 6,28 - 6,39 (2H, m, ArH), 4,32 - 4,44 (2H, m, COCHNH de l'indoline), 4,03 (2H, q, OCH₂), 3,75 (3H, s, H₃COOC), 3,29 - 3,33 (2H, m, CH₂ de l'indoline), 1,38 (3H, t, CH₃ de l'éthoxy).

### Préparation du butylamide de l'acide 4-éthoxyindoline-2(R/S)-carboxylique :

Du 4-éthoxyindoline-2(R/S)-carboxylate de méthyle (0,84 g, 3,8 mmol) a été dissous dans de la butylamine (40 ml), sous azote, et ensuite la solution a été agitée à reflux pendant trois heures. Après que le mélange ait été refroidi, la butylamine restante a été éliminée sous vide. Le produit brut a été purifié par chromatographie sur une colonne de gel de silice, en utilisant un mélange essence de pétrole : acétate d'éthyle 5:5 comme éluant. On a obtenu un solide jaune.
RMN ¹H (CDCl₃), δ (ppm) 6,99 - 7,05 (2H, m, CONH, ArH), 6,33 - 6,36 (2H, dd, ArH), 4,36 - 4,41 (1H, m, COCHN de l'indoline), 3, 98 - 4,11 (3H, m, NH, OCH₂), 3,52 - 3,59 (1H, m, CH(H) de l'indoline), 3,25 (2H, q, CONCH₂), 2,92 - 2,98 (1H, m, CH(H) de l'indoline), 1,27 - 1,50 (7H, m, CH₂CH₂ du butyle, CH₃ de l'éther), 0,89 (3H, t, CH₃ du butyle).

### Préparation du butylamide de l'acide 1-(N-benzyloxycarbonyl)-2(S)-aminobutyryl)-4-éthoxyindoline-2(S)-carboxylique :

Du butylamide de l'acide 4-éthoxyindoline-2(R/S)-carboxylique (0,720 g, 2,748 mmol) et de l'acide N-benzyloxycarbonyl-2(S)-aminobutyrique (0,787 g, 4,982 mmol) ont été dissous dans du chlorure de méthylène séché (5 ml). La solution a été refroidie jusqu'à 0°C, puis on a ajouté de la triéthylamine (1,39 ml, 9,947 mmol) et du chlorure bis(2-oxo-3-oxazolidinyl)phosphinique (1,268 g, 4,982 mmol). Le mélange a été agité à température ambiante pendant 22 heures, puis lavé avec de l'acide chlorhydrique 3N (3 x 50 ml), du bicarbonate de sodium à 5% (3 x 50 ml) et de l'eau (50 ml), et séché sur du sulfate de sodium. Après avoir retiré le solvant, le produit a été purifié par chromatographie sur une colonne de gel de silice, en utilisant un mélange essence de pétrole : acétate d'éthyle 3:2 comme éluant. On a obtenu un produit blanc - cassé; celui-ci a été recristallisé dans l'éther diéthylique.
RMN ¹H (CDCl₃), δ (ppm) 7,71 - 7,73 (1H, m, CONH), 7,15 - 7,37 (6H, m, ArH), 6,59 - 6,76 (2H, m, ArH), 4,82 - 5,77 (4H, m, NH de l'Abu, CH₂Ph, COCHN de l'indoline), 3,99 - 4,21 (3H, m, OCH₂, COCHN de l'Abu), 3,12 - 3,49 (4H, m, CH₂ de l'indoline, CONCH₂), 0,79 - 2,06 (15H, m, CH₂CH₂CH₃ du butyle, CH₃ de l'éthoxy, CH₂CH₃ de l'Abu).

### Préparation de l'oxalate du butylamide de l'acide 1-(2(S)-aminobutyryl)-4-éthoxyindoline-2(S)-carboxylique : R₁ = CH₂CH₃, R = R' = R₉ = R₁₀ = H, R₈ = OC₂H₅ ;

Du butylamide de l'acide 1-(N-benzyloxycarbonyl)-2(S)-aminobutyryl)-4-éthoxyindoline-2(S)-carboxylique (0,135 g, 28,1 mmol) a été dissous dans du méthanol sec, puis du palladium sur charbon activé (0,040 g) a été ajouté. Ce mélange a été hydrogéné pendant 2,5 heures à température ambiante. Après filtration, de l'acide oxalique (0,0290 g, 32,2 mmol) a été ajouté à la solution. La plus grande partie du méthanol a été éliminée par évaporation, puis de l'éther a été ajouté. On a obtenu un solide blanc.
Point de Fusion: 144,7 - 146,2°C.
C₁₉H₂₉N₃O₃ 1,0 (COOH)₂ 1,0 H₂O,
requis: C 55,37%, H 7,30%, N 9,22%,
trouvé: C 55,11%, H 7,09%, N 9,19%.
RMN ¹H (DMSO-d₆), δ (ppm) 8,50 - 8,53 (1H, m, CONHBu), 7,72 (1H, d, ArH), 7,18 (1H, d, ArH), 6,72 (1H, d, ArH), 5,04 - 5,06 (1H, m, COCHN de l'indoline), 4,03 - 4,06 (2H, m, CH₂O), 2,96 - 3,96 (5H, m, COCHN de l'Abu, CH₂ de l'indoline, CONCH₂), 1,75 - 1,92 (2H, m, CH₂ du butyle), 0,84 - 1,41 (10H, m, CH₂CH₃ de l'Abu, butyle).

### EXEMPLE 32 : voie 9

### Préparation du 2-azido-3-(2,3-diméthoxyphényl)-propénoate d'éthyle:

Des morceaux de sodium (3,678 g, 160 mmol) ont été ajoutés à de l'éthanol (200 ml), sur une période de 30 minutes. La solution résultante a été refroidie jusqu'à -18°C, et ensuite, sur une heure, un mélange de 2,3-diméthoxybenzaldéhyde (4,832 g, 40 mmol) et d'azidoacétate d'éthyle (20,64 g, 160 mmol) a été ajouté à une vitesse qui permettait de maintenir la température au dessous de -15°C. Après trois heures, la solution a été stockée à 5°C pendant deux jours, pour donner un produit cristallin qui a été recueilli et lavé avec de l'hexane froid pour donner un produit pur sous forme d'une huile jaune.
RMN ¹H (CDCl₃), δ (ppm) 7,78 - 7,82 (1H, dd, ArH), 7,32 (1H, s, ArCH), 7,04 - 7,12 (1H, t, ArH), 6,89 - 6,93 (1H, m, ArH), 4,37 (2H, q, OCH₂), 3,86 (3H, s, OCH₃), 3,83 (3H, s, OCH₃),1,39 (3H, t, CH₃).

### Préparation du 4,5-diméthoxyindole-2-carboxylate d'éthyle :

Du 2-azido-3-(2,3-diméthoxyphényl)-propénoate d'éthyle (4,0 g, 144 mmol) a été mis en suspension dans du toluène (120 ml) et le mélange a été chauffé à reflux pendant trois heures, puis refroidi et agité à température ambiante pendant la nuit. Les solides ont été recueillis par filtration pour donner le produit sous la forme d'un solide jaune.
RMN ¹H (CDCl₃), δ (ppm) 8,76 - 8,96 (1H, m, NH), 7,30 (1H, dd, ArH), 7,04 - 7,12 (2H, m, ArH), 4,36 (2H, q, O-CH₂-), 4,06 (3H, s, CH₃O), 3,86 (3H, s, CH₃O), 1,37 (3H, t, -CH₃).

### Préparation du 4,5-diméthoxyindoline-2(R/S)-carboxylate de méthyle :

Du 4,5-diméthoxyindole-2-carboxylate d'éthyle (0,500 g, 2 mmol) a été dissous dans du méthanol (10 ml), puis des copeaux de magnésium (0,2431 g, 10 mmol) ont été ajoutés tout en agitant, à température ambiante, sous une atmosphère d'azote. Après que la réaction ait démarrée, on a utilisé un bain d'eau pour maintenir la température de la réaction entre 5-15°C. Après la fin de la réaction, on a ajouté du dichlorométhane (200 ml), puis une solution de NH₄Cl (200 ml) au mélange. La phase organique a été séparée et la phase aqueuse a été extraite avec du dichlorométhane (3 x 50 ml). Les phases organiques combinées ont été séchées sur sulfate de magnésium et le solvant a été éliminé pour donner une huile marron.
RMN ¹H (CDCl₃), δ (ppm) 6,64 (1H, d, ArH), 6,38 (1H, d, ArH), 4,28 - 4,36 (2H, m, NH, COCHN de l'indoline), 3,85 (3H, s, H₃CO), 3,77, 3,75 (6H, s, H₃CO, H₃COO), 3,37 - 3,41 (2H, m, CH₂).

### Préparation du butylamide de l'acide 4,5-diméthoxyindoline-2(R/S)-carboxylique :

Du 4,5-diméthoxy-indoline-2(R/S)-carboxylate de méthyle (0,447 g, 1,89 mmol) a été dissous dans de la butylamine (35 ml), sous azote, et ensuite la solution a été agitée à reflux pendant trois heures. Après que le mélange ait refroidi, la butylamine restante a été éliminée sous vide. Le produit brut a été recristallisé dans l'éther diéthylique pour donner un solide blanc - cassé.
RMN ¹H (CDCl₃), δ (ppm) 7,06 - 7,18 (1H, m, CONH), 6,66 (1H, d, ArH), 6,39 (1H, d, ArH), 4,28 - 4,44 (1H, m, COCHN de l'indoline), 3,59 - 3,98 (2H, m, NH, CH(H) de l'indoline), 3,84 (3H, s, OCH₃), 3,79 (3H, s, OCH₃), 3,11 - 3,28 (3H, m, CH(H de l'indoline, CONCH₂), 1,30 - 1,52 (4H, m, CH₂CH₂)_{'} 0,90 (3H, t, CH₃

### Préparation du butylamide de l'acide 1-(N-benzyloxycarbonyl)-2(S)-aminobutyryl)-4,5-diméthoxyindoline-2(R/S)-carboxylique :

Du butylamide de l'acide 4,5-diméthoxyindoline-2(R/S)-carboxylique (0,300 g, 1,079 mmol) et de l'acide N-benzyloxycarbonyl-2(S)-aminobutyrique (0,3422 g, 1,444 mmol) ont été dissous dans du chlorure de méthylène séché (10 ml). La solution a été refroidie à 0°C, puis on a ajouté de la triéthylamine (0,49 ml, 3,46 mmol) et du chlorure bis(2-oxo-3-oxazolidinyl)phosphinique (0,4409 g, 1,732 mmol). Le mélange a été agité à température ambiante pendant 22 heures, puis lavé avec de l'acide chlorhydrique 3N (3 x 20 ml), du bicarbonate de sodium à 5% (3 x 20 ml) et de l'eau (20 ml), et séché sur du sulfate de sodium. Après avoir retiré le solvant, le résidu a été purifié par chromatographie sur une colonne de gel de silice, en utilisant un mélange dichlorométhane : acétate d'éthyle 3:2 comme éluant. On a obtenu un produit blanc - cassé.
RMN ¹H (CDCl₃), δ (ppm) 7,75 - 7,86 (1H, m, CONH), 7,10 - 7,35 (5H, m, ArH), 6,73 - 6,80 (2H, m, ArH), 4,82 - 5,36 (4H, m, PhCH₂, NH de l'Abu, COCHN de l'indoline), 3,14 - 4,8 (11H, m, COCHN de l'Abu, CH₂ de l'indoline, 2 x CH₃O, CONCH₂), 0,78 - 1,80 (12H, m, CH₂CH₂CH₃ du butyle, CH₂CH₃ de l'Abu).

### Préparation de l'oxalate du butylamide de l'acide 1-(2(S)-aminobutyryl)-4,5-diméthoxyindoline-2(R/S)-carboxylique : R₁ = CH₂CH₃, R = R' = R₁₀ = H, R₈ = R₉ = OCH₃ ;

Du butylamide de l'acide 1-(benzyloxycarbonyl)-2(S)-aminobutyryl)-4,5-diméthoxyindoline-2(R/S)-carboxylique (0,100 g, 0,20 mmol) a été dissous dans du méthanol sec (10 ml), puis du palladium sur charbon activé (0,038 g) a été ajouté. Ce mélange a été hydrogéné pendant 2,5 heures a température ambiante. Après filtration, de l'acide oxalique (0,020 g, 0,22 mmol) a été ajouté à la solution. La plus grande partie du méthanol a été éliminée par évaporation, puis de l'éther a été ajouté. On a obtenu un solide blanc - cassé.
Point de Fusion: 196,1 - 197,5°C. C₁₉H₂₉N₃O₄ 1,0 (COOH)₂ 1,0 H₂O,
requis: C 53,49%, H 7,05%, N 8,91%,
trouvé: C 53,46%, H 6,89%, N 8,80%.
RMN ¹H (DMSO-d₆), δ (ppm) 8,48 - 8,52 (1H, m, CONHBu), 7,79 (1H, d, ArH), 6,90 (1H, d, ArH), 5,03 - 5,06 (1H, m, COCHN de l'indoline), 3,57 - 3,77 (8H, m, 2 x CH₃O, CH(H) de l'indoline, COCHN de l'Abu), 3,08 - 3,12 (3H, m, CONCH₂, CH(H) de l'indoline),1,77 - 1,93 (2H, m, CH₂ de l'Abu), 1,25 - 1,42 (4H, m, CH₂CH₂ du butyle), 0,85 - 1,04 (6H, m, CH₃ de l'Abu, CH₃ du butyle).

### EXEMPLE 33 : voie 12

### Préparation du butylamide de l'acide 1-(N-benzyloxycarbonyl)-2(S)-aminobutyryl)-5-benzyloxyindoline-2(R/S)-carboxylique :

Du butylamide de l'acide 5-benzyloxyindoline-2(R/S)-carboxylique (0,648 g, 2 mmol), de l'acide N-benzyloxycarbonyl-2(S)-aminobutyrique (0,521 g, 2,2 mmol) et du dicyclohexylcarbodiimide (2,2 mmol) ont été dissous dans du dichlorométhane (70 ml) et agités pendant 18 h. Le mélange a été lavé avec une solution de NaHCO₃, les eaux de lavage ont été extraites avec de l'acétate d'éthyle et les portions organiques combinées ont été séchées (Na₂SO₄). Le solvant a été éliminé pour donner un solide qui a été purifié par chromatographie sur une colonne de gel de silice, en utilisant un mélange acétate d'éthyle : essence de pétrole 1:1, pour donner le diastéréoisomère (S/S) seul et les diastéréoisomères (S/S et S/R) mélangés.
RMN δ = 8,00 - 8,03 (1H, m, NH), 7,24 - 7,40 (10H, m, 2 x Ph), 6,78 - 6,90 (3H, m, ArH), 4,78 - 5,34 (6H, m, 2 x PhCH₂, NCHCO de l'Abu, NH), 4,3 - 4,18 (1H, m, NCHCO de l'indoline), 3,43 - 3,49 (2H, m, CH₂ de l'indoline), 3,14 - 3,18 (2H, m, CONHCH₂), 0,77 - 1,93 (12H, m, 2 x CH₃, 3 x CH₂).

### Préparation de l'oxalate du butylamide de l'acide 1-(2(S)-aminobutyryl)-5-hydroxyindoline-2(S)-carboxylique : R₁ = CH₂CH₃, R = R' = R₈ = R₁₀ = H, R₉ = OH ;

Du butylamide de l'acide N-benzyloxycarbonyl-2(S)-aminobutyryl-5-benzyloxyindoline-2(S)-carboxylique (200 mg, 0,36 mmol) a été dissous dans du méthanol (20 ml), et traité avec du palladium sur du charbon activé (80 mg). Le mélange a été hydrogéné à 80 psi pendant 2,5h. Le catalyseur a été retiré par filtration et le solvant a été réduit. De l'acide oxalique (36 mg) puis de l'éther ont été ajoutés pour fournir le produit sous la forme d'un solide blanc.
Point de Fusion: 156,5 - 158°C.
C₁₇H₂₅N₃O₃ 1,1 (COOH)₂ 0,3 H₂O 0,5(C₂H₅)₂O,
requis: C 55,23%, H 7,19%, N 9,12%
trouvé: C 54,99%, H 7,29%, N 9,08%.
RMN d = 8,42 - 8,43 (1H, m, NH), 7,91 (1H, d, ArH), 6,57 - 6,70 (2H, m, ArH), 5,59 - 4,87 (1H, m, NCHCO de l'Abu), 3,55 - 3,59 (1H, m, NCHCO de 1-indoline), 3,33 - 3,44 (2H, m, CONCH₂), 3,00 - 3,16 (2H, m, CH₂ de l'indoline), 0,84 - 1,87 (12H, m, 3 x CH₂, 2 x CH₃).

### EXEMPLE 34 : voie 12

### Préparation de l'oxalate du butylamide de l'acide 1-2(S)-aminobutyryl-5-hydroxyindoline-2(R/S)-carboxylique : R₁ = CH₂CH₃, R = R' = R₈ = R₁₀ = H , R₉ = OH ;

La procédure précédente a été répétée sur le butylamide de l'acide ,N-benzyloxycarbonyl-2(S)-aminobutyryl-5-benzyloxyindoline-2(R/S)-carboxylique (100 mg, 0,18 mmol) pour donner le produit désiré.
Point de Fusion: 129 - 130°C.
C₁₇H₂₅N₃O₃ 0,9(COOH)₂ 0,5H₂O 0,3(C₂H₅)₂O,
requis: C 55,63%, H 7,20%, N 9,74%,
trouvé: C 55,44%, H 7,31%, N 9,73%.
RMN δ = 8,43 - 8,44 (1H, m, CONH), 7,86 - 7,94 (1H, d, ArCH),
6,58 - 6,67 (2H, m, ArCH), 4,93 - 5,20 (1H, m, NCHCO de l'Abu),
3,36 - 3,81 (2H, m, NCHCO de l'indoline, CH(H) de l'indoline), 2,90 - 3,11 (3H, m, CH(H) de l'indoline, CONHCH₂), 0,84 - 1,74 (12H, m, 3 x CH₂, 2 x CH₃).

### EXEMPLE 35 : voie 8

### Préparation du 5-méthylindole-2-carboxylate de méthyle :

De l'acide 5-méthylindole-2-carboxylique (0,9 g, 5,13 mmol) a été dissous dans du méthanol (25 ml) et traité avec de l'acide sulfurique (0,5 ml). Le mélange a été maintenu à reflux pendant 4h. Le volume de solvant a été réduit et le résidu traité avec une solution d'hydrogéno-carbonate de sodium, et extrait avec de l'acétate d'éthyle. Les portions organiques ont été séchées et concentrées pour donner l'ester souhaité.

### Préparation du 5-méthylindoline-2(R/S)-carboxylate de méthyle :

Du 5-méthylindole-2-carboxylate de méthyle (0,95 g, 5 mmol) et des copeaux de magnésium (0,62 g, 25 mmol) ont été mis en suspension dans du méthanol (25 ml), et le mélange a été agité à 15°C pendant 2h. Le mélange a été filtré, dilué avec du chlorure de méthylène (100 ml) et lavé avec une solution de NH₄Cl. La phase organique a été séchée (Na₂SO₄) et le solvant retiré pour donner le produit brut qui a été purifié par chromatographie sur une colonne de gel de silice, en utilisant un mélange pétrole: acétate d'éthyle 7:3 comme éluant.
RMN δ = 6,63 - 6,93 (3H, m, ArCH), 4,33 - 4,41 (1H, m, NCHCO), 3,75 (3H, s, CH₃O), 3,29 - 3,36 (2H, m, CH₂), 2,24 (3H, s, CH₃Ar).

### Préparation du butylamide de l'acide 5-méthylindoline-2(R/S)-carboxylique :

Du 5-méthylindoline-2(R/S)-carboxylate de méthyle (0,3 g, 4,18 mmol) a été dissous dans de la butylamine (20 ml), et le mélange a été maintenu à reflux pendant 4h sous azote. L'excès d'amine a été retiré sous vide pour donner un solide qui a été recristallisé dans l'éther pour permettre de récolter le produit. RMN δ = 6,60 - 6,90 (3H, m, ArCH), 4,21 - 4,33 (2H, m, CONH, NCHCO), 3,43 - 3,48 (1H, m, CH(H) de l'indoline), 3,22 - 3,28 (2H, m, NCH₂), 3,02 - 3,18 (1H, m, CH(H) de l'indoline), 2,24 (3H, s, CH₃Ar), 1,29 - 1,50 (4H, m, 2 x CH₂), 0,86 - 0,93 (3H, m, CH₃).

### Préparation du butylamide de l'acide 1-N-benzyloxycarbonyl-2(S)-aminobutyryl-5-méthylindoline-2(R/S)-carboxylique :

On a ajouté, à une solution de butylamide de l'acide 5-méthylindoline-carboxylique (0,46 g, 2 mmol) et d'acide N-benzyloxycarbonyl-2(S)-aminobutyrique dans le dichlorométhane (10 ml), de la triéthylamine (0,84 ml) et du chlorure bis(2-oxo-3-oxazolidinyl)phosphinique (0,76 g) sous azote. Le mélange a été agité à température ambiante pendant 20 heures. La solution a été lavée avec une solution de NH₄Cl saturée et de l'eau. Les portions aqueuses combinées ont été extraites avec du chlorure de méthylène. Les fraction organiques combinées ont été séchées et le solvant a été retiré pour donner une huile jaune qui a été purifiée par chromatographie sur une colonne de gel de silice, en utilisant un mélange éther : pétrole 7:3 comme éluant. Le produit désiré a été obtenu sous la forme d'un solide blanc - cassé.
RMN δ = 7,97 - 8,00 (1H, m, NH), 7,24 - 7,38 (5H, m, ArCH), 6,89 - 7,07 (3H, m, ArCH), 4,79 - 5,31 (4H, m, PhCH₂, NH, NCHCO de l'Abu), 4,00 - 4,40 (1H, m, NCHCO de l'indoline), 3,42 - 3,57 (2H, m, CONHCH₂), 3,09 - 3,22 (2H, m, CH₂ de l'indoline), 2,29 (3H, s, CH₃Ar), 0,80 - 2,26 (12H, m, 3 x CH₂, 2 x CH₃).

### Préparation de l'oxalate du butylamide de l'acide 1-2 (S)-aminobutyryl-5-méthylindoline-2 (R/S)-carboxylique : R₁ = CH₂CH₃, R = R' = R₈ = R₁₀ = H, R₉ = CH₃ ;

Du butylamide de l'acide 1-N-benzyloxycarbonyl-2(S)-aminobutyryl-5-méthylindoline-2(R/S)-carboxylique (130 mg) a été dissous dans du méthanol (12 ml) et traité avec du palladium sur charbon activé (40 mg). Le mélange a été hydrogéné à 60 psi pendant 2,5h. Le catalyseur a été retiré par filtration et le volume de solvant a été réduit. Ce mélange a été traité avec de l'acide oxalique (28,8 mg) et de l'éther pour donner le produit désiré.
Point de Fusion: 110 - 2°C.
C₁₈H₂₇N₃O₂ (COOH)₂ 0,1 H₂O,
requis: C 58,70%, H 7,19%, N 10,27%,
trouvé: C 58,83%, H 7,20%, N 9,88%.
RMN δ = 8,45 - 8,46 (1H, m, CONH), 7,90 - 7,99 (1H, m, ArCH), 7,33 - 7,35 (2H, m, ArCH), 7,00 - 7,06 (1H, m, NH), 4,97 - 5,02 (2H, m, NCHCO de l'Abu, NH), 3,55 - 3,90 (5H, m, CONHCH₂, NCHCO et CH₂ de l'indoline), 2,23, 2,25 (3H, s, CH₃-Ar), 0,8 - 2,0 (12H, m, 3 x CH₂, 2 x CH₃).

### EXEMPLE 36 : voie 13

### Préparation de l'ester méthylique de l'acide 5-chloroindole-2-carboxylique :

De l'acide 5-chloroindole-2-carboxylique (0,95 g, 4,65 mmol) a été dissous dans du méthanol (35 ml) et traité avec de l'acide sulfurique (0,5 ml). Le mélange a été maintenu à reflux pendant 5h, refroidi, et le solvant a été retiré. Le résidu a été recristallisé deux fois dans le méthanol pour donner le produit.

### Préparation du 5-chloroindoline-2(R/S)-carboxylate de méthyle :

Du 5-chloroindole-2-carboxylate de méthyle (0,6 g, 2,87 mmol) et des copeaux de magnésium (0,34 g, 14,3 mmol) ont été mis en suspension dans du méthanol (40 ml), et le mélange a été agité pendant 8h. Le mélange a été filtré, traité avec du chlorure de méthylène (100 ml) et lavé avec une solution de NH₄Cl. La portion organique a été séchée (Na₂SO₄), et le solvant a été retiré pour donner un solide qui a été purifié par chromatographie sur une colonne de gel de silice, en utilisant un mélange pétrole : acétate d'éthyle 7:3 comme éluant. Le produit a été isolé sous la forme d'un solide jaune.

### Préparation du butylamide de l'acide 5-chloroindoline-2(R/S)-carboxylique :

Du 5-chloroindoline-2(R/S)-carboxylate de méthyle (0,63 g, 3 mmol) a été dissous dans de la butylamine (25 ml) et chauffé à reflux pendant 4h. L'excès d'amine a été retiré et le résidu a été cristallisé dans l'éther pour donner un solide jaune.

### Préparation du butylamide de l'acide 1-N-t-butoxycarbonyl-2(S)-aminobutyryl-5-chloroindoline-2(S)-carboxylique :

Du butylamide de l'acide 5-chloroindoline-2(R/S)-carboxylique (0,52 g, 2 mmol) et de l'acide N-t-butoxy-carbonyl-2(S)-aminobutyrique (0,45 g, 2,2 mmol), ont été dissous dans du chlorure de méthylène (20 ml) et traités avec du chlorure bis(2-oxo-3-oxazolidinyl)phosphinique (0,64 g, 2,5 mmol) et de la triéthylamine (0,5 g, 5 mmol). Le mélange a été agité à température ambiante pendant 12h, puis lavé avec de l'acide citrique à 10% (3 x 50 ml), et NaHCO₃ à 5% (3 x 50 ml) et de l'eau (50 ml). La phase organique a été séchée et concentrée. Le résidu a été purifié par chromatographie pour donner le produit.

### Préparation du trifluoroacétate de butylamide de l'acide 1-(2(S)-aminobutyryl)-5-chloroindoline-2(S)-carboxylique : R₁ = CH₂CH₃, R = R' = R₈ = R₁₀ = H, R₉ = Cl ;

Du butylamide de l'acide de 1-N-t-butoxycarbonyl-2(S)-aminobutyryl-5-chloroindoline-2(R/S)-carboxylique (50 mg, 0,114 mmol) a été dissous dans de l'acide trifluoro-acétique (2 ml) et du chlorure de méthylène (2 ml), et maintenu à température ambiante pendant 40 minutes. L'évaporation des solvants et l'addition d'éther diéthylique ont fourni le produit.
Point de Fusion: 83 - 84°C.
C₁₇H₂₄N₃O₂Cl 1,5 CF₃COOH,
requis: C 47,21%, H 5,05%, N 7,85%,
trouvé: C 47,43%, H 5,24%, N 8,42%.
RMN δ = 8,07 - 8,09 (1H, d, ArH), 7,27 - 7,36 (2H, m, ArH), 5,03 - 5,06 (1H, m, NCHCO de l'Abu), 3,59 - 3,70 (2H, m, NCHCO de l'indoline, CH(H) de l'indoline), 3,06 - 3,12 (3H, m, CH(H) de l'indoline, CONHCH₂), 1,77 - 1,94 (2H, m, CH₂ de l'Abu), 1,24 - 1,43 (4H, m, CH₂CH₂CH₃), 0,83 - 1,58 (6H, m, 2 x CH₃).

### EXEMPLE 37 : voie 14

### Préparation d'indoline-2(S)-carboxylate de méthyle :

De l'acide indoline-2(S)-carboxylique (10 g, 64 mmol) a été maintenu à reflux dans un mélange de méthanol (50 ml) et d'acide sulfurique (8 ml) pendant 5h. Le solvant a été retiré pour donner le produit sous la forme d'une huile.

### Préparation de l'ester méthylique de l'acide 1-(N-t-butoxycarbonyl-2(S)-aminobutyryl)-indoline-2(S)-carboxylique :

On a ajouté à une solution d'ester méthylique de l'acide indoline-2-carboxylique (2g, 11,3 mmol) et d'acide N-t-butoxycarbonyl-2(S)-aminobutyrique (2,6 g, 13,6 mmol) dans le chlorure de méthylène (15 ml), de la triéthylamine (3,15 ml) et du chlorure bis(2-oxo-3-oxazolidinyl)-phosphinique (5,75 g, 22,6 mmol) à 0°C sous azote. La réaction a été agitée pendant 2h à 5°C, puis jusqu'au lendemain à température ambiante. Le mélange a été filtré, et le filtrat a été lavé avec de l'eau (2 x 40 ml), et les portions aqueuses combinées ont été extraites avec du chlorure de méthylène. Les portions organiques combinées ont été séchées et concentrées pour donner un résidu qui a été purifié par chromatographie sur une colonne de gel de silice, en utilisant un mélange essence de pétrole : acétate d'éthyle 7:3 comme éluant. Le produit a été obtenu sous forme d'un solide blanc.

### (3-hydroxy)propylamide de l'acide 1-(N-t-butoxycarbonyl-2(S)-aminobutyryl)-indoline-2(S)-carboxylique :

De l'ester méthylique de l'acide 1-(N-t-butoxycarbonyl-2(S)-aminobutyryl)-indoline-2(S)-carboxylique (0,36 g, 1 mmol) a été dissous dans du méthanol (10 ml), et traité, sous azote, à 50°C, avec de la 3-hydroxypropylamine (10 ml). Le mélange a été agité à 50°C pendant 3h, et le solvant a été évaporé. Le résidu a été dissous dans du chlorure de méthylène (30 ml) et lavé avec du KHSO₄ M. La phase organique a été séchée (Na₂SO₄) et évaporée. Le résidu a été purifié par chromatographie sur colonne pour donner le produit.

**Trifluoroacétate de (3-hydroxy)propylamide de l'acide 1-(2(S)-aminobutyryl)-indoline-2(S)-carboxylique:R**_{**1**} **= CH**_{**2**}**CH**_{**3**}**, R**_{**2**} **= H, R = R' = R**_{**8**} **= R**_{**9**} **= R**_{**10**} **= H, n = 0 et m =** 1, R₃ = 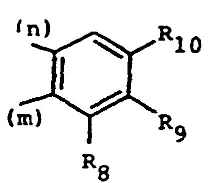 **R**_{**4**} **= CONHR**_{**5**}**, R**_{**5**} **= (CH**_{**2**}**)**_{**3**}**OH ;**

Du (3-hydroxy)propylamide de l'acide 1-(N-t-butoxycarbonyl)-2(S)-aminobutyryl)-indoline-2(S)-carboxylique (0,12 g) a été dissous dans du chlorure de méthylène (1 ml) et traité avec de l'acide trifluoroacétique (1 ml). La solution a été agitée à température ambiante pendant 20 minutes, et les solvants ont été retirés pour donner une huile. Une addition d'ether et une filtration ont donne le produit sous forme de solide.
Point de Fusion: 86 - 88°C.
C₁₆H₂₃N₃O₃ 1,5 CF₃COOH,
requis: C 47,97%, H 5,11%, N 8,67%,
trouvé: C 47,90%, H 5,18%, N 8,82%.
RMN δ = 8,11 (1H, d, ArH), 7,05 - 7,29 (3H, m, ArH), 5,02 - 5,04 (NCHCO de l'Abu) 4,37 - 4,65 (NCHCO de l'indoline, OH), 3,62 - 3,68 (2H, m, CH₂OH), 3,34 - 3,42 (1H, m, CH(H) de l'indoline), 3,09 - 3,21 (3H, m, CH(H) de l'indoline, CONHCH₂), 1,56 - 1,94 (4H, m, CH₂CH₂OH, CH₂CH₃), 0,94 - 1,1 (3H, m, CH₃).

### MESURE DE L'ACTIVITE TRIPEPTIDYLPEPTIDASE MEMBRANAIRE

Après avoir isolé et caractérisé la tripeptidylpeptidase membranaire précitée, les inventeurs ont mis au point une mesure de son activité décrite ci-après.

Des membranes de cerveau de rat ont été obtenues par centrifugation (200 000 x g min) d'un homogénat préparé dans 10 volumes de tampon phosphate de potassium 50 mM, pH 7,4.

Le culot de centrifugation soigneusement lavé est repris dans le tampon contenant 10 % de glycérol et 0,1 % de Brij 35 pour obtenir une concentration de 25 mg de protéines/ml.

Après 25 minutes de préincubation, les incubations sont pratiquées pendant 15 minutes à 37°C dans le tampon sous un volume de 0,1 ml contenant 0,1 % de Brij 35, 100 mM de bestatine, 25 µg de membrane et 50 µM de substrat (A-AP-Amc). La libération d'Amc est évaluée par fluorométrie.

### MISE AU POINT D'INHIBITEURS

L'effet des composés selon l'invention donnés en exemple ci-dessus a été évalué par mesure de l'activité tripeptidylpeptidase II membranaire. Le pouvoir inhibiteur de ces composés a été exprimé par leur constante de dissociation apparente (Ki), calculée à partir des valeurs de leur concentration inhibitrice 50 % et du K_{M} du substrat (23 µM).

Le tableau II suivant rapporte une série de valeurs caractéristiques des composés selon l'invention.

**TABLEAU II**

| INHIBITION DE L'ACTIVITE AMINOTRIPEPTIDYLPEPTIDASE MEMBRANAIRE DE CERVEAU | | |
|---|---|---|
| | n° exemple | Ki µM |
| | 1 | 0,9 |
| | 2 | 0,3 |
| | 3 | 0,270 |
| | 4 | 0,10 |
| | 5 | 0,5 |
| | 6 | 0,080 |
| | 7 | 0,320 |
| | 8 | 0,57 |
| | 9 | 0,00030 |
| | 10 | 0,00030 |
| | 11 | 0,320 |
| | 12 | 0,140 |
| | 13 | 0,040 |
| | 14 | 0,0052 |
| | 15 | 0,0060 |
| | 16 | 0,340 |
| | 17 | 0,012 |
| | 18 | 0,008 |
| | 19 | 0,077 |
| | 20 | 0,012 |
| | 21 | 1,0 |
| | 22 | 0,560 |
| | 23 | 0,143 |
| 2(S)-aminobutyryl-L-prolinamide* | connu | 0,57 |
| isomère (S,S) | 20 | 0,003 |
| isomère (S,R) | 20 | 0,233 |
| isomère (S,S) | 18 | 0,006 |
| isomère (S,R) | 18 | 0,241 |
| | 24 | 0,146 |
| | 25 | 0,026 |
| | 26 | 0,0075 |
| | 27 | 0,014 |
| | 28 | 0,864 |
| | 29 | 0,593 |
| | 30 | 0,032 |
| | 31 | 0,029 |
| | 32 | 0,0096 |
| | 33 | 0,0066 |
| | 34 | 0,0104 |
| | 35 | 0,0226 |
| | 36 | 0,0025 |
| | 37 | 0,0124 |

| | | |
|---|---|---|
| * R₁ = CH(CH₃)₂ ; R₂ = H ; n = O ou 1 et m = O ou 1 avec n différent de m ; R = R' = H ; R₃ = -(CH₂)₂- ; R₄ = CO-NH-R₅ ; R₅ = H | | |

Des effets remarquables des composés selon l'invention ont été mis en évidence en particulier pour les composés présentant un squelette indoline. Il est notable que le composé de l'exemple 22, par exemple, a un effet protecteur de CCK-8 endogène sur coupe de cortex cérébral de rat dépolarisé mis en évidence selon la méthode de Rose et al. (Proc. Natl. Acad. Sci. USA, 1988, 85 : 8326). A une concentration de 1 µM, il multiplie par 4 la récupération de CCK-8 libérée (mesurée par un radioimmunodosage).

L'activité anorexigène (pro-satiété) de ce même composé est mise en évidence chez le rongeur : chez la souris, à la dose de 10 mg/kg (par voie intraveineuse), ce composé diminue de 45 % la prise spontanée de nourriture solide (comprimé "UAR") mesurée sur une période de 30 à 90 minutes après libre accès à cette nourriture (période précédée par mise à jeun d'une nuit et administration de 0,2 ml d'un repas semi-liquide par voie orale).

Cet essai est complété par une évaluation de l'activité inhibitrice de TPP II "ex vivo" consistant à administrer un composé selon l'invention à un rongeur et à mesurer l'activité catalytique résiduelle sur membranes de cerveau ou d'un organe périphérique.

Par exemple, l'activité de TPP II hépatique mesurée chez la souris 90 minutes après administration de 10 mg/kg du composé de l'exemple 9 par voie orale est inhibée à environ 90-100 %.

### LISTE DE SEQUENCES

SEQ ID1

cgcccctcgtccgcgcgctgcctggcagtttacctcttccacgtccgtcctccagcttgc 60
gtcc ATG GCC ACC GCT GCG ACC GAG GAG CCT TTC CCT TTC CAT GGT 106
M A T A A T E E P F P F H G 14

CTT CTA CCA AAG AAA GAG ACT GGG GCC TCC TCC TTC CTG TGC CGC 151
L L P K K E T G A S S F L C R 29

TAC CCG GAG TAT GAC GGA CGC GGG GTG CTC ATC GCC GTC CTG GAC 196
Y P E Y D G R G V L I A V L D 44

ACA GGG GTT GAT CCC GGG GCC CCG GGC ATG CAG GTC ACG ACT GAT 241
T G V D P G A P G M Q V T T D 59

GGG AAA CCA AAA ATC ATT GAT ATC ATT GAT ACA ACA GGA AGT GGT 286
G K P K I I D I I D T T G S G 74

GAT GTG AAT ACT GCT ACA GAA GTA GAA CCA AAA GAT GGC GAA ATC 331
D V N T A T E V E P K D G E I 89

ACT GGT CTT TCT GGA AGA GTG CTT AAG ATT CCT GCA AAC TGG ACA 376
T G L S G R V L K I P A N W T 104

AAT CCT TCA GGA AAA TAT CAT ATT GGC ATT AAA AAT GGT TAT GAC 421
N P S G K Y H I G I K N G Y D 119

TTC TAT CCA AAG GCT CTC AAG GAA AGG ATA CAG AAA GAA CGG AAG 466
F Y P K A L K E R I Q K E R K 134

GAA AAA ATC TGG GAT CCA ATT CAC AGA GTT GCG CTT GCA GAA GCT 511
E K I W D P I H R V A L A E A 149

TGT AGA AAA CAA GAA GAA TTT GAT ATT GCC AAT AAT GGC TCT TCC 556
C R K Q E E F D I A N N G S S 164

CAA GCC AAT AAA CTA ATC AAG GAA GAG TTG CAG AGT CAA GTG GAA 601
Q A N K L I K E E L Q S Q [?] E 179

TTA CTT AAT TCT TTT GAG AAA AAG TAT AGT GAT CCA GGC CCT GTG 646
L L N S F E K K Y S D P G P V 194

TAT GAC TGC TTG GTG TGG CAT GAT GGT GAG ACC TGG AGA GCC TGT 691
Y D C L V W H D G E T W R A C 209

GTT GAT TCG AAT GAA AAT GGG GAC TTG GGT AAA TCT ACT GTA TTG 736
V D S N E N G D L G K S T V L 224

AGA AAC TAC AAA GAG GCT CAA GAA TAT GGT TCT TTT GGC ACA GCT 781
R N Y K E A Q E Y G S F G T A 239

GAG ATG CTG AAT TAC TCT GTT AAT ATT TAT GAT GAT GGA AAC CTG 826
E M L N Y S V N I Y D D G N L 254

CTC TCC ATT GTG ACC AGT GGA GGA GCT CAT GGA ACC CAT GTA GCG 871
L S I V T S G G A H G T H V A 269

AGT ATA GCT GCT GGG CAT TTT CCA GAA GAG CCT GAA CGG AAT GGA 916
S I A A G H F P E E P E R N G 284

GTT GCT CCT GGT GCT CAA ATT CTA TCC ATT AAG ATT GGT GAT ACA 961
V A P G A Q I L S I K I G D T 299

AGG CTA AGC ACC ATG GAA ACA GGC ACA GGC CTC ATC AGA GCT ATG 1006
R L S T M E T G T G L I R A M 314

ATA GAA GTT ATA AAT CAT AAG TGT GAT CTT GTC AAC TAC AGT TAT 1051
I E V I N H K C D L V N Y S Y 329

GGA GAA GCA ACT CAT TGG CCA AAT TCT GGG AGA ATT TGT GAA GTA 1096
G E A T H W P N S G R I C E V 344

ATT AAT GAA GCA GTA TGG AAA CAT AAT ACA ATT TAT GTT TCA AGT 1141
I N E A V W K H N T I Y V S S 359

GCT GGA AAT AAT GGT CCA TGC CTT TCT ACA GTG GGT TGT CCA GGA 1186
A G N N G P C L S T V G C P G 374

GGA ACT ACT TCC AGC GTG ATA GGT GTT GGA GCT TAT GTT TCT CCT 1231
G T T S S V I G V G A Y V S P 389

GAT ATG ATG GTT GCA GAG TAT TCA CTG AGA GAG AAA CTA CCT GCA 1276
D M M V A E Y S L R E K L P A 404

AAT CAA TAT ACA TGG TCT TCT AGA GGC CCA AGT GCT GAT GGG GCC 1321
N Q Y T W S S R G P S A D G A 419

CTT GGT GTG AGC ATC AGT GCA CCA GGA GGT GCT ATT GCT TCT GTG 1366
L G V S I S A P G G A I A S V 434

CCT AAC TGG ACA TTG AGG GGG ACT CAG CTA ATG AAT GGG ACA TCA 1411
P N W T L R G T Q L M N G T S 449

ATG TCT TCC CCC AAT GCC TGT GGT GGC ATT GCC CTG GTA CTT TCA 1456
M S S P N A C G G I A L V L S 464

GGG CTG AAA GCA AAT AAT GTT GAC TAT ACT GTT CAC TCA GTC AGA 1501
G L K A N N V D Y T V H S V R 479

AGA GCT CTA GAA AAC ACT GCA ATA AAA GCT GAC AAT ATA GAA GTA 1546
R A L E N T A I K A D N I E V 494

TTT GCC CAA GGA CAT GGA ATT ATT CAG GTT GAC AAA GCT TAT GAC 1591
F A Q G H G I I Q V D K A Y D 509

TAC CTC ATT CAG AAT ACA TCA TTT GCT AAC AGA TTA GGT TTT ACA 1636
Y L I Q N T S F A N R L G F T 524

GTT ACA GTT GGA AAT AAC CGT GGC ATC TAC CTC CGA GAT CCT GTT 1681
V T V G N N R G I Y L R D P V 539

CAG GTG GCT GCT CCT TCA GAT CAT GGT GTT GGC ATT GAG CCT GTA 1726
Q V A A P S D H G V G I E P V 554

TTT CCA GAG AAC ACA GAA AAC TCT GAA AAA ATA TCC TTC CAG CTT 1771
F P E N T E N S E K I S F Q L 569

CAT TTA GCT TTA ACT TCA AAT TCA TCT TGG GTT CAA TGT CCC AGC 1816
H L A L T S N S S W V Q C P S 584

CAT TTG GAA CTC ATG AAT CAA TGT CGG CAC ATA AAC ATA CGT GTG 1861
H L E L M N Q C R H I N I R V 599

GAC CCC AGG GGC TTA AGA GAA GGG TTA CAT TAT ACA GAG GTA TGT 1906
D P R G L R E G L H Y T E V C 614

GGG TAT GAT ATA GCA TCC CCC AAT GCA GGT CCT TTA TTC AGA GTT 1951
G Y D I A S P N A G P L F R V 629

CCA ATC ACT GCA GTT ATA GCA GCA AAA GTA AAT GAA TCA TCA CAT 1996
P I T A V I A A K V N E S S H 644

TAT GAT CTA GCC TTT ACA GAT GTA CAT TTT AAA CCT GGT CAG ATT 2041
Y D L A F T D V H F K P G Q I 659

CGA AGA CAT TTT GTT GAG GTT CCT GAG GGG GCA ACT TGG GCT GAA 2086
R R H F V E V P E G A T W A E 674

GTT ACC GTG TGT TCG TGT TCT TCT GAG GTA TCA GCA AAA TTT GTT 2131
V T V C S C S S E V S A K F V 689

CTT CAT GCA GTA CAG CTT GTG AAG CAG AGA GCA TAT CGA AGT CAT 2176
L H A V Q L V K Q R A Y R S H 704

GAA TTT TAT AAG TTT TGT TCC CTT CCA GAA AAA GGA ACA CTC ATT 2221
E F Y K F C S L P E K G T L I 719

GAA GCC TTT CCT GTT CTG GGT GGG AAA GCA ATT GAA TTT TGT ATT 2266
E A F P V L G G K A I E F C I 734

GCT CGT TGG TGG GCA AGT CTC AGT GAT GTC AAT ATT GAT TAT ACC 2311
A R W W A S L S D V N I D Y T 749

ATA TCA TTC CAT GGC ATA GTG TGT ACT GCA CCA CAG TTA AAC ATT 2356
I S F H G I V C T A P Q L N I 764

CAT GCA TCT GAA GGA ATC AAT CGT TTT GAT GTC CAG TCC TCT TTA 2401
H A S E G I N R F D V Q S S L 779

AAA TAT GAA GAT CTG GCT CCT TGC ATA ACT TTG AAG AGC TGG GTG 2446
K Y E D L A P C I T L K S W V 794

CAA ACA CTA CGC CCA GTA AAT GCA AAA ACC AGA CCT TTA GGA TCA 2491
Q T L R P V N A K T R P L G S 809

AGA GAT GTT TTG CCA AAT AAT CGC CAG CTT TAT GAG ATG GTC CTG 2536
R D V L P N N R Q L Y E M V L 824

ACA TAC AGC TTT CAT CAG CCC AAG AGC GGA GAA GTA ACA CCT AGT 2581
T Y S F H Q P K S G E V T P S 839

TGT CCA CTC CTT TGT GAA TTA TTA TAT GAG TCA GAA TTT GAC AGT 2626
C P L L C E L L Y E S E F D S 854

CAG TTG TGG ATT ATT TTT GAC CAG AAC AAA AGA CAG ATG GGC TCA 2671
Q L W I I F D Q N K R Q M G S 869

GGC GAT GCC TAT CCA CAT CAG TAT TCT TTG AAA TTG GAG AAA GGA 2716
G D A Y P H Q Y S L K L E K G 884

GAT TAT ACG ATT CGA TTA CAG ATT CGT CAT GAG CAA ATC AGT GAT 2761
D Y T I R L Q I R H E Q I S D 899

TTG GAT CGT CTC AAA GAT CTT CCG TTT ATT GTT TCG CAT AGG TTG 2806
L D R L K D L P F I V S H R L 914

TCT AAT ACC TTG AGC TTA GAT ATT CAT GAA AAT CAT AGC CTT GCA 2851
S N T L S L D I H E N H S L A 929

CTT CTA GGA AAG AAG AAA TCA AGC AGT TTG ACA TTA CCA CCC AAA 2896
L L G K K K S S S L T L P P K 944

TAT AAT CAG CCA TTC TTT GTT ACT TCC TTA CCT GAT GAT AAA ATA 2941
Y N Q P F F V T S L P D D K I 959

CCT AAG GGG GCA GGA CCC GGA TGC TAC CTT GCA GGC TCC TTG ACA 2986
P K G A G P G C Y L A G S L T 974

TTG TCA AAG ACT GAG CTT GGA AAG AAA GCT GAT GTG ATC CCT GTT 3031
L S K T E L G K K A D V I P V 989

CAT TAC TAT CTA ATA CCT CCA CCA ACA AAG ACT AAG AAT GGC AGC 3076
H Y Y L I P P P T K T K N G S 1004

AAA GAT AAA GAA AAG GAT TCA GAA AAA GAG AAA GAT TTG AAA GAA 3121
K D K E K D S E K E K D L K E 1019

GAG TTT ACT GAA GCT TTA CGA GAT CTT AAA ATT CAG TGG ATG ACC 3166
E F T E A L R D L K I Q W M T 1034

AAG CTG GAT TCT ACT GAC ATT TAC AAT GAA TTG AAA GAA ACA TAT 3211
K L D S T D I Y N E L K E T Y 1049

CCT GCT TAC CTT CCT TTG TAT GTT GCA CGT CTT CAT CAA TTA GAT 3256
P A Y L P L Y V A R L H Q L D 1064

GCT GAA AAG GAG CGG ATG AAA AGA CTT AAT GAA ATT GTT GAT GCT 3301
A E K E R M K R L N E I V D A 1079

GCC AAT GCT GTT ATT TCT CAC ATC GAT CAG ACC GCT CTC GCT GTT 3346
A N A V I S H I D Q T A L A V 1094

TAC ATT GCC ATG AAG ACT GAC CCC AGG CCT GAT GCA GCT ACT ATA 3391
Y I A M K T D P R P D A A T I 1109

AAA AAT GAT ATG GAC AAG CAG AAA TCT ACC CTG GTA GAT GCC CTC 3436
K N D M D K Q K S T L V D A L 1124

TGC AGG AAA GGA TGT GCT CTG GCA GAC CAC CTT CTT CAT GCA CAG 3481
C R K G C A L A D H L L H A Q 1139

CCC CAC GAT GGG GCA GCA GCT GGA GAT GCA GAA GCA AAA GAA GAG 3526
P H D G A A A G D A E A K E E 1154

GAA GGA GAA AGT ACC TTG GAA TCT CTA TCA GAA ACC TAT TGG GAA 3571
E G E S T L E S L S E T Y W E 1169

ACT ACA AAG TGG ACA GAT CTT TTT GAC ACT AAG GTT TTG ACA TTT 3616
T T K W T D L F D T K V L T F 1184

GCA TAC AAG CAT GCA TTA GTA AAT AAG ATG TAC GGG AGA GGC CTT 3661
A Y K H A L V N K M Y G R G L 1199

AAG TTT GCA ACC AAA CTC GTA GAA GAA AAA CCA ACA AAA GAA AAC 3706
K F A T K L V E E K P T K E N 1214

TGG AAA AAT TGT ATT CAA CTG ATG AAA TTA CTC GGA TGG ACC CAT 3751
W K N C I Q L M K L L G W T H 1229

TGT GCG TCT TTT ACT GAA AAC TGG CTC CCC ATC ATG TAT CCT CCT 3796
C A S F T E N W L P I M Y P P 1244

GAT TAT TGT GTA TTC TAA aatgggaaccaaaacgttaaatttcgaaagcagaa 3849
D Y C V F * 1250

aattttatagtgaacaaatatatgaacaaatgtgtggcatttctagtctaactaatgcat 3909
gtcttcatccactatcgaatactgatcattaaaactctatgtatttatcagagaactcaa 3969
tggtgtgtggcttcatacatgtaatgtagacagacctctgacatcatgctgctttcctgc 4029
tgcctcccacacttggctagggagggcagagcctgcctgccagccccaacctgggtggat 4089
gcagctgctcactgcaggagaggtttcatctcttaattttaactgtaaaacgtcatcc 4149

agttttattttataaatcaaaaaggttaaaacatgctaatttttccaattatatagag 4209
gccttaaaaatgctacattgggtgtagctaaattatttatttgactaaaaattacgagaa 4269
cataatttccagacttctaaaaataaattcaattaatgggtatggtggggaggtataaat 4329
acatggcaactgggaaaagaaacgtgttaatgtaatcttcactccggagtcacaacaagc 4389
aagttgtttttacagcatcctcaagtacacagcatcagaataagttaaaatttcatgtgt 4449
tggtgccagacagttgaatctatctggttttgtaaagatatacacagtatgtttataaca 4509
ttgaaatcatgtaaaatacatgaataaatgtgcaaaacaacaggcacagcacaccatatg 4569
cactctgatacctgtttttttataaataaaagtaaatattgaagctaaa 4617

SEQ ID2

ataatttgtttggtttcactgctgagtaccatgctatgatttctacactatctgcttatt 60
aaactgtttcttactgtttggaactgttatctaacagttatttcaaaagcattaggcac 120
agttcctgtgcctgtgtgcagatgtttccaggatgtataccagaaatggaattgctgagt 180
cagggagattcaggtttactaggtcatgtgtaacagcttttcattgagctggccttgaa 240
ctcttctgatattcctgcctccacctcccagctgctgtgattatggatatattccaccat 300
gcctggccaactgttaacttctttttttaatttatttttttttaaagatttatttt 360
attgtatgagtgcactgtagctgccttcagacacaccagaagagggcattggattccatt 420
acagatggttgtgagccaccatgtggttgctgggatttgaactcaggacctcaggaagag 480
cactcggtgctcttaaccactgagccatctctccagcccccaactgttaacttcttatac 540
tttgaattttcagacctgttttaatttcttatgtaggaaatatcagtgtattgtagg 598
TTT GAT TTG CAT TTT TAT AGT GAA ATC AGT TAT TTT TCT AAC AGA 643
F D L H F Y S E I S Y F S N R 15

TTT AGG CTT TTG GGT CCT CTC ATA GTG CTA TTA AAG TTC TTT TAC 688
F R L L G P L I V L L K F F Y 30

TTT TTC ATT TTC TGT TTT TTT TTT TTT TTT AAT ACT ACT AAG TTG 733
F F I F C F F F F F N T T K L 45

TCA ATT GCT TTT TTT TTT TTT CCG GAG CTG GGG ATC GAA CCC AGG 778
S I A F F F F P E L G I E P R 60

GCC TTG CGC TTG CTA GGC AAG CGC TCT ACC GCT GAG CTA AAT CTC 823
A L R L L G K R S T A E L N L 75

CAA CCC CTG TCA ATT GCT TTT TCA CTG ATT CCT GCA AAC TGG ACA
Q P L S I A F S L I P A N W T

AAT CCT TCA GGA AAA TAT CAT ATT GGC ATT AAA AAT GGT TAT GAC
N P S G K Y H I G I K N G Y D

TTC TAT CCA AAG GCT CTC AAG GAA AGG ATA CAG AAA GAA CGG AAG
F Y P K A L K E R I Q K E R K

GAA AAA ATC TGG GAT CCA ATT CAC AGA GTT GCG CTT GCA GAA GCT
E K I W D P I H R V A L A E A

TGT AGA AAA CAA GAA GAA TTT GAT ATT GCC AAT AAT GGC TCT TCC
C R K Q E E F D I A N N G S S

CAA GCC AAT AAA CTA ATC AAG GAA GAG TTG CAG AGT CAA GTG GAA
Q A N K L I K E E L Q S Q V E

TTA CTT AAT TCT TTT GAG AAA AAG TAT AGT GAT CCA GGC CCT GTG
L L N S F E K K Y S D P G P V

TAT GAC TGC TTG GTG TGG CAT GAT GGT GAG ACC TGG AGA GCC TGT
Y D C L V W H D G E T W R A C

GTT GAT TCG AAT GAA AAT GGG GAC TTG GGT AAA TCT ACT GTA TTG
V D S N E N G D L G K S T V L

AGA AAC TAC AAA GAG GCT CAA GAA TAT GGT TCT TTT GGC ACA GCT
R N Y K E A Q E Y G S F G T A

GAG ATG CTG AAT TAC TCT GTT AAT ATT TAT GAT GAT GGA AAC CTG
E M L N Y S V N I Y D D G N L

CTC TCC ATT GTG ACC AGT GGA GGA GCT CAT GGA ACC CAT GTA GCG
L S I V T S G G A H G T H V A

AGT ATA GCT GCT GGG CAT TTT CCA GAA GAG CCT GAA CGG AAT GGA
S I A A G H F P E E P E R N G

GTT GCT CCT GGT GCT CAA ATT CTA TCC ATT AAG ATT GGT GAT ACA
V A P G A Q I L S I K I G D T

AGG CTA AGC ACC ATG GAA ACA GGC ACA GGC CTC ATC AGA GCT ATG
R L S T M E T G T G L I R A M

ATA GAA GTT ATA AAT CAT AAG TGT GAT CTT GTC AAC TAC AGT TAT
I E V I N H K C D L V N Y S Y

GGA GAA GCA ACT CAT TGG CCA AAT TCT GGG AGA ATT TGT GAA GTA
G E A T H W P N S G R I C E V

ATT AAT GAA GCA GTA TGG AAA CAT AAT ACA ATT TAT GTT TCA AGT
I N E A V W K H N T I Y V S S

GCT GGA AAT AAT GGT CCA TGC CTT TCT ACA GTG GGT TGT CCA GGA
A G N N G P C L S T V G C P G

GGA ACT ACT TCC AGC GTG ATA GGT GTT GGA GCT TAT GTT TCT CCT
G T T S S V I G V G A Y V S P

GAT ATG ATG GTT GCA GAG TAT TCA CTG AGA GAG AAA CTA CCT GCA
D M M V A E Y S L R E K L P A

AAT CAA TAT ACA TGG TCT TCT AGA GGC CCA AGT GCT GAT GGG GCC
N Q Y T W S S R G P S A D G A

CTT GGT GTG AGC ATC AGT GCA CCA GGA GGT GCT ATT GCT TCT GTG
L G V S I S A P G G A I A S V

CCT AAC TGG ACA TTG AGG GGG ACT CAG CTA ATG AAT GGG ACA TCA
P N W T L R G T Q L M N G T S

ATG TCT TCC CCC AAT GCC TGT GGT GGC ATT GCC CTG GTA CTT TCA
M S S P N A C G G I A L V L S

GGG CTG AAA GCA AAT AAT GTT GAC TAT ACT GTT CAC TCA GTC AGA
G L K A N N V D Y T V H S V R

AGA GCT CTA GAA AAC ACT GCA ATA AAA GCT GAC AAT ATA GAA GTA
R A L E N T A I K A D N I E V

TTT GCC CAA GGA CAT GGA ATT ATT CAG GTT GAC AAA GCT TAT GAC
F A Q G H G I I Q V D K A Y D

TAC CTC ATT CAG AAT ACA TCA TTT GCT AAC AGA TTA GGT TTT ACA
Y L I Q N T S F A N R L G F T

GTT ACA GTT GGA AAT AAC CGT GGC ATC TAC CTC CGA GAT CCT GTT
V T V G N N R G I Y L R D P V

CAG GTG GCT GCT CCT TCA GAT CAT GGT GTT GGC ATT GAG CCT GTA
Q V A A P S D H G V G I E P V

TTT CCA GAG AAC ACA GAA AAC TCT GAA AAA ATA TCC TTC CAG CTT
F P E N T E N S E K I S F Q L

CAT TTA GCT TTA ACT TCA AAT TCA TCT TGG GTT CAA TGT CCC AGC
H L A L T S N S S W V Q C P S

CAT TTG GAA CTC ATG AAT CAA TGT CGG CAC ATA AAC ATA CGT GTG
H L E L M N Q C R H I N I R V

GAC CCC AGG GGC TTA AGA GAA GGG TTA CAT TAT ACA GAG GTA TGT
D P R G L R E G L H Y T E V C

GGG TAT GAT ATA GCA TCC CCC AAT GCA GGT CCT TTA TTC AGA GTT
G Y D I A S P N A G P L F R V

CCA ATC ACT GCA GTT ATA GCA GCA AAA GTA AAT GAA TCA TCA CAT
P I T A V I A A K V N E S S H

TAT GAT CTA GCC TTT ACA GAT GTA CAT TTT AAA CCT GGT CAG ATT
Y D L A F T D V H F K P G Q I

CGA AGA CAT TTT GTT GAG GTT CCT GAG GGG GCA ACT TGG GCT GAA
R R H F V E V P E G A T W A E

GTT ACC GTG TGT TCG TGT TCT TCT GAG GTA TCA GCA AAA TTT GTT
V T V C S C S S E V S A K F V

CTT CAT GCA GTA CAG CTT GTG AAG CAG AGA GCA TAT CGA AGT CAT
L H A V Q L V K Q R A Y R S H

GAA TTT TAT AAG TTT TGT TCC CTT CCA GAA AAA GGA ACA CTC ATT
E F Y K F C S L P E K G T L I

GAA GCC TTT CCT GTT CTG GGT GGG AAA GCA ATT GAA TTT TGT ATT
E A F P V L G G K A I E F C I

GCT CGT TGG TGG GCA AGT CTC AGT GAT GTC AAT ATT GAT TAT ACC
A R W W A S L S D V N I D Y T

ATA TCA TTC CAT GGC ATA GTG TGT ACT GCA CCA CAG TTA AAC ATT
I S F H G I V C T A P Q L N I

CAT GCA TCT GAA GGA ATC AAT CGT TTT GAT GTC CAG TCC TCT TTA
H A S E G I N R F D V Q S S L

AAA TAT GAA GAT CTG GCT CCT TGC ATA ACT TTG AAG AGC TGG GTG
K Y E D L A P C I T L K S W V

CAA ACA CTA CGC CCA GTA AAT GCA AAA ACC AGA CCT TTA GGA TCA
Q T L R P V N A K T R P L G S

AGA GAT GTT TTG CCA AAT AAT CGC CAG CTT TAT GAG ATG GTC CTG
R D V L P N N R Q L Y E M V L

ACA TAC AGC TTT CAT CAG CCC AAG AGC GGA GAA GTA ACA CCT AGT
T Y S F H Q P K S G E V T P S

TGT CCA CTC CTT TGT GAA TTA TTA TAT GAG TCA GAA TTT GAC AGT
C P L L C E L L Y E S E F D S

CAG TTG TGG ATT ATT TTT GAC CAG AAC AAA AGA CAG ATG GGC TCA
Q L W I I F D Q N K R Q M G S

GGC GAT GCC TAT CCA CAT CAG TAT TCT TTG AAA TTG GAG AAA GGA
G D A Y P H Q Y S L K L E K G

GAT TAT ACG ATT CGA TTA CAG ATT CGT CAT GAG CAA ATC AGT GAT
D Y T I R L Q I R H E Q I S D

TTG GAT CGT CTC AAA GAT CTT CCG TTT ATT GTT TCG CAT AGG TTG
L D R L K D L P F I V S H R L

TCT AAT ACC TTG AGC TTA GAT ATT CAT GAA AAT CAT AGC CTT GCA
S N T L S L D I H E N H S L A

CTT CTA GGA AAG AAG AAA TCA AGC AGT TTG ACA TTA CCA CCC AAA
L L G K K K S S S L T L P P K

TAT AAT CAG CCA TTC TTT GTT ACT TCC TTA CCT GAT GAT AAA ATA
Y N Q P F F V T S L P D D K I

CCT AAG GGG GCA GGA CCC GGA TGC TAC CTT GCA GGC TCC TTG ACA
P K G A G P G C Y L A G S L T

TTG TCA AAG ACT GAG CTT GGA AAG AAA GCT GAT GTG ATC CCT GTT
L S K T E L G K K A D V I P V

CAT TAC TAT CTA ATA CCT CCA CCA ACA AAG ACT AAG AAT GGC AGC
H Y Y L I P P P T K T K N G S

AAA GAT AAA GAA AAG GAT TCA GAA AAA GAG AAA GAT TTG AAA GAA
K D K E K D S E K E K D L K E

GAG TTT ACT GAA GCT TTA CGA GAT CTT AAA ATT CAG TGG ATG ACC
E F T E A L R D L K I Q W M T

AAG CTG GAT TCT ACT GAC ATT TAC AAT GAA TTG AAA GAA ACA TAT
K L D S T D I Y N E L K E T Y

CCT GCT TAC CTT CCT TTG TAT GTT GCA CGT CTT CAT CAA TTA GAT
P A Y L P L Y V A R L H Q L D

GCT GAA AAG GAG CGG ATG AAA AGA CTT AAT GAA ATT GTT GAT GCT
A E K E R M K R L N E I V D A

GCC AAT GCT GTT ATT TCT CAC ATC GAT CAG ACC GCT CTC GCT GTT
A N A V I S H I D Q T A L A V

TAC ATT GCC ATG AAG ACT GAC CCC AGG CCT GAT GCA GCT ACT ATA
Y I A M K T D P R P D A A T I

AAA AAT GAT ATG GAC AAG CAG AAA TCT ACC CTG GTA GAT GCC CTC
K N D M D K Q K S T L V D A L

TGC AGG AAA GGA TGT GCT CTG GCA GAC CAC CTT CTT CAT GCA CAG
C R K G C A L A D H L L H A Q

CCC CAC GAT GGG GCA GCA GCT GGA GAT GCA GAA GCA AAA GAA GAG
P H D G A A A G D A E A K E E

GAA GGA GAA AGT ACC TTG GAA TCT CTA TCA GAA ACC TAT TGG GAA
E G E S T L E S L S E T Y W E

ACT ACA AAG TGG ACA GAT CTT TTT GAC ACT AAG GTT TTG ACA TTT
T T K W T D L F D T K V L T F

GCA TAC AAG CAT GCA TTA GTA AAT AAG ATG TAC GGG AGA GGC CTT
A Y K H A L V N K M Y G R G L

AAG TTT GCA ACC AAA CTC GTA GAA GAA AAA CCA ACA AAA GAA AAC
K F A T K L V E E K P T K E N

TGG AAA AAT TGT ATT CAA CTG ATG AAA TTA CTC GGA TGG ACC CAT
W K N C I Q L M K L L G W T H

TGT GCG TCT TTT ACT GAA AAC TGG CTC CCC ATC ATG TAT CCT CCT
C A S F T E N W L P I M Y P P

GAT TAT TGT GTA TTC TAA aatgggaaccaaaacgttaaatttcgaaagcagaa
D Y C V F *

aattttatagtgaacaaatatatgaacaaatgtgtggcatttctagtctaactaatgcat
gtcttcatccactatcgaatactgatcattaaaactctatgtatttatcagagaactcaa
tggtgtgtggcttcatacatgtaatgtagacagacctctgacatcatgctgctttcctgc
tgcctcccacacttggctagggagggcagagcctgcctgccagccccaacctgggtggat
gcagctgctcactgcaggagaggttttcatctcttaattttaactgtaaaacgtcatcc

agttttatttataaatcaaaaggttaaaacatgctaaattttccaattatatagag
gccttaaaaatgctacattgggtgtagctaaattatttatttgactaaaattacgagaa
cataatttccagacttctaaaataaattcaattaatgggtatggtggggaggtataaat
acatggcaactgggaaaagaaacgtgttaatgtaatcttcactccggagtcacaacaagc
aagttgtttttacagcatcctcaagtacacagcatcagaataagttaaaatttcatgtgt
tggtgccagacagttgaatctatctggttttgtaaagatatacacagtatgtttataaca
ttgaaatcatgtaaaatacatgaataaatgtgcaaaacaacaggcacagcacaccatatg
cactctgatacctgtttttataaataaaagtaaatattgaagctaaa

## Revendications

1. Procédé de criblage de médicaments destinés à traiter chez l'homme ou l'animal des troubles ou affections liés à l'inactivation ou dégradation exagérée, ou pouvant être traités en retardant la dégradation physiologique de neuropeptides endogènes, **caractérisé en ce qu'**il consiste à mettre en contact une molécule candidate avec une tripeptidylpeptidase membranaire et à mesurer l'activité de cette enzyme, ladite tripeptidylpeptidase membranaire étant codée par la séquence nucléotidique donnée par les identifieurs SEQ ID n°1 et SEQ ID n°2.

2. Procédé selon la revendication 1 , **caractérisé en ce que** la tripeptidylpeptidase correspond à l'identificateur de séquence SEQ ID N°1 de la liste de séquences annexée.

3. Procédé selon la revendication 1 ou 2, **caractérisé en ce que** la tripeptidylpeptidase correspond à l'identificateur de séquence SEQ ID N°2 de la liste de séquence annexée.

4. Procédé selon la revendication 1, **caractérisé en ce qu'**il comprend le fait d'incuber des membranes de cerveau, par exemple de rat, préparées par centrifugation d'un homogénat, en présence d'un substrat d'aminotripeptidylpeptidase et d'une molécule candidate, inhibiteur potentiel de l'activité enzymatique.

5. Procédé selon l'une des revendications 1 à 4, **caractérisé en ce que** les troubles ou affections sont liés à l'inactivation de la cholécystokinine (CCK).

6. Composé de formule générale (I) suivante : 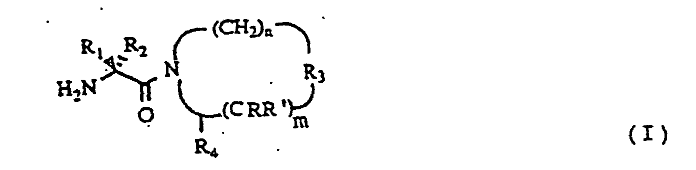 dans laquelle :
- R₁ représente un hydrogène ou un groupe- alkyle en C₁-C₄ linéaire ou ramifié ;
- R₂ représente un hydrogène ou un groupe alkyle en C₁-C₂ ;
l'un au moins de R₁ et R₂ représentant un hydrogène ;
- n = 0 ou 1 et m = 0 ou 1 avec n différent de m ;
- R et R' représentent chacun indépendamment un hydrogène ou un groupe alkyle en C₁-C₂ ;
- R₃ représente un radical bivalent constitué d'une chaîne alkyle -(CH₂)₂-, -CH₂-CB(cis.F)-, -CH₂-CH(CH₂Ph)-, d'un motif 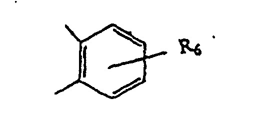
où R₆ représente H, F, OCH₃ ou OCH₂Ph, 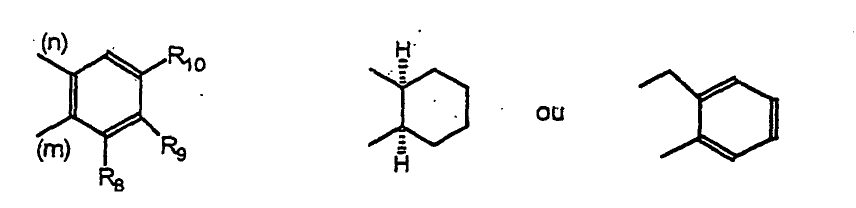 où R₈, R₉ et R₁₀ représentent chacun un atome d'hydrogène ou d'halogène, un groupe O (alkyle en C₁-C₄), OCH₂Ph, OH ou alkyle en C₁-C₄, y comprise R₆, (m) et (n) indiquant le sens de liaison respectivement au groupe (CH₂)ₙ (ou à N si n = 0) et au groupe (CRR')ₘ (ou à CHR₄ si m = 0).
- R₄ représente un groupe' amide CO-NH-R₅ où R₅ représente un hydrogène ou un groupe alkyle en C₁-C₆ linéaire ou ramifié, -(CH₂)₃-SCH₃, -CH₂Ph, -CH₂C₆H₁₁, (CH₂)₃OH, 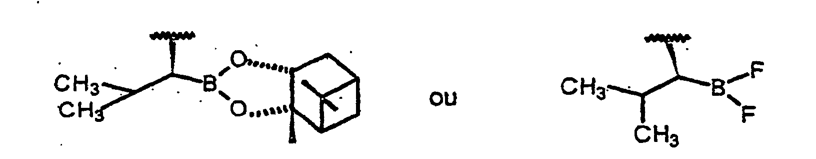 ainsi que leurs sels ou hydrates correspondants,
sous réserve que :
i) lorsque
R₂ = R' = R = H ; R₃ = -CH₂-CH₂-; n + m = 1 ; R₄ = -CO-NH-R₅ ; et R₅ = H alors
R₁ est distinct de -CH₃; -CH₂-CH₃; -(CH₂)₂-CH₃; -(CH₂)₃-CH₃; -CH(CH₃)₂ ; -CH₂CH(CH₃)₂ ; et -CH(CH₃)CH₂-CH₃ ;
ii) lorsque
R₂ = R' = R = H ; R₃ = -CH₂-CH₂-; n + m = 1 ; R₄ = -CO-NH-R₅ ; et R₅ = -CH₂-CH₃ ;
alors
R₁ est distinct de -CH₃ ; et -CH₂CH(CH₃)₂ ;
iii) lorsque
R₂ = R' = R = H ; R₃ = -CH₂-CH₂- ; n + m = 1 ; R₄ = -CO-NH-R₅; et R₅ = -(CH₂)₄-CH₃, -CH(CH₃)₂, CH₃ ou le motif : 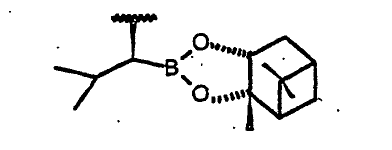 alors
R₁ est distinct de -CH₃.

7. Composé selon la revendication 6, de formule générale (I') suivante : 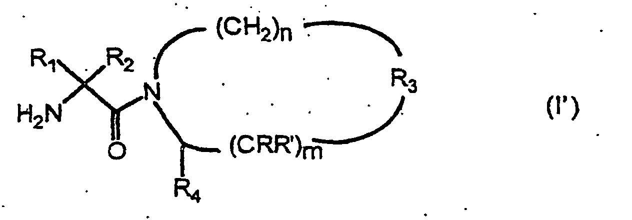 dans laquelle
- R₁ représente un hydrogène ou un groupe alkyle en C₁-C₄ linéaire ou ramifié ;
- R₂ représente un hydrogène ou un groupe alkyle en C₁-C₂ ; l'un au moins de R₁ et R₂ représentant un hydrogène ;
- n = 0 ou 1 et m = 0 ou 1 avec n différent de m ;
- R₃ représente un radical bivalent constitué d'une chaîne alkyle -(CH₂)₂-, -CH₂-CH(cis.F)-, -CH₂-CH(CH₂Ph)-, d'un motif 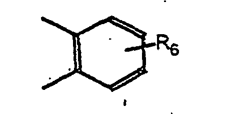 où R₆ représente H, F, OCH₃ ou OCH₂Ph, 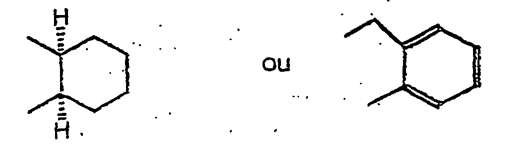
- R₄ représente un groupe amide CO-NH-R₅ où R₅ représente un hydrogène ou un groupe alkyle en C₁-C₈ linéaire ou ramifié, -(CH₂)₃-SCH₃, -CH₂Ph, -CH₂C₆H₁₁,

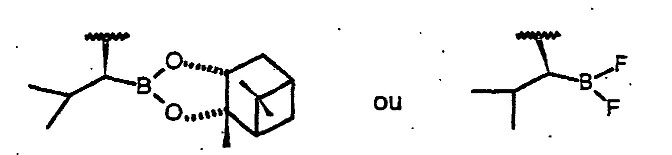

8. Composé selon la revendication 6 ou 7, **caractérisé en ce que** R₃ représente -(CH₂)₂-.

9. Composé selon la revendication 6 ou 7, **caractérisé en ce que** R₃ représente -CH₂-CH(cis.F)-.

10. Composé selon la revendication 6 ou 7, **caractérisé en ce que** R₃ représente -CH₂-CH(CH₂Ph)-.

11. Composé selon la revendication 6 ou 7, **caractérisé en ce que** n = 0 et m = 1.

12. Composé selon la revendication 11, **caractérisé en ce que** R₃ représente

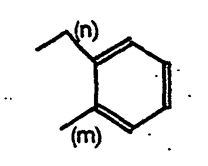

13. Composé selon la revendication 11, **caractérisé en ce que** R, R' représentent un hydrogène et R₃ représente

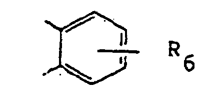

14. Composé selon la revendication 11, **caractérisé en ce que** R₃ représente

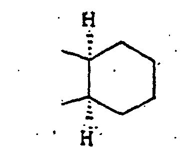

15. Composé selon la revendication 11, **caractérisé en ce que** R₃ représente le motif

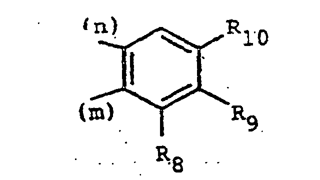

16. Composé selon la revendication 6 ou 7, **caractérisé en ce que** n = 1 et m = 0.

17. Composé selon la revendication 16, **caractérisé en ce que** R₃ représente

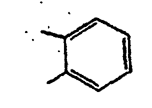

18. Composé selon l'une des revendications 6 à 17,
**caractérisé en ce que** R₅ représente le groupe n.butyle

19. Composé selon l'une quelconque des revendications 6 à 18, **caractérisé en ce que** R₂ représente un hydrogène.

20. Composé selon l'une des revendications 6 à 19,
**caractérisé en ce que** R₁ représente un hydrogène.

21. Composé selon l'une quelconque des revendications 6 à 20, **caractérisé en ce qu'**il est choisi parmi le groupe comprenant :
- L-valyl-L-proline n.hexylamide ;
- 1-(2(S)-aminobutyryl)-L-proline 3-(méthylthio)propyl-amide ;
- 1-(2(S)-aminobutyryl)-L-proline n.pentylamide ;
- 1-(2(S)-aminobutyryl)-L-proline n.butylamide ;
- 1-(2(S)-aminobutyryl)-L-proline [2(S)-méthyl]butylamide ;
- 1-(2(S)-aminobutyryl)-L-proline n.propylamide ;
- 1-(2(S)-aminobutyryl)-L-proline iso.butylamide ;
- L-valyl-L-proline n.butylamide ;
- borohydrate de L-alanyl-L-prolyl-difluoro-L-borovaline ;
- 1-(2(S)-aminobutyryl)-(4(S)-fluoro)-L-proline n.butyl-amide ;
- 1-(2(S)-aminobutyryl)-(4(S)-benzyl)-L-proline n.butyl-amide ;
- acide 2-(2(S)-aminobutyryl)-1,2,3,4-tétrahydro-3(S)-iso-quinolinecarboxylique n.butylamide ;
- acide 1-(2(S)-aminobutyryl)-2(S)-indolinecarboxylique n.butylamide ;
- acide 1-(2(S)-aminobutyryl)-2(S)-indolinecarboxylique n.propylamide ;
- acide 1-(2(S)-aminobutyryl)-2(S)-indolinecarboxylique méthylamide ;
- acide 1-(2(S)-aminobutyryl)-2(S)-indolinecarboxylique éthylamide ;
- acide 1-(2(S)-aminobutyryl)-2(R/S)-(5-méthoxy)indoline carboxylique n.butylamide ;
- acide 1-(2(S)-aminobutyryl)-2(R/S)-(6-méthoxy)indoline carboxylique n.butylamide ;
- acide 1-(2(S)-aminobutyryl)-2(R/S)-(5-fluoro)indoline carboxylique n.butylamide ;
- acide 1-(2(S)-aminobutyryl)-2(R/S)-(5-benzyloxy)indoliné carboxylique n.butylamide ;
- acide 1-(2(S)-aminobutyryl)-2(S)-[(3aS,7aS)-perhydroj-indolinecarboxylique n.butylamide ;
- acide 2-(2(S)-aminobutyryl)-1(R/S)-isoindoline carboxylique n.butylamide ;
ainsi que leurs sels ou hydrates correspondants.

22. Composé selon la revendication 14, **caractérisé en ce qu'**il est choisi parmi le groupe comprenant :
- acide 1-(2(S)-aminobutyryl)-2(S)-indolinécarboxylique n.butylamide ;
- acide. 1-(2(S)-amïnobutyryl)-2(S)-indolinecarboxylique n.propylamide ;
- acide 1-(2(S)-aminobutyryl)-2(S)-indolinecarboxylique éthylamide ;
- acide 1-(2(S)-aminobutyryl)-2(R/S)-(5-méthoxy)indoline carboxylique n.butylamide ;
- acide 1-(2(S)-aminobutyryl)-2(R/S)-(5-fluoro)indoline carboxylique n.butylamide
ainsi que leurs sels ou hydrates correspondants.

23. Composé selon la revendication 15, **caractérisé en ce qu'**il est choisi parmi le groupe comprenant :
- acide 1-(L-valyl)-5-méthoxyindoline-2(R/S)-carboxylique butylamide
- acide 1-(L-alanyl)-5-méthoxyindoline-2(R/S)-carboxylique butylamide
- acide 1-(L-alanyl)-5-méthoxyindoline-2(S)-carboxylique butylamide
- acide 1-(2(S)aminobutyryl)-4-méthaxy-indoline-2(R/S)-carboxylique butylamide
- acide 1-(2(S) aminobutyryl)-3,3-diméthylindoline2(R/S) carboxylique butylamide
- acide 1-(2(S)-aminobutyryl)-3(R)-méthylindoline-2(R) carboxylique butylamide et acide 1-(2(S)-aminobutyryl)-2(S)-méthylindoline-2(S)-carboxylique butylamide
- acide 1-(2(S)-aminobutyryl)-3(R)-méthylindoline-2(S) carboxylique butylamide et acide 1-(2(S)-aminobutyryl)-3(S)-méthylihdoline-2(R)-carboxylique butylamide
- acide 1-(2(S)-aminobutyryl)-4-éthoxyindoline-2(S)-carboxylique butylamide
- acide 1-(2(S)-aminobutyryl)-4,5-diméthoxyindoline-2(R/S)-carboxylique butylamide
- acide 1-2(S)-aminobutyryl-5-hydroxyindoline 2(S) carboxylique butylamide
- acide 1-2(S)-aminobutyryl-5-hydroxyindoline 2(R/S) carboxylique butylamide
- acide 1-2S-aminobutyryl-5-méthylindoline 2(R/S) carboxylique butylamide
- acide 1-(2(S)-aminobutyryl)-5-chloroindoline-2(S)-carboxylique butylamide
- acide 1-(2(S)-aminobutyryl) indoline-2(S)-carboxylique (3-hydroxy)propylamide
ainsi que leurs sels ou hydrates correspondants.

24. Procédé pour la préparation d'un composé de formule générale (I) donnée ci-dessus dans laquelle :
- R₁ représente un groupe alkyle en C₁-C₄ linéaire ou ramifié ;
- R₂ représente un hydrogène ;
- n = 0 ou 1 et m = 0 ou 1 avec n différent de m et R₃ représente -(CH₂)₂- ; et
- R et R' représentent un hydrogène
- R₄ représente CO-NH-R₅,
où R₅ représente un hydrogène ou un groupe alkyle en C₁-C₆ linéaire ou ramifié, -(CH₂)₃-S-CH₃, -CH₂Ph ; Sous réserve que R₁ est différent de CH₂CH₃, (CH₂)₂CH₃, (CH₂)₃CH₃, CH(CH₂) CH₂CH(CH₃)₂, CH(CH₃)CH₂CH₃ lorsque R₃ est un hydrogène, R₁ est différent de CH₂CH(CH₃)₂ lorsque R₅ est le groupe CH₂CH₃ et R₁ est différent de CH₃ lorsque R₅ est le groupe -(CH₂)₄-CH₃, -CH(CH₃)₂, -CH₂CH₃, -CH₃ ou un atome d'hydrogène, ou le motif 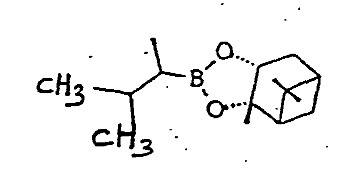 **caractérisé en ce qu'**il comprend :
i) la formation d'un composé de formule (III) 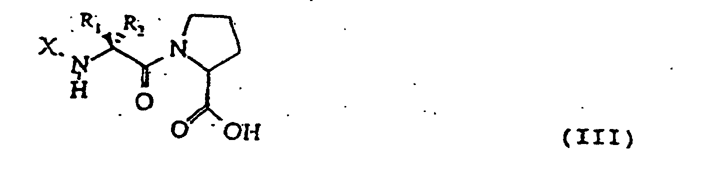 dans laquelle R₁ et R₂ ont les significations données ci-dessus et X représente un groupe protecteur, à partir d'un composé de formule (II) 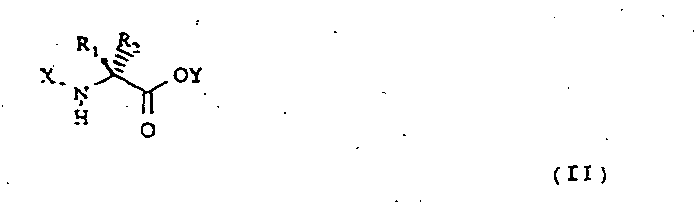 estérifié sur sa fonction acide par un groupement Y et dans laquelle X, R₁ et R₂ ont les significations données précédemment, par réaction avec la L-proline ;
ii) l'amidation de la fonction acide du compose (III) avec l'aminé appropriée R₅NH₂ où R₅ a la signification donnée ci-dessus, pour former le dérivé (IV) 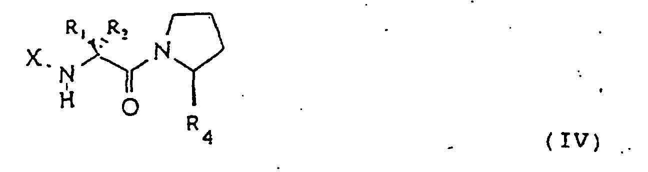 protégé sur sa fonction aminé primaire par le groupe X ;
iii) l'élimination du groupe X du dérivé (IV), pour obtenir le composé (I) désiré.

25. Procédé pour la préparation d'un composé de formule générale (I) donnée ci-dessus dans laquelle :
- R₁ représente un groupe alkyle en C₁-C₄ linéaire ou ramifié ;
- R₂ représente un hydrogène ou un groupe méthyle ;
- n = 0 ou 1 et m = 0 ou 1 avec n différent de m et R₃ représente un groupe -(CH₂)₂- ; et
- R et R' représentent un hydrogène
- R₄ représente un groupe CO-NH-R₅ où R₅ représente un hydrogène ou un groupe alkyle en C₁-C₆ linéaire ou ramifié ;
sous réserve que lorsque R₂ représente un hydrogène, alors R₁ est différent de CH₂CH₃, (CH₂)₂CH₃, (CH₂)₃CH₃, CH(CH₃)₂, CH₂CH(CH₃)₂, CH(CH₃)CH₂CH₃ lorsque R₅ est un hydrogène, R₁ est différent de CH₂CH(CH₃)₂ lorsque R₅ est le groupe CH₂CH₃ et R₁ est différent de CH₃ lorsque R₅ est le groupe -(CH₂)₄-CH₃, -CH(CH₃)₂, -CH₂CH₃, -CH₃ ou un atome d'hydrogène,
**caractérisé en ce qu'**il comprend :
i) la formation d'un composé de formule (IV) 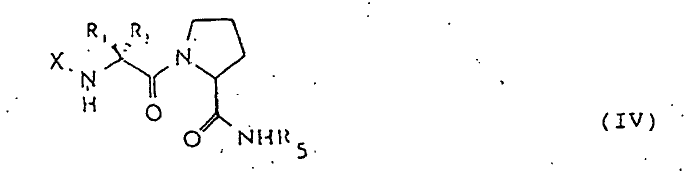 dans laquelle R₁, R₂ et R₅ ont les significations données ci-dessus et X représente un groupe protecteur, à partir d'un composé de formule (II) 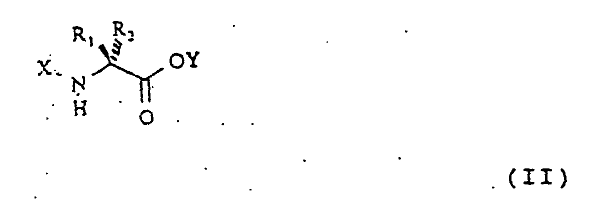 estérifié sur sa fonction acide à l'aide d'un groupe Y et dans laquelle R₁, R₂ et X ont les significations données précédemment, par réaction avec une prolineamide de formule (V) 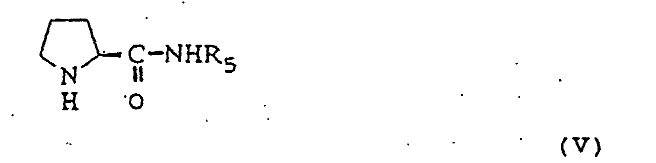 dans laquelle R₅ a la signification donnée ci-dessus ;
ii) l'élimination du groupe protecteur X du composé (IV), pour obtenir le composé (I) désiré.

26. Procédé pour la préparation d'un composé de formule générale (I) donnée ci-dessus dans laquelle :
- R₁ représente un groupe alkyle en C₁-C₄ linéaire ou ramifié ;
- R₂ représente un hydrogène ;
- n = 0 et m = 1 et R₃ représente un groupe 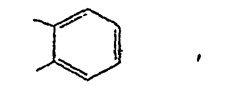
- CH₂-CH(cis.F)-, 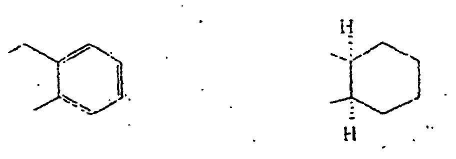
- R et R' représentent un hydrogène ;
- R₄ représente un groupe CO-NH-R₅ où R₅ représente un hydrogène ou un groupe alkyle en C₁-C₆ linéaire ou ramifié ;
**caractérisé en ce qu'**il comprend :
i) la préparation d'un composé de formule (X) 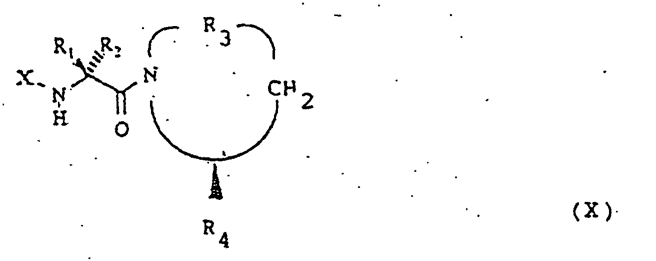 dans laquelle R₁, R₂, R₃ et R₄ ont les significations données ci-dessus et X représente un groupe protecteur, par réaction d'un composé de formule (VIII) 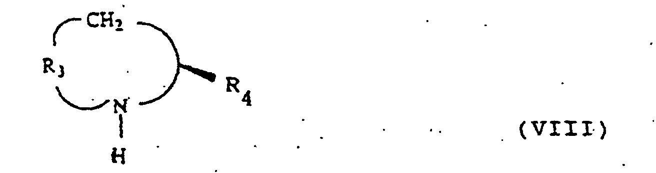
CH2
R3
R4
N (VIII)
H
 avec un composé de formule (IX) 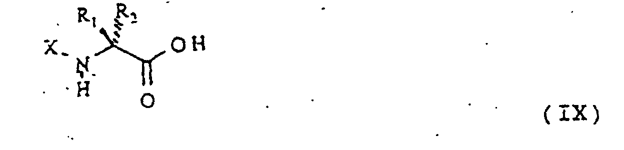 dans lesquelles R₁, R₂, R₄ et X ont les significations données précédemment ;
ii) l'élimination du groupement X du composé (X), pour former le composé (I) désiré.

27. Procédé pour la préparation d'un composé de formule générale (I) donnée ci-dessus dans laquelle :
- R₁ représente un groupe alkyle en C₁-C₄ linéaire ou ramifié ;
- R₂ représente un hydrogène ;
- n = 1 et m = 0 et R₃ représente un groupe -CH(CH₂Ph)-CH₂-, ou 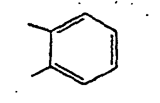
- R₄ représente un groupe CO-NH-R₅ où R₅ représente un hydrogène ou un groupe alkyle en C₁-C₆ linéaire ou ramifié ;
**caractérisé en ce qu'**il comprend :
i) l'obtention d'un composé (XII) 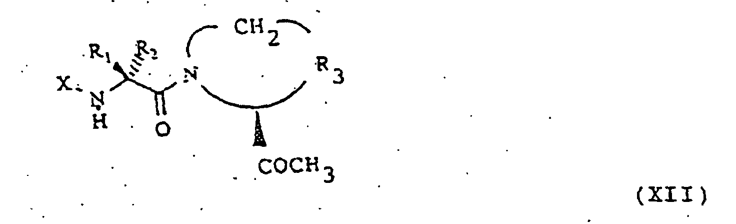 protégé sur sa fonction amine primaire par un groupe protecteur X et dans laquelle R₁, R₂ et R₃ ont les significations données ci-dessus, par réaction du composé de formule (IX) 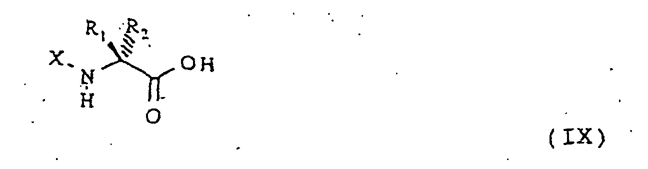 avec le composé de formule (XI) 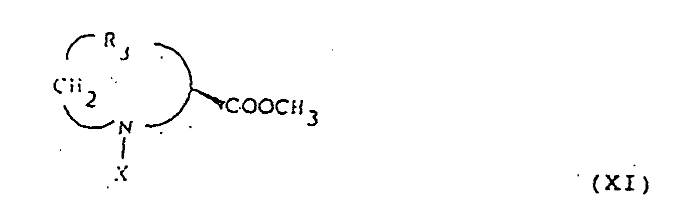 dans lesquelles R₁, R₂ et R₃ ont les significations données précédemment et X représente un groupe protecteur ;
ii) l'hydrolyse de la fonction ester du composé (XII) ainsi obtenu pour former le composé de formule (XIII) 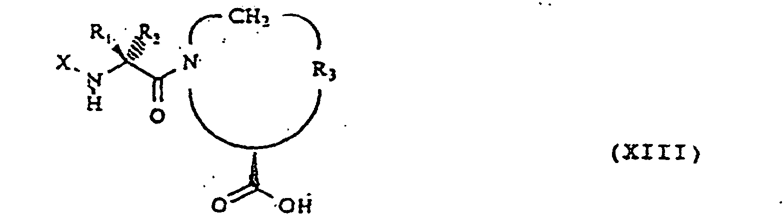 dans laquelle R₁, R₂, R₃ et X ont les significations données précédemment ;
iii) l'amidation de la fonction acide du composé (XIII) à l'aide de l'amine R₅NH₂ appropriée pour former le dérivé de formule (XIV) 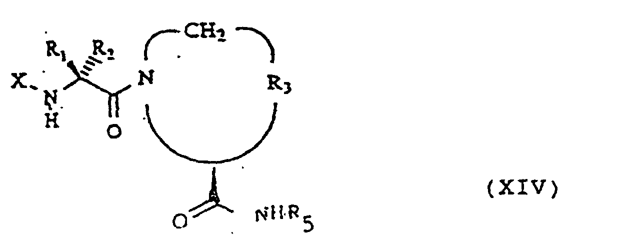 dans laquelle R₁, R₂, R₃, R₅ et X ont les significations données ci-dessus ;
iv) l'élimination du groupe protecteur X du composé (XIV) pour former le composé (I) désiré.

28. Procédé pour la préparation d'un composé de formule générale (I) donnée ci-dessus dans laquelle :
- R₁ représente un groupe méthyle ;
- R₂ représente un hydrogène ;
- n = 0 ou 1 et m = 0 ou 1 avec n différent de m et R₃ représente un groupe -(CH₂)₂- ; et
- R et R' représentent un hydrogène ;
- R₄ représente un groupe CO-NH-R₅ où R₅ représente (CH₃)₂-CH-BF₂ ;
**caractérisé en ce qu'**il comprend :
i) l'élimination du motif pinane du composé de formule (I) dans laquelle R₁, R₂, n, m R₃ et R₄ ont les significations données ci-dessus et R₅ représente 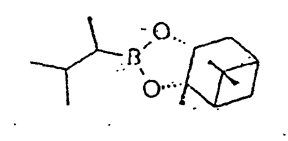 par action du trichlorure de bore dans du chlorure de méthylène suivie d'une hydrolyse, pour obtenir le dérivé (XXI) 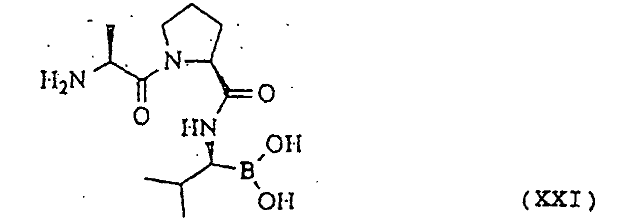
ii) le fait de faire réagir le composé (XXI) avec l'acide fluorhydrique pour former le composé (I) désiré.

29. Procédé pour la préparation d'un composé de formule générale (I) donnée ci-dessus, dans laquelle :
- R₁ représente un groupe alkyle en C₁-C₄ linéaire ou ramifié ;
- R₂ représente un hydrogène ;
- n = 0 et m = 1 et R₃ représente le motif 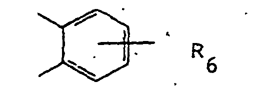
où R₆ représente un groupe OCH₃, OCH₂Ph, F ; et
- R et R' représentent un hydrogène ;
- R₄ représente un groupe Co-NH-R₅ où R₅ représente un groupe alkyle en C₁-C₆ linéaire ou ramifié ;
**caractérisé en ce qu'**il comprend :
i) la formation de l'amide de formule (XXXXIV) 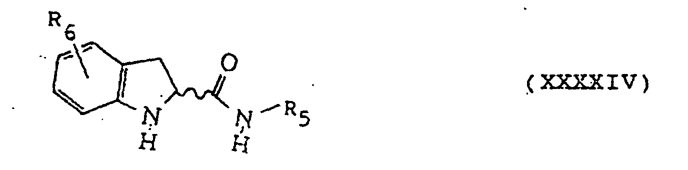 à partir de l'ester de formule (XXXXIII) 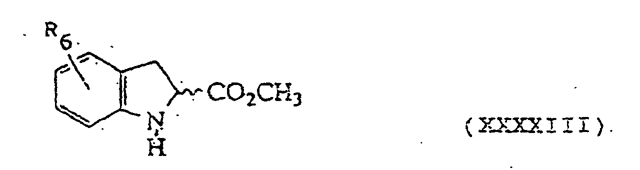 dans lesquelles R₆ a la signification donnée ci-dessus, par réaction avec l'amine R₅NH₂ appropriée ;
ii) la réaction du composé (XXXXIV) avec le composé de formule (IX) 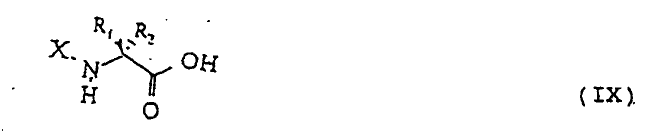 dans laquelle R₁ et R₂ ont les significations précitées et X représente un groupement protecteur, pour former le composé de formule (XXXXV) 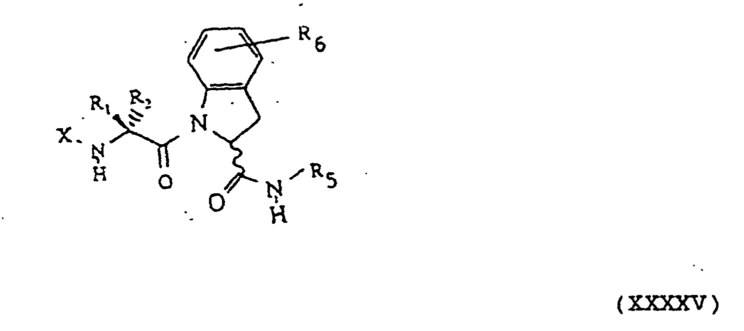 dans laquelle R₁, R₂, R₅, R₆ et X ont les significations données précédemment ;
iii) l'élimination du groupement X pour former le composé (I) désiré.

30. Procédé selon la revendication 29, **caractérisé en ce que** l'on prépare l'ester méthylique (XXXXIII)
i) par action du nitrite de sodium en présence d'acide chlorhydrique, sur le composé de formule (XXXIX) 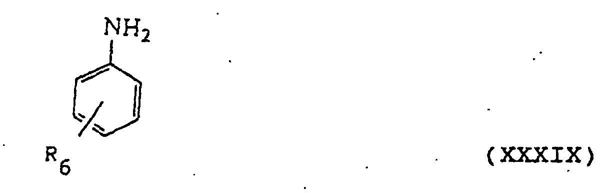 pour former le composé de formule (XXXX) 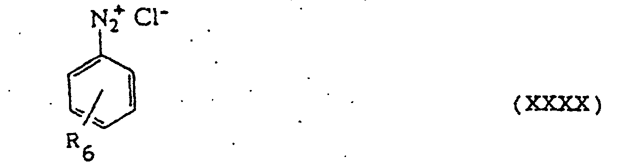 dans lesquelles R₆ a la signification indiquée précédemment
ii) par addition de 2-méthylacétoacétate d'éthyle au composé (XXXX) ainsi obtenu, en présence de nitrite de sodium dans l'éthanol, pour former le composé de formule (XXXXI) 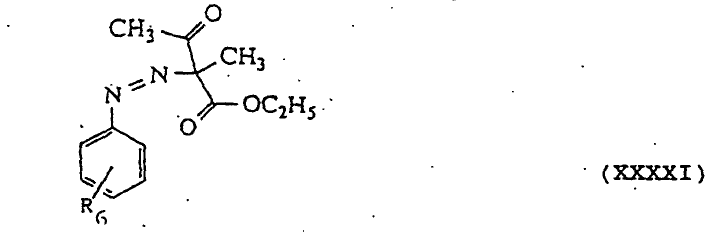 dans laquelle R₆ a la signification précitée ;
iii) par cyclisation en milieu acide, pour former l'ester éthylique (XXXXII) 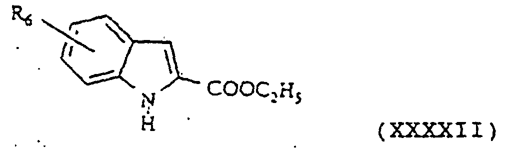
iv) par échange à partir de l'ester éthylique (XXXXII) ainsi obtenu, en présence de magnésium dans du méthanol.

31. Procédé pour la préparation d'un composé de formule générale (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 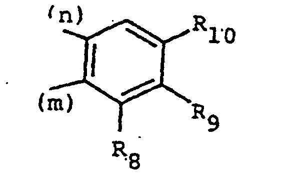
où R₈ et R₁₀ représentent un hydrogène et R₉ représente un groupe O (alkyle en C₁-C₄) ou alkyle en C₁-C₄
- R₄ représente un groupe amide CO-NH-R₅ où R₅ représente un groupe alkyle en C₁-C₆ linéaire ou ramifié ;
**caractérisé en ce qu'**il comprend :
i) la formation de l'amide de formule (49) 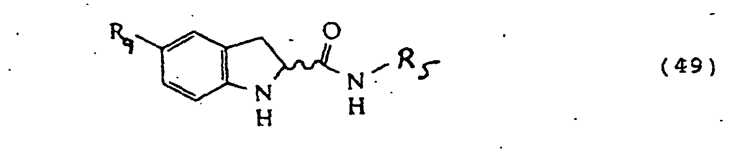 à partir de l'ester de formule (48) 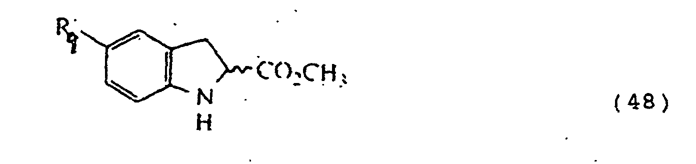 dans lesquelles R₉ et R₅ ont la signification donnée ci-dessus, par réaction avec l'amine R₅NH₂ appropriée ;
ii) la réaction du composé (49) avec le composé de formule (IX) 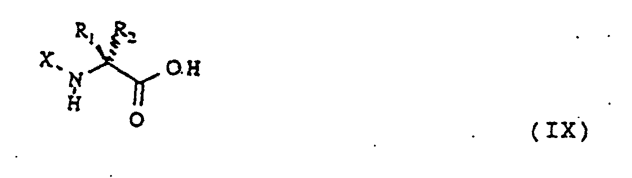 dans laquelle R₁ et R₂ ont les significations précitées et X représente un groupement protecteur, pour former le composé de formule (50) 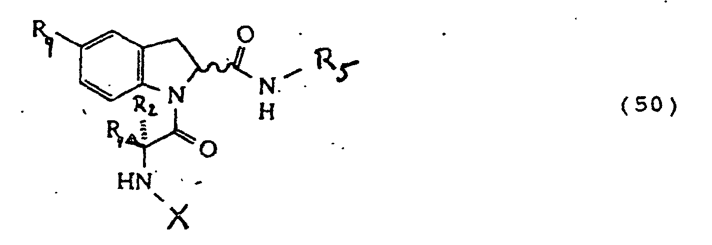 dans laquelle R₁, R₂, R₉ et X ont les significations données précédemment ;
iii) l'élimination du groupement X pour former le composé (I) désiré.

32. Procédé selon la revendication 31, **caractérisé en ce que** l'ester méthylique (48) est obtenu à partir de l'acide (46) correspondant 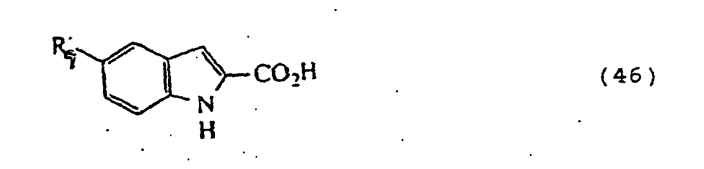 où R₉ a la signification donné précédemment
i) par traitement dans de l'éthanol ou du méthanol avec de l'acide sulfurique concentré, pour conduire à l'ester (47) correspondant 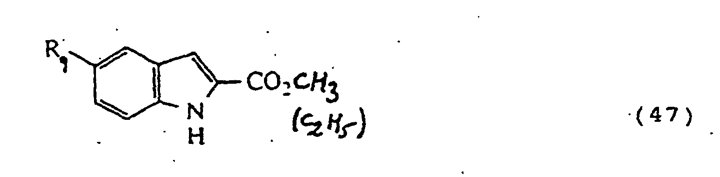 où R₉ est tel que défini ci-dessus
ii) puis le composé (47) est traité avec du magnésium dans le méthanol.

33. Procédé pour la préparation d'un composé de formule générale (I) donnée ci-dessus, dans laquelle
- R₁ représente un groupe alkyle en C₁-C₄ linéaire ou ramifié ;
- R₂, R et R' représentent chacun un hydrogène ;
- n = 0 et m = 1 et R₃ représente le motif 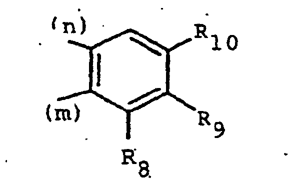
où R₈ et R₉ représentent un hydrogène ou un groupe o(alkyle en C₁-C₄), R₈ et R₉ ne pouvant représenter simultanément un hydrogène et R₁₀ représente un hydrogène
- R₄ représente un groupe amide CONHR₅ où R₅ est un groupe alkyle en C₁-C₆ linéaire ou ramifié,
**caractérisé en ce qu'**il comprend :
i) la formation du composé (54) 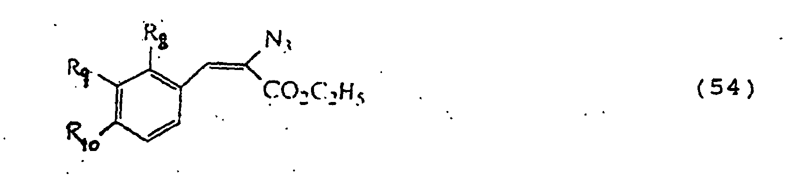 par réaction de l'aldéhyde (53) correspondant 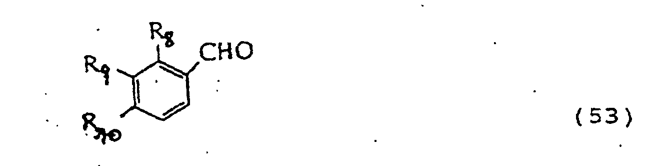 où R₈, R₉ et R₁₀ sont tels que définis ci-dessus
avec de l'azidoacétate d'éthyle (52) 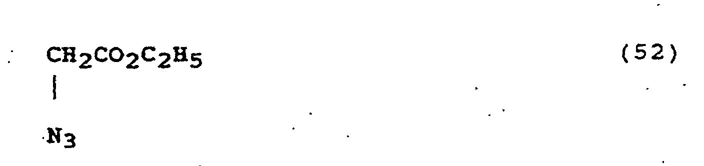
ii) la cyclisation du composé (54) pour conduire au composé (55) 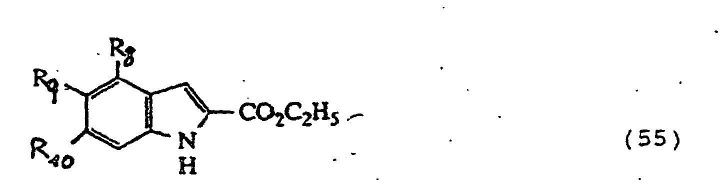 où R₈, R₉ et R₁₀ ont les significations données précédemment
iii) la formation de l'ester méthylique (56) 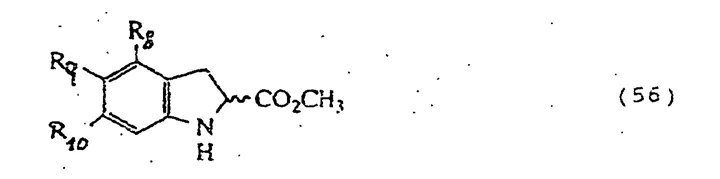 où R₈, R₉ et R₁₀ ont les significations données précédemment
à partir du composé (55) en présence de magnésium dans du méthanol
iv) la réaction de l'ester (56) obtenu avec l'amine R₅NH₂ appropriée pour former l'amide (57) 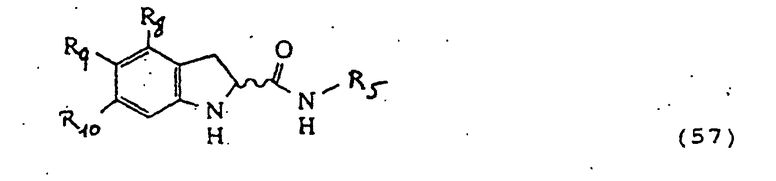 où R₈, R₉ et R₁₀ sont tels que définis ci-dessus
v) la réaction du composé (57) avec le composé de formule (IX) 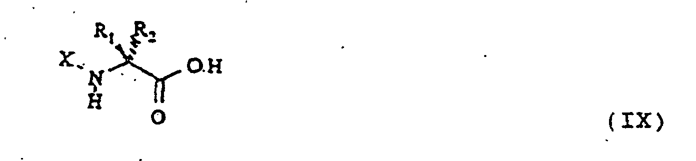 où R₁ et R₂ ont la signification précitée et X représente un groupement protecteur, pour former le composé (58) 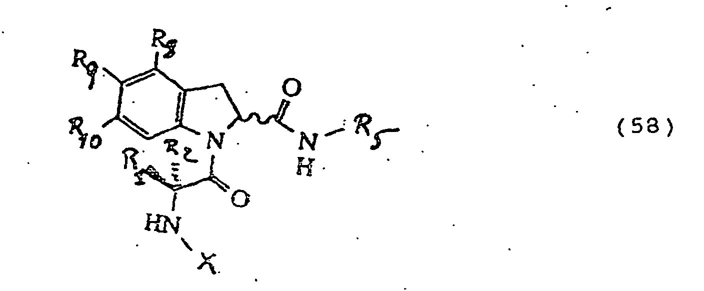 où R₁, R₂, R₅, R₈, R₉, R₁₀ et X ont la signification donnée précédemment,
vi) l'élimination du groupement X pour former le composé (I) désiré.

34. Procédé pour la préparation d'un composé de formule (I) donnée ci-dessus, dans laquelle :
- R₁ représente un groupe alkyle en C₁-C₄ linéaire ou ramifié ;
- R₂ représente un hydrogène ;
- l'un des substituants R ou R' représente un hydrogène et l'autre un groupe alkyle en C₁-C₂.
- n = 0 et m = 1 et R₃ représente le motif 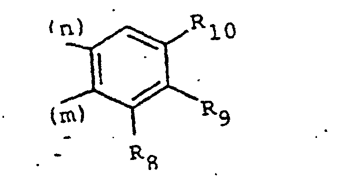 avec R₈, R₉ et R₁₀ représentant un hydrogène
- R₄ représente un groupe amide CONHR₅ où R₅ est un groupe alkyle en C₁-C₆ linéaire ou ramifié,
**caractérisé en ce qu'**il comprend :
i) la formation du composé (59) 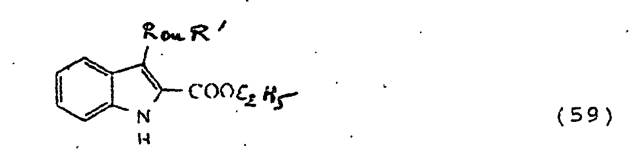 où R et R' sont tels que définis ci-dessus
par réaction entre la phényl hydrazine et l'acide 2-céto-butyrique en milieu acide
ii) la formation du composé (60) à partir du composé (59) obtenu 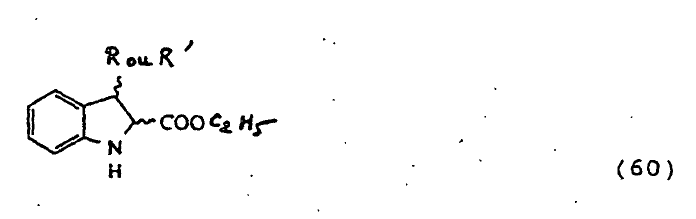 où R et R' sont tels que définis ci-dessus
en présence de magnésium dans le méthanol,
iii) la formation de l'amide (61) correspondant au composé (60) par réaction avec l'amine appropriée R₅NH₂ 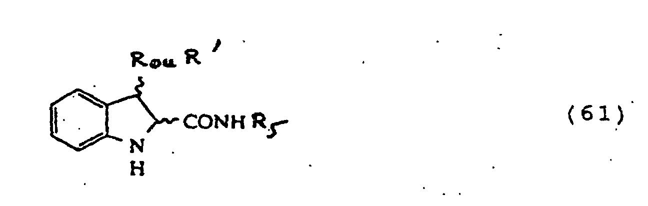 où R et R' sont tels que définis ci-dessus
iv) la séparation des isomères cis (61a) d'une part et des isomères trans (61b) d'autre part du composé (61) 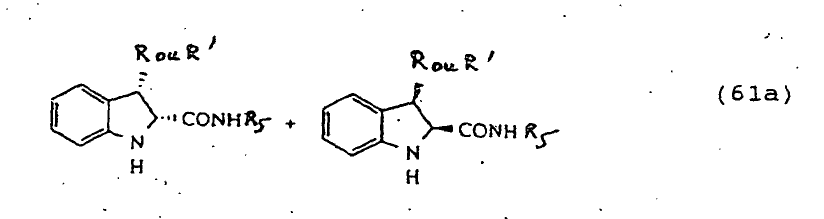 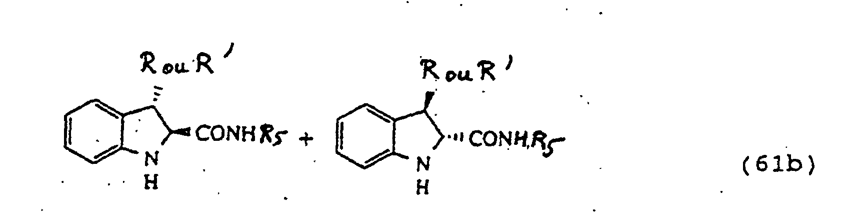
v) la réaction respectivement des composés (61a) et (61b) avec le composé (IX) 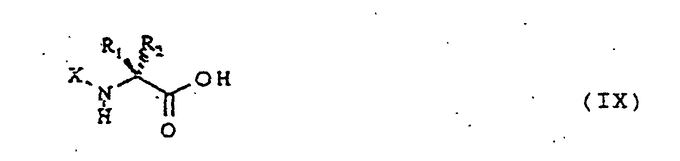 où R₁ et R₂ ont la signification donnée précédemment et X représente un groupe protecteur, pour former respectivement les mélanges (62a) et (62b) 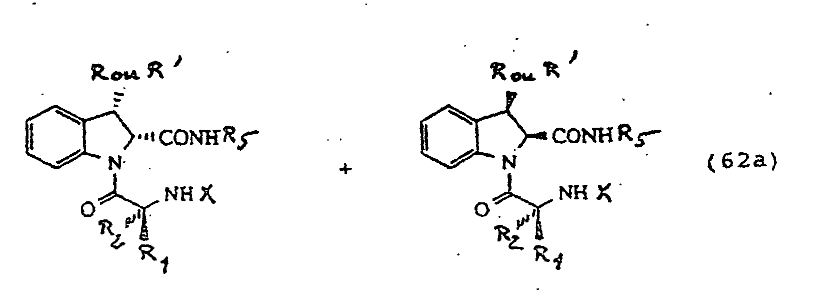 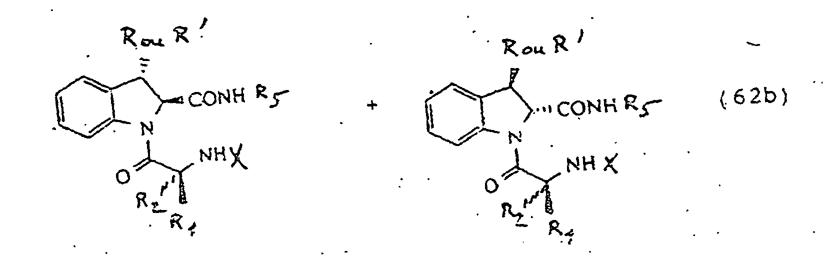 où R₁, R₂, R, R' et R₅ ont la signification donnée précédemment
vi) l'élimination du groupement protecteur X conduisant au composé (I) désiré sous forme respectivement des paires cis (63a) et trans (63b).

35. Procédé pour la préparation d'un composé de formule (I) donnée ci-dessus, dans laquelle :
- R₁ représente un groupe alkyle en C₁-C₄ linéaire ou ramilfié ;
- R₂ représente un hydrogène ;
- R et R' représentent chacun un groupe alkyle en C₁-C₂, identiques ou différents ;
- n = 0 et m = 1 et R₃ représente le motif 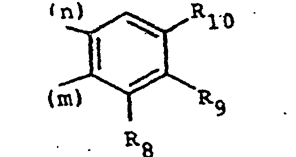
où R₈, R₉ et R₁₀ représentent chacun un hydrogène ;
- R₄ représente un groupe amide CONHR₅ où R₅ est un groupe alkyle en C₁-C₆ linéaire ou ramifié ;
**caractérisé en ce qu'**il comprend :
i) la formation du composé (65) de formule suivante : 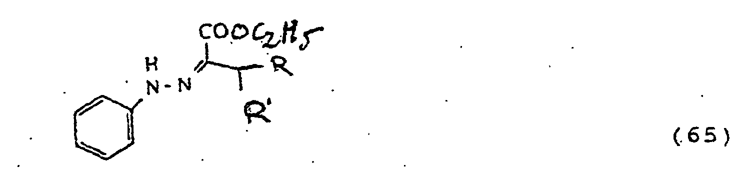 par réaction de la phénylhydrazine avec un composé 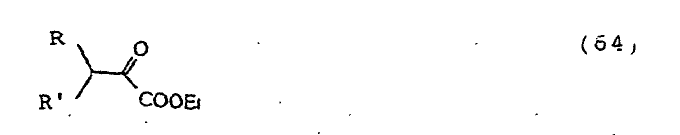
ii) la cyclisation du composé (65) en milieu acide, pour former le composé (66) suivant : 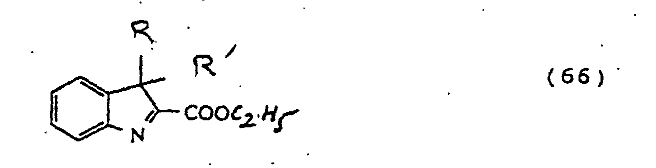 où R et R' sont tels que définis ci-dessus
iii) l'hydrogénation du composé (66) conduisant au composé (67) suivant 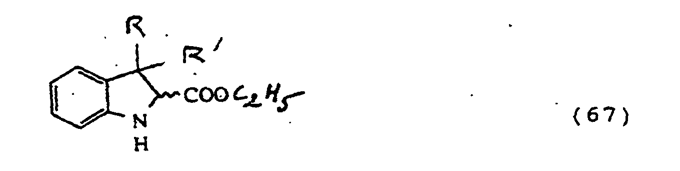 où R et R' sont tels que définis ci-dessus
iv) la formation de l'amide correspondant (68) par action de LiNHR₅ où R₅ a la signification précitée 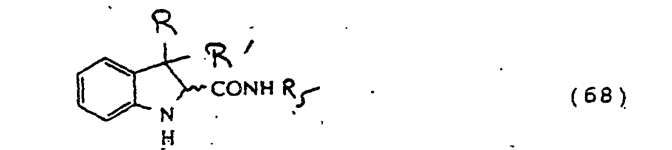 où R et R' sont tels que définis ci-dessus
v) la réaction du composé (68) avec le composé de formule (IX) 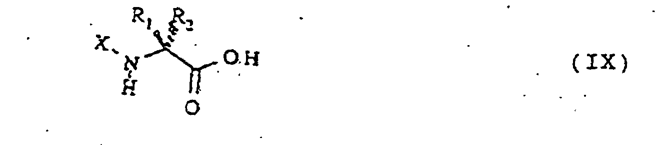 où R₁ et R₂ ont la signification donnée ci-dessus et X est un groupement protecteur, pour former le composé (69) 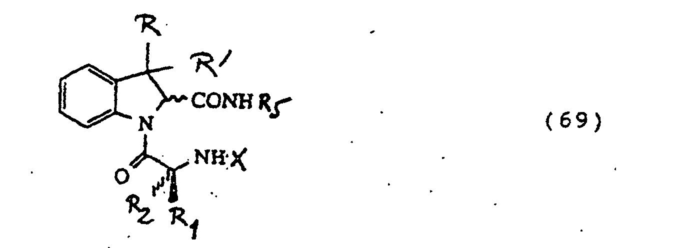 où R et R' sont tels que définis ci-dessus
vi) l'élimination du groupement X pour former le composé (I) désiré.

36. Procédé pour la préparation d'un composé de formule (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 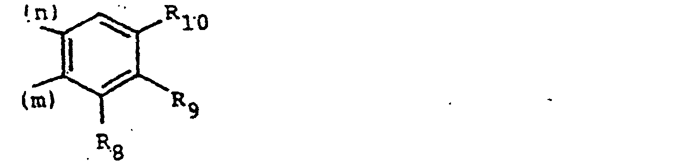
où R₉ représente un groupe OH et R₈ et R₁₀ représentent tous deux un hydrogène ;
- R₄ représente un groupe amide CONHR₅ où R₅ est un groupe alkyle en C₁-C₆ linéaire ou ramifié ;
**caractérisé en ce qu'**il comprend :
i) la formation du composé (71) 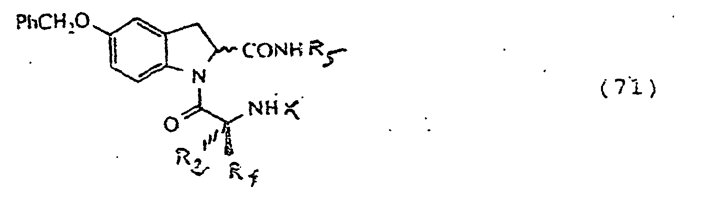 où R₉ représente un groupe OCH₂Ph, R₅ a la signification donnée précédemment et X représente un groupe protecteur, par réaction du composé (70). 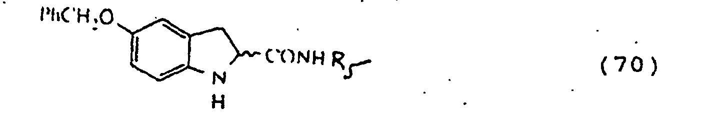 où R₅ est tel que défini ci-dessus, avec un composé 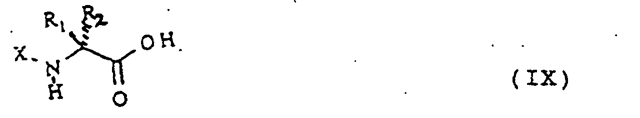 dans lequel R₁ et R₂ ont la signification donnée ci-dessus et X représente un groupement protecteur
ii) l'élimination des groupes CH₂Ph et X du composé (71) pour former le composé (I) désiré.

37. Procédé pour la préparation d'un composé de formule (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 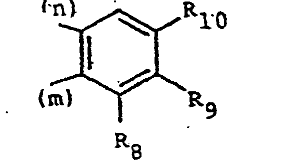
où R₉ représente un atome d'halogène et R₈ et R₁₀ représentent chacun un hydrogène
- R₄ représente un groupe amide CONHR₅ où R₅ est un groupe alkyle en C₁-C₆ linéaire ou ramifié ;
**caractérisé en ce qu'**il comprend :
i) la formation de l'ester méthylique (73) 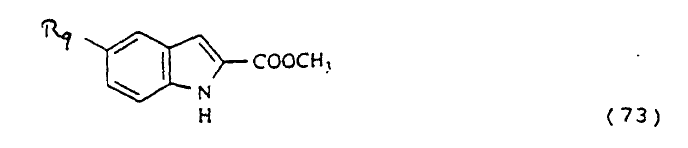 à partir de l'acide (72) correspondant 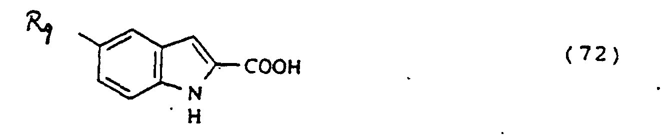 où R₉ est tel que défini ci-dessus,
avec de l'acide sulfurique concentré dans du méthanol,
ii) la formation du composé (74) 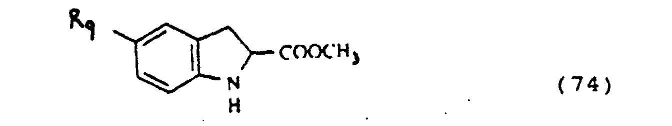 où R₉ est tel que défini ci-dessus,
à partir du composé (73), avec du magnésium dans du méthanol,
iii) la formation de l'amide (75) correspondant par réaction avec l'amine appropriée R₅NH₂ 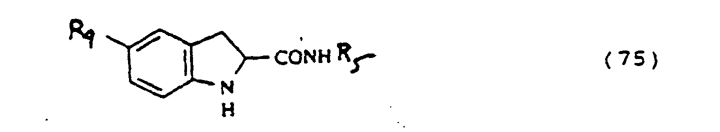 où R₅ et R₉ sont tels que définis ci-dessus
iv) la réaction du composé (75) avec un composé (IX) 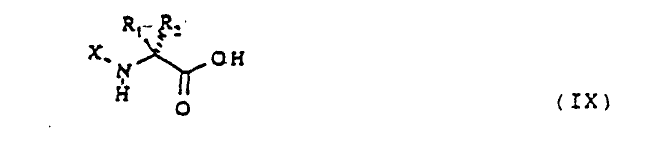 dans lequel R₁ et R₂ ont les significations données précédemment et X représente un groupe protecteur, pour former le composé (76) 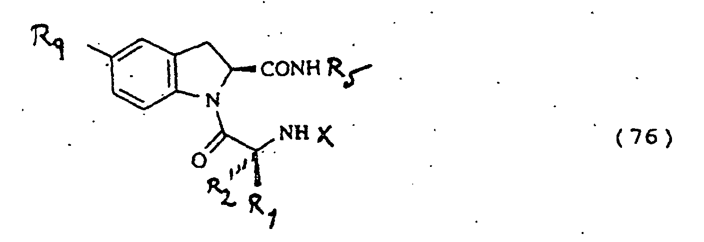 où R₁, R₂, R₅, R₉ et X ont les significations données précédemment
v) l'élimination du groupement X pour former le composé (I) désiré.

38. Procédé de préparation d'un composé de formule (I) donnée ci-dessus, dans laquelle :
- R₁ représente un groupe alkyle en C₁-C₄ linéaire ou ramifié ;
- R₂, R et R' représentent chacun un hydrogène ;
- n = 0 et m = 1 et R₃ représente le motif 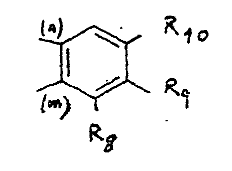
où R₈, R₉ et R₁₀ représentent chacun un hydrogène;
- R₄ représente un groupe amide CONHR₅ où R₅ est un groupe (CH₂)₃OH
**caractérisé en ce qu'**il comprend :
i) la réaction de l'ester méthylique de l'acide indoline 2S-carboxylique avec un composé de formule (IX) 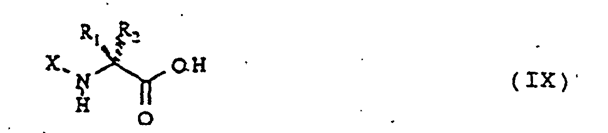 où R₁ et R₂ ont la signification donnée ci-dessus et X représente un groupe protecteur, pour former le composé (77) suivant 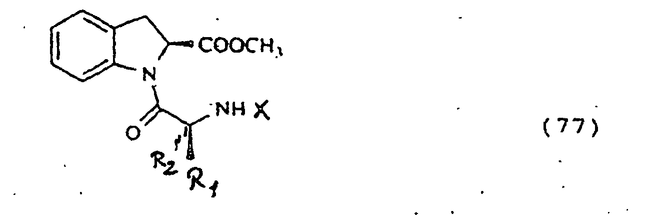 où R₁ et R₂ et X sont tels que définis ci-dessus
ii) la formation de l'amide (78) 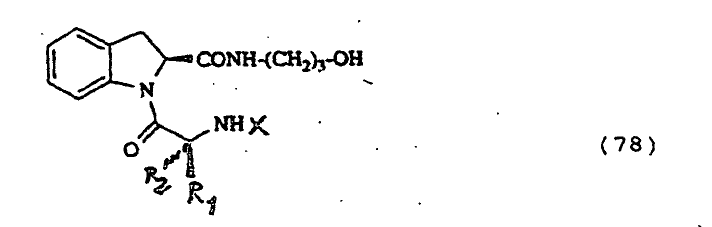 où R₁ et R₂ ont la signification précédente
à partir du composé (77), par action de la 3-hydroxypropylamine dans le méthanol
iii) l'élimination du groupement X pour former le composé (1) désiré.

39. Utilisation d'un composé de formule générale (I) selon l'une quelconque des revendications 6 à 23 pour la préparation d'un médicament pour le traitement chez l'homme ou l'animal des troubles ou affections liés à l'inactivation ou dégradation exagérée, ou pouvant être traités en retardant la dégradation physiologique de neuropeptides endogènes, désactivés par la tripeptidylpeptidase membranaire définie à la revendication 1.

40. Utilisation selon la revendication 39, **caractérisée en ce que** les troubles ou affections sont liés à l'inactivation de la cholécystokinine (CCK).

41. Utilisation selon la revendication 40, **caractérisée en ce que** les troubles ou affections sont des troubles de la prise alimentaire, de l'humeur, cognitifs ou moteurs, notamment l'anorexie, la schizophrénie, la maladie de Parkinson et les dépressions ainsi que les troubles du transit gastro-intestinal tel que le syndrome du côlon irritable, la boulimie ou les états d'obésité pathologique.

42. Utilisation selon la revendication 41, **caractérisé en ce que** le composé de formule générale (I) est tel que défini selon l'une quelconque des revendications 7 à 23.

43. Utilisation selon la revendication 41 ou 42, **caractérisée en ce que** les troubles ou affections sont liés à l'inactivation de la cholécystokinine (CCK).

44. Utilisation selon la revendication 43,
**caractérisée en ce que** les troubles ou affections sont des troubles de la prise alimentaire, de l'humeur, cognitifs ou moteurs, notamment l'anorexie, la schizophrénie, la maladie de Parkinson et les dépressions ainsi que les troubles du transit gastro-intestinal tel que le syndrome du côlon irritable, la boulimie ou les états d'obésité pathologique.

45. Composition pharmaceutique comprenant un composé selon l'une quelconque des revendications 6 à 23 dans un excipient pharmaceutiquement acceptable.

46. Composition pharmaceutique selon la revendication 45, **caractérisée en ce qu'**elle comprend un composé selon la revendication 21, 22 ou 23.

## Patentansprüche

1. Verfahren zum Sichten von Arzneimitteln, die bestimmt sind für die Behandlung von Störungen oder Erkrankungen von Menschen oder Tieren, welche verknüpft sind mit der Inaktivierung oder dem übermäßigen Abbau oder welche behandelt werden können durch Verzögern des physiologischen Abbaus von endogenen Neuropeptiden, **dadurch gekennzeichnet, daß** es darin besteht, ein Kandidaten-Molekül mit einer Membran-Tripeptidylpeptidase in Kontakt zu bringen und die Aktivität dieses Enzyms zu messen, wobei diese Membran-Tripeptidylpeptidase durch die Nucleotidsequenz codiert wird, welche durch die Identifikatoren SEQ ID N°1 und SEQ ID N°2 gegeben ist.

2. Verfahren nach Anspruch 1, **dadurch gekennzeichnet, daß** die Tripeptidylpeptidase dem Identifikator der Sequenz ID N°1 des beigefügten Sequenzprotokolls entspricht.

3. Verfahren nach Anspruch 1 oder 2, **dadurch gekennzeichnet, daß** die Tripeptidylpeptidase dem Identifikator der Sequenz ID N°2 des beigefügten Sequenzprotokolls entspricht.

4. Verfahren nach Anspruch 1, **dadurch gekennzeichnet, daß** es darin besteht, Membranen des Gehirns, beispielsweise von Ratten, die durch Zentrifugieren eines Homogenisats hergestellt worden sind, in Gegenwart eines Substrats für Aminotripeptidylpeptidase und eines Kandidaten-Moleküls, welches einen potentiellen Inhibitor der enzymatischen Aktivität darstellt, zu inkubieren.

5. Verfahren nach einem der Ansprüche 1 bis 4, **dadurch gekennzeichnet, daß** die Störungen oder Erkrankungen mit der Inaktivierung von Cholecystokinin (CCK) verknüpft sind.

6. Verbindung der folgenden allgemeinen Formel (I): 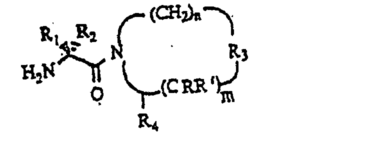 in der:
- R₁ Wasserstoff oder eine geradkettige oder verzweigte C₁-C₄-Alkylgruppe bedeutet;
- R₂ Wasserstoff oder eine C₁-C₂-Alkylgruppe bedeutet;
wobei mindestens einer der Reste R₁ und R₂ Wasserstoff bedeutet;
- n = 0 oder 1 und m = 0 oder 1 mit der Maßgabe bedeuten, daß n von m verschieden ist;
- R und R' jeweils unabhängig voneinander Wasserstoff oder eine C₁-C₂-Alkylgruppe bedeuten;
- R₃ einen zweiwertigen Rest bedeutet, welcher gebildet ist durch eine Alkylkette -(CH₂)₂-, -CH₂-CH(cis.F)-, -CH₂-CH(CH₂Ph)-, oder einen Rest 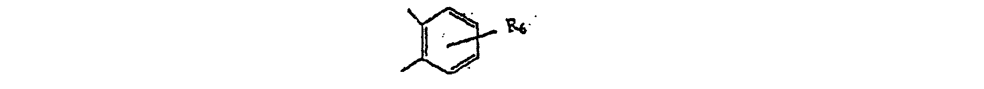
worin R₆ H, F, OCH₃ oder OCH₂Ph darstellt, 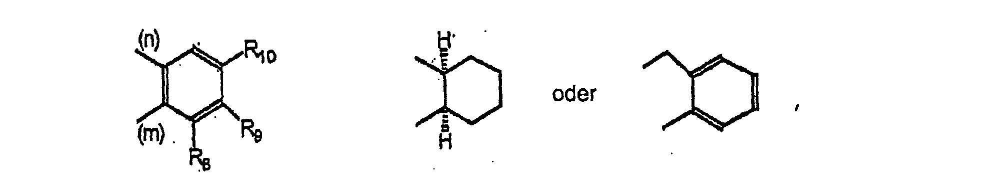 worin R₈, R₉ und R₁₀ jeweils ein Wasserstoffatom oder Halogenatom, eine Gruppe O(C₁-C₄-Alkyl), OCH₂Ph, OH oder C₁-C₄-Alkyl einschließlich R₆ bedeuten und (m) und (n) die Richtung der Bindung zu der Gruppe (CH₂)ₙ (oder N, wenn n = 0 bedeutet) beziehungsweise zu der Gruppe (CRR')ₘ (oder CHR₄, wenn m = 0 bedeutet) angeben,
- R₄ eine Amidgruppe CO-NH-R₅ bedeutet, worin R₅ Wasserstoff oder eine geradkettige oder verzweigte C₁-C₆-Alkylgruppe oder eine Gruppe -(CH₂)₃-SCH₃, -CH₂Ph, -CH₂C₆H₁₁, (CH₂)₃)OH, 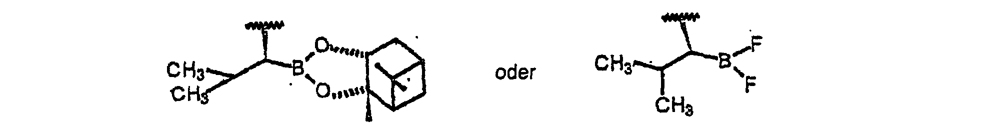
darstellt,
sowie deren entsprechende Salze oder Hydrate,
mit der Maßgabe, daß:
i) wenn R₂ = R' = R = H ; R₃ = -CH₂-CH₂- ; n + m = 1 ; R₄ = -CO-NH-R₅ ; und R₅ = H bedeuten,
dann
R₁ verschieden ist von -CH₃ ; -CH₂-CH₃ ; -(CH₂)₂-CH₃ , -(CH₂)₃-CH₃ ; -CH(CH₃)₂ ; -CH₂CH(CH₃)₂ ; und -CH(CH₃)CH₂-CH₃ ;
ii) wenn
R₂ = R' = R = H; R₃ = -CH₂-CH₂- ; n + m = 1 ; R₄ = -CO-NH-R₅ ; und R₅ = -CH₂-CH₃ bedeuten;
dann
R₁ verschieden ist von -CH₃ ; und -CH₂CH(CH₃)₂ ;
iii) wenn
R₂ = R' = R = H ; R₃ = -CH₂-CH₂- ; n + m = 1 ; R₄ = -CO-NH-R₅ ; und R₅ = -(CH₂)₄-CH₃, -CH(CH₃)₂), CH₃ oder den Rest: 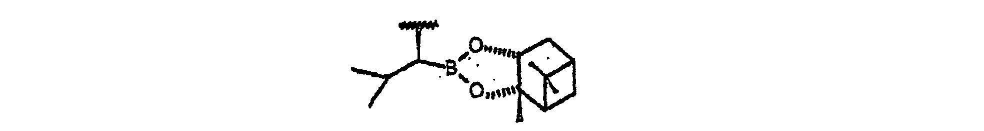 bedeuten,
dann
R₁ verschieden ist von -CH₃.

7. Verbindung nach Anspruch 6 der folgenden allgemeinen Formel (I'): 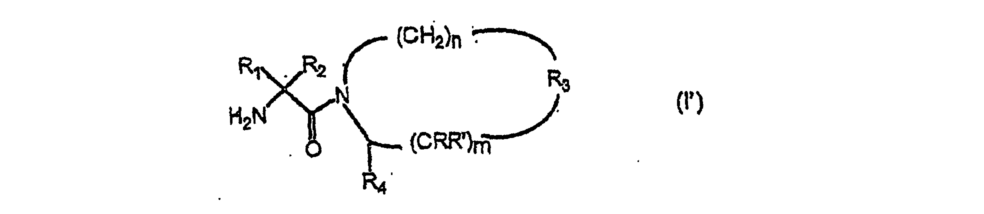 in der
- R₁ Wasserstoff oder eine geradkettige oder verzweigte C₁-C₄-Alkylgruppe bedeutet;
- R₂ Wasserstoff oder eine C₁-C₂-Alkylgruppe bedeutet;
wobei mindestens einer der Reste R₁ und R₂ Wasserstoff bedeutet;
- n = 0 oder 1 und m = 0 oder 1 bedeuten, wobei n von m verschieden ist;
- R₃ einen zweiwertigen Rest bedeutet, welcher gebildet ist durch eine Alkylkette -(CH₂)₂-, -CH₂-CH(cis.F)-, -CH₂-CH(CH₂Ph)-, einen Rest 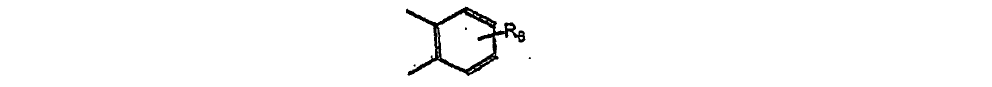
worin R₆ H, F, OCH₃ oder OCH₂Ph darstellt, 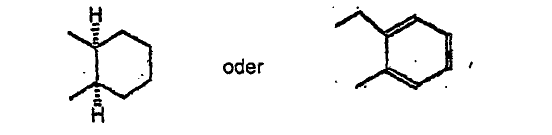
- R₄ eine Amidgruppe CO-NH-R₅ bedeutet, worin R₅ Wasserstoff oder eine geradkettige oder verzweigte C₁-C₆-Alkylgruppe, -(CH₂)₃-CH₃, -CH₂Ph, -CH₂C₆H₁₁, 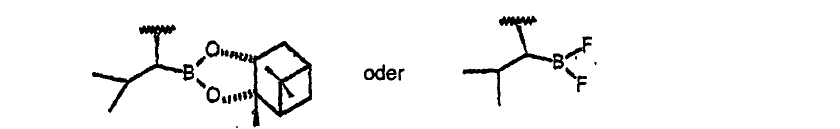 darstellt.

8. Verbindung nach Anspruch 6 oder 7, **dadurch gekennzeichnet, daß** R₃ -(CH₂)₂- bedeutet.

9. Verbindung nach Anspruch 6 oder 7, **dadurch gekennzeichnet, daß** R₃ -CH₂-CH(cis.F)- bedeutet.

10. Verbindung nach Anspruch 6 oder 7, **dadurch gekennzeichnet, daß** R₃ -CH₂-CH(CH₂Ph)- bedeutet.

11. Verbindung nach Anspruch 6 oder 7, **dadurch gekennzeichnet, daß** n = 0 und m = 1 bedeuten.

12. Verbindung nach Anspruch 11, **dadurch gekennzeichnet, daß** R₃ 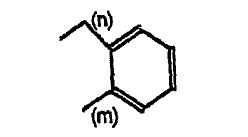 bedeutet.

13. Verbindung nach Anspruch 11, **dadurch gekennzeichnet, daß** R und R' Wasserstoff und R₃ 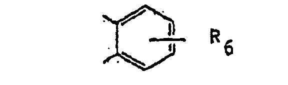 bedeuten.

14. Verbindung nach Anspruch 11, **dadurch gekennzeichnet, daß** R₃ 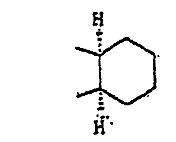 bedeutet.

15. Verbindung nach Anspruch 11, **dadurch gekennzeichnet, daß** R₃ den Rest 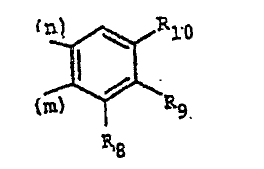 bedeutet.

16. Verbindung nach Anspruch 6 oder 7, **dadurch gekennzeichnet, daß** n = 1 und m = 0 bedeuten.

17. Verbindung nach Anspruch 16, **dadurch gekennzeichnet, daß** R₃ 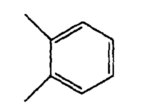 bedeutet.

18. Verbindung nach einem der Ansprüche 6 bis 17, **dadurch gekennzeichnet, daß** R₅ die n-Butylgruppe bedeutet.

19. Verbindung nach einem der Ansprüche 6 bis 18, **dadurch gekennzeichnet, daß** R₂ Wasserstoff bedeutet.

20. Verbindung nach einem der Ansprüche 6 bis 19, **dadurch gekennzeichnet, daß** R₁ Wasserstoff bedeutet.

21. Verbindung nach einem der Ansprüche 6 bis 20, **dadurch gekennzeichnet, daß** sie ausgewählt ist aus der Gruppe, welche:
- L-Valyl-L-prolin-n-hexylamid;
- 1-(2(S)-Aminobutyryl)-L-prolin-3-(methylthio)-propylamid;
- 1-(2(S)-Aminobutyryl)-L-prolin-n-pentylamid;
- 1-(2(S)-Aminobutyryl)-L-prolin-n-butylamid;
- 1-(2(S)-Aminobutyryl)-L-prolin-[2(S)-methyl)-butylamid;
- 1-(2(S)-Aminobutyryl)-L-prolin-n-propylamid;
- 1-(2(S)-Aminobutyryl)-L-prolin-isobutylamid;
- L-Valyl-L-prolin-n-butylamid;
- L-Alanyl-L-prolyl-difluor-L-borvalin-Hydroborat;
- 1-(2(S)-Aminobutyryl)-(4(S)-fluor)-L-prolin-n-butylamid;
- 1-(2(S)-Aminobutyryl)-(4(S)-benzyl-L-prolin-n-butylamid;
- 2-(2(S)-Amiobutyryl)-1,2,3,4-tetrahydro-3(S)-isochinolincarbonsäure-n-butylamid;
- 1-(2(S)-Aminobutyryl)-2(S)-indolincarbonsäure-n-butylamid;
- 1-(2(S)-Aminobutyryl)-2(S)-indolincarbonsäure-n-propylamid;
- 1-(2(S)-Aminobutyryl)-2-(S)-indolincarbonsäure-methylamid;
- 1-(2(S)-Aminobutyryl)-2(S)-indolincarbonsäure-ethylamid;
- 1-(2(S)-Aminobutyryl)-2(R/S)-(5-methoxy)-indolincarbonsäure-n-butylamid;
- 1-(2(S)-Aminobutyryl)-2(R/S)-(6-methoxy)-indolincarbonsäure-n-butylamid;
- 1-(2(S)-Aminobutyryl)-2(R/S)-(5-fluor)-indolincarbonsäure-n-butylamid;
- 1-(2(S)-Aminobutyryl)-2(R/S)-(5-benzyloxy)-indolincarbonsäure-n-butylamid;
- 1-(2(S)-Aminobutyryl)-2(S)-[(3aS,7aS)-perhydro]-indolincarbonsäure-n-butylamid;
- 2-(2(S)-Aminobutyryl)-1(R/S)-isoindolincarbonsäure-n-butylamid;
sowie deren entsprechende Salze und Hydrate umfaßt.

22. Verbindung nach Anspruch 14, **dadurch gekennzeichnet, daß** sie aus der Gruppe ausgewählt ist, die:
- 1-(2(S)-Aminobutyryl)-2(S)-indolincarbonsäure-n-butylamid;
- 1-(2(S)-Aminobutyryl)-2(S)-indolincarbonsäure-n-propylamid;
- 1-(2(S)-Aminobutyryl)-2(S)-indolincarbonsäure-ethylamid;
- 1-(2(S)-Aminobutyryl)-2(R/S)-(5-methoxy)-indolincarbonsäure-n-butylamid;
- 1-(2(S)-Aminobutyryl)-2(R/S)-(5-fluor)-indolincarbonsäure-n-butylamid;
sowie deren entsprechende Salze und Hydrate umfaßt.

23. Verbindung nach Anspruch 15, **dadurch gekennzeichnet, daß** sie aus der Gruppe ausgewählt ist, die:
- 1-(L-Valyl)-5-methoxyindolin-2(R/S)-carbonsäure-butylamid;
- 1-(L-Alanyl)-5-methoxyindolin-2(R/S)-carbonsäure-butylamid;
- 1-(L-Alanyl)-5-methoxyindolin-2(S)-carbonsäure-butylamid;
- 1-(2(S)-Aminobutyryl)-4-methoxy-indolin-2(R/S)-carbonsäure-butylamid;
- 1-(2(S)-Aminobutyryl)-3,3-dimethylindolin-2(R/S)-carbonsäure-butylamid;
- 1-(2(S)-Aminobutyryl)-3(R)-methylindolin-2(R)-carbonsäure-butylamid und 1-(2(S)-Aminobutyryl)-2(S)-methylindolin-2(S)-carbonsäure-butylamid;
- 1-(2(S)-Aminobutyryl)-3(R)-methylindolin-2(S)-carbonsäure-butylamid und 1-(2(S)-Aminobutyryl)-3(S)-methylindolin-2(R)-carbonsäure-butylamid;
- 1-(2(S)-Aminobutyryl)-4-ethoxyindolin-2(S)-carbonsäure-butylamid;
- 1-(2(S)-Aminobutyryl)-4,5-dimethoxyindolin-2(R/S)-carbonsäure-butylamid;
- 1-2(S)-Aminobutyryl-5-hydroxyindolin-2(S)-carbonsäure-butylamid;
- 1-2(S)-Aminobutyryl-5-hydroxyindolin-2(R/S)-carbonsäure-butylamid;
- 1-2(S)-Aminobutyryl-5-methylindolin-2(R/S)-carbonsäure-butylamid;
- 1-(2(S)-Aminobutyryl)-5-chlorindolin-2(S)-carbonsäure-butylamid;
- 1-(2(S)-Aminobutyryl)-indolin-2(S)-carbonsäure-(3-hydroxy)-propylamid;
sowie deren entsprechende Salze und Hydrate umfaßt.

24. Verfahren zur Herstellung einer Verbindung der oben angegebenen allgemeinen Formel (I), in der:
- R₁ eine geradkettige oder verzweigte C₁-C₄-Alkylgruppe bedeutet;
- R₂ Wasserstoff bedeutet;
- n = 0 oder 1 und m = 0 oder 1 bedeuten, wobei n von m verschieden ist und R₃ -(CH₂)₂- bedeutet; und
- R und R' Wasserstoff bedeuten,
- R₄ CO-NH-R₅ bedeutet,
worin R₅ Wasserstoff oder eine geradkettige oder verzweigte C₁-C₆-Alkylgruppe, -(CH₂)₃-S-CH₃, -CH₂Ph bedeutet;
mit der Maßgabe, daß R₁ von CH₂CH₃, (CH₂)₂CH₃, (CH₂)₃CH₃, CH(CH₃)₂, CH₂CH(CH₃)₂, CH(CH₃)CH₂CH₃ verschieden ist, wenn R₅ Wasserstoff bedeutet, R₁ von CH₂CH(CH₃)₂ verschieden ist, wenn R₅ die Gruppe CH₂CH₃ bedeutet und R₁ von CH₃ verschieden ist, wenn R₅ die Gruppe -(CH₂)₄-CH₃, -CH(CH₃)₂, -CH₂CH₃, -CH₃ oder ein Wasserstoffatom oder den Rest 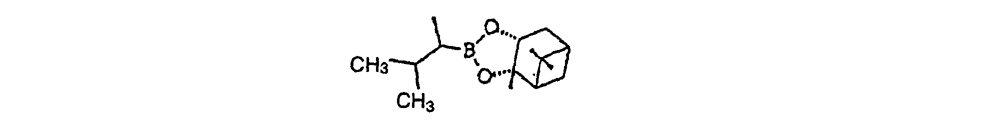 bedeutet,
**dadurch gekennzeichnet, daß** es umfaßt:
i) die Bildung einer Verbindung der Formel (III) 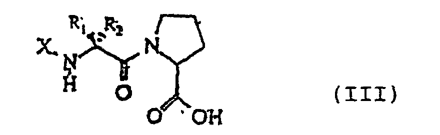 in der R₁ und R₂ die oben angegebenen Bedeutungen besitzen und X eine Schutzgruppe darstellt, ausgehend von einer Verbindung der Formel (II) 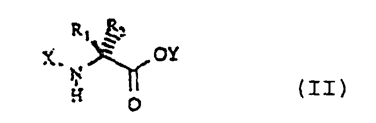 welche an ihrer Säurefunktion durch eine Gruppe Y verestert ist und in der X. R₁ und R₂ die oben angegebenen Bedeutungen besitzen, durch Reaktion mit L-Prolin;
ii) die Amidierung der Säurefunktion der Verbindung (III) mit dem geeigneten Amin R₅NH₂, worin R₅ die oben angegebenen Bedeutungen besitzt, zur Bildung des Derivats (IV) 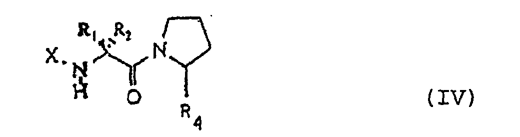 welche an ihrer primären Aminfunktion durch die Gruppe X geschützt ist;
iii) die Eliminierung der Gruppe X des Derivats (IV) zur Bildung der gewünschten Verbindung (I).

25. Verfahren zur Herstellung einer Verbindung der oben angegebenen allgemeinen Formel (I), in der:
- R₁ eine geradkettige oder verzweigte C₁-C₄-Alkylgruppe bedeutet;
- R₂ Wasserstoff oder eine Methylgruppe bedeutet;
- n = 0 oder 1 und m = 0 oder 1 mit der Maßgabe bedeuten, daß n von m verschieden ist und R₃ eine Gruppe -(CH₂)₂- bedeutet; und
- R und R' Wasserstoff bedeuten
- R₄ eine Gruppe CO-NH-R₅ darstellt, worin R₅ Wasserstoff oder eine geradkettige oder verzweigte C₁-C₆-Alkylgruppe bedeutet;
mit der Maßgabe, daß R₂ Wasserstoff bedeutet, dann R₁ von CH₂CH₃, (CH₂)₂CH₃, (CH₂)₃CH₃, CH(CH₃)₂. CH₂CH(CH₃)₂, CH(CH₃)CH₂CH₃ verschieden ist, wenn R₅ Wasserstoff bedeutet, R₁ von CH₂CH(CH₃)₂ verschieden ist, wenn R₅ die Gruppe CH₂CH₃ bedeutet, und R₁ von CH₃ verschieden ist, wenn R₅ die Gruppe -(CH₂)₄-CH₃. -CH(CH₃)₂. -CH₂CH₃, -CH₃ oder ein Wasserstoffatom bedeutet,
**dadurch gekennzeichnet, daß** es umfaßt:
i) die Bildung einer Verbindung der Formel (IV): 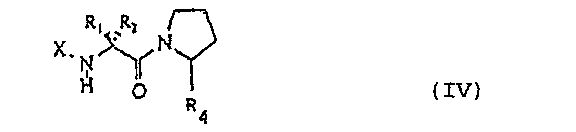 in der R₁, R₂ und R₅ die oben angegebenen Bedeutungen besitzen und X eine Schutzgruppe darstellt, ausgehend von einer Verbindung der Formel (II) 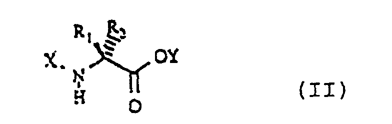 welche an ihrer Säurefunktion mit einee Gruppe Y verestert ist und in der R₁, R₂ und X die oben angegebenen Bedeutungen besitzen, durch Reaktion mit einem Prolinamid der Formel (V) 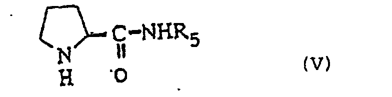 in der R₅ die oben angegebenen Bedeutungen besitzt;
ii) die Eliminierung der Schutzgruppe X der Verbindung (IV) zur Bildung der gewünschten Verbindung (I).

26. Verfahren zur Herstellung einer Verbindung der oben angegebenen allgemeinen Formel (I), in der:
- R₁ eine geradkettige oder verzweigte C₁-C₄-Alkylgruppe bedeutet;
- R₂ Wasserstoff bedeutet;
- n = 0 und m = 1 und R₃ eine Gruppe 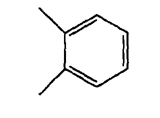 -CH₂-CH(cis.F)- 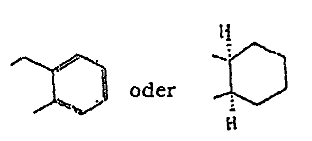 bedeuten,
- R und R' Wasserstoff bedeuten;
- R₄ eine Gruppe CO-NH-R₅ darstellt, worin R₅ Wasserstoff oder eine geradkettige oder verzweigte C₁-C₆-Alkylgruppe darstellt;
**dadurch gekennzeichnet, daß** es umfaßt:
i) die Herstellung einer Verbindung der Formel (X) 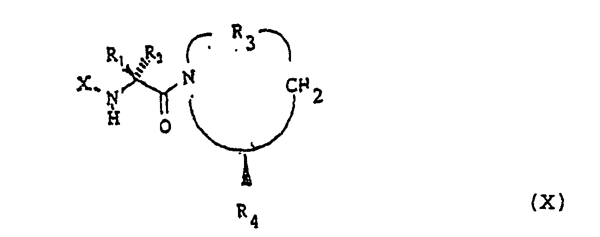 in der R₁, R₂, R₃ und R₄ die oben angegebenen Bedeutungen besitzen und X eine Schutzgruppe darstellt, durch Reaktion einer Verbindung der Formel (VIII) 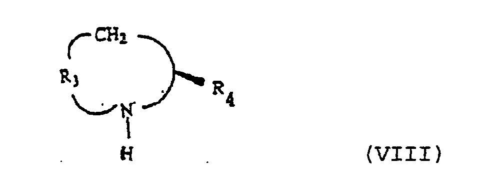 mit einer Verbindung der Formel (IX) 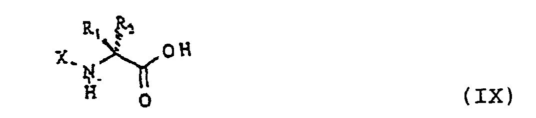 in denen R₁, R₂, R₄ und X die oben angegebenen Bedeutungen besitzen;
ii) die Eliminierung der Gruppe X der Verbindung (X) zur Bildung der gewünschten Verbindung (I).

27. Verfahren zur Herstellung einer Verbindung der oben angegebenen allgemeinen Formel (I), in der:
- R₁ eine geradkettige oder verzweigte C₁-C₄-Alkylgruppe bedeutet;
- R₂ Wasserstoff bedeutet;
- n = 1 und m = 0 und R₃ eine Gruppe -CH(CH₂Ph)-CH₂-; oder 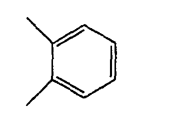 bedeuten;
- R₄ eine Gruppe CO-NH-R₅ bedeutet, worin R₅ Wasserstoff oder eine geradkettige oder verzweigte C₁-C₆-Alkylgruppe darstellt;
**dadurch gekennzeichnet, daß** es umfaßt:
i) die Bildung einer Verbindung der Formel (XII) 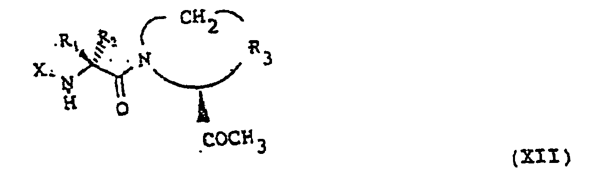 welche an der primären Aminfunktion durch eine Schutzgruppe X geschützt ist und in der R₁, R₂ und R₃ die oben angegebenen Bedeutungen besitzen, durch Reaktion der Verbindung der Formel (IX) 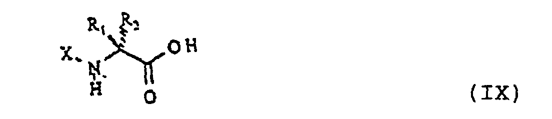 mit der Verbindung der Formel (XI) 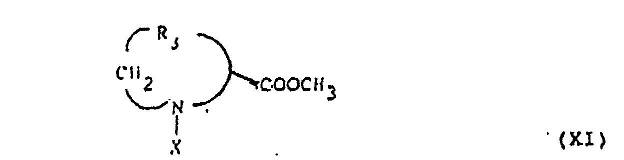 in denen R₁, R₂ und R₃ die oben angegebenen Bedeutungen besitzen und X eine Schutzgruppe darstellt;
ii) die Hydrolyse der Esterfunktion der in dieser Weise erhaltenen Verbindung (XII) zur Bildung der Verbindung der Formel (XIII) 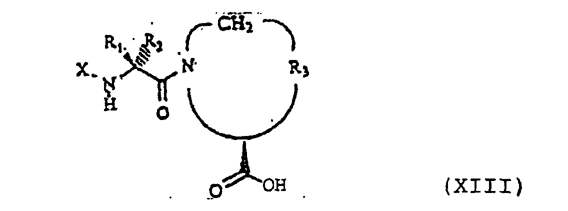 in der R₁, R₂. R₃ und X die oben angegebenen Bedeutungen besitzen;
iii) die Amidierung der Säurefunktion der Verbindung (XIII) mit Hilfe des geeigneten Amins R₅NH₂ zur Bildung des Derivats der Formel (XIV) 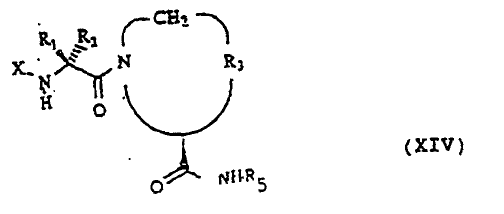 in der R₁, R₂, R₃, R₅ und X die oben angegebenen Bedeutungen besitzen;
iv) die Eliminierung der Schutzgruppe X der Verbindung (XIV) zur Bildung der gewünschten Verbindung (I).

28. Verfahren zur Herstellung einer Verbindung der oben angegebenen allgemeinen Formel (I), in der:
- R₁ eine Methylgruppe bedeutet;
- R₂ Wasserstoff bedeutet;
- n = 0 oder 1 und m = 0 oder 1, worin n von m verschieden ist, und R₃ eine Gruppe -(CH₂)₂- bedeutet; und
- R und R' Wasserstoff bedeuten;
- R₄ eine Gruppe CO-NH-R₅ bedeutet, worin R₅ (CH₃)₂-CH-BF₂ darstellt;
**dadurch gekennzeichnet, daß** es umfaßt:
i) die Eliminierung des Pinan-Rests der Verbindung der Formel (I), in der R₁, R₂, n, m, R₃ und R₄ die oben angegebenen Bedeutungen besitzen und R₅ 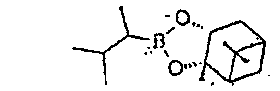 darstellt,
durch Einwirkung von Bortrichlorid in Methylenchlorid, gefolgt von einer Hydrolyse zur Bildung des Derivats (XXI) 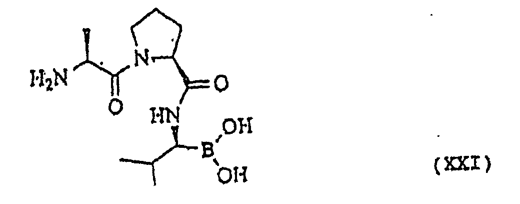
ii) die Umsetzung der Verbindung (XXI) mit Fluorwasserstoffsäure zur Bildung der gewünschten Verbindung (I).

29. Verfahren zur Herstellung einer Verbindung der oben angegebenen allgemeinen Formel (I), in der:
- R₁ eine geradkettige oder verzweigte C₁-C₄-Alkylgruppe bedeutet;
- R₂ Wasserstoff bedeutet;
- n = 0 und m = 1 und R₃ den Rest 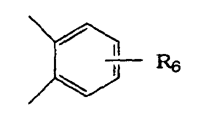 bedeuten, worin R₆ eine Gruppe OCH₃, OCH₂Ph und F darstellt; und
- R und R' Wasserstoff bedeuten; und
- R₄ eine Gruppe CO-NH-R₅ bedeutet, worin R₅ eine geradkettige oder verzweigte C₁-C₆-Alkylgruppe darstellt;
**dadurch gekennzeichnet, daß** es umfaßt:
i) die Bildung des Amids der Formel (XXXXIV) 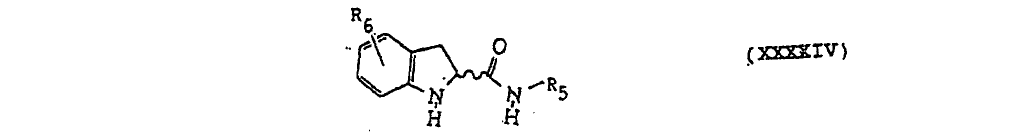 ausgehend von dem Ester der Formel (XXXXIII) 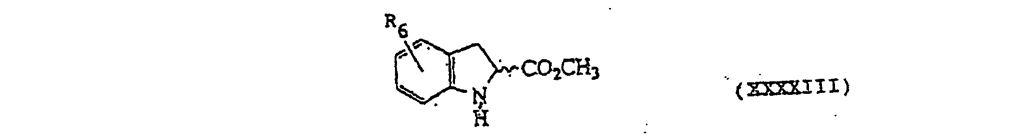 in denen R₆ die oben angegebenen Bedeutungen besitzt, durch Reaktion mit dem geeigneten Amin R₅NH₂;
ii) die Umsetzung der Verbindung (XXXIV) mit der Verbindung der Formel (IX) 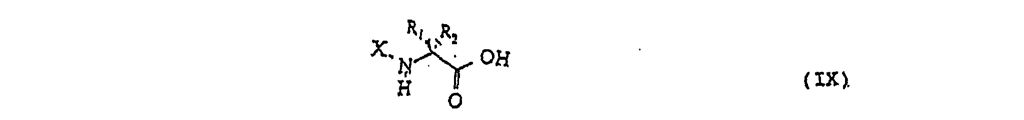 in der R₁ und R₂ die oben angegebenen Bedeutungen besitzen und X eine Schutzgruppe darstellt, zur Bildung der Verbindung der Formel (XXXXV) 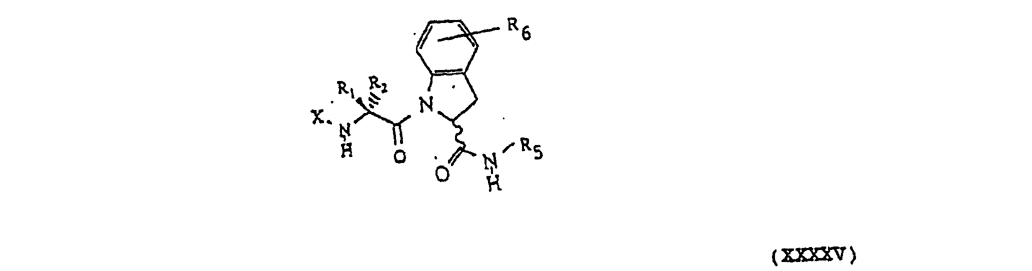 in der R₁, R₂, R₅, R₆ und X die oben angegebenen Bedeutungen besitzen;
iii) die Eliminierung der Gruppe X zur Bildung der gewünschten Verbindung (I).

30. Verfahren nach Anspruch 29, **dadurch gekennzeichnet, daß** man den Methylester (XXXXIII) herstellt
i) durch Einwirkung von Natriumnitrit in Gegenwart von Chlorwasserstoffsäure auf die Verbindung der Formel (XXXIX) 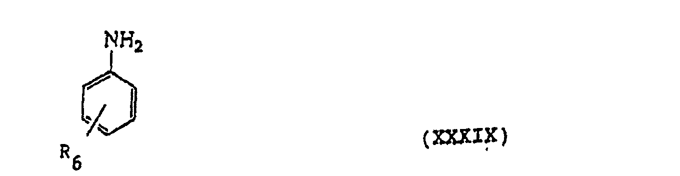 zur Bildung der Verbindung der Formel (XXXX) 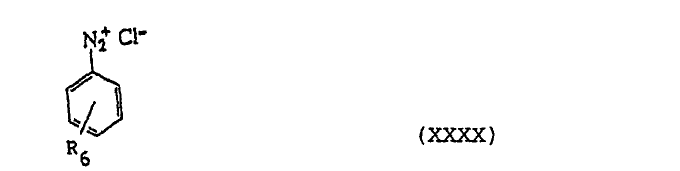 in denen R₆ die oben angegebene Bedeutung besitzt,
ii) durch Addition von 2-Methylacetoessigsäureethylester an die in dieser Weise erhaltene Verbindung (XXXX) in Gegenwart von Natriumnitrit in Ethanol zur Bildung der Verbindung der Formel (XXXXI) 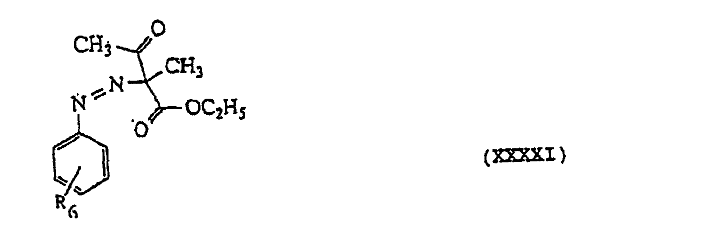 in der R₆ die oben angegebene Bedeutung besitzt;
iii) durch Cyclisieren in saurem Medium zur Bildung des Ethylesters (XXXXII) 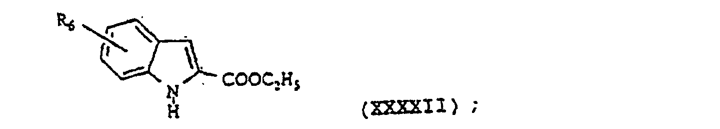
iv) durch Austausch ausgehend von dem in dieser Weise erhaltenen Ethylester (XXXXII) in Gegenwart von Magnesium in Methanol.

31. Verfahren zur Herstellung einer Verbindung der oben angegebenen allgemeinen Formel (I), in der:
- R₁ eine geradkettige oder verzweigte C₁-C₄-Alkylgruppe bedeutet;
- R₂, R und R' jeweils Wasserstoff bedeuten;
- n = 0 und m = 1 und R₃ den Rest 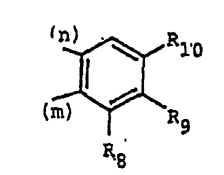 bedeuten, in der R₈ und R₁₀ Wasserstoff und R₉ eine Gruppe O(C₁-C₄-Alkyl) oder eine C₁-C₄-Alkylgruppe bedeuten und
- R₄ eine Amidgruppe CO-NH-R₅ bedeutet, worin R₅ eine geradkettige oder verzweigte C₁-C₆-Alkylgruppe darstellt;
**dadurch gekennzeichnet, daß** es umfaßt:
i) die Bildung des Amids der Formel (49) 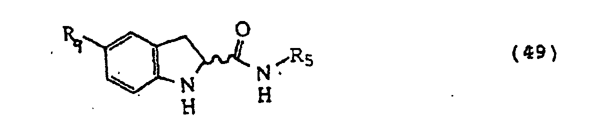 ausgehend von dem Ester der Formel (48) 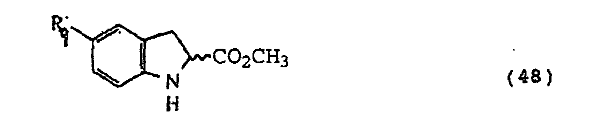 in denen R₉ und R₅ die oben angegebenen Bedeutungen besitzen, durch Umsetzen mit dem geeigneten Amin R₅NH₂;
ii) die Reaktion der Verbindung (49) mit der Verbindung der Formel (IX) 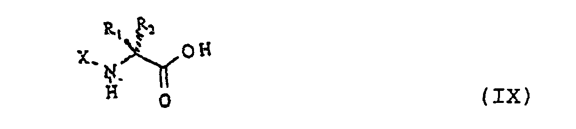 in der R₁ und R₂ die oben angegebenen Bedeutungen besitzen und X eine Schutzgruppe darstellt, zur Bildung der Verbindung der Formel (50) 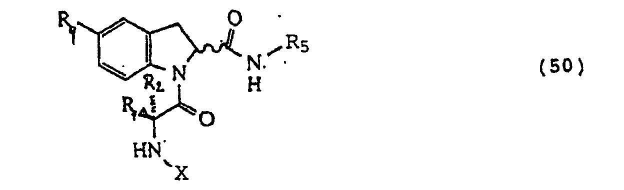 in der R₁, R₂, R₉ und X die oben angegebenen Bedeutungen besitzen;
iii) die Eliminierung der Gruppe X zur Bildung der gewünschten Verbindung (I).

32. Verfahren nach Anspruch 31, **dadurch gekennzeichnet, daß** man den Methylester (48) erhält ausgehend von der entsprechenden Säure (46) 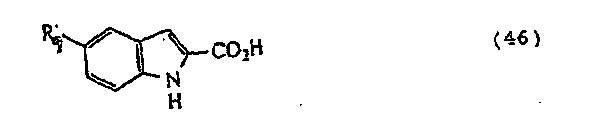 in der R₉ die oben angegebenen Bedeutungen besitzt,
i) durch Behandeln mit konzentrierter Schwefelsäure in Ethanol oder Methanol zur Bildung des entsprechenden Esters (47) 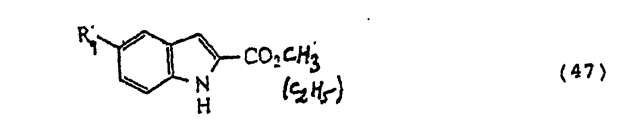 in der R₉ die oben angegebenen Bedeutungen besitzt,
ii) Behandeln der Verbindung (47) mit Magnesium in Methanol.

33. Verfahren zur Herstellung einer Verbindung der obigen allgemeinen Formel (I), in der:
- R₁ eine geradkettige oder verzweigte C₁-C₄-Alkylgruppe bedeutet;
- R₂, R und R' jeweils Wasserstoff bedeuten;
- n = 0 und m = 1 und R₃ den Rest 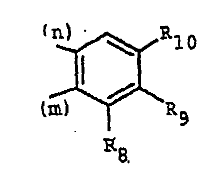 bedeuten, worin R₈ und R₉ Wasserstoff oder eine Gruppe O (C₁-C₄)-Alkyl),
wobei R₈ und R₉ nicht gleichzeitig Wasserstoff darstellen, und R₁₀ Wasserstoff bedeuten, und
- R₄ eine Amidgruppe CONHR₅ bedeutet, worin R₅ eine geradkettige oder verzweigte C₁-C₆-Alkylgruppe darstellt,
**dadurch gekennzeichnet, daß** es umfaßt:
i) die Bildung der Verbindung (54) 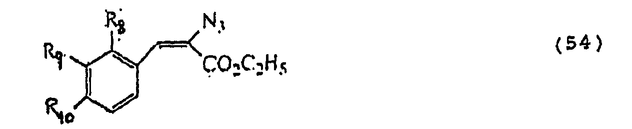 durch Umsetzen des entsprechenden Aldehyds (53) 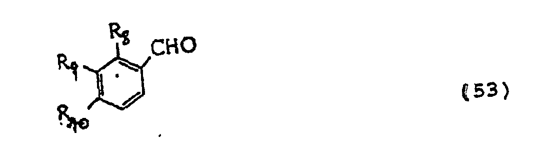 in der R₈, R₉ und R₁₀ die oben angegebenen Bedeutungen besitzen, mit dem Azidoessigsäureethylester (52) 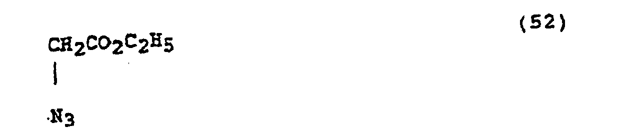
ii) die Cyclisierung der Verbindung (54) zur Bildung der Verbindung (55) 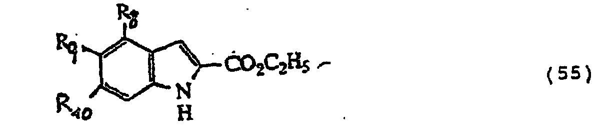 worin R₈, R₉ und R₁₀ die oben angegebenen Bedeutungen besitzen;
iii) die Bildung des Methylesters (56) 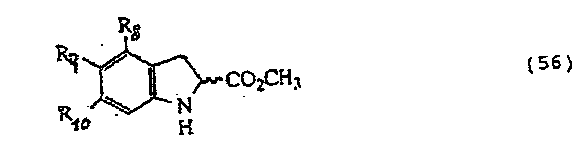 in der R₈, R₉ und R₁₀ die oben angegebenen Bedeutungen besitzen,
ausgehend von der Verbindung (55) in Gegenwart von Magnesium in Methanol;
iv) die Reaktion des erhaltenen Esters (56) mit dem geeigneten Amin R₅NH₂ zur Bildung des Amids (57) 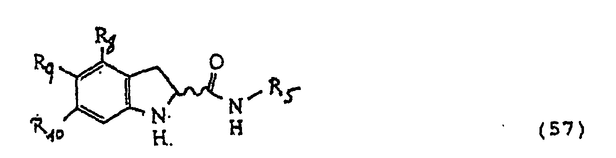 in der R₈, R₉ und R₁₀ die oben angegebenen Bedeutungen besitzen;
v) die Umsetzung der Verbindung (57) mit der Verbindung der Formel (IX) 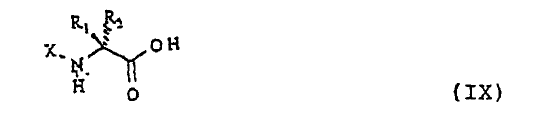 in der R₁ und R₂ die oben angegebenen Bedeutungen besitzen und X eine Schutzgruppe darstellt, zur Bildung der Verbindung (58) 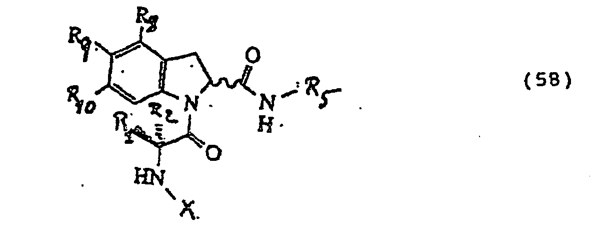 in der R₁, R₂, R₅, R₈, R₉, R₁₀ und X die oben angegebenen Bedeutungen besitzen;
vi) die Eliminierung der Gruppe X zur Bildung der gewünschten Verbindung (i).

34. Verfahren zur Herstellung einer Verbindung der oben angegebenen Formel (I), in der:
- R₁ eine geradkettige oder verzweigte C₁-C₄-Alkylgruppe bedeutet;
- R₂ Wasserstoff bedeutet;
- einer der Substituenten R und R' Wasserstoff und der andere eine C₁-C₂-Alkylgruppe bedeuten;
- n = 0 und m = 1 und R₃ den Rest 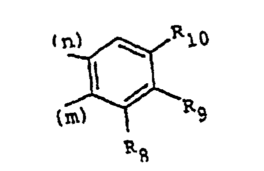 bedeuten, worin R₈, R₉ und R₁₀ Wasserstoff darstellen;
- R₄ eine Amidgruppe CONHR₅ bedeutet, worin R₅ eine geradkettige oder verzweigte C₁-C₆-Alkylgruppe darstellt,
**dadurch gekennzeichnet, daß** es umfaßt:
i) die Bildung der Verbindung (59) 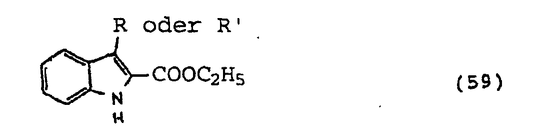 worin R und R' die oben angegebenen Bedeutungen besitzen,
durch Umsetzen von Phenylhydrazin mit 2-Ketobuttersäure in saurem Medium;
ii) die Bildung der Verbindung (60) ausgehend von der erhaltenen Verbindung (59) 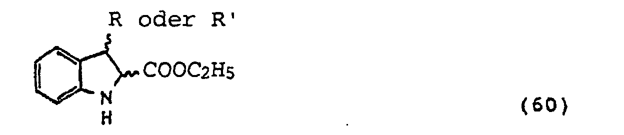 worin R und R' die oben angegebenen Bedeutungen besitzen, in Gegenwart von Magnesium in Methanol,
iii) die Bildung des der Verbindung (60) entsprechenden Amids (61) durch Umsetzen mit dem geeigneten Amin R₅NH₂ 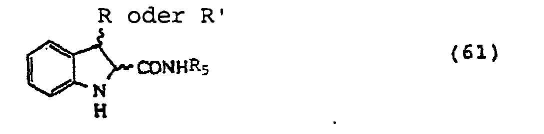 worin R und R' die oben angegebenen Bedeutungen besitzen;
iv) die Trennung der cis-Isomeren (61a) einerseits und der trans-Isomeren (61b) andererseits der Verbindung (61) 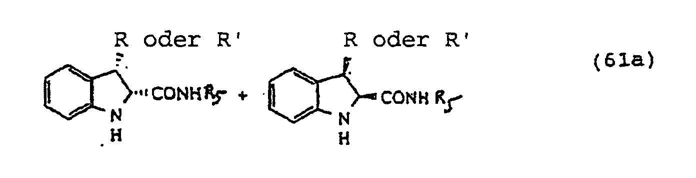 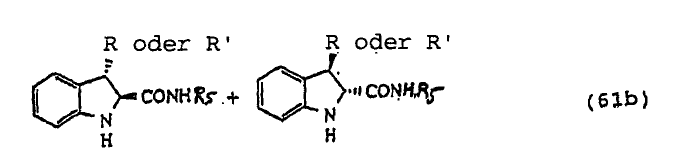
v) die Umsetzung der Verbindungen (61a) und (61b) mit der Verbindung (IX) 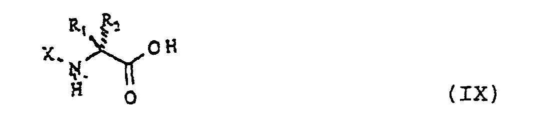 in der R₁ und R₂ die oben angegebenen Bedeutungen besitzen und X eine Schutzgruppe darstellt, zur Bildung der Mischungen (62a) und (62b) 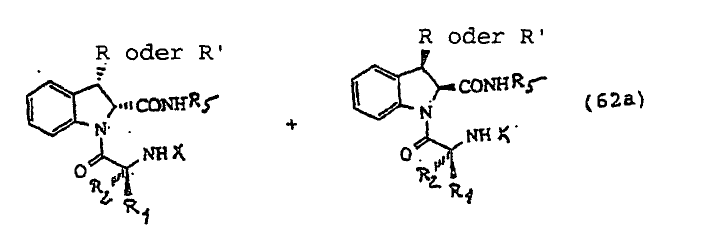 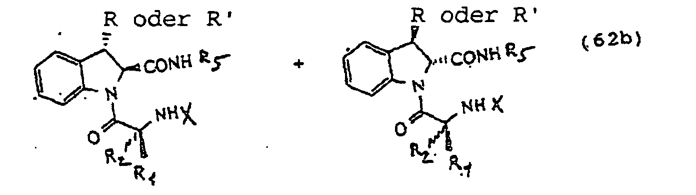 in denen R₁, R₂, R, R' und R₅ die oben angegebenen Bedeutungen besitzen;
vi) die Eliminierung der Schutzgruppe X unter Bildung der gewünschten Verbindung (I) in Form der cis-Paare (63a) bzw. trans-Paare (63b).

35. Verfahren zur Herstellung einer Verbindung der oben angegebenen Formel (I), in der:
- R₁ eine geradkettige oder verzweigte C₁-C₄-Alkylgruppe bedeutet;
- R₂ Wasserstoff bedeutet;
- R und R' jeweils gleichartige oder verschiedene C₁-C₂-Alkylgruppen bedeuten;
- n = 0 und m = 1 und R₃ den Rest 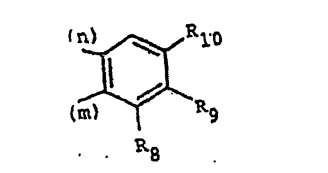 bedeuten, worin R₈, R₉ und R₁₀ jeweils Wasserstoff darstellen; und
- R₄ eine Amidgruppe CONHR₅ darstellt, worin R₅ eine geradkettige oder verzweigte C₁-C₆-Alkylgruppe bedeutet;
**dadurch gekennzeichnet, daß** es umfaßt:
i) die Bildung der Verbindung (65) der folgenden Formel: 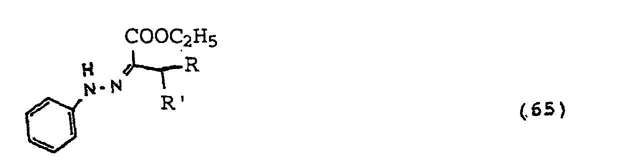 durch Umsetzen von Phenylhydrazin mit einer Verbindung 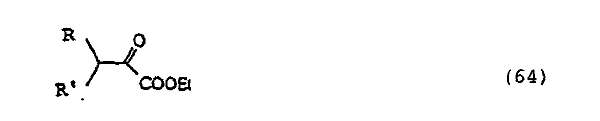
ii) die Cyclisierung der Verbindung (65) in saurem Medium zur Bildung der folgenden Verbindung (66): 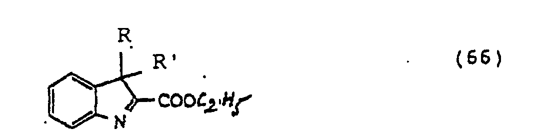 in der R und R' die oben angegebenen Bedeutungen besitzen,
iii) die Hydrierung der Verbindung (66) zur Bildung der folgenden Verbindung (67) 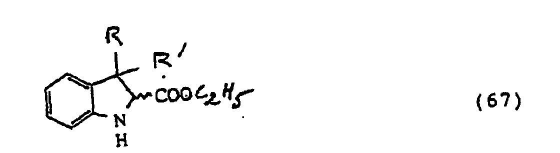 worin R und R' die oben angegebenen Bedeutungen besitzen,
iv) die Bildung des entsprechenden Amids (68) durch Reaktion mit LiNHR₅,
worin R₅ die oben angegebenen Bedeutungen besitzt, 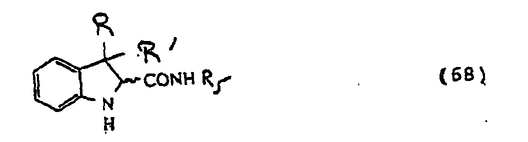 worin R und R' die oben angegebenen Bedeutungen besitzen;
v) die Umsetzung der Verbindung (68) mit der Verbindung der Formel (IX) 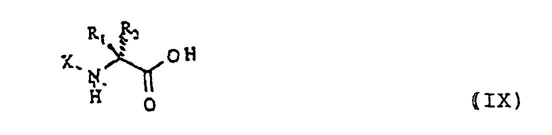 in der R₁ und R₂ die oben angegebenen Bedeutungen besitzen und X eine Schutzgruppe darstellt, zur Bildung der Verbindung (69) 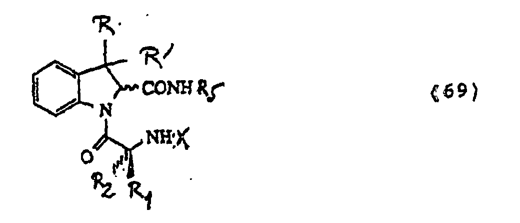 in der R und R' die oben angegebenen Bedeutungen besitzen;
vi) die Eliminierung der Gruppe X zur Bildung der gewünschten Verbindung (I).

36. Verfahren zur Herstellung einer Verbindung der oben angegebenen Formel (I), in der:
- R₁ eine geradkettige oder verzweigte C₁-C₄-Alkylgruppe bedeutet;
- R₂, R und R' jeweils Wasserstoff bedeuten;
- n = 0 und m = 1 und R₃ den Rest 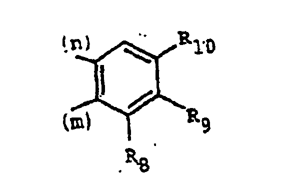 bedeuten, worin R₉ eine OH-Gruppe und R₈ und R₁₀ beide Wasserstoff bedeuten; und
- R₄ eine Amidgruppe CONHR₅ darstellt, in der R₅ eine geradkettige oder verzweigte C₁C₆-Alkylgruppe darstellt;
**dadurch gekennzeichnet, daß** es umfaßt:
i) die Bildung der Verbindung (71) 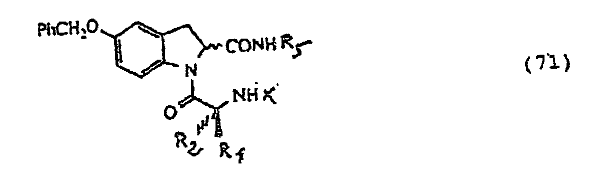 in der R₉ eine Gruppe OCH₂Ph bedeutet, R₅ die oben angegebenen Bedeutungen besitzt und X eine Schutzgruppe darstellt, durch Umsetzen der Verbindung (70) 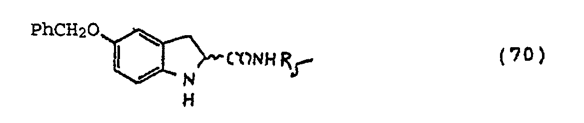 in der R₅ die oben angegebenen Bedeutungen besitzt, mit einer Verbindung (IX) 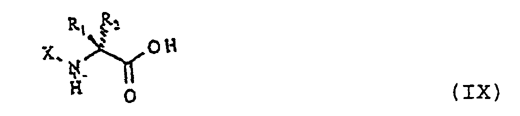 in der R₁ und R₂ die oben angegebenen Bedeutungen besitzen und X eine Schutzgruppe darstellt;
ii) die Eliminierung der Gruppen CH₂Ph und X der Verbindung (71) zur Bildung der gewünschten Verbindung (I).

37. Verfahren zur Herstellung einer Verbindung der oben angegebenen Formel (I), in der:
- R₁ eine geradkettige oder verzweigte C₁-C₄-Alkylgruppe bedeutet;
- R₂, R und R' jeweils Wasserstoff bedeuten;
- n = 0 und m = 1 und R₃ den Rest 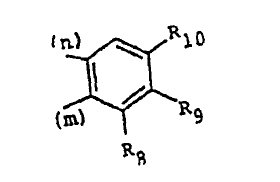 bedeuten, worin R₉ ein Halogenatom darstellt und R₈ und R₁₀ jeweils Wasserstoff bedeuten; und
- R₄ eine Amidgruppe CONHR₅ darstellt, worin R₅ eine geradkettige oder verzweigte C₁-C₆-Alkylgruppe bedeutet;
**dadurch gekennzeichnet, daß** es umfaßt:
i) die Bildung des Methylesters (73) 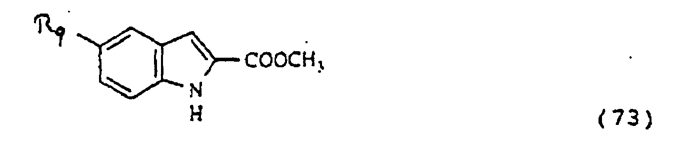 ausgehend von der entsprechenden Säure (72) 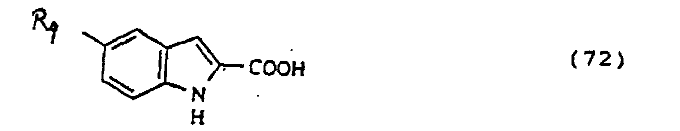 in der R₉ die oben angegebenen Bedeutungen besitzt,
mit konzentrierter Schwefelsäure in Methanol;
ii) die Bildung der Verbindung (74) 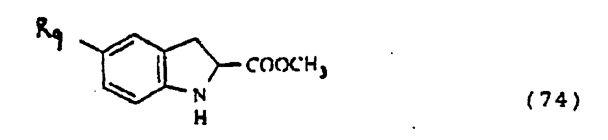 in der R₉ die oben angegebenen Bedeutungen besitzt,
ausgehend von der Verbindung (73) mit Magnesium in Methanol;
iii) die Bildung des entsprechenden Amids (75) durch Umsetzen mit dem geeigneten Amin R₅NH₂ 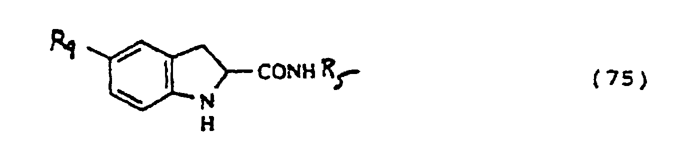 in der R₅ und R₉ die oben angegebenen Bedeutungen besitzen;
iv) die Umsetzung der Verbindung (75) mit einer Verbindung (IX) 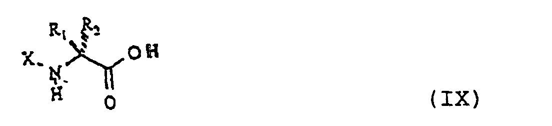 in der R₁ und R₂ die oben angegebenen Bedeutungen besitzen und X eine Schutzgruppe darstellt, zur Bildung der Verbindung (76) 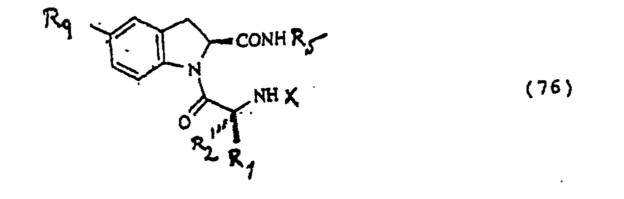 in der R₁, R₂, R₅, R₉ und X die oben angegebenen Bedeutungen besitzen;
v) die Eliminierung der Gruppe X zur Bildung der gewünschten Verbindung (I).

38. Verfahren zur Herstellung einer Verbindung der oben angegebenen Formel (I), in der:
- R₁ eine geradkettige oder verzweigte C₁-C₄-Alkylgruppe bedeutet;
- R₂, R und R' jeweils Wasserstoff bedeuten;
- n = 0 und m = 1 und R₃ den Rest 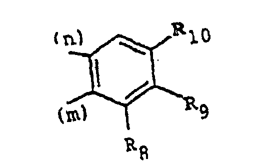 bedeuten, in der R₈, R₉ und R₁₀ jeweils Wasserstoff bedeuten; und
- R₄ eine Amidgruppe CONHR₅ bedeutet, worin R₅ eine Gruppe (CH₂)₃OH darstellt,
**dadurch gekennzeichnet, daß** es umfaßt:
i) die Reaktion des Methylesters der Indolin-2S-carbonsäure mit einer Verbindung der Formel (IX) 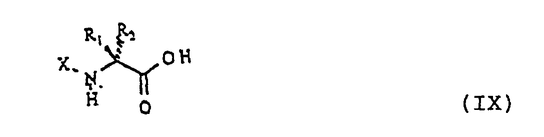 in der R₁ und R₂ die oben angegebenen Bedeutungen besitzen und X eine Schutzgruppe darstellt, zur Bildung der folgenden Verbindung (77) 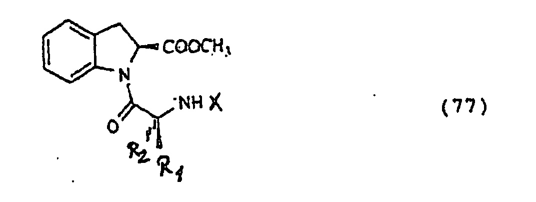 in der R₁, R₂ und X die oben angegebenen Bedeutungen besitzen;
ü) die Bildung des Amids (78) 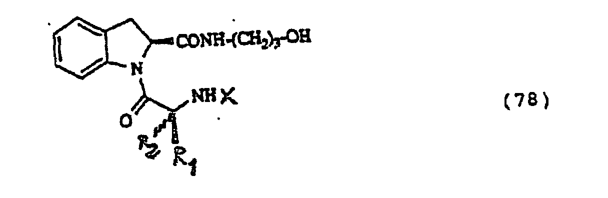 in der R₁ und R₂ die oben angegebenen Bedeutungen besitzen,
ausgehend von der Verbindung (77) durch Einwirkung von 3-Hydroxypropoylamin in Methanol; und
iii) die Eliminierung der Gruppe X zur Bildung der gewünschten Verbindung (I).

39. Verwendung einer Verbindung der allgemeinen Formel (I) nach einem der Ansprüche 6 bis 23 für die Herstellung eines Arzneimittels zur Behandlung von Störungen oder Erkrankungen von Menschen oder Tieren, welche verknüpft sind mit der Inaktivierung oder dem übermäßigen Abbau oder welche behandelt werden können durch Verzögern des physiologischen Abbaus von endogenen Neuropeptiden, welche durch die in Anspruch 1 definierte Membran-Tripeptidylpeptidase desaktiviert werden können.

40. Verwendung nach Anspruch 39, **dadurch gekennzeichnet, daß** die Störungen oder Erkrankungen mit der Inaktivierung von Cholecystokinin (CCK) verknüpft sind.

41. Verwendung nach Anspruch 40, **dadurch gekennzeichnet, daß** die Störungen oder Erkrankungen Störungen sind der Nahrungsaufnahme, des Gemüts, kognitive oder motorische Störungen, insbesondere Anorexie, Schizophrenie, der Parkinsonschen Krankheit und Depressionen sowie Störungen des Magen-Darm-Durchgangs, wie des Reizdarmsyndroms, der Bulimie oder von pathologischen Fettsuchtszuständen.

42. Verwendung nach Anspruch 41, **dadurch gekennzeichnet, daß** die Verbindung der allgemeinen Formel (I) eine in einem der Ansprüche 7 bis 23 definierte ist.

43. Verwendung nach Anspruch 41 oder 42, **dadurch gekennzeichnet, daß** die Störungen oder Erkrankungen mit der Inaktivierung von Cholecystokinin (CCK) verknüpft sind.

44. Verwendung nach Anspruch 43, **dadurch gekennzeichnet, daß** die Störungen oder Erkrankungen Störungen sind der Nahrungsaufnahme, des Gemüts, kognitive oder motorische Störungen, insbesondere Anorexie, Schizophrenie, der Parkinsonschen Krankheit und Depressionen sowie Störungen des Magen-Darm-Durchgangs, wie des Reizdarmsyndroms, der Bulimie oder von pathologischen Fettsuchtszuständen.

45. Pharmazeutische Zubereitung umfassend eine Verbindung nach einem der Ansprüche 6 bis 23 in einem pharmazeutisch annehmbaren Trägermaterial.

46. Pharmazeutische Zubereitung nach Anspruch 45, **dadurch gekennzeichnet, daß** sie eine Verbindung nach Anspruch 21, 22 oder 23 umfaßt.

## Claims

1. Process for screening medicaments intended for treating disorders or complaints connected with the inactivation or excessive breakdown, or capable of being treated by inhibiting the physiological breakdown, of endogenous neuropeptides, in humans or animals, **characterised in that** it comprises contacting a candidate molecule with a membrane tripeptidylpeptidase and measuring the activity of this enzyme, said membrane tnpepddylpeptidase being coded by the nucleotide sequence indicated by the identifiers SEQ ID no. 1 and SEQ ID no. 2.

2. Process according to claim 1, **characterised in that** the tripeptidylpeptidase corresponds to the sequence identifier SEQ ID no. 1 of the attached sequence listing.

3. Process according to claim 1 or 2, **characterised in that** the tripeptidylpepridase corresponds to the sequence identifier SEQ ID no. 2 of the attached sequence listing.

4. Process according to claim 1, **characterised in that** it comprises incubating brain membranes, e.g. from rats, prepared by centrifuging a homogenised preparation, in the presence of an aminouipeptidylpeptidase substrate and a candidate molecule, which is a potential inhibitor of the enzyme activity.

5. Process according to one of claims 1 to 4, **characterised in that** the disorders or complaints are connected with the inactivation of cholecystokinin (CCK).

6. Compound of the following general formula (I): 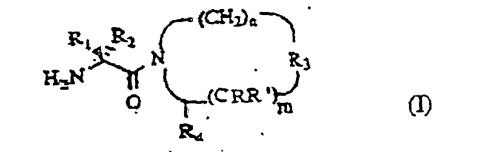 wherein:
R₁ denotes a hydrogen or a straight-chain or branched C₁-C₄ alkyl group;
R₂ denotes a hydrogen or a C₁-C₂-alkyl group;
at least one of R₁ and R₂ denotes a hydrogen;
n = 0 or 1 and m = 0 or 1 while n is different from m;
R and R' each independently denote a hydrogen or a C₁-C₂-alkyl group;
R₃ denotes a divalent radical consisting of a -(CH₂)₂-, -CH₂-CH(cis.F)-or -CH₂-CH(CH₂Ph)- alkyl chain, a motif 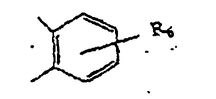 wherein R₆ denotes H, F, OCH₃ or OCH₂Ph, 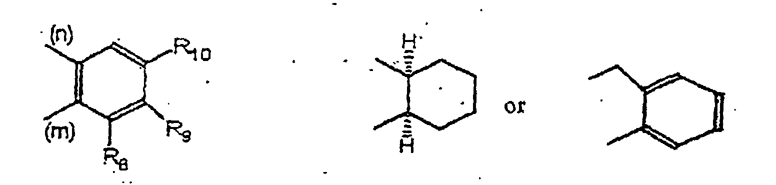 wherein R₈, R₉ and R₁₀ each denote a hydrogen or halogen atom, an O(C₁₋C₄- alkyl), OCH₂Ph, OH or C₁-C₄-alkyl group, including R₆, where (m) and (n) indicate the direction of bonding to the group (CH₂)ₙ (or to N if n = 0) and to the group (CRR')ₘ (or to CHR₄ if m = 0), respectively,
R₄ denotes a CO-NH-R₅ amide group where R₅ denotes a hydrogen or a straight-chain or branched C₁-C₆-alkyl, (CH₂)₃-SCH₃, -CH₂Ph, -CH₂C₆H₁₁, (CH₂)₃OH group, 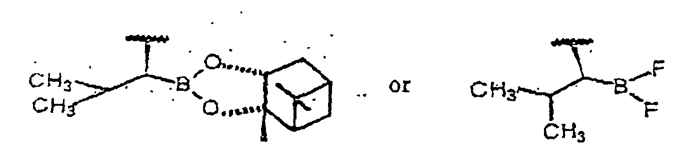
and the corresponding salts or hydrates thereof,
with the proviso that:
i) when
R₂ = R' = R = H; R₃ = -CH₂-CH₂-; n + m = 1; R₄ = -CO-NH-R₅; and R₅ = H, then
R₁ is other than -CH₃; -CH₂-CH₃; -(CH₂)₂-CH₃; -(CH₂)₃-CH₃; -CH(CH₃)₂ -CH₂CH(CH₃)₂; and -CH(CH₃)CH₂-CH₃;
ii) when
R₂ = R' = R = H; R₃ = -CH₂-CH₂-; n + m = 1; R₄ = -CO-NH-R₅; and R₅ = -CH₂-CH₃, then
R₁ is other than -CH₃; and -CH₂CH(CH₃)₂;
iii) when
R₂ = R' = R = H; R₃ = -CH₂-CH₂-; n + m = 1; R₄ = -CO-NH-R₅; and R₅ = -(CH₂)₄-CH₃, -CH(CH₃)₂, CH3 or the motif: 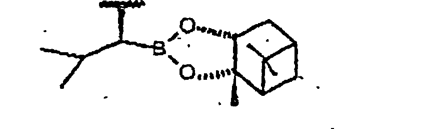 then
R₁ is other than -CH₃.

7. Compound according to claim 6 of the following general formula (I'): 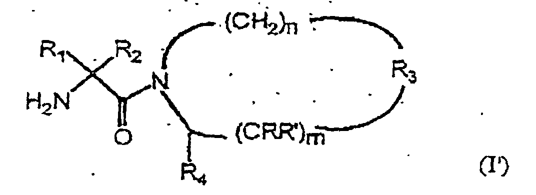 wherein:
R₁ denotes a hydrogen or a straight-chain or branched C₁-C₄ alkyl group;
R₂ denotes a hydrogen or a C₁-C₂-alkyl group;
at least one of R₁ and R₂ denotes a hydrogen;
n = 0 or 1 and m = 0 or 1 while n is different from m;
R₃ denotes a divalent radical consisting of a -(CH₂)₂-, -CH₂-CH(cis.F)-or-CH₂-CH(CH₂Ph)- alkyl chain, a motif 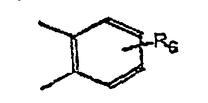 wherein R₆ denotes H, F, OCH₃ or OCH₂Ph, 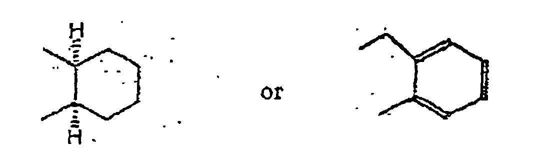
R₄ denotes a CO-NH-R₅ amide group where R₅ denotes a hydrogen or a straight-chain or branched C₁-C₈-alkyl, -(CH₂)₃-SCH₃, -CH₂Ph, - CH₂C₆H₁₁,

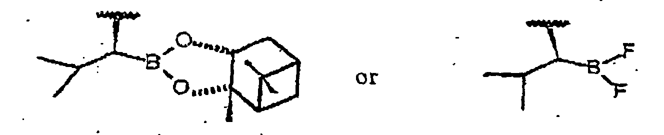

8. Compound according to claim 6 or 7, **characterised in that** R₃ denotes -(CH₂)₂-.

9. Compound according to claim 6 or 7, **characterised in that** R₃ denotes -CH₂-CH(cis.F)-.

10. Compound according to claim 6 or 7, **characterised in that** R₃ denotes -CH₂-CH(CH₂Ph)-.

11. Compound according to claim 6 or 7, **characterised in that** n = 0 and m = 1.

12. Compound according to claim 11, **characterised in that** R₃ denotes

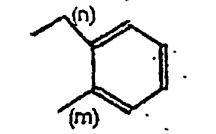

13. Compound according to claim 11, **characterised in that** R, R' denote a hydrogen and R₃ denotes

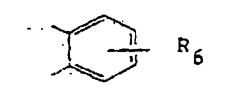

14. Compound according to claim 11, **characterised in that** R₃ denotes

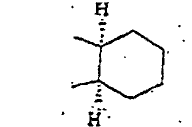

15. Compound according to claim 11, **characterised in that** R₃ denotes the motif

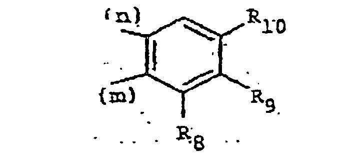

16. Compound according to claim 6 or 7, **characterised in that** n = 1 and m = 0.

17. Compound according to claim 16, **characterised in that** R₃ denotes

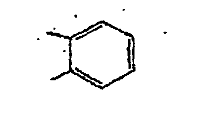

18. Compound according to one of claims 6 to 17, **characterised in that** R₅ denotes the n-butyl group.

19. Compound according to any one of claims 6 to 18, **characterised in that** R₂ denotes a hydrogen.

20. Compound according to one of claims 6 to 19, **characterised in that** R₁ denotes a hydrogen.

21. Compound according to any one of claims 6 to 20, **characterised in that** it is selected from among the group comprising:
L-valyl-L-proline n-hexylamide;
1-(2(S)-aminobutyryl)-L-proline-3-(methylthio)propyl-amide;
1-(2(S)-aminobutyryl)-L-proline-n-pentylamide;
1-(2(S)-aminobutyryl)-L-proline-n-butylamide;
1-(2(S)-aminobutyryl)-L-proline-[2(S)-methyl]butylamide;
1-(2(S)-aminobutyryl)-L-proline-n-propylamide;
1-(2(S)-aminobutyryl)-L-proline-isobutylamide;
L-valyl-L-proline n-butylamide;
L-alanyl-L-prolyl-difluoro-L-borovaline borohydrate;
1-(2(S)-aminobutyryl)-(4(S)-fluoro)- L-proline-n-butylamide;
1-(2(S)-aminobutyryl)-(4(S)-benzyl)- L-proline-n-butylamide;
2-(2(S)-aminobutyryl)-1,2,3,4-tetrahydro-3(S)-isoquinolinecarboxylic acid n-butylamide;
1-(2(S)-aminobutyryl)-2(S)-indolinecarboxylic acid n-butylamide;
1-(2(S)-aminobutyryl)-2(S)-indolinecarboxylic acid n-propylamide;
1-(2(S)-aminoburyryl)-2(S)-indolinecarboxylic acid methylamide;
1-(2(S)-aminobutyryl-2(S)-indolinecarboxylic acid ethylamide;
1-(2(S)-aminobutyryl)-2(R/S)-(5-methoxy)indolinecarboxylic acid n-butylamide;
1-(2(S)-aminobutyryl)-2(R/S)-(6-methoxy)indolinecarboxylic acid n-burylamide;
1-(2(S)-aminobutyryl)-2(R/S)-(5-fluoro)indolinecarboxylic acid n-butylamide;
1-(2(S)-aminobutyryl)-2(R/S)-(5-benzyloxy)indolinecarboxylic acid n-burylamide;
1-(2(S)-aminobutyryl)-2(S)-[(3aS,7aS)-perhydro]indolinecarboxylic acid n-butylamide;
2-(2(S)-aminobutyryl)-1(R/S)-isoindolinecarboxylic acid n-butylamide;
and the corresponding salts or hydrates thereof.

22. Compound according to claim 14, **characterised in that** it is selected from the group comprising:
1-(2(S)-aminobutyryl)-2(S)-indolinecarboxylic acid n-butylamide;
1-(2(S)-aminobutyryl)-2(S)-indolinecarboxylic acid n-propylamide;
1-(2(S)-aminoburyryl)-2(S)-indolinecarbohylic acid ethylamide;
1-(2(S)-aminobutyryl)-2(R/S)-(5-methoxy)indolinecarboxylic acid n-burylamide;
1-(2(S)-aminobutyryl)-2(R/S)-(5-fluoro)indolinecarboxylic acid n-butylamide
and the corresponding salts or hydrates thereof.

23. Compound according to claim 15, **characterised in that** it is selected from the group comprising:
1-(L-valyl)-5-methoxyindoline-2(R/S)-carboxylic acid butylamide;
1-(L-alanyl)-5-methoxyindoline-2(R/S)-carboxylic acid butylamide;
1-(L-alanyl)-5-methoxyindoline-2(S)-carboxylic acid butylamide;
1-(2(S)-aminobutyryl)-4-methoxyindoline-2(R/S)-carboxylic acid butylamide;
1-(2(S)-aminobutyryl)-3,3-dimethylindoline-2(R/S)-carboxylic acid butylamide;
1-(2(S)-aminobutyryl)-3(R)-methylindoline-2(R)-carboxylic acid butylamide and 1-(2(S)-aminobutyryl)-2(S)-methylindoline-2(S)-carboxylic acid butylamide;
1-(2(S)-aminobutyryl)-3 (R)-methylindoline-2(S)-carboxylic acid butylamide and 1-(2(S)-aminobutyryl)-3(S)-methylindoline-2(R)-carboxylic acid butylamide;
1-(2(S)-aminobutyryl)-4-ethoxyindoline-2(S)-carboxylic acid butylamide;
1-(2(S)-aminobutyryl)-4,5-dimethoxyindoline-2(R/S)-carboxylic acid butylamide;
1-(2(S)-aminobutyryl)-5-hydroxyindoline-2(S)-carboxylic acid butylamide;
1-(2(S)-aminobutyryl)-5-hydroxyindoline-2(R/S)-carboxylic acid butylamide;
1-(2(S)-aminobutyryl)-5-methylindoline-2(R/S)-carboxylic acid butylamide;
1-(2(S)-aminobutyryl)-5-chloroindoline-2(S)-carboxylic acid butylamide; and
1-(2(S)-aminobutyryl)indoline-2(S)-carboxylic acid (3-hydroxy)propylamide;
and the corresponding salts or hydrates thereof.

24. Process for preparing a compound of general formula (I) given hereinbefore wherein:
R₁ denotes a straight-chain or branched C₁-C₄ alkyl group;
R₂ denotes a hydrogen;
n = 0 or 1 and m = 0 or 1 while n is different from m and R₃ denotes -(CH₂)₂-; and
R and R' denote a hydrogen;
R₄ denotes CO-NH-R₅,
wherein R₅ denotes a hydrogen or a straight-chain or branched C₁₋C₆ alkyl group, -(CH₂)₃-S-CH₃, -CH₂ Ph;
with the proviso that R₁ is different from -CH₂CH₃, -(CH₂)₂CH₃,
-(CH₂)₃CH₃; -CH(CH₂), -CH₂CH(CH₃)₂, CH(CH₃)CH₂CH₃ when R₅ is a hydrogen, R₁ is different from -CH₂CH(CH₃)₂ when R₅ is the group CH₂CH₃ and R₁ is different from CH₃ when R₅ is the group -(CH₂)₄-CH₃, -CH(CH₃)₂, -CH₂-CH₃, -CH₃ or a hydrogen atom or the motif 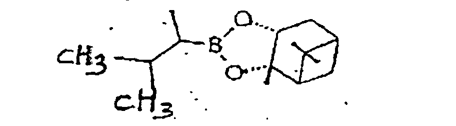 **characterised in that** it comprises:
i) forming a compound of formula (III) 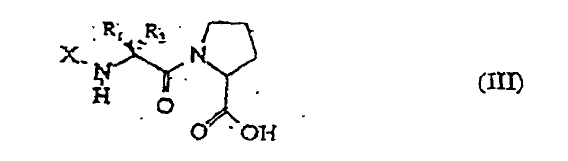 wherein R₁ and R₂ have the meanings given hereinbefore and X denotes a protective group, from a compound of formula (II) 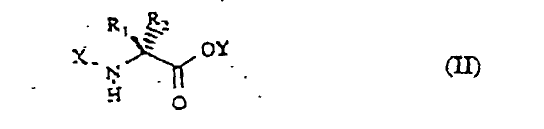 esterified at its acid function by a group Y and wherein X, R₁ and R₂ have the meanings given hereinbefore, by reacting with L-proline;
ii) amidating the acid function of the compound (III) with the appropriate amine R₅NH₂ wherein R₅ has the meaning given hereinbefore, to form the derivative (IV) 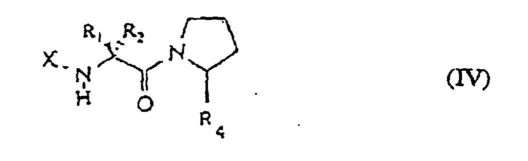 protected at its primary amine function by the group X;
ii) eliminating the group X from the derivative (IV), to obtain the desired compound (I).

25. Process for preparing a compound of general formula (I) given hereinbefore, wherein:
R₁ denotes a straight-chain or branched C₁-C₄ alkyl group;
R₂ denotes a hydrogen or a methyl group;
n = 0 or 1 and m = 0 or 1 while n is different from m and R₃ denotes a group -(CH₂)₂-; and
R and R' denote a hydrogen;
R₄ denotes a CO-NH-R₅ group wherein R₅ denotes a hydrogen or a stnight-chain or branched C₁-C₆ alkyl group;
with the proviso that when R₂ denotes a hydrogen, R₁ is different from
-CH₂CH₃, -(CH₂)₂CH₃, -(CH₂)₃CH₃; -CH(CH₃) ₂, -CH₂CH(CH₃)₂, CH(CH₃)CH₂CH₃ when R₅ is a hydrogen, R₁ is different from
-CH₂CH(CH₃)₂ when R₅ is the group CH₂CH₃ and R₁ is different from CH₃ when R₅ is the group -(CH₂)₄-CH₃, -CH(CH₃)₂, -CH₂-CH₃, -CH₃ or a hydrogen atom,
**characterised in that** it comprises:
i) forming a compound of formula (IV) 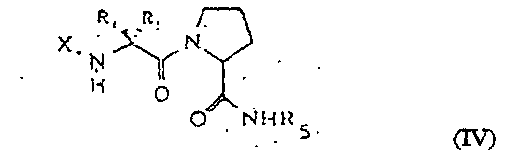 wherein R₁, R₂ and R₅ have the meanings given hereinbefore and X denotes a protective group, from a compound of formula (II) 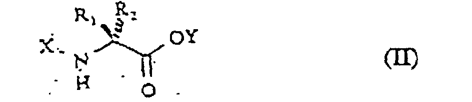 esterified at its acid function by a group Y and wherein R₁, R₂ and X have the meanings given hereinbefore, by reacting with a prolineamide of formula (V) 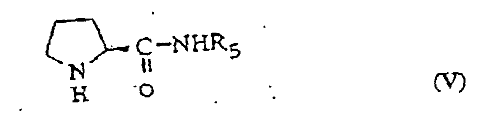 wherein R₅ is as hereinbefore defined;
ii) eliminating the procecuve group X from the compound (IV), to obtain the desired compound (I).

26. Process for preparing a compound of general formula (I) given hereinbefore, wherein:
R₁ denotes a straight-chain or branched C₁-C₄ alkyl group;
R₂ denotes a hydrogen;
n = 0 and m = 1 and R₃ denotes a group 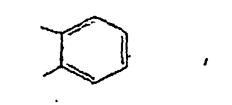
-CH₂-CH(cis.F)-, 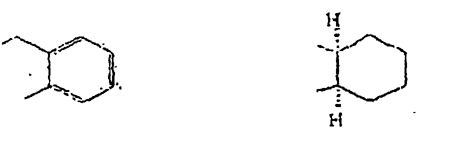
R and R' denote a hydrogen;
R₄ denotes a group CO-NH-R₅ wherein R₅ denotes a hydrogen or a straight-chain or branched C₁₋C₆ alkyl group;
**characterised in that** it comprises:
i) preparing a compound of formula (X) 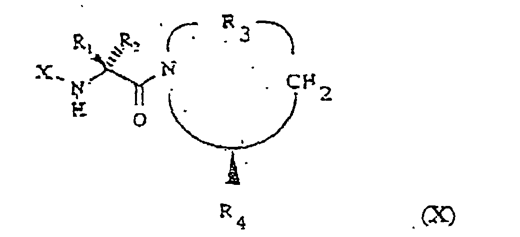 wherein R₁, R₂, R₃ and R₄ have the meanings given hereinbefore and X denotes a protective group, by reacting a compound of formula (VIII) 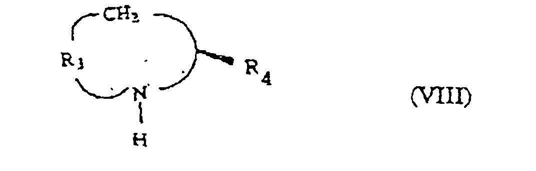 with a compound of formula (IX) 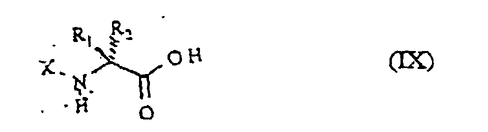 wherein R₁, R₂, R₄ and X have the meanings given hereinbefore;
ii) eliminating the group X from the compound (X), to form the desired compound (I).

27. Process for preparing a compound of general formula (I) given hereinbefore, wherein:
R₁ denotes a straight-chain or branched C₁-C₄ alkyl group;
R₂ denotes a hydrogen;
n = 1 and m = 0 and R₃ denotes a group -CH(CH₂Ph)-CH₂-, or 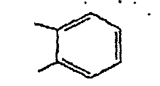
R₄ denotes a group CO-NH-R₅ wherein R₅ denotes a hydrogen or a straight-chain or branched C₁-C₆ alkyl group;
**characterised in that** it comprises:
i) preparing a compound of formula (XFI) 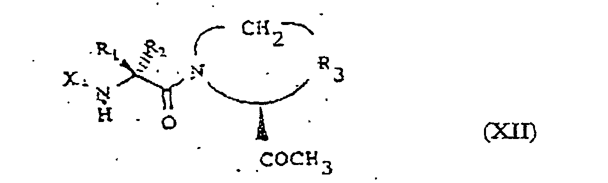 protected at its primary amine function by a protective group X and wherein R₁, R₂ and R₃ have the meanings given hereinbefore, by reacting the compound of formula (IX) 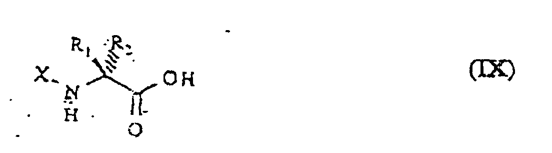 with a compound of formula (XI) 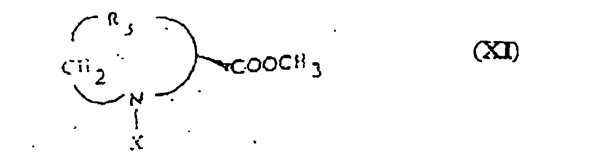 wherein R₁, R₂ and R₃ have the meanings given hereinbefore and X denotes a protective group;
ii) hydrolysing the ester function of the compound (XII) thus obtained to form the compound of formula (XIII) 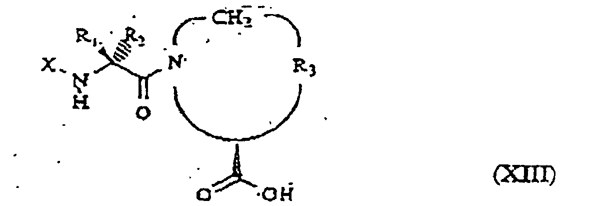 wherein R₁, R₂, R₃ and X have the meanings given hereinbefore;
iii) amidating the acid function of the compound (XIII) using the appropriate amine R₅NH₂ to form the derivative of formula (XIV) 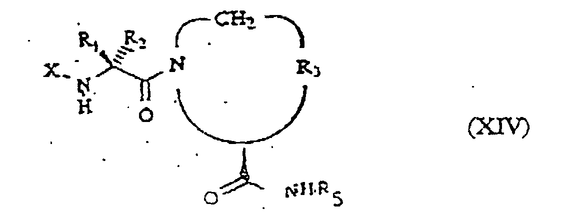 wheiein R₁, R₂, R₃, R₅ and X have the meanings given hereinbefore;
iv) eliminating the protective group X from the compound (XIV) to form the desired compound (I).

28. Process for preparing a compound of general formula (I) given hereinbefore, wherein:
R₁ denotes a methyl group;
R₂ denotes a hydrogen;
n = 0 or 1 and m = 0 or 1 while n is different from m and R₃ denotes a group - (CH₂)₂-; and
R and R' denote a hydrogen;
R₄ denotes a group CO-NH-R₅ wherein R₅ denotes (CH₃)₂-CH-BF₂;
**characterised in that** it comprises:
i) eliminating the pinane motif from the compound of formula (I) wherein R₁, R₂, n, m, R₃ and R₄ have the meanings given hereinbefore and R₅ denotes 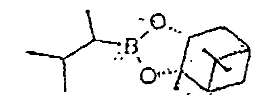 by the action of boron trichloride in methylene chloride followed by hydrolysis, to obtain the derivative (XXI) 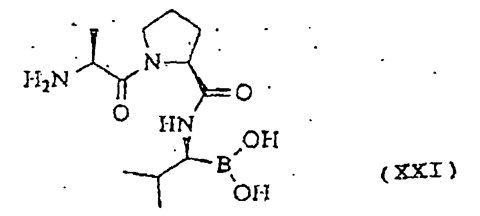
ii) reacting the compound (XXI) with hydrofluoric acid to form the desired compound (I).

29. Process for preparing a compound of general formula (I) given hereinbefore, wherein:
R₁ denotes a straight-chain or branched C₁-C₄ alkyl group;
R₂ denotes a hydrogen;
n = 0 and m = 1 and R₃ denotes the motif 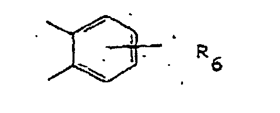 where R₆ denotes a group OCH₃, OCH₂Ph, F; and
R and R' denote a hydrogen;
R₄ denotes a group CO-NH-R₅ wherein R₅ denotes a straight-chain or branched C₁-C₆ alkyl group;
**characterised in that** it comprises:
i) forming the amide of formula (XXXXIV) 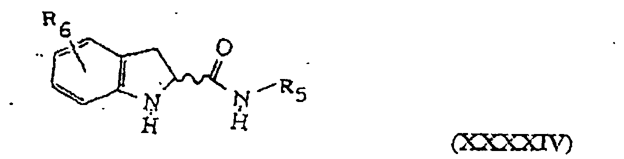 from the ester of formula (XXXXIII) 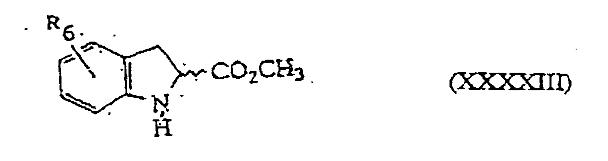 wherein R₆ has the meaning given hereinbefore, by reacting with the appropriate amine R₅NH₂;
ii) reacting the compound (XXXXIV) with the compound of formula (IX) 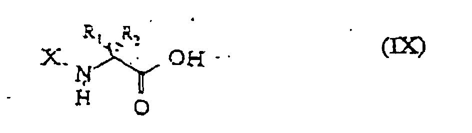 wherein R₁ and R₂ have the meanings given hereinbefore and X denotes a protective group, to form the compound of formula (XXXXV) 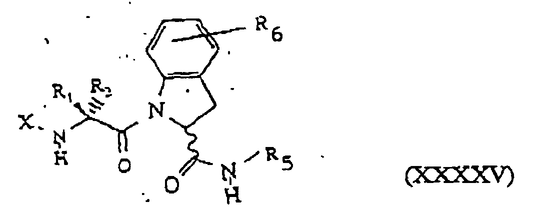 wherein R₁, R₂, R₅, R₆ and X have the meanings given hereinbefore;
iii) eliminating the group X to form the desired compound (I).

30. Process according to claim 29, **characterised in that** the methyl ester (XXXXIII) is prepared
i) by the action of sodium nitrite in the presence of hydrochloric acid on the compound of formula (XXXIX) 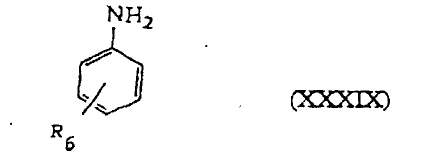 to form the compound of formula (XXXX) 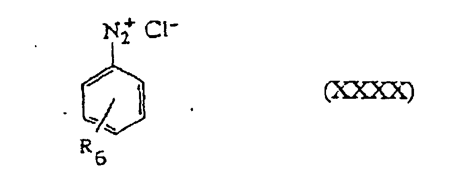 wherein R₆ has the meaning given hereinbefore
ii) by adding ethyl 2-methylacetoacetate to the compound (XXXX) thus obtained, in the presence of sodium nitrite in ethanol, to form the compound of formula (XXXXI) 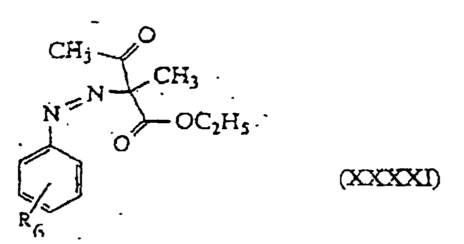 wherein R₆ has the meaning given hereinbefore;
iii) by cyclisation in an acid medium, to form the ethyl ester (XXXXII) 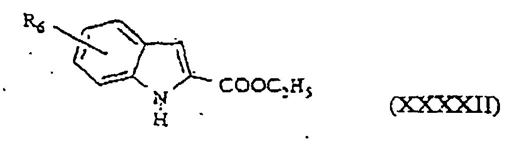
iv) by exchange, starting from the ethyl ester (XXXXII) thus obtained, in the presence of magnesium in methanol.

31. Process for preparing a compound of general formula (I) given hereinbefore, wherein:
R₁ denotes a straight-chain or branched C₁-C₄ alkyl group;
R₂, R and R' each denote a hydrogen;
n = 0 and m = 1 and R₃ denotes the motif 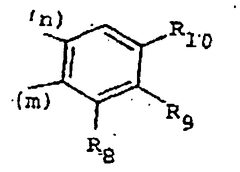 where R₈ and R₁₀ denote a hydrogen and R₉ denotes an O(C₁-C₄-alkyl) or C₁-C₄ alkyl group,
R₄ denotes an amide group CO-NH-R₅ wherein R₅ denotes a straight-chain or branched C₁-C₆ alkyl group;
**characterised in that** it comprises:
i) forming the amide of formula (49) 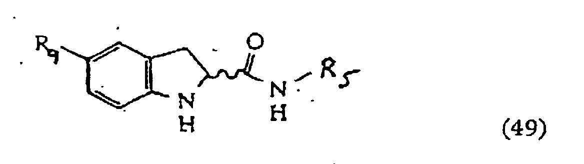 from the ester of formula (48) 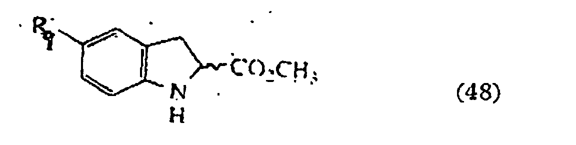 wherein R₉ and R₅ have the meanings given hereinbefore, by reacting with the appropriate amine R₅NH₂;
ii) reacting the compound (49) with the compound of formula (IX) 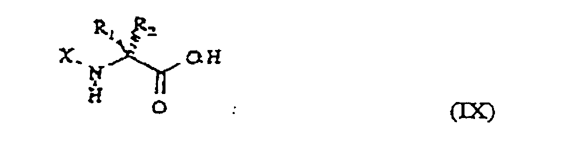 wherein R₁ and R₂ have the meanings given hereinbefore and X denotes a protective group, to form the compound of formula (50) 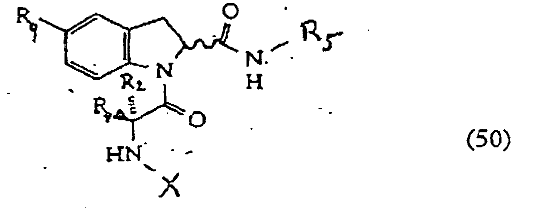 wherein R₁, R₂, R₉ and X have the meanings given hereinbefore;
iii) eliminating the group X to form the desired compound (I).

32. Process according to claim 31, **characterised in that** the methyl ester (48) is obtained from the corresponding acid (46) 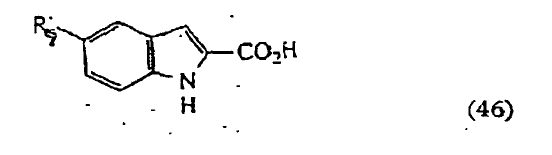 wherein R₉ is as hereinbefore defined
i) by treatment in ethanol or methanol with concentrated sulphuric acid, to produce the corresponding ester (47) 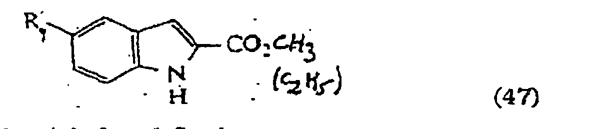 wherein R₉ is as hereinbefore defined
ii) then the compound (47) is treated with magnesium in methanol.

33. Process for preparing a compound of general formula (I) given hereinbefore, wherein
R₁ denotes a straight-chain or branched C₁-C₄ alkyl group;
R₂, R and R' each denote a hydrogen;
n = 0 and m = 1 and R₃ denotes the motif 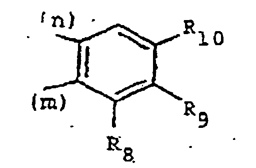 where R₈ and R₉ denote a hydrogen or an O(C₁-C₄-alkyl) group, while R₈ and R₉ cannot simultaneously represent a hydrogen, and R₁₀ denotes a hydrogen,
R₄ denotes an amide group CO-NH-R₅ wherein R₅ denotes a straight-chain or branched C₁-C₆ alkyl group;
**characterised in that** it comprises:
i) forming the compound (54) 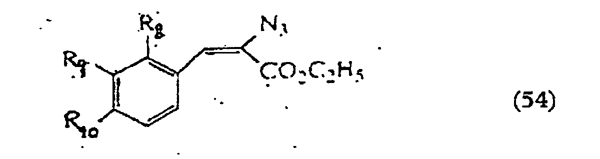 by reacting the corresponding aldehyde (53) 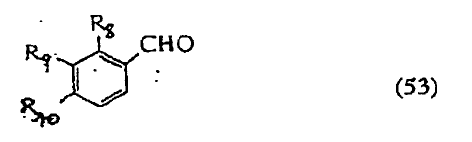 wherein R₈, R₉ and R₁₀ are defined as hereinbefore,
with ethyl azidoacetate (52) 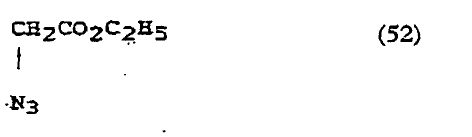
ii) cyclising the compound (54) to yield the compound (55) 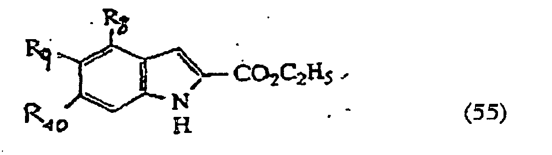 wherein R₈, R₉ and R₁₀ are defined as hereinbefore,
iii) forming the methyl ester (56) 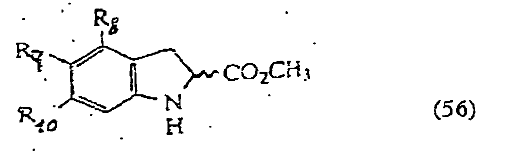 wherein R₈, R₉ and R₁₀ are defined as hereinbefore,
from the compound (55) in the presence of magnesium in methanol
(iv) reacting the ester (56) obtained with the appropriate amine R₅NH₂ to form the amide (57) 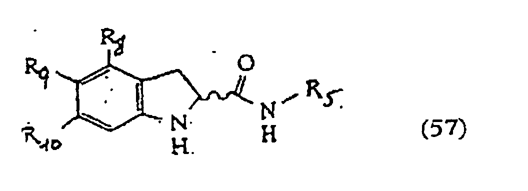 wherein R₈, R₉ and R₁₀ are defined as hereinbefore,
v) reacting the compound (57) with the compound of formula (IX) 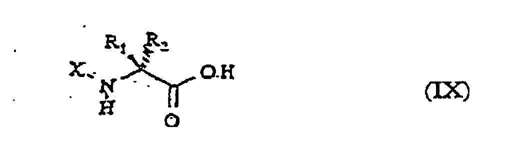 wherein R₁ and R₂ are as hereinbefore defined and X denotes a protective group, to form the compound (58) 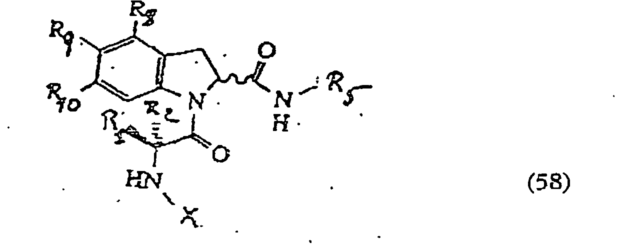 wherein R₁, R₂ , R₅ , R₈, R₉, R₁₀ and X are as hereinbefore defined,
vi) eliminating the group X to form the desired compound (I).

34. Process for preparing a compound of formula (I) given hereinbefore,
wherein:
R₁ denotes a straight-chain or branched C₁-C₄ alkyl group;
R₂ denotes a hydrogen;
one of the substituents R or R' denotes a hydrogen and the other denotes a C₁-C₂-alkyl group,
n = 0 and m = 1 and R₃ denotes the motif 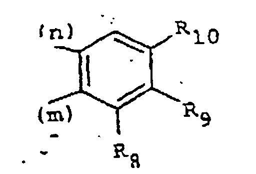 where R8, R₉ and R₁₀ denote a hydrogen,
R₄ denotes a group CONHR₅ wherein R₅ is a straight-chain or branched C₁-C₅ alkyl group,
**characterised in that** it comprises:
i) forming the compound (59) 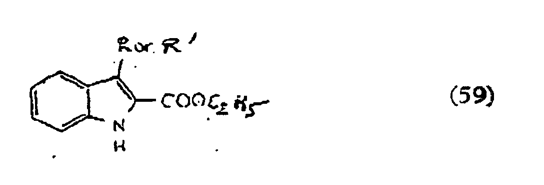 where R and R' are as hereinbefore defined,
by reacting phenylhydrazine and 2-ketobutyric acid in an acid medium;
ii) forming the compound (60) from the compound (59) obtained 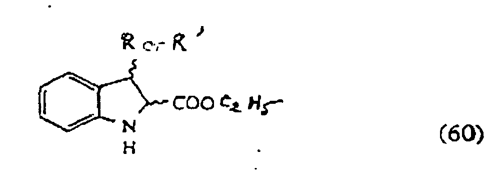 where R and R' are as hereinbefore defined,
in the presence of magnesium in methanol,
iii) forming the amide (61) corresponding to the compound (60) by reacting with the appropriate amine R₅NH₂ 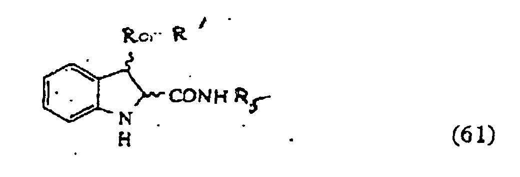 where R and R' are as hereinbefore defined,
iv) separating the cis isomers (61a) on the one hand and the trans isomers (61b) on the other hand of the compound (61) 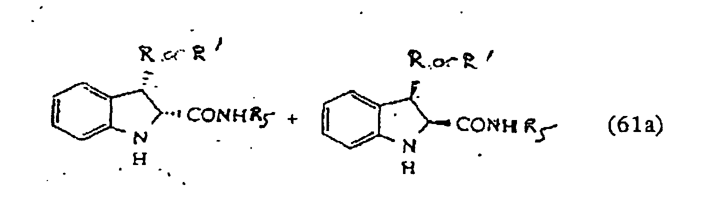 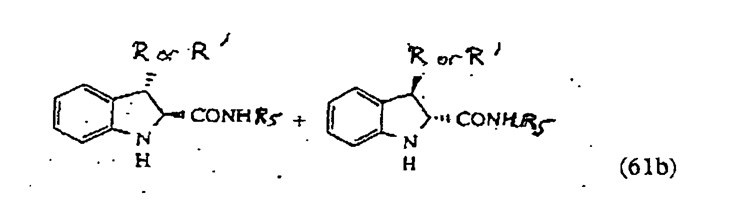
v) reacting the compounds (61a) and (61b), respectively, with the compound (IX) 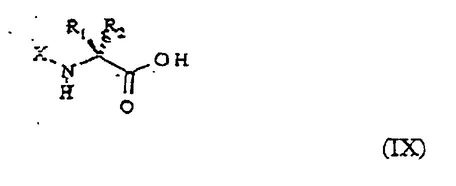 wherein R₁ and R₂ are as hereinbefore defined and X denotes a protective group, to form the mixtures (62a) and (62b), respectively 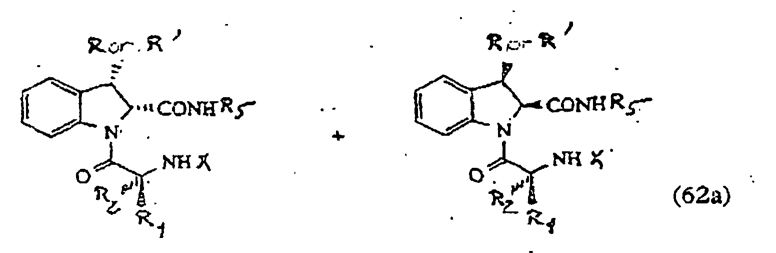 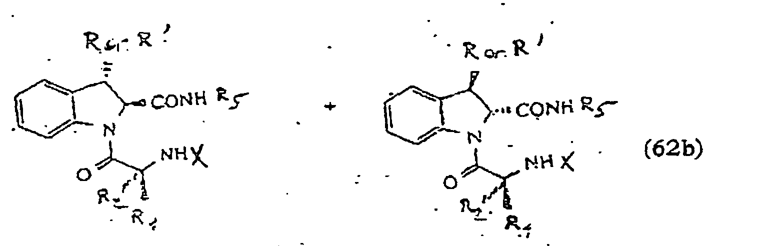 wherein R₁, R₂, R, R' and R₅ have the meanings given hereinbefore;
vi) eliminating the protective group X, leading to the desired compound (I) in the form of the cis (63a) and trans (63b) pairs, respectively.

35. Process for preparing a compound of formula (I) given hereinbefore,
wherein:
R₁ denotes a straight-chain or branched C₁-C₄ alkyl group;
R₂ denotes a hydrogen;
R and R' each denote a C₁-C₂-alkyl group, which may be identical or different,
n = 0 and m = 1 and R₃ denotes the motif 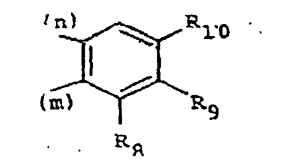 where R₈, R₉ and R₁₀ each denote a hydrogen,
R₄ denotes a CONHR₅ amide group wherein R₅ is a straight-chain or branched C₁-C₆ alkyl group,
**characterised in that** it comprises:
i) forming the compound (65) of the following formula: 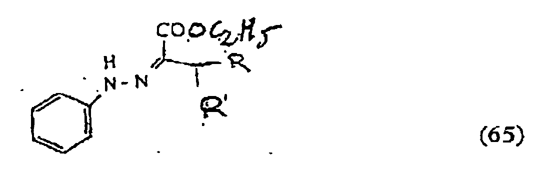 by reacting phenylhydrazine with a compound 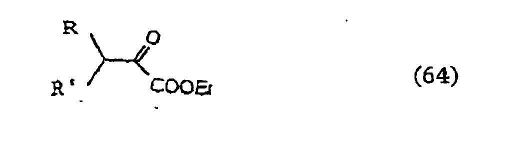
ii) cyclising the compound (65) in an acid medium to form the following compound (66): 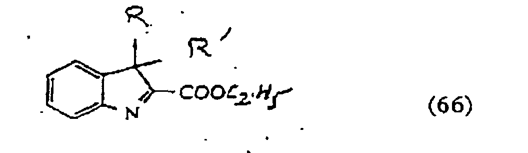 where R and R' are as hereinbefore defined,
iii) hydrogenating the compound (66), yielding the following compound (67) 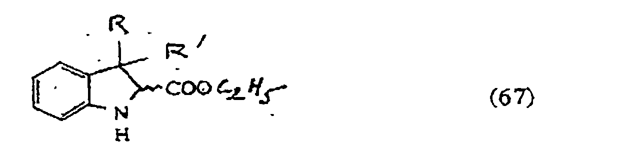 where R and R' are as hereinbefore defined,
iv) forming the corresponding amide (68) by the action of LiNHR₅
wherein R₅ is as hereinbefore defined 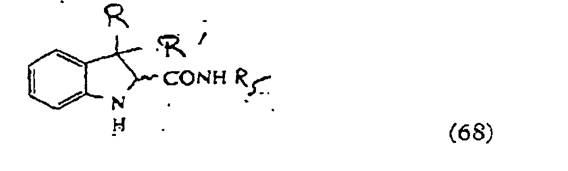 where R and R' are as hereinbefore defined,
v) reacting the compound (68) with the compound of formula (IX) 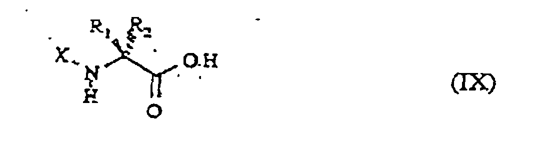 where R₁ and R₂ are as hereinbefore defined and X is a protective group, to form the compound (69) 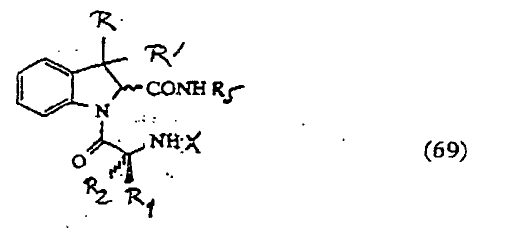 where R and R' are as hereinbefore defined,
vi) eliminating the group X to form the desired compound (I).

36. Process for preparing a compound of formula (I) given hereinbefore,
wherein:
R₁ denotes a straight-chain or branched C₁-C₄ alkyl group;
R₂, R and R' each denote a hydrogen;
n = 0 and m = 1 and R₃ denotes the motif 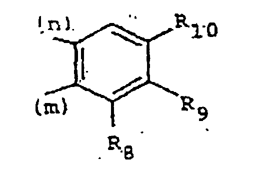
wherein Rg denotes an OH group and R₈ and R₁₀ both denote a hydrogen;
R₄ denotes a CONHR₅ amide group wherein R₅ is a straight-chain or branched C₁-C₆ alkyl group,
**characterised in that** it comprises:
i) forming the compound (71) 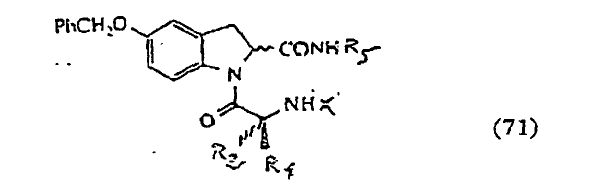 wherein R₉ denotes an OCH₂Ph group, R₅ is as hereinbefore defined and X denotes a protective group, by reacting the compound (70) 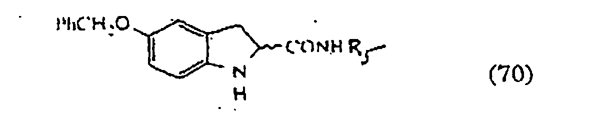 wherein R₅ is as hereinbefore defined, with a compound (IX) 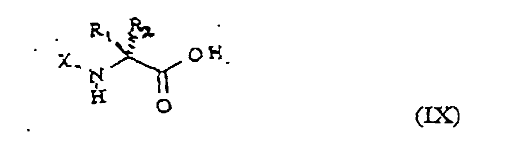 wherein R₁ and R₂ are as hereinbefore defined and X denotes a protective group,
ii) eliminating the groups CH₂Ph and X from the compound (71) to form the desired compound (I).

37. Process for preparing a compound of formula (I) given hereinbefore,
wherein:
R₁ denotes a straight-chain or branched C₁-C₄ alkyl group;
R₂, R and R' each denote a hydrogen;
n = 0 and m = 1 and R₃ denotes the motif 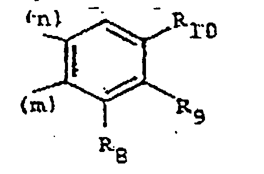 where R₉ denotes a halogen atom and R₈ and R₁₀ each denote a hydrogen,
R₄ denotes a CONHR₅ amide group wherein R₅ is a straight-chain or branched C₁-C₆ alkyl group,
**characterised in that** it comprises:
i) forming the methyl ester (73) 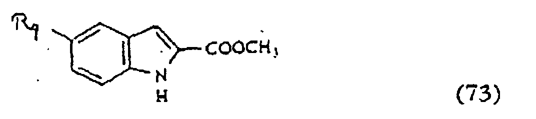 from the corresponding acid (72) 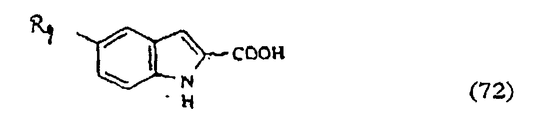 wherein R₉ is as hereinbefore defined,
with concentrated sulphuric acid in methanol,
ii) forming the compound (74) 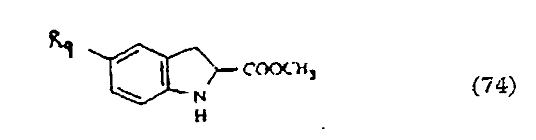 wherein R₉ is as hereinbefore defined,
from the compound (73), with magnesium in methanol,
iii) forming the corresponding amide (75) by reaction with the appropriate amine R₅NH₂ 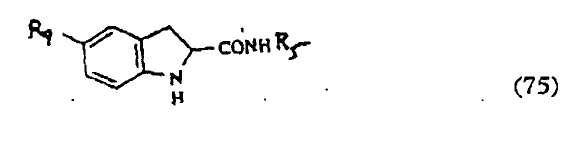 wherein R₅ and R₉ are as hereinbefore defined
iv) reacting the compound (75) with a compound (IX) 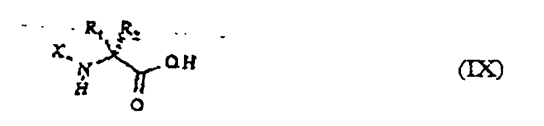 wherein R₁ and R₂ are as hereinbefore defined and X denotes a protective group, to form the compound (76) 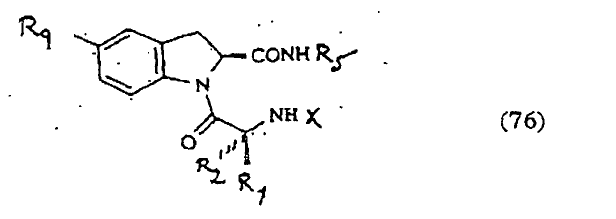 wherein R₁, R₂ , R₅ , R₉ and X are as hereinbefore defined,
v) eliminating the group X to form the desired compound (I).

38. Process for preparing a compound of formula (I) given hereinbefore, wherein:
R₁ denotes a straight-chain or branched C₁-C₄ alkyl group;
R₂, R and R' each denote a hydrogen;
n = 0 and m = 1 and R₃ denotes the motif 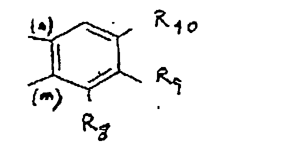 where R₈, R₉ and R₁₀ each denote a hydrogen,
R₄ denotes a CONHR₅ amide group wherein R₅ is a (CH₂)₃OH group,
**characterised in that** it comprises:
i) reacting the methyl ester of indoline-2S-carboxylic acid with a compound of formula (IX) 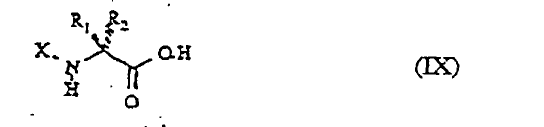 wherein R₁ and R₂ are as hereinbefore defined and X denotes a protective group, to form the following compound (77) 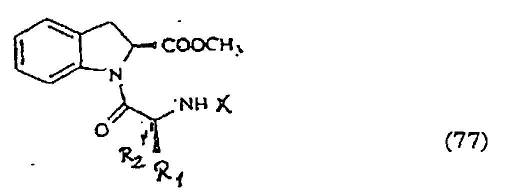 wherein R₁ and R₂ and X are as hereinbefore defined,
ii) forming the amide (78) 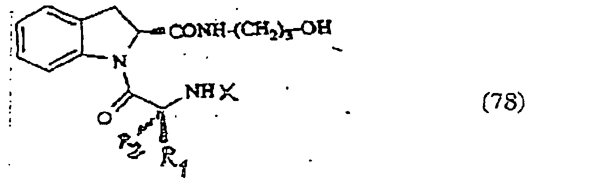 wherein R₁ and R₂ are as hereinbefore defined, from the compound (77) by the action of 3-hydroxypropylamine in methanol,
iii) eliminating the group X to form the desired compound (I).

39. Use of a compound of general formula (I) according to any one of claims 6 to 23 for the preparation of a medicament for treating disorders or complaints in humans or animals connected with the inactivation or excessive breakdown, or capable of being treated by inhibiting the physiological breakdown, of endogenous neuropeptides, deactrivared by membrane tripeptidylpeptidase as defined in claim 1.

40. Use according to claim 39, **characterised in that** the disorders or complaints are associated with the inactivation of cholecystokinin (CCK).

41. Use according to claim 40, **characterised in that** the disorders or complaints are disorders of food intake, mood, cognitive or motor disorders, notably anorexia, schizophrenia, Parkinson's disease and depression, as well as gascro-intestinal transit disorders such as irritable bowel syndrome, bulimia or morbid obesity.

42. Use according to claim 41, **characterised in that** the compound of general formula (I) is as defined in any one of claims 7 to 23.

43. Use according to claim 41 or 42, **characterised in that** the disorders or complaints are associated with the inactivation of cholecystokinia. (CCK).

44. Use according to claim 43, **characterised in that** the disorders or complaints are disorders of food intake, mood, cognitive or motor disorders, notably anorexia, schizophrenia, Parkinson's disease and depression, as well as gastro-intestinaI transit disorders such as irritable bowel syndrome, bulimia or morbid obesity.

45. Pharmaceutical composition comprising a compound according to any one of claims 6 to 23 in a pharmaceutically acceptable excipient.

46. Pharmaceutical composition according to claim 45, **characterised in that** it comprises a compound according to claim 21, 22 or 23.
